# EUROPEAN PATENT APPLICATION

(11) **EP 1 780 201 A2**
(43) Date of publication of application: **02.05.2007**
(21) Application number: 06077243.1
(22) Date of filing: 18.01.2002
(51) Int. Cl.: C07D 213/81, C07D 471/04, C07C 229/36, C07D 401/12, C07D 213/89, A61P 37/00

(54) **PHENYLALANINE ENAMIDE DERIVATIVES POSSESSING A CYCLOBUTENE GROUP, FOR USE AS INTEGRIN INHIBITORS**

(30) Priority: 22.02.2001 GB 0104418; 08.06.2001 GB 0114000; 16.11.2001 GB 0127562
(62) Divisional of application: 02715515.9
(71) Applicant: Celltech R&D Limited, Slough, Berkshire SL1 3WE (GB)
(72) Inventor: Bailey, Stuart, Slough Berkshire SL1 3WE (GB); Brown, Julien, Slough Berkshire SL1 3WE (GB); Brand, Stephen, Slough Berkshire SL1 3WE (GB); Johnson, James, Slough Berkshire SL1 3WE (GB); Porter, John, Slough Berkshire SL1 3WE (GB); Head, John, Slough Berkshire SL1 3WE (GB)
(74) Representative: Mercer, Christopher Paul

(57) **Abstract**

Phenylalanine enamide derivatives of formula (1) are described: 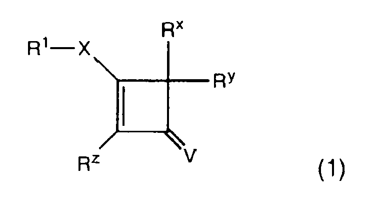 wherein R¹, R^{x}, R^{y}, R^{z}, X and V are as defined in claim 1.
The compounds are able to inhibit the binding of integrins to their ligands and are of use in the prophylaxis and treatment of immuno or inflammatory disorders or disorders involving the inappropriate growth or migration of cells.

## Description

This invention-relates to a series of phenylalanine enamide derivatives, to compositions containing them, to processes for their preparation, and to their use in medicine.

Over the last few years it has become increasingly clear that the physical interaction of inflammatory leukocytes with each other and other cells of the body plays an important role in regulating immune and inflammatory responses [Springer, T. A., Nature, 346, 425, (1990); Springer, T. A., Cell, 76, 301, (1994)]. Specific cell surface molecules collectively referred to as cell adhesion molecules mediate many of these interactions.

The adhesion molecules have been sub-divided into different groups on the basis of their structure. One family of adhesion molecules which is believed to play a particularly important role in regulating immune and inflammatory responses is the integrin family. This family of cell surface glycoproteins has a typical non-covalently linked heterodimer structure. At least 16 different integrin alpha chains and 8 different integrin beta chains have been identified [Newman, P. et al, Molecular Medicine Today, 304, (1996)]. The members of the family are typically named according to their heterodimer composition although trivial nomenclature is widespread in the field. Thus the integrin α4β1 consists of the integrin alpha 4 chain associated with the integrin beta 1 chain, but is also widely referred to as Very Late Antigen 4 or VLA-4. Not all of the potential pairings of integrin alpha and beta chains have yet been observed in nature and the integrin family has been subdivided into a number of subgroups based on the pairings that have been recognised to date [Sonnenberg, A., Current Topics in Microbiology and Immunology, 184, 7, (1993)].

The importance of integrin function in normal physiological responses is highlighted by two human deficiency diseases in which integrin function is defective. Thus in the disease termed Leukocyte Adhesion Deficiency (LAD) there is a defect in one of the families of integrins expressed on leukocytes [Marlin, S. D. et al, J. Exp. Med. 164, 855, (1986)]. Patients suffering from this disease have a reduced ability to recruit leukocytes to inflammatory sites and suffer recurrent infections, which in extreme cases may be fatal. In the case of patients suffering from the disease termed Glanzman's thrombasthenia (a defect in a member of the beta 3 integrin family) there is a defect in blood clotting (Hodivala-Dilke, K. M., J. Clin. Invest. 103, 229, (1999)].

The potential to modify integrin function in such a way as to beneficially modulate cell adhesion has been extensively investigated in animal models using specific antibodies and peptides that block various functions of these molecules [e.g. Issekutz, T. B., J. lmmunol. 149, 3394, (1992); Li, Z. et al, Am. J. Physiol. 263, L723, (1992); Mitjans, F. et al, J. Cell Sci. 108, 2825, (1995); Brooks, P. C. et al, J. Clin. Invest. 96, 1815, (1995); Binns, R. M. et al, J. Immunol. 157, 4094, (1996); Hammes, H.-P. et al, Nature Medicine 2, 529, (1996); Srivata, S. et al, Cardiovascular Res. 36, 408 (1997)]. In particular an anti α₄β₇-antibody has demonstrated both clinical and histologic improvement of inflammatory activity and disease in a non-human primate model of inflammatory bowel disease [Hesterberg, P.E. et al, Gastroenterol, 111, 1373-80 (1996)]. A number of monoclonal antibodies which block integrin function are currently being investigated for their therapeutic potential in human disease, and one, ReoPro, a chimeric antibody against the platelet integrin αllbβ3 is in use as a potent antithrombotic agent for use in patients with cardiovascular complications following coronary angioplasty.

Integrins recognize both cell surface and extracellular matrix ligands, and ligand specificity is determined by the particular alpha-beta subunit combination of the molecule [Newman, P., *ibid*]*.* One particular integrin subgroup of interest involves the α4 chain which can pair with two different beta chains β1 and β7 [Sonnenberg, A., *ibid*]*.* The α4β1 pairing occurs on many circulating leukocytes (for example lymphocytes, monocytes, eosinophils and basophils) although it is absent or only present at low levels on circulating neutrophils. α4β1 binds to an adhesion molecule (Vascular Cell Adhesion Molecule-1 also known as VCAM-1) frequently up-regulated on endothelial cells at sites of inflammation [Osborne, L., Cell, 62, 3, (1990)]. The molecule has also been shown to bind to at least three sites in the matrix molecule fibronectin [Humphries, M. J. et al, Ciba Foundation Symposium, 189, 177, (1995)]. Based on data obtained with monoclonal antibodies in animal models it is believed that the interaction between α4β1 and ligands on other cells and the extracellular matrix plays an important role in leukocyte migration and activation [Yednock, T. A. et al, Nature, 356, 63, (1992); Podolsky, D. K. et al, J. Clin. Invest. 92, 372, (1993); Abraham, W. M. et al, J. Clin. Invest. 93, 776, (1994)].

The integrin generated by the pairing of α4 and β7 has been termed LPAM-1 [Holzmann, B. and Weissman, I. L., EMBO J. 8, 1735, (1989)]. The α4β7 pairing is expressed on certain sub-populations of T and B lymphocytes and on eosinophils [Erle, D. J. et al, J. Immunol. 153, 517 (1994)]. Like α4β1, α4β7 binds to VCAM-1 and fibronectin. In addition, α4β7 binds to an adhesion molecule believed to be involved in the homing of leukocytes to mucosal tissue such as gastrointestinal mucosa termed MAdCAM-1 [Berlin, C. et al, Cell, 74, 185, (1993)]. MAdCAM-1 is preferentially expressed in the gastrointestinal track. The interaction between α4β7 and MAdCAM-1 may also be important at sites of inflammation outside of mucosal tissue [Yang, X.-D. et al, PNAS, 91, 12604, (1994)].

Regions of the peptide sequence recognized by α4β1 and α4β7 when they bind to their ligands have been identified. α4β1 seems to recognise LDV, IDA or REDV peptide sequences in fibronectin and a QIDSP sequence in VCAM-1 [Humphries, M. J. *et al, ibid*] whilst α4β7 recognises a LDT sequence in MAdCAM-1 [Birskin, M. J. et al, J. Immunol. 156, 719, (1996)]. There have been several reports of inhibitors of these interactions being designed from modifications of these short peptide sequences [Cardarelli, P. M. et al, J. Biol. Chem., 269, 18668, (1994); Shorff, H. N.et al, Biorganic Med. Chem. Lett., 6, 2495, (1996); Vanderslice, P. et al, J. Immunol., 158, 1710, (1997)]. It has also been reported that a short peptide sequence derived from the α4β1 binding site in fibronectin can inhibit a contact hypersensitivity reaction in a trinitrochlorobenzene sensitised mouse [Ferguson, T. A., et al, PNAS, 88, 8072, (1991)].

Since the alpha 4 subgroup of integrins are predominantly expressed on leukocytes their inhibition can be expected to be beneficial in a number of immune or inflammatory disease states. However, because of the ubiquitous distribution and wide range of functions performed by other members of the integrin family it is important to be able to identify selective inhibitors of the alpha 4 subgroup.

We have now found a group of compounds which are potent and selective inhibitors of α4 integrins. Members of the group are able to inhibit α4 integrins such as α4β1 and/or α4β7 at concentrations at which they generally have no or minimal inhibitory action on α integrins of other subgroups. These compounds possess the additional advantages of good pharmacokinetic properties, especially low plasma clearance and good absorption properties making them particularly suitable for oral dosing.

Thus according to one aspect of the invention we provide a compound of formula (1): 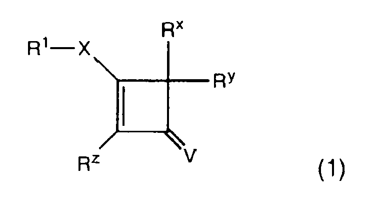 wherein
R¹ is a group Ar¹L²Ar²Alk- in which:
Ar¹ is an optionally substituted aromatic or heteroaromatic group;
L² is a covalent bond or a linker atom or group;
Ar² is an optionally substituted arylene or heteroarylene group;
and Alk is a chain 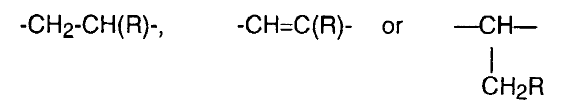
in which R is a carboxylic acid (-CO₂H) or a derivative or biostere thereof;
X is an -O- or -S- atom or -N(R²)- group in which:
R² is a hydrogen atom or a C₁₋₆alkyl group;
V is an oxygen (O) or sulphur (S) atom;
R^{x}, R^{y} and R^{z} which may be the same or different is each an atom or group -L¹(Alk¹)ₙ(R³)ᵥ in which L¹ is a covalent bond or a linker atom or group, Alk¹ is an optionally substituted aliphatic or heteroaliphatic chain, R³ is a hydrogen or halogen atom or group selected from -OR^{3a} [where R^{3a} is a hydrogen atom or an optionally substituted straight or branched C₁₋₆alkyl group or C₃₋₈cycloalkyl group], -SR^{3a}, -CN or an optionally substituted cycloaliphatic, heterocycloaliphatic, polycycloaliphatic, heteropolycycloaliphatic, aromatic or heteroaromatic group, n is zero or the integer 1 and v is the integer 1, 2 or 3 provided that when n is zero and L¹ is a covalent bond v is the integer 1;
or R^{z} is an atom or group as previously defined and R^{x} and R^{y} are joined together to form an optionally substituted spiro linked cycloaliphatic or heterocycloaliphatic group;
and the salts, solvates, hydrates and N-oxides thereof.

It will be appreciated that compounds of formula (1) may have one or more chiral centres, and exist as enantiomers or diastereomers. The invention is to be understood to extend to all such enantiomers, diastereomers and mixtures thereof, including racemates. Formula (1) and the formulae hereinafter are intended to represent all individual isomers and mixtures thereof, unless stated or shown otherwise. In addition, compounds of formula (1) may exist as tautomers, for example keto (CH₂C=O)-enol (CH=CHOH) tautomers. Formula (1) and the formulae hereinafter are intended to represent all individual tautomers and mixtures thereof, unless stated otherwise.

Optionally substituted aromatic groups represented by Ar¹ when present in the group R¹ include for example optionally substituted monocyclic or bicyclic fused ring C₆₋₁₂aromatic groups, such as phenyl, 1- or 2-naphthyl, 1- or 2-tetrahydronaphthyl, indanyl or indenyl groups.

Optionally substituted heteroaromatic groups represented by the group Ar¹ when present in the group R¹ include for example optionally substituted C₁₋₉heteroaromatic groups containing for example one, two, three or four heteroatoms selected from oxygen, sulphur or nitrogen atoms. In general, the heteroaromatic groups may be for example monocyclic or bicyclic fused ring heteroaromatic groups. Monocyclic heteroaromatic groups include for example five- or six-membered heteroaromatic groups containing one, two, three or four heteroatoms selected from oxygen, sulphur or nitrogen atoms. Bicyclic heteroaromatic groups include for example eight- to thirteen-membered fused-ring heteroaromatic groups containing one, two or more heteroatoms selected from oxygen, sulphur or nitrogen atoms.

Particular examples of heteroaromatic groups of these types include pyrrolyl, furyl, thienyl, imidazolyl, N-C₁₋₆alkylimidazolyl, oxazolyl, isoxazolyl, thiazolyl, isothiazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl, 1,3,4-thiadiazole, pyridyl, pyrimidinyl, pyridazinyl, pyrazinyl, 1,3,5-triazinyl, 1,2,4-triazinyl, 1,2,3-triazinyl, benzofuryl, [2,3-dihydro]benzofuryl, [2,3-dihydro]benzothienyl, benzothienyl, benzotriazolyl, indolyl, isoindolyl, benzimidazolyl, imidazo[1,2-a]pyridyl, benzothiazolyl, benzoxazolyl, benzisoxazolyl, benzopyranyl, [3,4-dihydro]benzopyranyl, quinazolinyl, quinoxalinyl, naphthyridinyl, e.g. 2,6-naphthyridinyl, or 2,7-naphthyridinyl, pyrido[3,4-b]pyridyl, pyrido[3,2-b]pyridyl, pyrido[4,3-b]pyridyl, quinolinyl, isoquinolinyl, tetrazolyl, 5,6,7,8-tetrahydroquinolinyl, 5,6,7,8-tetrahydro-isoquinolinyl, and imidyl, e.g. succinimidyl, phthalimidyl, or naphthalimidyl such as 1,8-naphthalimidyl.

Each aromatic or heteroaromatic group represented by the group Ar¹ may be optionally substituted on any available carbon or, when present, nitrogen atom. One, two, three or more of the same or different substituents may be present and each substituent may be selected for example from an atom or group -L³(Alk²)ₜL⁴(R⁴)ᵤ in which L³ and L⁴, which may be the same or different, is each a covalent bond or a linker atom or group, t is zero or the integer 1, u is an integer 1, 2 or 3, Alk² is an optionally substituted aliphatic or heteroaliphatic chain and R⁴ is a hydrogen or halogen atom or a group selected from optionally substituted C₁₋₆alkyl or C₃₋₈cycloalkyl, -OR⁵ [where R⁵ is a hydrogen atom, an optionally substitued C₁₋₆alkyl or C₃₋₈cycloalkyl group], -SR⁵, -NR⁵R⁶ [where R⁶ is as just defined for R⁵ and may be the same or different], -NO₂, -CN, -CO₂R⁵, -SO₃H, -SOR⁵, -SO₂R⁵, -SO₃R⁵, -OCO₂R⁵, -CONR⁵R⁶, -OCONR⁵R⁶, -CSNR⁵R⁶, -COR⁵, -OCOR⁵, -N(R⁵)COR⁶, -N(R⁵)CSR^{6,} -SO₂N(R⁵)(R⁶), -N(R⁵)SO₂R⁶, N(R⁵)CON(R⁶)(R⁷) [where R⁷ is a hydrogen atom, an optionally substituted C₁₋₆alkyl or C₃₋₈cycloalkyl group], -N(R⁵)CSN(R⁶)(R⁷) or -N(R⁵)SO₂N(R⁶)(R⁷), provided that when t is zero and each of L³ and L⁴ is a covalent bond then u is the integer 1 and R⁴ is other than a hydrogen atom.

When L³ and/or L⁴ is present in these substituents as a linker atom or group it may be any divalent linking atom or group. Particular examples include -O- or -S- atoms or -C(O)-, -C(O)O-, -OC(O)-, -C(S)-, -S(O)-,-S(O)₂-, -N(R⁸)- [where R⁸ is a hydrogen atom or an optionally substituted straight or branched C₁₋₆alkyl group], -CON(R⁸)-, -OC(O)N(R8)-, -CSN(R⁸)-, -N(R⁸)CO-, -N(R⁸)C(O)O-, -N(R⁸)CS-, -S(O)₂N(R⁸)-, -N(R⁸)S(O)₂-, -N(R⁸)O-, -ON(R8)-, -N(R⁸)N(R⁸)-, -N(RB)CON(R8)-, -N(R8)CSN(R⁸)-, or -N(R⁸)SO₂N(R⁸)- groups. Where the linker group contains two R⁸ substituents, these may be the same or different.

When R^{3a}, R⁴, R⁵, R⁶, R⁷ and/or R⁸ is present as a C₁₋₆alkyl group it may be a straight or branched C₁₋₆alkyl group, e.g. a C₁₋₃alkyl group such as a methyl, ethyl or i-propyl group. C₃₋₈cycloalkyl groups represented by R^{3a}, R⁴, R⁵, R⁶ and/or R⁷ include C₃₋₆cycloalkyl groups e.g. cyclopropyl, cyclobutyl, cyclopentyl and cyclohexyl groups. Optional substituents which may be present on such alkyl or cycloalkyl groups include for example one, two or three substituents which may be the same or different selected from halogen atoms, for example fluorine, chlorine, bromine or iodine atoms, or hydroxy or C₁₋₆alkoxy e.g. methoxy or ethoxy groups.

When the groups R⁵ and R⁶ or R⁶ and R⁷ are both C₁₋₆alkyl groups these groups may be joined, together with the N atom to which they are attached, to form a heterocyclic ring. Such heterocyclic rings may be optionally interrupted by a further heteroatom selected from -O-, -S- or-N(R⁵)-. Particular examples of such heterocyclic rings include piperidinyl, morpholinyl, thiomorpholinyl, pyrrolidinyl, imidazolidinyl and piperazinyl rings.

When Alk² is present as an optionally substituted aliphatic or heteroaliphatic chain it may be any optionally substituted aliphatic or heteroaliphatic chain as described hereinafter for Alk¹.

Halogen atoms represented by R⁴ in the optional Ar¹ substituents include fluorine, chlorine, bromine, or iodine atoms.

Examples of the substituents represented by -L³(Alk¹)ₜL⁴(R⁴)ᵤ when present in Ar¹ groups in compounds of the invention include atoms or groups -L³Alk²L⁴R⁴, -L³Alk²R⁴, -L3R4, -R⁴ and -Alk²R⁴ wherein L³, Alk², L⁴ and R⁴ are as defined above. Particular examples of such substituents include -L³CH₂L⁴R⁴, -L³CH(CH₃)L⁴R⁴, -L³(CH₂)₂L⁴R⁴, -L³CH₂R⁴, -L³CH(CH₃)R⁴, -L³(CH₂)₂R⁴, -CH₂R⁴, -CH(CH₃)R⁴, -(CH₂)₂R⁴ and -R⁴ groups.

Thus Ar¹ in compounds of the invention may be optionally substituted for example by one, two, three or more halogen atoms, e.g. fluorine, chlorine, bromine or iodine atoms, and/or C₁₋₆alkyl, e.g. methyl, ethyl, n-propyl, i-propyl, n-butyl or t-butyl, C₃₋₈cycloalkyl, e.g. cyclopropyl, cyclobutyl, cyclopentyl or cyclohexyl, C₁₋₆hydroxyalkyl, e.g. hydroxymethyl, hydroxyethyl or -C(OH)(CF₃)₂, carboxyC₁₋₆alkyl, e.g. carboxyethyl, C₁-₆alkylthio e.g. methylthio or ethylthio, carboxyC₁₋₆alkylthio, e.g. carboxymethylthio, 2-carboxyethylthio or 3-carboxypropylthio, C₁₋₆alkoxy, e.g. methoxy or ethoxy, hydroxyC₁₋₆alkoxy, e.g. 2-hydroxyethoxy, haloC₁₋₆alkyl, e.g. -CF₃, -CHF₂, -CH₂F, haloC₁₋₆alkoxy, e.g. -OCF₃, -OCHF₂,-OCH₂F, C₁₋₆alkylamino, e.g. methylamino or ethylamino, amino (-NH₂), aminoC₁₋₆alkyl, e.g. aminomethyl or aminoethyl, C₁₋₆dialkylamino, e.g. dimethylamino or diethylamino, C₁₋₆alkylaminoC₁₋₆alkyl, e.g. ethylaminoethyl, C₁₋₆dialkylaminoC₁₋₆alkyl, e.g. diethylaminoethyl, aminoc,C₁₋₆alkoxy, e.g. aminoethoxy, C₁₋₆alkylaminoC₁₋₆alkoxy, e.g. methylaminoethoxy, C₁₋₆dialkylaminoC₁₋₆alkoxy, e.g. dimethylaminoethoxy, diethylaminoethoxy, diisopropylaminoethoxy or dimethylaminopropoxy, nitro, cyano, amidino, hydroxyl (-OH), formyl [HC(O)-], carboxyl (-CO₂H),-CO₂R⁵ e.g. -CO₂CH₃ or -CO₂C(CH₃)₃, C₁₋₆alkanoyl e.g. acetyl, thiol (-SH), thioC₁₋₆alkyl, e.g. thiomethyl or thioethyl, sulphonyl (-SO₃H), -SO₃R⁵, C₁₋₆alkylsulphinyl, e.g. methylsulphinyl, C₁₋₆alkylsulphonyl, e.g. methylsulphonyl, aminosulphonyl (-SO₂NH₂), C₁₋₆alkylaminosulphonyl, e.g. methylaminosulphonyl or ethylaminosulphonyl, C₁₋₆dialkylaminosulphonyl, e.g. dimethylaminosulphonyl or diethylaminosulphonyl, phenylaminosulphonyl, carboxamido (-CONH₂), C₁₋₆alkylaminocarbonyl, e.g. methylaminocarbonyl or ethylaminocarbonyl, C₁₋₆dialkylaminocarbonyl, e.g. dimethylaminocarbonyl or diethylaminocarbonyl, aminoC₁₋₆alkylaminocarbonyl, e.g. aminoethylaminocarbonyl, C₁₋₆alkylaminoC₁₋₆alkylaminocarbonyl, e.g. ethylaminoethylaminocarbonyl, C₁₋₆dialkylaminoC₁₋₆alkylaminocarbonyl, e.g. diethylaminoethylaminocarbonyl, aminocarbonylamino, C₁₋₆alkylaminocarbonylamino, e.g. methylaminocarbonylamino or ethylaminocarbonylamino, C₁₋₆dialkylaminocarbonylamino, e.g. dimethylaminocarbonylamino or diethylaminocarbonylamino, C₁₋₆alkylaminocabonylC₁₋₆alkylamino, e.g. methylaminocarbonylmethylamino, aminothiocarbonylamino, C₁₋₆alkylaminothiocarbonylamino, e.g. methylaminothiocarbonylamino or ethylaminothiocarbonylamino, C₁₋₆dialkylaminothiocarbonylamino, e.g. dimethylaminothiocarbonylamino or diethylaminothiocarbonylamino, C₁₋₆alkylaminothiocarbonylC₁₋₆alkylamino, e.g. ethylaminothiocarbonylmethylamino, C₁₋₆alkylsulphonylamino, e.g. methylsulphonylamino or ethylsulphonylamino, C₁₋₆dialkylsulphonylamino, e.g. dimethylsulphonylamino or diethylsulphonylamino, aminosulphonylamino (-NHSO₂NH₂), C₁₋₆alkylaminosulphonylamino, e.g. methylaminosulphonylamino or ethylaminosulphonylamino, C₁₋₆dialkylaminosulphonylamino, e.g. dimethylaminosulphonylamino or diethylaminosulphonylamino, C₁₋₆alkanoylamino, e.g. acetylamino, aminoC₁₋₆alkanoylamino e.g. aminoacetylamino, C₁₋₆dialkylaminoC₁₋₆alkanoylamino, e.g. dimethylaminoacetylamino, C₁₋₆alkanoylaminoC₁₋₆alkyl, e.g. acetylaminomethyl, C₁₋₆alkanoylaminoC₁₋₆alkylamino, e.g. acetamidoethylamino, C₁₋₆alkoxycarbonylamino, e.g. methoxycarbonylamino, ethoxycarbonylamino or t-butoxycarbonylamino groups.

L² when present as part of the group R¹ in compounds of the invention may be a linker atom or group L^{2a} or a linker -(Alk³)L^{2a}-, where Alk³ is an optionally substituted aliphatic or heteroaliphatic chain which may be any such chain as described hereinafter for Alk¹, and L^{2a} may be any linker atom or group as described hereinbefore for L³.

Optionally substituted arylene groups represented by Ar² when present as part of the group R¹ include those aromatic groups as previously described for Ar¹.

Optionally substituted heteroarylene groups represented by Ar² when present as part of the group R¹ include those heteroaromatic groups as previously described for Ar¹.

Each divalent arylene or heteroarylene group represented by Ar² may be attached to the remainder of the molecule through any available ring carbon or nitrogen atoms.

The arylene and heteroarylene groups represented by Ar² may be optionally substituted by one, two or more substituents selected from the atoms or groups -L³(Alk²)ₜL⁴(R⁴)ᵤ described herein. Where two of these atoms or groups are present they may be the same or different.

When the group R² is present in compounds of the invention as a C₁₋₆alkyl group it may be for example a straight or branched C₁₋₆alkyl group e.g. a C₁₋₃alkyl group such as a methyl or ethyl group.

When the group R is present in R¹ in compounds of the invention as a derivative of a carboxylic acid it may be for example an acyclic or cyclic carboxylic acid ester or an amide. Particular acyclic esters and amides include -CO₂Alk⁷ and -CONR⁵R⁶ groups as defined herein. When R is a biostere of a carboxylic acid it may be for example a tetrazole or other acid such as phosphonic acid, phosphinic acid, sulphonic acid, sulphinic acid or boronic acid or an acylsulphonamide group.

Esters (-CO₂Alk⁷) and amide (-CONR⁵R⁶) derivatives of the carboxylic acid group (-CO₂H) in compounds of formula (1) may advantageously be used as prodrugs of the active compound. Such prodrugs are compounds which undergo biotransformation to the corresponding carboxylic acid prior to exhibiting their pharmacological effects and the invention particularly extends to prodrugs of the acids of formula (1). Such prodrugs are well known in the art, see for example International Patent Application No. WO00/23419, Bodor, N. (Alfred Benzon Symposium, 1982, 17, 156-177), Singh, G. et al (J. Sci. Ind. Res., 1996, 55, 497-510) and Bundgaard, H., (Design of Prodrugs, 1985, Elsevier, Amsterdam).

Esterified carboxyl groups represented by the group -CO₂Alk⁷ include groups wherein Alk⁷ is a straight or branched optionally substituted C₁₋₈alkyl group such as a methyl, ethyl, n-propyl, i-propyl, n-butyl, i-butyl, s-butyl, t-butyl, pentyl or neopentyl group; an optionally substituted C₂₋₈alkenyl group such as a propenyl e.g. 2-propenyl or butenyl e.g. 2-butenyl or 3-butenyl group, an optionally substituted C₂₋₈alkynyl group such as a ethynyl, propynyl e.g. 2-propynyl or butynyl e.g. 2-butynyl or 3-butynyl group, an optionally substituted C₃-₈cycloalkyl group such as a cyclopropyl, cyclobutyl, cyclopentyl, cyclohexyl or cycloheptyl group; an optionally substituted C₃₋₈heterocycloalkyl group such as a tetrahydrofuanyl e.g. tetrahydrofuran-3-yl, pyrrolidinyl e.g. 1-methylpyrrolidinyl such as 1-methylpyrrolidin-3-yl, piperidinyl e.g. 1-methylpiperidinyl such as 1-methylpiperidin-4-yl, tetrahydropyranyl e.g. tetrahydropyran-4-yl or 2-oxo-[1,3]dioxol-4-yl e.g. 5-methyl-2-oxo-[1,3]dioxol-4-yl group; an optionally substituted C₃₋₈cycloalkylC₁₋₈alkyl group such as a cyclopentylmethyl, cyclohexylmethyl or cyclohexylethyl group; an optionally substituted C₃₋₈heterocycloalkylC₁₋₈alkyl group such as a morpholinyl-N-ethyl, thiomorpholinyl-N-methyl, pyrrolidinyl-N-ethyl, pyrrolidinyl-N-propyl, piperidinyl-N-ethyl, pyrazolidinyl-N-methyl or piperazinyl-N-ethyl group; an optionally substituted C₁₋₆alkyloxyC₁₋₆alkyl group such as a methyloxyethyl or propyloxyethyl group; an optionally substituted hydroxyC₁₋₆alkyl group such as a hydroxyethyl e.g. 2-hydroxyethyl or hydroxypropyl e.g. 2-hydroxypropyl, 3-hydroxypropyl or 2,3-dihydroxypropyl group; an optionally substituted C₁₋₆alkylthioC₁₋₆alkyl group such as an ethylthioethyl group; an optionally substituted C₁₋₆alkylsulfinylC₁₋₆alkyl group such as an methylsulfinylethyl group; an optionally substituted C₁₋₆alkylsulfonylC₁₋₆alkyl group such as an methylsulfonylmethyl group; an optionally substituted C₃₋₈cycloalkyloxyC₁₋₆alkyl group such as a cyclohexyloxymethyl group; an optionally substituted C₃₋₈cycloalkylthioC₁₋₆alkyl group such as a cyclopentylthiomethyl group; an optionally substituted C₃₋₈cycloalkylsulfinylC₁₋₆alkyl group such as a cyclopentyl-sulfinylmethyl group; an optionally substituted C₃₋₈cycloalkylsulfonylC₁₋₆alkyl group such as a cyclopentylsulfonylmethyl group; an optionally substituted C₁₋₆alkyloxycarbonylC₁₋₆alkyl group such as isobutoxy-carbonylpropyl group; an optionally substituted C₁₋₆alkyloxycarbonylC₁₋₆alkenyl group such as isobutoxycarbonylpentenyl group; an optionally substituted C₁₋₆alkyloxycarbonyloxyC₁₋₆alkyl group such as an ethyloxycarbonyloxymethyl or isopropoxycarbonyloxyethyl e.g 1-(isopropoxycarbonyloxy)ethyl or 2-(isopropoxycarbonyloxy)ethyl group; an optionally substituted C₁₋₆alkyloxycarbonyloxyC₁₋₆alkenyl group such as a isopropoxycarbonyloxybutenyl group, an optionally substituted C₃₋₈cycloalkyloxycarbonyloxyC₁₋₆alkyl group such as a cyclohexyloxycarbonyloxyethyl, e.g. a 2-(cyclohexyloxycarbonyloxy)ethyl group, an optionally substituted N-di-C₁₋₈alkylaminoC₁₋₈alkyl group such as a N-dimethylaminoethyl or N-diethylaminoethyl group; an optionally substituted N-C₆₋₁₂aryl-N-C₁₋₆alkylaminoC₁₋₆alkyl group such as a N-phenyl-N-methylaminomethyl group; an optionally substituted N-di-C₁₋₈alkylcarbamoylC₁₋₈alkyl group such as a N-diethylcarbamoylmethyl group; an optionally substituted C₆₋₁₂arylC₁₋₆alkyl group such as an optionally substituted benzyl, phenylethyl, phenylpropyl, 1-naphthylmethyl or 2-naphthylmethyl group; an optionally substituted heteroC₆₋₁₀arylC₁₋₆alkyl group, such as a pyridinylmethyl e.g. pyridin-4-ylmethyl or imidazolylethyl e.g. 2-imidazol-1-ylethyl group; a C₆₋₁₂aryl group such as an optionally substituted phenyl, 1-naphthyl or 2-naphthyl group; a C₆₋₁₂aryloxyC₁₋₈alkyl group such as an optionally substituted phenyloxymethyl, phenyloxyethyl, 1-naphthyloxymethyl, or 2-naphthyloxymethyl group; a C₆₋₁₂arylthioC₁₋₈alkyl group such as an optionally substituted phenylthioethyl group; a C₆₋₁₂aryisulfinylC₁₋₈alkyl group such as an optionally substituted phenyl-sulfinylmethyl group; a C₆₋₁₂arylsulfonylC₁₋₈alkyl group such as an optionally substituted phenylsulfonylmethyl group; an optionally substituted C₁₋₈alkanoyloxyC₁₋₈alkyl group, such as a acetoxymethyl, ethoxycarbonyloxyethyl, pivaloyloxymethyl, propionyloxyethyl or propionyloxypropyl group; an optionally substituted C₄₋₈imidoC₁₋₈alkyl group such as a succinimidomethyl or phthalamidoethyl group; a C₆₋₁₂aroyloxyC₁₋₈alkyl group such as an optionally substituted benzoyloxyethyl or benzoyloxypropyl group or a triglyceride such as a 2-substituted triglyceride e.g. a 1,3-di-C₁₋₈alkylglycerol-2-yl group such as a 1,3-diheptylglycerol-2-yl group. Optional substituents present on the Alk⁷ group include R^{13a} substituents described below.

It will be appreciated that in the forgoing list of Alk⁷ groups the point of attachment to the remainder of the compound of formula (1) is via the last described part of the Alk⁷ group. Thus, for example a methoxyethyl group would be attached by the ethyl group, whilst a morpholinyl-N-ethyl group would be attached via the N-ethyl group.

It will be further appreciated that in the forgoing list of Alk⁷ groups, where not specifically mentioned, alkyl groups may be replaced by alkenyl or alkynyl groups where such groups are as previously defined for Alk¹. Additionally these alkyl, alkenyl or alkynyl groups may optionally be interrupted by one, two or three linker atoms or groups where such linker atoms and groups are as previously defined for L³.

Further prodrugs of compounds of formula (1) include cyclic esters where X is a -N(R²)- group in which R² becomes a C₁₋₆alkyl joining chain, especially a -CH₂- or -CH₂CH₂- chain, which is also connected to the acid group R to form a cyclic ester of formula (1a):

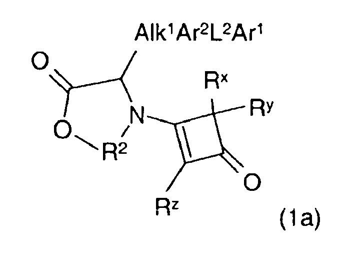

When present in the group R^{x}, R^{y} and/or R^{z} in compounds of formula (1) the linker atom or group represented by L¹ may be any linker atom or group as described above for the linker atom or group L³. In addition L¹ may also be a -Se- atom.

When Alk¹ is present in the group R^{x}, RY and/or R^{z} in compounds of formula (1) as an optionally substituted aliphatic chain it may be an optionally substituted C₁₋₁₀aliphatic chain. Particular examples include optionally substituted straight or branched chain C₁₋₆alkylene, C₂₋₆alkenylene or C₂₋₆alkynylene chains.

Particular examples of aliphatic chains represented by Alk¹ include optionally substituted -CH₂-, -(CH₂)₂-, -CH(CH₃)CH₂-, -(CH₂)₂CH₂-,-(CH₂)₃CH₂-, -CH(CH₃)(CH₂)₂-, -CH₂CH(CH₃)CH₂-, -C(CH₃)₂CH₂-, CH₂C(CH₃)₂CH₂-, -(CH₂)₂C(CH₃)₂CH₂-, -(CH₂)₄CH₂-, -(CH₂)₅CH₂-,-CHCH-, -CHCHCH₂-, -CH₂CHCH-, -CHCHCH₂CH₂-, -CH₂CHCHCH₂-,-(CH₂)₂CHCH-, -CC-, -CCCH₂-, -CH₂CC-, -CCCH₂CH₂-, -CH₂CCCH₂- or -(CH₂)₂CC- chains.

Heteroaliphatic chains represented by Alk¹ when present in the group R^{x}, R^{y} and/or R^{z} in compounds of formula (1) include the aliphatic chains just described for Alk¹ but with each additionally containing one, two, three or four heteroatoms or heteroatom-containing groups. Particular heteroatoms or groups include atoms or groups L⁵ where L⁵ is as defined above for L³ when L³ is a linker atom or group. Each L⁵ atom or group may interrupt the aliphatic chain, or may be positioned at its terminal carbon atom to connect the chain to an adjoining atom or group. Particular examples include optionally substituted -CH₂L⁵-, -CH₂CH₂L⁵-, -L⁵CH₂-,-L⁵CH₂CH₂-, -L⁵CH(CH₃)CH₂-, -L⁵CH₂CH(CH₃)CH₂-,-L⁵CH₂CH₂CH(CH₃)-, -L⁵C(CH₃)₂CH₂-, -CH₂L⁵CH₂CH₂-, -(CH₂)₂L⁵CH₂-,-(CH₂)₃L⁵CH₂-, -L⁵(CH₂)₃-, -L⁵(CH₂)₄-, -CH₂L⁵CH₂CHL⁵CH₂- and -(CH₂)₂L⁵CH₂CH₂- chains.

The optional substituents which may be present on aliphatic or heteroaliphatic chains represented by Alk¹ include one, two, three or more substituents where each substituent may be the same or different and is selected from halogen atoms, e.g. fluorine, chlorine, bromine or iodine atoms, or -OH, -CO₂H, -CO₂R⁹, where R⁹ is an optionally substituted straight or branched C₁₋₆alkyl group as defined above for R⁴, -CONHR⁹, -CON(R⁹)₂, -COR⁹, e.g. -COCH₃, C₁₋₆alkoxy, e.g. methoxy or ethoxy, thiol, -S(O)R⁹, -S(O)₂R⁹, C₁₋₆alkylthio e.g. methylthio or ethylthio, amino or substituted amino groups. Substituted amino groups include -NHR⁹ and -N(R⁹)₂ groups. Where two R⁹ groups are present in any of the above substituents these may be the same or different.

Optionally substituted cycloaliphatic groups represented by the group R³ when present in the group R^{x}, R^{y} and/or R^{z} in compounds of the invention include optionally substituted C₃₋₁₀cycloaliphatic groups. Particular examples include optionally substituted C₃₋₁₀cycloalkyl, e.g. C₃₋₈cycloalkyl or C₃₋₁₀cycloalkenyl, e.g C₃₋₈cycloalkenyl groups.

Optionally substituted heterocycloaliphatic groups represented by the group R³ when present in the group R^{x}, R^{y} and/or R^{z} include optionally substituted C₃₋₁₀heterocycloaliphatic groups. Particular examples include optionally substituted C₃₋₁₀heterocydoalkyl, e.g. C₃₋₇heterocycloalkyl, or C₃₋₁₀heterocycloalkenyl, e.g. C₃₋₇hetercycloalkenyl groups, each of said groups containing one, two, three or four heteroatoms or heteroatom-containing groups L⁵ as defined above.

Optionally substituted polycycloaliphatic groups represented by the group R³ when present in the group R^{x}, R^{y} and/or R^{z} include optionally substitued C₇₋₁₀ bi- or tricycloalkyl or C₇₋₁₀bi- or tricycloalkenyl groups. Optionally substituted heteropolycycloaliphatic groups represented by the group R³ include the optionally substituted polycycloaliphatic groups just described, but with each group additionally containing one, two, three or four L⁵ atoms or groups.

Particular examples of cycloaliphatic, polycycloaliphatic, heterocycloaliphatic and heteropolycycloaliphatic groups represented by the group R³ include optionally substituted cyclopropyl, cyclobutyl, cyclopentyl, cyclohexyl, cycloheptyl, cyclooctyl, cyclopropenyl, cyclobutenyl, cyclopentenyl, cyclohexenyl, cycloheptenyl, cyclooctenyl, 2-cyclobuten-1-yl, 2-cyclopenten-1-yl, 3-cyclopenten-1-yl, adamantyl, norbornyl, norbornenyl, tetrahydrofuranyl, tetrahydrothiophenyl, tetrahydrothiophene-1-oxide, tetrahydrothiophene-1,1-dioxide, pyrroline, e.g. 2- or 3-pyrrolinyl, pyrrolidinyl, pyrrolidinone, oxazolidinyl, oxazolidinone, dioxolanyl, e.g. 1,3-dioxolanyl, imidazolinyl, e.g. 2-imidazolinyl, imidazolidinyl, pyrazolinyl, e.g. 2-pyrazolinyl, pyrazolidinyl, pyranyl, e.g. 2- or 4-pyranyl, tetrahydropyranyl, tetrahydrothiopyranyl, tetrahydrothiopyran-1-oxide, tetrahydrothiopyran-1,1-dioxide, piperidinyl, piperidinone, dioxanyl e.g. 1,3-dioxanyl or 1,4-dioxanyl, morpholinyl, morpholinone, dithianyl, e.g. 1,3-dithianyl or 1,4-dithianyl, thiomorpholinyl, piperazinyl, 1,3,5-trithianyl, oxazinyl, e.g. 2H-1,3-, 6H-1,3-, 6H-1,2-, 2H-1,2- or 4H-1,4- oxazinyl, 1,2,5-oxathiazinyl, isoxazinyl, e.g. o-or p-isoxazinyl, oxathiazinyl, e.g. 1,2,5 or 1,2,6-oxathiazinyl, or 1,3,5,-oxadiazinyl groups.

The optional substituents which may be present on the cycloaliphatic, polycycloaliphatic, heterocycloaliphatic or heteropolycycloaliphatic groups represented by the group R³ include one, two, three or more substituents each selected from halogen atoms, e.g. fluorine, chlorine, bromine or iodine atoms, or C₁₋₆alkyl, e.g. methyl, ethyl, propyl or i-propyl, haloC₁₋₆alkyl, e.g. halomethyl or haloethyl such as difluoromethyl or trifluoromethyl, optionally substituted by hydroxyl, e.g. -C(OH)(CF₃)₂, C₁₋₆alkoxy, e.g. methoxy, ethoxy or propoxy, haloC₁₋₆alkoxy, e.g. halomethoxy or haloethoxy such as difluoromethoxy or trifluoromethoxy, thiol, C₁₋₆alkylthio e.g. methylthio, ethylthio or propylthio, or -(Alk⁴)_{g}R¹⁰ groups in which Alk⁴ is a straight or branched C₁₋₃alkylene chain, g is zero or an integer 1 and R¹⁰ is a -OH, -SH, -N(R¹¹)2, (in which R¹¹ is an atom or group as defined herein for R⁷) -CN, -CO₂R¹¹, -NO₂, -CON(R¹¹)₂,-CSN(R¹¹)₂, -COR¹¹, -CSN(R¹¹)₂, -N(R¹¹)COR¹¹, -N(Rll)CSR11,-SO₂N(R¹¹)₂, -N(RH)SO₂R¹¹, -N(R¹¹)CON(R¹¹)₂, -N(R¹¹)CSN(R¹¹), N(R¹¹)SO₂N(R¹¹)₂ or optionally substituted phenyl group. Where two R¹¹ atoms or groups are present in these substituents these may be the same or different or joined to form a heterocyclic ring as previously described when R⁵ and R⁶ are joined together. Optionally substituted phenyl groups include phenyl substituted by one, two or three of the R¹³ groups described below.

Additionally, when the group R³ is a heterocycloaliphatic group containing one or more nitrogen atoms each nitrogen atom may be optionally substituted by a group -(L⁶)ₚ(Alk⁵)_{q}R¹² in which L⁶ is -C(O)-, -C(O)O-,-C(S)-, -S(O)₂-, -CON(R⁸)-, -CSN(R⁸)- or SO₂N(R⁸)-; p is zero or an integer 1; Alk⁵ is an optionally substituted aliphatic or heteroaliphatic chain; q is zero or an integer 1; and R¹² is a hydrogen atom or an optionally substituted cycloaliphatic, heterocycloaliphatic, polycycloaliphatic, polyheterocycloaliphatic, aromatic or heteroaromatic group.

C₁₋₃alkylene chains represented by Alk⁴ include -CH₂-, -CH₂CH₂-,-CH₂CH₂CH₂-, -CH(CH₃)CH₂- and -CH₂CH(CH₃)- chains.

Optionally substituted aliphatic or heteroaliphatic chains represented by Alk⁵ include those optionally substituted chains described above for Alk¹. Optional substituents which may be present on these groups include those described above in relation to Alk¹.

Cycloaliphatic, heterocycloaliphatic, polycycloaliphatic or polyheterocycloaliphatic groups represented by R¹² include those groups just described for the group R³. Optional substituents which may be present on those groups include those described above in relation to R³ cycloaliphatic groups.

Aromatic or heteroaromatic groups represented by R¹² include those groups described herein for the group Ar¹. Optional substituents which may be present on these groups include those R¹³ optional substituents described hereinafter.

When the group R³ is an optionally substituted aromatic or heteroaromatic group it may be for example an aromatic or heteroaromatic group as described herein for the group Ar¹.

Optional substituents which may be present on the aromatic or heteroaromatic groups represented by the group R³ include one, two, three or more substituents, each selected from an atom or group R¹³ in which R¹³ is -R^{13a} or -Alk⁶(R^{13a})ₘ, where R^{13a} is a halogen atom, or an amino (-NH₂), substituted amino, nitro, cyano, amidino, hydroxyl (-OH), substituted hydroxyl, formyl, carboxyl (-CO₂H), esterified carboxyl, thiol (-SH), substituted thiol, -COR¹⁴ [where R¹⁴ is an -Alk⁶(R^{13a})ₘ, aryl or heteroaryl group], -CSR¹⁴, -SO₃H, -SOR¹⁴, -SO₂R¹⁴, -SO₃R¹⁴, -SO₂NH₂, -SO₂NHR¹⁴, SO₂N(R¹⁴)₂, -CONH₂, -CSNH₂, -CONHR¹⁴, -CSNHR¹⁴,-CON[R¹⁴]₂, -CSN(R¹⁴)₂, -N(R¹¹)SO₂R¹⁴, -N(SO₂R¹⁴)₂,-NH(R¹¹)SO₂NH₂, -N(R¹¹)SO₂NHR¹⁴, -N(R¹¹)SO₂N(R¹⁴)₂, -N(R¹¹)COR¹⁴, -N(R¹¹)CONH₂, -N(R¹¹)CONHR¹⁴, -N(R¹¹)CON(R¹⁴)₂, -N(R¹¹)CSNH₂, -N(R¹¹)CSNHR¹⁴, -N(R¹¹)CSN(R¹⁴)₂, -N(R¹¹)CSR¹⁴, -N(R¹¹)C(O)OR¹⁴, -SO₂NHet¹ [where -NHet¹ is an optionally substituted C₅₋₇cyclicamino group optionally containing one or more other -O- or -S- atoms or -N(R¹¹)-, -C(O)-, -C(S)-, S(O) or -S(O)₂ groups], -CONHet¹, -CSNHet¹, -N(R¹¹)SO₂NHet¹, -N(R¹¹)CONHet¹, -N(R¹¹)CSNHet¹, -SO₂N(R¹¹)Het² [where Het² is an optionally substituted monocyclic C₅₋₇carbocyclic group optionally containing one or more -O- or -S- atoms or -N(R¹¹)-, -C(O)- or -C(S)- groups], -Het², -CON(R¹¹)Het², -CSN(R¹¹)Het², -N(R¹¹)CON(R¹¹)Het², -N(R¹¹)CSN(R¹¹)Het², aryl or heteroaryl group; Alk⁶ is a straight or branched C₁₋₆alkylene, C₂₋₆alkenylene or C₂₋₆alkynylene chain, optionally interrupted by one, two or three -O- or -S-atoms or -S(O)ₙ [where n is an integer 1 or 2] or -N(R¹⁵)- groups [where R¹⁵ is a hydrogen atom or C₁₋₆alkyl, e.g. methyl or ethyl group]; and m is zero or an integer 1, 2 or 3. It will be appreciated that when two R¹¹ or R¹⁴ groups are present in one of the above substituents, the R¹¹ or R¹⁴ groups may be the same or different.

When in the group -Alk⁶(R^{13a})ₘ m is an integer 1, 2 or 3, it is to be understood that the substituent or substituents R^{13a} may be present on any suitable carbon atom in -Alk⁶. Where more than one R^{13a} substituent is present these may be the same or different and may be present on the same or different atom in -Alk⁶. Clearly, when m is zero and no substituent R^{13a} is present the alkylene, alkenylene or alkynylene chain represented by Alk⁶ becomes an alkyl, alkenyl or alkynyl group.

When R^{13a} is a substituted amino group it may be for example a group-NHR¹⁴ [where R¹⁴ is as defined above] or a group -N(R¹⁴)₂ wherein each R¹⁴ group is the same or different.

When R^{13a} is a halogen atom it may be for example a fluorine, chlorine, bromine, or iodine atom.

When R^{13a} is a substituted hydroxyl or substituted thiol group it may be for example a group -OR¹⁴ or a -SR¹⁴ or -SC(=NH)NH₂ group respectively.

Esterified carboxyl groups represented by the group R^{13a} include groups of formula -CO₂Alk⁸ wherein Alk⁸ is a straight or branched, optionally substituted C₁₋₈alkyl group such as a methyl, ethyl, n-propyl, i-propyl, n-butyl, i-butyl, s-butyl or t-butyl group; a C₆₋₁₂arylC₁₋₈alkyl group such as an optionally substituted benzyl, phenylethyl, phenylpropyl, 1-naphthylmethyl or 2-naphthylmethyl group; a C₆₋₁₂aryl group such as an optionally substituted phenyl, 1-naphthyl or 2-naphthyl group; a C₆₋₁₂aryloxyC₁₋₈alkyl group such as an optionally substituted phenyloxymethyl, phenyloxyethyl, 1-naphthyloxymethyl, or 2-naphthyloxymethyl group; an optionally substituted C₁₋₈alkanoyloxyC₁₋₈alkyl group, such as a pivaloyloxymethyl, propionyloxyethyl or propionyloxypropyl group; or a C₆₋₁₂aroyloxyC₁₋₈alkyl group such as an optionally substituted benzoyloxyethyl or benzoyloxypropyl group. Optional substituents present on the Alk⁸ group include R^{13a} substituents described above.

When Alk⁶ is present in or as a substituent it may be for example a methylene, ethylene, n-propylene, i-propylene, n-butylene, i-butylene, s-butylene, t-butylene, ethenylene, 2-propenylene, 2-butenylene, 3-butenylene, ethynylene, 2-propynylene, 2-butynylene or 3-butynylene chain, optionally interrupted by one, two, or three -O- or -S-, atoms or -S(O)-, -S(O)₂- or -N(R⁸)- groups.

Aryl or heteroaryl groups represented by the groups R^{13a} or R¹⁴ include mono- or bicyclic optionally substituted C₆₋₁₂aromatic or C₁₋₉heteroaromatic groups as described above for the group Ar¹. The aromatic and heteroaromatic groups may be attached to the remainder of the compound of formula (1) by any carbon or hetero e.g. nitrogen atom as appropriate.

When -NHet¹ or -Het² forms part of a substituent R¹³ each may be for example an optionally substituted pyrrolidinyl, pyrazolidinyl, piperazinyl, morpholinyl, thiomorpholinyl, piperidinyl or thiazolidinyl group. Additionally Het² may represent for example, an optionally substituted cyclopentyl or cyclohexyl group. Optional substituents which may be present on -NHet¹ or -Het² include those optional substituents described above in relation to aliphatic chains represented by Alk¹.

Particularly useful atoms or groups represented by R¹³ include fluorine, chlorine, bromine or iodine atoms, or C₁₋₆alkyl, e.g. methyl, ethyl, n-propyl, i-propyl, n-butyl or t-butyl, optionally substituted phenyl, pyridyl, pyrimidinyl, pyrrolyl, furyl, thiazolyl, thienyl, morpholinyl, thiomorpholinyl, piperazinyl, e.g. t-butyloxycarbonylpiperazinyl, pyrrolidinyl, dioxolanyl, dioxanyl, oxazolidinyl, thiazolidinyl, imidazolidinyl or piperidinyl, C₁₋₆hydroxyalkyl, e.g. hydroxymethyl or hydroxyethyl, carboxyC₁₋₆alkyl, e.g. carboxyethyl, C₁₋₆alkylthio e.g. methylthio or ethylthio, carboxyC₁₋₆alkylthio, e.g. carboxymethylthio, 2-carboxyethylthio or 3-carboxypropylthio, C₁₋₆alkoxy, e.g. methoxy or ethoxy, hydroxyC₁₋₆alkoxy, e.g. 2-hydroxyethoxy, optionally substituted phenoxy, pyridyloxy, thiazolyoxy, phenylthio or pyridylthio, C₄₋₇cycloalkyl, e.g. cyclobutyl, cyclopentyl, C₅₋₇cycloalkoxy, e.g. cyclopentyloxy, haloC₁₋₆alkyl, e.g. trifluoromethyl, haloC₁₋₆alkoxy, e.g. trifluoromethoxy, C₁₋₆alkylamino, e.g. methylamino, ethylamino or propylamino, C₆₋₁₂arylC₁₋₆alkylamino, e.g. benzylamino, 4-fluorobenzylamino or 4-hydroxyphenylethylamino, amino (-NH₂), aminoC₁₋₆alkyl, e.g. aminomethyl or aminoethyl, C₁₋₆dialkylamino, e.g. dimethylamino or diethylamino, aminoC₁₋₆alkylamino, e.g. aminoethylamino or aminopropylamino, optionally substituted Het¹NC₁₋₆alkylamino, e.g. 3-morpholinopropylamino, C₁₋₆alkylaminoC₁₋₆alkyl, e.g. ethylaminoethyl, C₁₋₆dialkylaminoC₁₋₆alkyl, e.g. diethylaminoethyl, aminoC₁₋₆alkoxy, e.g. aminoethoxy, C₁₋₆alkylaminoC₁₋₆alkoxy, e.g. methylaminoethoxy, C₁₋₆dialkylaminoC₁₋₆alkoxy, e.g. dimethylaminoethoxy, diethylaminoethoxy, diisopropylaminoethoxy, or dimethylaminopropoxy, hydroxyC₁₋₆alkylamino, e.g. 2-hydroxyethylamino, 3-hydroxypropylamino or 3-hydroxybutylamino, imido, such as phthalimido or naphthalimido, e.g. 1,8-naphthalimido, nitro, cyano, amidino, hydroxyl (-OH), formyl [HC(O)-], carboxyl (-CO₂H),-CO₂Alk⁸ [where Alk⁸ is as defined above], C₁₋₆alkanoyl e.g. acetyl, propyryl or butyryl, optionally substituted benzoyl, thiol (-SH), thioC₁₋₆alkyl, e.g. thiomethyl or thioethyl, -SC(=NH)NH₂, sulphonyl (-SO₃H), -SO₃Alk⁸, C₁₋₆alkylsulphinyl, e.g. methylsulphinyl, ethylsulphinyl or propylsulphinyl, C₁₋₆alkylsulphonyl, e.g. methylsulphonyl, ethylsulphonyl or propylsulphonyl, aminosulphonyl (-SO₂NH₂), C₁₋₆alkylaminosulphonyl, e.g. methylaminosulphonyl, ethylaminosulphonyl or propylaminosulphonyl C₁₋₆dialkylaminosulphonyl, e.g. dimethylaminosulphonyl or diethylaminosulphonyl, phenylaminosulphonyl, carboxamido (-CONH₂), C₁₋₆alkylaminocarbonyl, e.g. methylaminocarbonyl, ethylaminocarbonyl or propylaminocarbonyl, C₁₋₆dialkylaminocarbonyl, e.g. dimethylaminocarbonyl or diethylaminocarbonyl, aminoC₁₋₆alkylaminocarbonyl, e.g. aminoethylaminocarbonyl, C₁₋₆alkylaminoC₁₋₆alkylaminocarbonyl, e.g. methylaminoethylaminocarbonyl, C₁₋₆dialkylaminoC₁₋₆alkylaminocarbonyl, e.g. diethylaminoethylaminocarbonyl, aminocarbonylamino, C₁₋₆alkylaminocarbonylamino, e.g. methylaminocarbonylamino or ethylaminocarbonylamino, C₁₋₆dialkylaminocarbonylamino, e.g. dimethylaminocarbonylamino or diethylaminocarbonylamino, C₁₋₆alkylaminocabonylC₁₋₆alkylamino, e.g. methylaminocarbonylmethylamino, aminothiocarbonylamino, C₁₋₆alkylaminothiocarbonylamino, e.g. methylaminothiocarbonylamino or ethylaminothiocarbonylamino, C₁₋₆dialkylaminothiocarbonylamino, e.g. dimethylaminothiocarbonylamino or diethylaminothiocarbonylamino, C₁₋₆alkylaminothiocarbonylC₁₋₆alkylamino, e.g. ethylaminothiocarbonylmethylamino,-CONHC(=NH)NH₂, C₁₋₆alkylsulphonylamino, e.g. methylsulphonylamino or ethylsulphonylamino, haloC₁₋₆alkylsulphonylamino, e.g. trifluoromethylsulphonylamino, C₁₋₆dialkylsulphonylamino, e.g. dimethylsulphonylamino or diethylsulphonylamino, optionally substituted phenylsulphonylamino, aminosulphonylamino (-NHSO₂NH₂), C₁₋₆alkylaminosulphonylamino, e.g. methylaminosulphonylamino or ethylaminosulphonylamino, C₁₋₆dialkylaminosulphonylamino, e.g. dimethylaminosulphonylamino or diethylaminosulphonylamino, optionally substituted morpholinesulphonylamino or morpholinesulphonylC₁₋₆alkylamino, optionally substituted phenylaminosulphonylamino, C₁₋₆alkanoylamino, e.g. acetylamino, aminoC₁₋₆alkanoylamino e.g. aminoacetylamino, C₁₋₆dialkylaminoC₁₋₆alkanoylamino, e.g. dimethylaminoacetylamino, C₁₋₆alkanoylaminoC₁₋₆alkyl, e.g. acetylaminomethyl, C₁₋₆alkanoylaminoC₁₋₆alkylamino, e.g. acetamidoethylamino, C₁₋₆alkoxycarbonylamino, e.g. methoxycarbonylamino, ethoxycarbonylamino or t-butoxycarbonylamino or optionally substituted benzyloxy, pyridylmethoxy, thiazolylmethoxy, benzyloxycarbonylamino, benzyloxycarbonylaminoC₁₋₆alkyl e.g. benzyloxycarbonylaminoethyl, thiobenzyl, pyridylmethylthio or thiazolylmethylthio groups.

Where desired, two R¹³ substituents may be linked together to form a cyclic group such as a cyclic ether, e.g. a C₁₋₆alkylenedioxy group such as methylenedioxy or ethylenedioxy.

It will be appreciated that where two or more R¹³ substituents are present, these need not necessarily be the same atoms and/or groups. In general, the substituent(s) may be present at any available ring position in the aromatic or heteroaromatic group represented by R³.

When the groups R^{x} and R^{y} are joined together to form an optionally substituted spiro linked cycloaliphatic or heterocycloaliphatic group joined to the cyclobutenone ring as defined by formula (1) it may be any such cycloaliphatic or heterocycloaliphatic group as previously described for R³. Optional substituents which may be present on such spiro linked cycloaliphatic or heteroaliphatic groups include those optional substituents as described in relation to R³.

The presence of certain substituents in the compounds of formula (1) may enable salts of the compounds to be formed. Suitable salts include pharmaceutically acceptable salts, for example acid addition salts derived from inorganic or organic acids, and salts derived from inorganic and organic bases.

Acid addition salts include hydrochlorides, hydrobromides, hydroiodides, alkylsulphonates, e.g. methanesulphonates, ethanesulphonates, or isothionates, arylsulphonates, e.g. p-toluenesulphonates, besylates or napsylates, phosphates, sulphates, hydrogen sulphates, acetates, trifluoroacetates, propionates, citrates, maleates, fumarates, malonates, succinates, lactates, oxalates, tartrates and benzoates.

Salts derived from inorganic or organic bases include alkali metal salts such as sodium or potassium salts, alkaline earth metal salts such as magnesium or calcium salts, and organic amine salts such as morpholine, piperidine, dimethylamine or diethylamine salts.

Particularly useful salts of compounds according to the invention include pharmaceutically acceptable salts, especially acid addition pharmaceutically acceptable salts.

In the compounds according to the invention the group R¹ is preferably an Ar¹L²Ar²Alk- group. In compounds of this type Ar¹ is preferably an optionally substituted phenyl, monocyclic heteroaromatic or bicyclic heteroaromatic group. Particularly useful monocyclic heteroaromatic groups are optionally substituted five- or six-membered heteroaromatic groups as described previously, especially five- or six-membered heteroaromatic groups containing one or two heteroatoms selected from oxygen, sulphur or nitrogen atoms. Nitrogen-containing groups are especially useful, particularly pyridyl or pyrimidinyl groups. Particularly useful substituents present on these monocyclic Ar¹ groups include halogen atoms or alkyl, haloalkyl, -OR⁵, -SR⁵, -NR⁵R⁶, -CO₂H, -CO₂CH₃, -NO₂, -N(R⁵)COR⁶ or -CN groups as described above in relation to the compounds of formula (1). Particularly useful bicyclic heteroaromatic groups represented by Ar¹ include optionally substituted ten-membered fused-ring heteroaromatic groups containing one, two or three, especially one or two heteroatoms, especially nitrogen atoms. Particular examples include optionally substituted naphthyridinyl, especially 2,6-naphthyridinyl, 2,7-naphthyridinyl, quinolinyl and isoquinolinyl, especially isoquinolin-1-yl groups. Particular optional substituents include those just described for monocyclic heteroaromatic groups. Additionally, in compounds according to the invention X is preferably an -N(R²)- group and V is preferably an oxygen atom.

A particularly useful group of compounds according to the invention has the formula (2a): 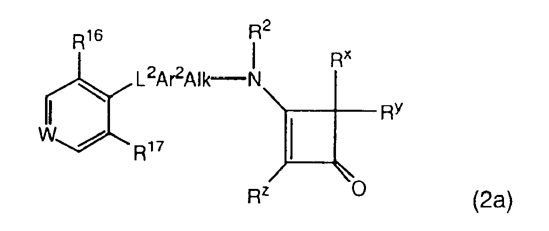 wherein -W= is -CR¹⁸=, -N= or -N(O)=;
R¹⁶, R¹⁷ and R¹⁸, which may be the same or different is each a hydrogen atom or an atom or group -L³(Alk²)ₜL⁴(R⁴)ᵤ in which L³, Alk², t, L⁴, R⁴ and u are as defined previously;
L², Ar², Alk, R², R^{x}, R^{y} and R^{z} are as defined for formula (1);
and the salts, solvates, hydrates and N-oxides thereof.

In one preferred class of compounds of formula (2a) where W is a -CR¹⁸= group R¹⁸ is a hydrogen atom. In another preferred class of compounds R¹⁸ is a preferred atom or group as hereinafter defined for R¹⁶, especially a C₁₋₆alkoxy, especially a methoxy or ethoxy, group.

In another preferred class of compounds of formula (2a) W is a -N= or -N(O)= group.

R¹⁶ and R¹⁷ in compounds of formula (2a) is each preferably as particularly described above for compounds of formula (1), other than a hydrogen atom. Particularly useful R¹⁶ and R¹⁷ substituents include halogen atoms, especially fluorine or chlorine atoms, or C₁₋₆alkyl, especially methyl, ethyl or isopropyl, haloC₁₋₆alkyl especially halomethyl, most especially -CF₃, -CHF₂ or -CH₂F, C₁₋₆alkoxy especially methoxy or etoxy or haloC₁₋₆alkoxy especially halomethoxy, most especially -OCF₃, -OCHF₂ or -OCH₂F groups.

A further particularly useful group of compounds according to the invention has the formula (2b): 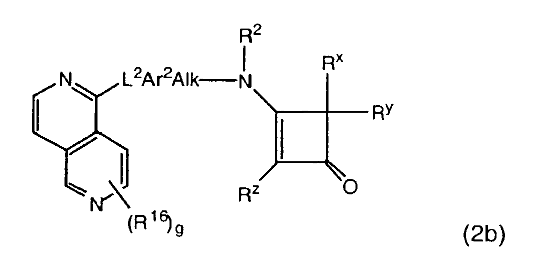 wherein g is the integer 1, 2, 3 or 4;
R¹⁶, is an atom or group -L³(Alk²)ₜL⁴(R⁴)ᵤ in which L³, Alk², t, L⁴, R⁴ and u are as defined previously;
L2, Ar², Alk, R², R^{x}, R^{y} and R^{z} are as defined for formula (1);
and the salts, solvates, hydrates and N-oxides thereof.

Particularly useful R¹⁶ substituents when present in compounds of formula (2b) include halogen atoms, especially fluorine, chlorine or bromine atoms, or C₁₋₆alkyl e.g. methyl, ethyl or isopropyl, haloC₁-₆alkyl, especially halomethyl, most especially -CF₃, C₁-₆alkoxyl, especially methoxy, haloC₁₋₆alkoxy, especially halomethoxy, most especially -OCF₃, -CN,-CO₂CH₃, -NO₂, amino (-NH₂), substituted amino (-NR⁵R⁶) especially-NHCH₃ and -N(CH₃)₂, -N(R⁵)COCH₃, especially -NHCOCH₃ groups or optionally substituted phenyl, furyl, thienyl, imidazolyl, pyridyl and pyrimidinyl groups.

A further particularly useful group of compounds according to the invention has the formula (2c): 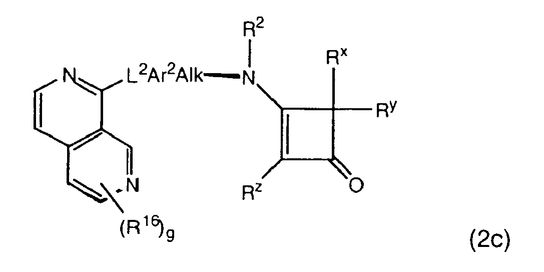 wherein R¹⁶, g, L², Ar², Alk, R², R^{x}, R^{y} and R^{z} are as defined for formula (2b);
and the salts, solvates, hydrates and N-oxides thereof.

Each R¹⁶ atom or group in compounds of formula (2c) may be independently selected from an atom or group -L³(Alk²)ₙL⁴(R⁴)ᵤ as previously particularly defined for compounds of formula (2b).

A further particularly useful group of compounds according to the invention has the formula (2d): 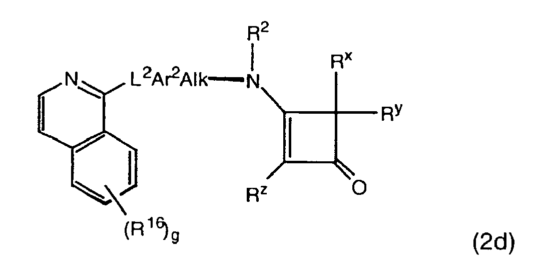 wherein R¹⁶, g, L², Ar², Alk, R², R^{x}, R^{y} and R^{z} are as defined for formula (2b):
and the salts, solvates, hydrates and N-oxides thereof.

Each R¹⁶ atom or group in compounds of formula (2d) may be independently selected from an atom or group -L³(Alk²)ₜL⁴(R⁴)ᵤ as previously defined for compounds of formula (2b).

In one preferred class of compounds of formula (2d) at least one R¹⁶ atom or group is present at the 3-position of the isoquinoline ring. In a preferred group of compounds of this class R¹⁶ is an optionally substituted phenyl ring. Optional substituents which may be present on the phenyl ring include halogen atoms, especially fluorine or chlorine atoms, or C₁₋₆alkyl, especially methyl, ethyl or isopropyl, haloC₁₋₆alkyl especially halomethyl, most especially -CF₃, -CHF₂ or -CH₂F, C₁₋₆alkoxy especially methoxy or etoxy or haloC₁₋₆alkoxy especially halomethoxy, most especially -OCF₃,-OCHF₂ or -OCH₂F groups.

It will be understood that compounds according to formulae (2a), (2b), (2c) and (2d) include, where applicable, the corresponding hydroxy tautomers.

Alk in compounds of the invention is preferably: 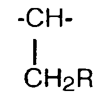 or, especially, -CH₂CH(R)-.

In one preferred class of compounds of formulae (1), (2a), (2b), (2c) and (2d) R is a -CO₂H group.

In another prefered class of compounds of formulae formulae (1), (2a), (2b), (2c) and (2d) R is an esterified carboxyl group of formula -CO₂Alk⁷ which may advantageously be used as a prodrug of the active compound. In this class of compound Alk⁷ is preferably a C₁₋₈alkyl group, especially a methyl, ethyl, propyl, i-propyl, butyl, t-butyl, pentyl or neopenyl group; an optionally substituted C₃₋₈cycloalkyl group, especially a cyclopropyl, cyclobutyl, cyclopentyl, cyclohexyl or cycloheptyl group; an optionally substituted C₃₋₈heterocycloalkyl group especially a tetrahydrofuanyl e.g. tetrahydrofuran-3-yl, pyrrolidinyl e.g. 1-methylpyrrolidinyl such as 1-methylpyrrolidin-3-yl, piperidinyl e.g. 1-methylpiperidinyl such as 1-methylpiperidin-4-yl, tetrahydropyranyl e.g. tetrahydropyran-4-yl or 2-oxo-[1,3]dioxol-4-yl e.g. 5-methyl-2-oxo-[1,3]dioxol-4-yl group; an optionally substituted C₆₋₁₀aryl group, especially a phenyl group; an optionally substituted C₆₋₁₀arylC₁₋₆alkyl group, especially a benzyl group; an optionally substituted heteroC₆₋₁₀arylC₁₋₆alkyl group, especially a pyridinylC₁₋₃alkyl group such as pyridinylmethyl e.g. pyridin-4-ylmethyl or pyridinylethyl e.g. pyridine-4-ylethyl or a imidazolylC₁₋₃alkyl group such as imidazolylethyl e.g. 2-imidazol-1-ylethyl or imidazolylpropyl e.g. 2-imidazol-1-ylpropyl group; an optionally substituted hydroxyC₁₋₆alkyl group, especially a hydroxyethyl e.g. 2-hydroxyethyl or hydroxypropyl e.g. 3-hydroxypropyl or 2,3-dihydroxypropyl group; an optionally substituted C₃₋₈heterocycloalkylC₁₋₆alkyl group, especially a morpholinyl-N-ethyl group; an optionally substituted N-di-C₁₋₈alkylaminoC₁₋₈alkyl group, especially a N-dimethylaminoethyl or N-diethylaminoethyl group; or an optionally substituted C₁₋₆alkyloxyC₁₋₆alkyl group, especially a methyloxyethyl group. Especially preferred esterified carboxyl groups include -CO₂CH₃,-CO₂CH₂CH₃, -CO₂CH₂CH₂CH₃, -CO₂CH(CH₃)₂ and -CO₂C(CH₃)₃ groups. A most especially preferred esterified carboxyl group is-CO₂(hydroxyC₁₋₆alkyl), especially -CO₂CH₂CH₂OH.

In general in compounds of formula (1) when X is a -N(R²) group and in particular in compounds of formulae (2a), (2b), (2c) and (2d) R² is preferably a hydrogen atom.

In one preferred class of compounds of formula (2a) L² is preferably L^{2a} where L^{2a} is a -CON(R⁸)- group [where R⁸ is preferably a hydrogen atom or a C₁₋₃alkyl group], especially a -CONH- group or a -(Alk³)L^{2a}- group, especially a -(Alk³)O- group [where Alk³ is preferably a C₁₋₃alkyl group], most especially a -CH₂O- group. In this class of compounds -W= is preferably -N= or -N(O)=. Most preferably W is -N=.

In another preferred class of compounds of formula (2a) L² is preferably a covalent bond. In this class of compounds -W= is preferably -C(R¹⁸)=, where R¹⁸ is as hereinbefore generally and particularly defined.

In general in compounds of formulae (2b), (2c) and (2d) L² is preferably L^{2a} where L^{2a} is an -O- atom or -N(R⁸)- group [where R⁸ is preferably a hydrogen atom or a C₁₋₃alkyl group]. An especially useful -N(R⁸)- group is -NH-.

The group Ar² in compounds of formulae (1), (2a), (2b), (2c) and (2d) is preferably an optionally substituted phenylene or optionally substituted pyridinediyl group or formula: 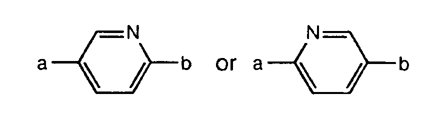 where a and b signify the points of attachment of L² and Alk respectively. Most preferably Ar² is an optionally substituted 1,4-phenylene group.

Particularly preferred optional substituents which may be present on Ar² in compounds of the invention include halogen atoms, especialy fluorine, chlorine or bromine atoms, or C₁₋₆alkyl e.g. methyl, ethyl or i-propyl, haloC₁₋₆alkyl especially halomethyl, most especialy -CF₃, C₁₋₆alkoxy especially methoxy or haloC₁₋₆alkoxy, especially halomethoxy, most especially -OCF₃, -CN, -CO₂CH₃, -NO₂, amino (-NH₂), substituted amino (NR⁵R⁶) especially -NHCH₃ and -N(CH₃)₂ and -N(R⁵)COCH₃, especially-NHCOCH₃ groups.

In one generally preferred class of compounds of formulae (1), (2a), (2b), (2c) and (2d) R^{x}, R^{y} and/or R^{z} is an optionally substituted alkyl group, most preferably an optionally substituted C₁₋₈alkyl group such as a methyl, ethyl, n-propyl, i-propyl, n-butyl, n-heptyl, or n-hexyl group. Particularly preferred optional substituents which may be present on such R^{x}, R^{y} and/or R^{z} alkyl groups include halogen atoms, especially fluorine or chlorine atoms, C₁₋₆alkoxy groups, especially methoxy, haloC₁₋₆alkoxy groups, especially -OCF₃, -CN, -CO₂CH₃, -NO₂, substituted amino (-NR⁵R⁶) especially -NHCH₃ and -N(CH₃)₂ and optionally substituted phenyl groups where the optional substituents are as herein defined for optional substituents on Ar².

In another preferred class of compounds of formulae (1), (2a), (2b), (2c) and (2d) R^{z} is a hydrogen atom.

In another preferred class of compounds of formulae (1), (2a), (2b), (2c) and (2d) R^{x} is a hydrogen atom.

In another preferred class of compounds of formulae (1), (2a), (2b), (2c) and (2d) R^{z} is a halogen atom, especially a fluorine, chlorine, bromine or iodine atom, most especially a chlorine or bromine atom.

In another preferred class of compounds of formulae (1), (2a), (2b), (2c) and (2d) R^{z} is a group -L¹(Alk¹)ₙR³. In this class of compounds L¹ is preferably a covalent bond or an -O-, -S- or -Se- atom or -S(O)- or -N(R⁸)-, especially -NH- or -N(CH₃)- group. Most preferably L¹ is a -S- atom or-S(O)- group. In this class of compounds R³ is preferably a hydrogen atom or an optionally substituted C₃₋₁₀cycloaliphatic, especially C₃₋₇cycloalkyl group, most especially an optionally substituted cyclopentyl, cyclohexyl or cycloheptyl group; or an optionally substituted C₃₋₁₀heterocycloaliphatic, especially C₃₋₇heterocycloalkyl group, most especially an optionally substituted piperidinyl, piperazinyl, pyrrolidinyl, imidazolidinyl, dithianyl or pyrazolidinyl group, or an optionally substituted C₆₋₁₂aromatic group, preferably an optionally substituted phenyl group or an optionally substituted C₁₋₉heteroaromatic group, preferably an optionally substituted monocyclic C₁₋₉heteroaromatic group, most preferably a 5- or 6-membered monocyclic heteroaromatic group containing one, two , three or four heteroatoms selected from oxygen, sulphur or nitrogen atoms, especially an optionally substituted furyl, thienyl, imidazolyl e.g. 1-methylimidazol-2-yl, triazolyl, tetrazolyl, pyridyl, pyrimidinyl or pyrazinyl group. Optional substituents which may be present on such heterocycloaliphatic groups include those substituents as described hereinafter when R^{x} and R^{y} are joined to form an optionally substituted spiro linked heterocycloaliphatic group. Optional substituents which may be present on such aromatic and heteroaromatic groups include those substituents as described hereinbefore in relation to R¹⁶ substituents in compounds of formula (2a). In one preferred group of compounds of this class n is zero. In another preferred group of compounds of this class L¹ is a covalent bond and n is zero. In this group of compounds R³ is preferrably an optionally substituted C₃₋₁₀cycloaliphatic, C₃₋₁₀heterocycloaliphatic, C₆₋₁₂aromatic or monocyclic C₁₋₉heteroaromatic group as just described. In a further preferred group of compounds of this class n is the integer 1 and Alk¹ is preferably an optionally substituted aliphatic chain, most preferably an optionally substituted C₁₋₆alkylene chain, especially a -CH₂-, -CH₂CH₂- or -CH₂CH(CH₃)- chain. In a further preferred group of compounds of this class L¹ is a covalent bond, n is the integer 1 and Alk¹ is preferably an optionally substituted aliphatic chain, most preferably an optionally substituted C₁₋₆alkylene chain, especially a-CH₂-, -CH₂CH₂-, -CH₂CH₂CH₂- or -CH₂CH(CH₃)- chain. In a further preferred group of compounds of this class L¹ is a preferred atom or group as just described, most especially a -S- atom, n is the integer 1 and Alk¹ is preferably an optionally substituted aliphatic chain, most preferably an optionally substituted C₁₋₆alkylene chain, especially a -CH₂-, -CH₂CH₂-,-CH₂CH₂CH₂- or -CH₂CH(CH₃)- chain. In this class of compounds R³ is preferably a hydrogen atom.

Most especially useful R^{z} groups which may be present in compounds of the invention include a hydrogen or halogen atom, especially fluorine, chlorine, bomine or iodine atom or a group of formula -L¹(Alk¹)ₙR³ as just defined, especially an alkyl group as previously described or a hydroxyl (-OH); C₁₋₆alkoxymethoxy, ethoxy or i-propoxy; C₃₋₇cycloalkyl, especially cyclopentyl or cyclohexyl; C₁₋₆alkylsulfanyl, especially methyl- ethyl- or i-propylsulfanyl; C₁₋₆alkylsulfinyl, especially methyl- ethyl- or i-propylsulfinyl; C₃₋₇heterocycloalkyl, especially piperidinyl most especially piperidin-3-yl such as 1-methylpiperidin-3-yl or dithianyl especially [1,3]dithian-2-yl; C₆₋₁₂arylselenenyl, especially phenylselenenyl; C₆₋₁₂arylsulfanyl, especially phenylsulfanyl or pentafluorophenylsulfanyl; monocyclic C₁₋₉heteroaromaticsulfanyl, especially tetrazol-5-ylsulfanyl most especially 1-methyl-1H-terazol-5-ylsulfanyl or imidazolylsulfanyl especially imidazol-2-ylsulfanyl most especially 1-methyl-1H-imidazol-2-ylsulfanyl; monocyclic C₁₋₉heteroaromatic, especially pyridinyl most especially pyridin-3-yl, 1-methylpyridinium or pyrazinyl especially pyrazin-2-yl; or a C₆₋₁₂arylC₁₋₃alkyl, especially benzyl group.

In another preferred class of compounds of formulae (1), (2a), (2b), (2c) and (2d) R^{x} and R^{z} is each a hydrogen atom.

In another preferred class of compounds of formulae (1), (2a), (2b), (2c) and (2d) R^{x} is a hydrogen atom and R^{z} is a halogen atom, especially a fluorine, chlorine, bromine or iodine atom, most especially a chlorine or bromine atom, or R^{z} is a group -L¹(Alk¹)ₙR³ as just described.

In another preferred class of compounds of formulae (1), (2a), (2b), (2c) and (2d) R^{x} and R^{y} is each a hydrogen atom and R^{z} is a halogen atom, especially a fluorine, chlorine, bromine or iodine atom, most especially a chlorine or bromine atom, or R^{z} is a group -L¹(Alk¹)ₙR³ as just described.

In another preferred class of compounds of formulae (1), (2a), (2b), (2c) and (2d) R^{x} is a hydrogen atom and RY is an optionally substituted alkyl group as just described for generally preferred alkyl groups.

In another preferred class of compounds of formulae (1), (2a), (2b), (2c) and (2d) R^{x} and R^{z} is each a hydrogen atom and R^{y} is an optionally substituted alkyl group as just described.

In another preferred class of compounds of formulae (1), (2a), (2b), (2c) and (2d) R^{x} is a hydrogen atom, R^{z} is a halogen atom, especially a fluorine, chlorine, bromine or iodine atom, most especially a chlorine or bromine atom or R^{z} is a group -L¹(Alk¹)ₙR³, especially a group as just particularly described, and R^{y} is an optionally substituted alkyl group as just described for generally preferred alkyl groups.

In another preferred class of compounds of formulae (1), (2a), (2b), (2c) and (2d) R^{x} is a hydrogen atom and R^{y} and R^{z} is each an optionally substituted alkyl group as just described for generally preferred alkyl groups.

In another preferred class of compounds of formulae (1), (2a), (2b), (2c) and (2d) R^{x} and R^{y} is each an optionally substituted alkyl group as just described for generally preferred alkyl groups.

In another preferred class of compounds of formulae (1), (2a), (2b), (2c) and (2d) R^{x} and R^{y} is each an optionally substituted alkyl group as just described for generally preferred alkyl groups and R^{z} is a hydrogen atom.

In another preferred class of compounds of formulae (1), (2a), (2b), (2c) and (2d) R^{x} and R^{y} is each an optionally substituted alkyl group as just described for generally preferred alkyl groups and R^{z} is a halogen atom, especially a fluorine, chlorine, bromine or iodine atom, most especially a chlorine or bromine atom, or R^{z} is a group -L¹(Alk¹)ₙR³ as just described.

In another preferred class of compounds of formulae (1), (2a), (2b), (2c) and (2d) R^{x}, R^{y} and R^{z} is each an optionally substituted alkyl group as just described for generally preferred alkyl groups.

In another preferred class of compounds of formulae (1), (2a), (2b), (2c) and (2d) R^{x} and R^{y} are joined to form an optionally substituted spiro linked cycloaliphatic group particularly a C₃₋₁₀cycloaliphatic group, most particularly a C₃₋₈cycloalkyl group, especially an optionally substituted cyclopentyl cyclohexyl, cycloheptyl or cyclooctyl group, or a C₃₋₈cycloalkenyl group, especially an optionally substituted cyclopentenyl, cyclohexenyl, cycloheptenyl or cyclooctenyl group group. Particularly preferred optional substituents which may be present on such spiro linked cycloaliphatic groups include halogen atoms, especially fluorine or chlorine atoms, C₁₋₆alkyl groups, especially methyl, ethyl, propyl or i-propyl, C₁₋₆alkoxy groups, especially methoxy or ethoxy, haloC₁₋₆alkoxy groups, especially -OCF₃, -CN, -CO₂CH₃, -NO₂ and substituted amino (-N(R¹¹)₂), especially -NHCH₃ and -N(CH₃)₂ groups. In a preferred group of compounds of this class R^{z} is a hydrogen atom. In another preferred group of compounds of this class R^{z} is an alkyl group as just described. In a further preferred group of compounds of this class R^{z} is a halogen atom, especially a fluorine, chlorine, bromine or iodine atom, most especially a chlorine or bromine atom, particularly a bromine atom. In a still further preferred group of compounds of this class R^{z} is a group -L¹(Alk¹)ₙR³ as just described.

In another preferred class of compounds of formulae (1), (2a), (2b), (2c) and (2d) R^{x} and R^{y} are joined to form an optionally substituted spiro linked heterocycloaliphatic group, particularly an optionally substituted C₃₋₁₀heterocycloaliphatic group, most particularly an optionally substituted C₃₋₇heterocycloalkyl group, especially an optionally substituted C₃₋₇heterocycloalkyl group containing one or two -O-, -S-, -S(O)-, -S(O)₂-,-NH- or -C(O)- heteroatoms or heteroatom-containing groups. Especially preferred optionally substituted heterocycloaliphatic groups include optionally substituted 5- and 6-membered heterocycloalkyl groups containing one heteroatom or heteroatom-containing group as just described, especially optionally substituted pyrrolidinyl, tetrahydrofuranyl, tetrahydrothiophenyl, tetrahydrothiophene-1-oxide, tetrahydrothiophene-1,1-dioxide, piperidinyl, tetrahydropyranyl, tetrahydrothiopyranyl tetrahydrothiopyran-1-oxide or tetrahydrothiopyran-1,1-dioxide groups. Particularly preferred optional substituents which may be present on such spiro linked heterocycloaliphatic groups include halogen atoms, especially fluorine or chlorine atoms, C₁₋₆alkyl groups, especially methyl, ethyl, propyl or i-propyl, C₁₋₆alkoxy groups, especially methoxy or ethoxy, haloC₁₋₆alkoxy groups, especially -OCF₃, -CN, -CO₂CH₃, -NO₂ and substituted amino (-N(R¹¹)₂), especially -NHCH₃ and -N(CH₃)₂ groups. In addition when the spiro linked heterocycloaliphatic group contains a nitrogen atom this may be substituted by a group -(L⁶)ₚ(Alk⁵)_{q}R¹² where L⁶ is preferably -C(O)- or -S(O)₂-, Alk⁵ is preferably an optionally substituted C₁₋₆alkylene chain, especially a -CH₂-, -(CH₂)₂- or-CH(CH₃)CH₂- chain or an optionally substituted heteroC₁₋₆alkylene chain, especially -CH₂L⁵-, -CH₂CH₂L⁵-, -L⁵CH₂- or -L⁵CH₂CH₂ chain where L⁵ is an -O- or -S- atom or -NH or -N(CH₃)- group and R¹² is a hydrogen atom or an optionally substituted phenyl ring where preferred optional substituents include those atoms and groups as defined hereinbefore for R¹⁶ in relation to formula (2b). In one preferred group of compounds of this class R^{z} is a hydrogen atom. In another preferred group of compounds of this class R^{z} is an alkyl group as just described. In a further preferred group of compounds of this class R^{z} is a halogen atom, especially a fluorine, chlorine, bromine or iodine atom, most especially a chlorine or bromine atom. In a still further preferred group of compounds of this class R^{z} is a group -L¹(Alk¹)ₙR³ as just described.

Particularly useful compounds of the invention include:
(2*S*)-2-[(3-Oxospiro[3.5]non-1-en-1-yl)amino]-3-{4-[(3,5-dichloroisonicotinoyl)amino]phenyl}propanoic acid
(2*S*)-2-[(3-Oxospiro[3.5]non-1-en-1-yl)amino]-3-{4-[(3-methyl[2,7]naphthyridin- 1 -yl)oxy]phenyllpropanoic acid
(2*S*)-2-[(2-Bromo-3-oxospiro[3.5]non-1-en-1-yl)amino]-3-{4-[(3,5-dichloroisonicotinoyl)amino]phenyl}propanoic acid
(2*S*)-2-[(2-Bromo-4,4-dimethyl-3-oxo-1-cyclobutenyl)amino]-3-{4-[(3,5-dichloroisonicotinoyl)amino]phenyl}propanoic acid
(2*S*)-2-[(2-Bromo-3-oxospiro[3.5]non-1-en-1-yl)amino]-3-{4-[(2,7)naphthyridin-1-yloxy]phenyl}propanoic acid
(2*S*)-2-[(2-Bromo-3-oxo-7-oxaspiro[3.5]non-1-en-1-yl)amino]-3-{4-[(3,5-dichloroisonicotinoyl)amino]phenyl}propanoic acid
(2*S*)-2-{(2-Bromo-3-oxospiro[3,5]non-1-en-1-yl)amino}-3-(2,6-dimethoxy[1,1'-biphenyl]-4-yl)propanoic acid
(2*S*)-2-[(3-Oxospiro[3,6]dec-1-en-1-yl)amino]3-{4-[(3,5-dichloroisonicotinoyl)amino]phenyl}propanoic acid
(2*S*)-2-[(2-Bromo-3-oxospiro[3.6]dec-1-en-1-yl)amino]-3-{4-[(3,5-dichloroisonicotinoyl)amino]phenyl}propanoic acid
(2*S*)-2-{[4,4-Dimethyl-2-(phenylselenenyl)-3-oxo-1-cyclobutenyl] amino}-3-{4-[(3,5-dichloroisonicotinoyl)amino]phenyl}propanoic acid
(2*S*)-2-[(2-Bromo-7-methoxy-3-oxospiro[3.5]non-1-en-1-yl)amino]-3-{4-[(3,5-dichloroisonicotinoyl)amino]phenyl}propanoic acid
(2*S*)-2-{(2-Bromo-3-oxospiro[3.5]non-1-en-1-yl)amino}-3-{4-[(3-methyl[2.7]naphthyridin-1-yl)oxy]phenyl}propanoic acid
(2*S*)-2-{[2-(Phenylsulfanyl)-4,4-dimethyl-3-oxo-1-cyclobutenyl]-amino}-3-{4-[(3,5-dichloroisonicotinoyl)amino]phenyl}propanoic acid
(2*S*)-2-[(2-Chloro-3-oxospiro[3.5]non-1-en-1-yl)amino]-3-{4-[(3,5-dichloroisonicotinoyl)amino]phenyl}propanoic acid
(2*S*)-3-{4-[(3,5-Dichloroisonicotinoyl)amino]phenyl}-2-(3-oxo-2-pyridin-3-yl-spiro[3.5]non-1-en-1-ylamino)-propanoic acid
(2*S*)-2-[(2-Iodo-3-oxospiro[3.5]non-1-en-1-yl)amino]-3-{4-[(3,5-dichloroisonicotinoyl)amino]phenyl}propanoic acid
(2*S*)-2-[(2-Bromo-3-oxospiro[3.5]non-1-en-1-yl)amino]-3-{5-[(3,5-dichloroisonicotinoyl)amino]pyridin-2-yl}propanoic acid
(2*S*)-2-[(2-Chloro-3-oxospiro[3.5]non-1-en-1-yl)amino]-3-{5-[(3,5-dichloroisonicotinoyl)amino]pyridin-2-yl}propanoic acid
(2*S*)-2-[(2-Chloro-3-oxo-7-oxa-spiro[3.5]non-1-en-1-yl)amino]-3-{4-[(3,5-dichloroisonicotinoyl)amino]phenyl}propanoic acid
(2*S*)-2-[(2-Chloro-3-oxo-spiro[3.6]dec-1-en-1-yl)amino]-3-{4-[(3,5-dichloroisonicotinoyl)amino]phenyl}propanoic acid
(2*S*)-2-[4,4-Dimethyl-2-(1-methyl-1H-tetrazol-5-ylsulfanyl)-3-oxo-cyclobut-1-enylamino]-3-{4-[(3,5-dichloroisonicotinoyl)amino]phenyl}propanoic acid
(2*S*)-2-[(2-Bromo-3,7,7-trioxo-7λ⁶-thia-spiro[3,5]non-1-en-1-yl)amino]-3-{4-[(3,5-dichloroisonicotinoyl)amino]phenyl}propanoic acid
(2*S*)-2-[(2-Chloro-3-oxo-spiro[3,4]oct-1-en-1-yl)amino]-3-{4-[(3,5-dichloroisonicotinoyl)amino]phenyl}propanoic acid
(2*S*)-2-[(2-Bromo-3-oxo-spiro[3,4]oct-1-en-1-yl)amino]-3-{4-[(3,5-dichloroisonicotinoyl)amino]phenyl}propanoic acid
(2*S*)-2-[(2-Methylsulfanyl-3-oxo-spiro[3.5]non-1-en-1-yl)amino]-3-{4-[(3,5-dichloroisonicotinoyl)amino]phenyl}propanoic acid
(2*S*)-2-(2-Fluoro-3-oxo-spiro[3.5]non-1-en-1-ylamino)3-{4-[(3,5-dichloroisonicotinoyl)amino]phenyl}propanoic acid
(*2S*)-2-[(2-Fluoro-3-oxo-7-oxa-spiro[3.5]non-1-en-1-yl)amino]-3-{4-[(3,5-dichloroisonicotinoyl)amino]phenyl}propanoic acid
(*2S*)-2-[(4,4-Dimethyl-2-methylsulfanyl-3-oxo-cyclobut-1-enyl)amino]-3-{4-[(3,5-dicloroisonicotinoyl)amino]phenyl}propanoic acid
*(2S)*-2-[(2-Isopropylsulfanyl-4,4-dimethyl-3-oxo-cyclobut-1-enyl)amino]-3-{4-[(3,5-dichloroisonicotinoyl)amino]phenyl}propanoic acid
(2*S)*-2-[(2-Isopropylsulfanyl-3-oxo-spiro[3.5]non-1-en-1-yl)amino]-3-{4-[(3,5-dichloroisonicotinoyl)amino]phenyl}propanoic acid
(2*S*)-2-[(2-Isopropylsulfanyl-4,4-dimethyl-3-oxo-cyclobut-1-enyl)amino]-3-[4-([2,7]naphthyridin-1-ylamino)phenyl]propanoic acid
(2*S*)-2-[(2-Isopropylsulfanyl-3-oxo-spiro[3.5]non-1-en-1-yl)amino]-3-[4-([2,7]naphthyridin-1-ylamino)phenyl]propanoic acid
(2*S*)-2-[(2-lsopropylsulfanyl-3-oxo-7-oxa-spiro[3.5]non-1-en-1-yl)amino]-3-[4-([2,7]naphthyridin-1-ylamino)phenyl]propanoic acid
(2*S*)-2-[(2-Bromo-3-oxo-spiro[3.4]octa-1,6-dien-1-yl)amino]-3-{4-[(3,5-dichloroisonicotinoyl)amino]phenyl}propanoic acid
(2*S*)-2-[(4,4-Dimethyl-3-oxo-2-pentafluorophenylsulfanyl-cyclobut-1-enyl)amino]-3-{4-[(3,5-dichloroisonicotinoyl)amino]phenyl}propanoic acid
(*2S*)-2-[(4,4-Dimethyl-3-oxo-2-pyrazin-2-yl-cyclobut-1-enyl)amino]-3-{4[(3,5-dichloroisonicotinoyl)amino]phenyl}propanoic acid
(2*S*)-*2*-[(7-Acetyl-2-bromo-3-oxo-7-aza-spiro[3.5]non-1-en-1-yl)amino]-3-{4-[(3,5-dichloroisonicotinoyl)amino]phenyl}propanoic acid
(2*S*)-2-{[2-(Isopropylsulfanyl)-3-oxo-7-oxaspiro[3.5]non-1-en-yl)]amino}-3-(2,6-dimethoxy[1,1'-biphenyl]-4-yl)propanoic acid
(*2S*)-2-[(2-Cyclohexy)-3-oxo-spiro[3.5]non-1-en-1-yl)amino]-3-{4-[(3,5-dichloroisonicotinoyl)amino]phenyl}propanoic acid
(2*S*)-3-{4-[(3,5-Dichloroisonicotinoyl)amino]phenyl}-2-(2-methyl-3-oxo-spiro[3.5]non-1-en-1-ylamino)propanoic acid
(2*S*)-3-{4-[(3,5-Dichloroisonicotinoyl)amino]phenyl}-2-(3-oxo-2-propylspiro[3.5]non-1-en-1-ylamino)propanoic acid
(2*S*)-3-{4-[(3,5-Dichloroisonicotinoyl)aminolphenyl}-2-(2-methyl-3-oxo-7-oxa-spiro[3.5]non-1-en-1-ylamino)propanoic acid
(2*S*)-3-{4-[(3,5-Dichloroisonicotinoyl)amino]phenyl}-2-(3-oxo-2-propyl-7-oxa-spiro[3.5]non-1-en-1-ylamino)propanoic acid
(2*S*)-3-{4-[(3,5-Dichloroisonicotinoyl)amino]phenyl}-2-(2-[1,3]dithian-2-yl-3-oxo-spiro[3.5]non-1-en-1-ylamino)propanoic acid
(2*S*)-3-{4-[(3,5-Dichloroisonicotinoyl)amino]phenyl)-2-(2-ethyl-3-oxo-spiro[3.5]non-1-en-1-ylamino)propanoic acid
(2*S*)-3-{4-[(3,5-Dichloro-1-oxy-pyridine-4-carbonyl)amino]phenyl}-2-(3-oxo-spiro[3.5]non-1-en-1-ylamino)propanoic acid
(2*S*)-2-(2-Bromo-3-oxo-spiro[3.5]non-1-en-1-ylamino)-3-{4-[(3,5-dichloro-1-oxy-pyridine-4-carbonyl)amino]phenyl}propanoic acid
(2*S*)-2-(2-Chloro-3-oxo-spiro[3.5]non-1-en-1-ylamino)-3-{4-[(3,5-dichloro-1-oxy-pyridine-4-carbonyl)amino]phenyl}propanoic acid
(*2S*)-3-{4-[(3,5-Dichloro-1-oxy-pyridine-4-carbonyl)-amino]-phenyl}-2-(2-methanesulfinyl-4,4-dimethyl-3-oxo-cyclobut-1-enylamino)-propanoic acid
(*2S*)-2-(2-Bromo-3-oxo-spiro[3.5]non-1-en-1-ylamino)-3-[4-(3-methyl-[2,7]naphthyridin-1-ylamino)phenyl]propanoic acid
(*2S*)-2-(2-Bromo-3-oxo-spiro[3.5]non-1-en-1-ylamino)-3-[4-([2,7]naphthyridin-1-ylamino)phenyl]propanoic acid
(2*S*)-2-(2-Bromo-4,4-dimethyl-3-oxo-cyclobut-1-enylamino)-3-[4-(3-methyl-[2,7]naphthyridin-1-yloxy)phenyl]propanoic acid
(2*S*)-2-[(2-Bromo-3-oxo-spiro[3,4]oct-1-en-1-yl)amino]-3-{4-[(3,5-dichloroisonicotinoyl)amino]phenyl}propanoic acid
and the salts, solvates, hydrates, N-oxides and carboxylic acid esters thereof.

Particularly useful carboxylic acid esters thereof include the methyl, ethyl, propy, i-propyl and t-butyl esters.

Most especially useful compounds of the invention include:
(2*S*)-2-[(2-Bromo-3-oxospiro[3.5]non-1-en-1-yl)amino]-3-[4-[(3,5-dichloroisonicotinoyl)amino]phenyl}propanoic acid
(2*S*)-2-[(2-Bromo-3-oxo-7-oxaspiro[3.5]non-1-en-1-yl)amino]-3-{4-[(3,5-dichloroisonicotinoyl)amino]phenyl}propanoic acid
(2*S*)-2-[(2-Bromo-3-oxospiro[3.6]dec-1-en-1-yl)amino]-3-{4-[(3,5-dichloroisonicotinoyl)amino]phenyl}propanoic acid
(2*S*)-2-{(2-Bromo-3-oxospiro[3.5]non-1-en-1-yl)amino}-3-{4-[(3-methyl[2,7]naphthyridin-1-yl)oxy]phenyl}propanoic acid
(2*S*)-2-{[2-(Phenylsulfanyl)-4,4-dimethyl-3-oxo-1-cyclobutenyl]-amino}-3-{4-[(3,5-dichloroisonicotinoyl)amino]phenyl}propanoic acid
(2*S*)-2-[(2-Chloro-3-oxospiro[3.5]non-1-en-1-yl)amino]-3-{4-[(3,5-dichloroisonicotinoyl)amino]phenyl}propanoic acid
(2S)-3-{4-[(3,5-Dichloroisonicotinoyl)amino]phenyl}-2-(3-oxo-2-pyridin-3-yl-spiro[3.5]non-1-en-1-ylamino)propanoic acid
(2*S*)-2-[(2-Iodo-3-oxospiro[3.5]non-1-en-1-yl)amino]-3-{4-[(3,5-dichloroisonicotinoyl)amino]phenyl}propanoic acid
(2*S*)-2-[(2-Bromo-3-oxospiro[3.5]non-1-en-1-yl)amino]-3-{5-[(3,5-dichloroisonicotinoyl)amino]pyridin-2-yl}propanoic acid
(2*S*)-2-[(2-Chloro-3-oxospiro[3.5]non-1-en-1-yl)amino]-3-{5-[(3,5-dichloroisonicotinoyl)amino]pyridin-2-yl}propanoic acid
(2*S*)-2-[(2-Chloro-3-oxo-7-oxa-spiro[3.5]non-1-en-1-yl)amino]-3-{4-[(3,5-dichloroisonicotinoyl)amino]phenyl}propanoic acid
(2*S*)-2-[(2-Chloro-3-oxo-spiro[3.6]dec-1-en-1-yl)amino]-3-{4-[(3,5-dichloroisonicotinoyl)amino]phenyl}propanoic acid
(2*S*)-2-[(2-Bromo-3,7,7-trioxo-7λ⁶-thia-spiro[3.5]non-1-en-1-yl)amino]-3-{4-[(3,5-dichloroisonicotinoyl)amino]phenyl}propanoic acid
(2*S*)-2-[(2-Chloro-3-oxo-spiro[3.4]oct-1-en-1-yl)amino]-3-{4-[(3,5-dichloroisonicotinoyl)amino]phenyl}propanoic acid
(2*S*)-2-[(2-Bromo-3-oxo-spiro[3.4]oct-1-en-1-yl)amino]-3-{4-[(3,5-dichloroisonicotinoyl)amino]phenyl}propanoic acid
(*2S*)-2-[(2-Methylsulfanyl-3-oxo-spiro[3.5]non-1-en-1-yl)amino]-3-{4-[(3,5-dichloroisonicotinoyl)amino]phenyl}propanoic acid
(2*S*)-2-(2-Fluoro-3-oxo-spiro[3.5]non-1-en-1-ylamino)3-{4-[(3,5-dichloroisonicotinoyl)amino]phenyl}propanoic acid
(2*S*)-2-[(2-Fluoro-3-oxo-7-oxa-spiro[3.5]non-1-en-1-yl)amino]-3-{4-[(3,5-dichloroisonicotinoyl)amino]phenyl}propanoic acid
(2*S*)-2-[(4,4-Dimethyl-2-methylsulfanyl-3-oxo-cyclobut-1-enyl)amino]-3-{4-[(3,5-dichloroisonicotinoyl)amino]phenyl}propanoic acid
(2*S*)-2-[(2-Isopropylsulfanyl-4,4-dimethyl-3-oxo-cyclobut-1-enyl)amino]-3-{4-[(3,5-dichloroisonicotinoyl)amino]phenyl}propanoic acid
(2*S*)-2-[(2-Isopropylsulfanyl-3-oxo-spiro[3.5]non-1-en-1-yl)amino]-3-{4-[(3,5-dichloroisonicotinoyl)amino]phenyl}propanoic acid
(2*S*)-2-[(2-Isopropylsulfanyl-4,4-dimethyl-3-oxo-cyclobut-1-enyl)amino]-3-[4-([2,7]naphthyridin-1-ylamino)phenyl]propanoic acid
(2S)-2-[(2-Isopropylsulfanyl-3-oxo-spiro[3.5]non-1-en-1-yl)amino]-3-[4-([2,7]naphthyridin-1-ylamino)phenyl]propanoic acid
(2*S*)-2-[(2-Isopropylsulfanyl-3-oxo-7-oxa-spiro[3.5]non-1-en-1-yl)amino]-3-[4-([2,7]naphthyridin-1-ylamino)phenyl]propanoic acid
(2*S*)-2-[(2-Bromo-3-oxo-spiro[3.4]octa-1,6-dien-1-yl)amino]-3-{4-[(3,5-dichloroisonicotinoyl)amino]phenyl}propanoic acid
(*2S*)-2-[(7-Acetyl-2-bromo-3-oxo-7-aza-spiro[3.5]non-1-en-1-yl)amino]-3-{4-[(3,5-dichloroisonicotinoyl)amino]phenyl}propanoic acid
(2*S*)-2-[(2-Cyclohexyl-3-oxo-spiro[3.5]non-1-en-1-yl)amino]-3-[4-[(3,5-dichloroisonicotinoyl)amino]phenyl}propanoic acid
(2*S*)3-{4-[(3,5-Dichloroisonicotinoyl)amino]phenyl}-2-(2-methyl-3-oxo-spiro[3.5]non-1-en-1-ylamino)propanoic acid
(2*S*)-3-{4-[(3,5-Dichloroisonicotinoyl)amino]phenyl}-2-(3-oxo-2-propy)-spiro[3.5]non-1-en-1-ylamino)propanoic acid
(2*S*)-3-{4-[(3,5-Dichloroisonicotinoyl)amino]phenyl}-2-(2-[1,3]dithian-2-yl-3-oxo-spiro[3.5]non-1-en-1-ylamino)propanoic acid
(2*S*)-2-(2-Bromo-3-oxo-spiro[3.5]non-1-en-1-ylamino)-3-{4-[(3,5-dichloro-1-oxy-pyridine-4-carbonyl)amino]phenyl}propanoic acid
2-(2-Chloro-3-oxo-spiro[3.5]non-1-en-1-ylamino)-3-{4-[(3,5-dichloro-1-oxypyridine-4-carbonyl)amino]phenyl}propanoic acid
and the salts, solvates, hydrates, N-oxides and carboxylic acid esters thereof.

Particularly useful carboxylic acid esters thereof include the methyl, ethyl, propy, i-propyl and t-butyl esters.

Particularly useful ester prodrugs of compounds of the invention include:
Ethyl (2S)-2-(2-bromo-3-oxo-spiro[3.5]non-1-en-1-ylamino)-3-{4-[(3,5-dichloroisonicotinoyl)amino]phenyl}propanoate
Isopropyl (2S)-2-(2-bromo-3-oxo-spiro[3.5]non-1-en-1-yiamino)-3-{4-[(3,5-dichloroisonicotinoyl)amino]phenyl}propanoate
t-Butyl (2S)-2-(2-bromo-3-oxo-spiro[3.5]non-1-en-1-ylamino)-3-{4-[(3,5-dichloroisonicotinoyl)amino]phenyl}propanoate
1-Methyl-piperidin-4-yl (2*S*)-2-(2-bromo-3-oxo-spiro[3.5]non-1-en-1-ylamino)-3-{4-[(3,5-dichloroisonicotinoyl)amino]phenyl}propanoate
Phenyl (2*S*)-2-(2-bromo-3-oxo-spiro[3.5]non-1-en-1-ylamino)-3-{4-[(3,5-dichloroisonicotinoyl)amino]phenyl}propanoate
Cyclopentyl (2*S*)-2-(2-bromo-3-oxo-spiro[3.5]non-1-en-1-ylamino)-3-{4-[(3,5-dichloroisonicotinoyl)amino]phenyl}propanoate
2-Imidazol-1-yl-ethyl (2*S*)-2-(2-bromo-3-oxo-spiro[3.5]non-1-en-1-ylamino)-3-{4-[(3,5-dichloroisonicotinoyl)amino]phenyl}propanoate
Neopentyl (*2S*)-2-(2-bromo-3-oxo-spiro[3.5]non-1-en-1-ylamino)-3-{4-[(3,5-dichloroisonicotinoyl)amino]phenyl}propanoate
Tetrahydro-furan-3-yl (*2S*)-2-(2-bromo-3-oxo-spiro[3.5]non-1-en-1-ylamino)-3-{4-[(3,5-dichloroisonicotinoyl)amino]phenyl}propanoate
Pyridin-4-ylmethyl (*2S*)-2-(2-bromo-3-oxo-spiro[3.5]non-1-en-1-ylamino)-3-{4-[(3,5-dichloroisonicotinoyl)amino]phenyl}propanoate
Tetrahydropyran-4-yl (*2S*)-2-(2-bromo-3-oxo-spiro[3.5]non-1-en-1-ylamino)-3-{4-[(3,5-dichloroisonicotinoyl)amino]phenyl}propanoate
5-Methyl-2-oxo-[1,3]dioxol-4-ylmethyl (*2S*)-2-(2-bromo-3-oxo-spiro[3.5]non-1-en-1-ylamino)-3-{4-[(3,5-dichloroisonicotinoyl)amino]-phenyl}propanoate
1-Methyl-pyrrolidin-3-yl (2S)-2-(2-bromo-3-oxo-spiro[3.5]non-1-en-1-ylamino)-3-{4-[(3,5-dichloroisonicotinoyl)amino]phenyl}propanoate
2,3-Dihydroxypropyl (2S)-2-(2-bromo-3-oxo-spiro[3.5]non-1-en-1-ylamino)-3-{4-[(3,5-dichloroisonicotinoyl)amino]phenyl}propanoate
Tetrahydrofuran-2-ylmethyl (2S)-2-(2-bromo-3-oxo-spiro[3.5]non-1-en-1- ylamino)-3-{4-[(3,5-dichloroisonicotinoyl)amino]phenyl}propanoate
and the salts, solvates, hydrates and N-oxides thereof.

Compounds according to the invention are potent and selective inhibitors of α4 integrins. The ability of the compounds to act in this way may be simply determined by employing tests such as those described in the Examples hereinafter.

The compounds are of use in modulating cell adhesion and in particular are of use in the prophylaxis and treatment of diseases or disorders including inflammation in which the extravasation of leukocytes plays a role and the invention extends to such a use and to the use of the compounds for the manufacture of a medicament for treating such diseases or disorders,

Diseases or disorders of this type include inflammatory arthritis such as rheumatoid arthritis vasculitis or polydermatomyositis, multiple sclerosis, allograft rejection, diabetes, inflammatory dermatoses such as psoriasis or dermatitis, asthma and inflammatory bowel disease.

For the prophylaxis or treatment of disease the compounds according to the invention may be administered as pharmaceutical compositions, and according to a further aspect of the invention we provide a pharmaceutical composition which comprises a compound of formula (1) together with one or more pharmaceutically acceptable carriers, excipients or diluents.

Pharmaceutical compositions according to the invention may take a form suitable for oral, buccal, parenteral, nasal, topical or rectal administration, or a form suitable for administration by inhalation or insufflation.

For oral administration, the pharmaceutical compositions may take the form of, for example, tablets, lozenges or capsules prepared by conventional means with pharmaceutically acceptable excipients such as binding agents (e.g. pregelatinised maize starch, polyvinylpyrrolidone or hydroxypropyl methylcellulose); fillers (e.g. lactose, microcrystalline cellulose or calcium hydrogen phosphate); lubricants (e.g. magnesium stearate, talc or silica); disintegrants (e.g. potato starch or sodium glycollate); or wetting agents (e.g. sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents, emulsifying agents, nonaqueous vehicles and preservatives. The preparations may also contain buffer salts, flavouring, colouring and sweetening agents as appropriate.

Preparations for oral administration may be suitably formulated to give controlled release of the active compound.

For buccal administration the compositions may take the form of tablets or lozenges formulated in conventional manner.

The compounds for formula (1) may be formulated for parenteral administration by injection e.g. by bolus injection or infusion. Formulations for injection may be presented in unit dosage form, e.g. in glass ampoule or multi dose containers, e.g. glass vials. The compositions for injection may take such forms as suspensions, solutions or emulsions in oily or aqueous vehicles, and may contain formulatory agents such as suspending, stabilising, preserving and/or dispersing agents. Alternatively, the active ingredient may be in powder form for constitution with a suitable vehicle, e.g. sterile pyrogen-free water, before use.

In addition to the formulations described above, the compounds of formula (1) may also be formulated as a depot preparation. Such long acting formulations may be administered by implantation or by intramuscular injection.

For nasal administration or administration by inhalation, the compounds for use according to the present invention are conveniently delivered in the form of an aerosol spray presentation for pressurised packs or a nebuliser, with the use of suitable propellant, e.g. dichlorodifluoromethane, trichloro-fluoromethane, dichlorotetrafluoroethane, carbon dioxide or other suitable gas or mixture of gases.

The compositions may, if desired, be presented in a pack or dispenser device which may contain one or more unit dosage forms containing the active ingredient. The pack or dispensing device may be accompanied by instructions for administration.

The quantity of a compound of the invention required for the prophylaxis or treatment of a particular condition will vary depending on the compound chosen, and the condition of the patient to be treated. In general, however, daily dosages may range from around 100ng/kg to 100mg/kg e.g. around 0.01 mg/kg to 40mg/kg body weight for oral or buccal administration, from around 10ng/kg to 50mg/kg body weight for parenteral administration and around 0.05mg to around 1000mg e.g. around 0.5mg to around 1000mg for nasal administration or administration by inhalation or insufflation.

The compounds of the invention may be prepared by a number of processes as generally described below and more specifically in the Examples hereinafter. In the following process description, the symbols Ar¹, Ar², Alk, R¹, R², R³, L¹, L², Alk¹, R^{x}, RY, R^{z} and n when used in the formulae depicted are to be understood to represent those groups described above in relation to formula (1) unless otherwise indicated. In the reactions described below, it may be necessary to protect reactive functional groups, for example hydroxy, amino, thio or carboxy groups, where these are desired in the final product, to avoid their unwanted participation in the reactions. Conventional protecting groups may be used in accordance with standard practice [see, for example, Green, T. W. in "Protective Groups in Organic Synthesis", John Wiley and Sons, 1999]. In some instances, deprotection may be the final step in the synthesis of a compound of formula (1) and the processes according to the invention described hereinafter are to be understood to extend to such removal of protecting groups. For convenience the processes described below all refer to a preparation of a compound of formula (1) but clearly the description applies equally to the preparation of compounds of formula (2).

Thus according to a further aspect of the invention, a compound of formula (1) in which R is a -CO₂H group may be obtained by hydrolysis of an ester of formula (1 a): 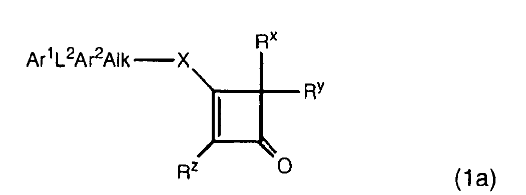 where Alk represents a group 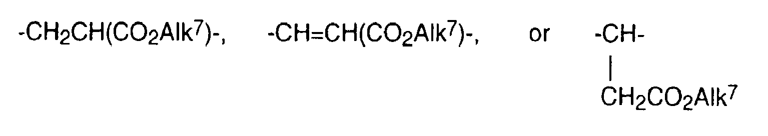 [where Alk⁷ is an alkyl group for example a C₁₋₆alkyl group].

The hydrolysis may be performed using either an acid or a base depending on the nature of Alk⁷, for example an organic acid such as trifluoroacetic acid or an inorganic base such as lithium, sodium or potassium hydroxide optionally in an aqueous organic solvent such as an amide e.g. a substituted amide such as dimethylformamide, an ether e.g. a cyclic ether such as tetrahydrofuran or dioxane or an alcohol e.g. methanol at a temperature from ambient to the reflux temperature. Where desired, mixtures of such solvents may be used.

According to a further aspect of the invention a compound of formula (1) may be prepared by condensation of a compound of formula (3): 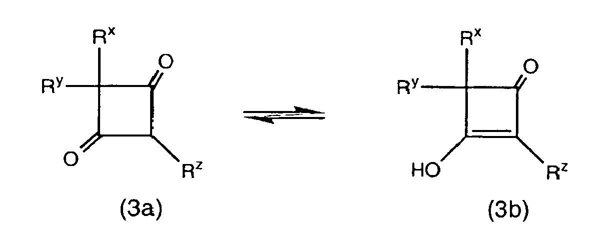 where compounds of formula (3) exist as two tautomeric isomers, (3a) and (3b) in solution with an amine of formula R¹R²NH, an alcohol of formula R¹OH or a thiol of formula R¹SH.

The reaction may be performed in an inert solvent or mixture of solvents, for example a hydrocarbon such as an aromatic hydrocarbon e.g. benzene or toluene and/or a halogenated hydrocarbon such as 1,2-dichloroethane, or dichloromethane at a temperature from 0°C to the reflux temperature. Where necessary, for example when a salt of an amine R¹R²NH is used, an organic base such as diisopropylethylamine can be added.

Any carboxylic acid group present in the intermediate of formula (3) or the amine R¹R²NH, alcohol R¹OH or thiol R¹SH may need to be protected during the displacement reaction, for example as an ethyl ester. The desired acid may then be obtained through subsequent hydrolysis, for example as particularly described above and generally described below.

The displacement reaction may also be carried out on an intermediate of formula 4 (see below) under the conditions just described.

Where desired the displacement reaction may also be performed on an intermediate of formulae (3), R¹R²NH, R¹OH or R¹SH which is linked, for example via its R, R¹ or R³ group, to a solid support, such as a polystyrene resin. After the reaction the desired compound of formula (1) may be displaced from the support by any convenient method, depending on the original linkage chosen.

Intermediates of formulae (3) R¹R²NH, R¹ OH and R¹SH may be obtained from simpler, known compounds by one or more standard synthetic methods employing substitution, oxidation, reduction or cleavage reactions. Particular substitution approaches include conventional alkylation, arylation, heteroarylation, acylation, thioacylation, halogenation, sulphonylation, nitration, formylation and coupling procedures. It will be appreciated that these methods may also be used to obtain or modify other compounds of formulae (1) and (2a), (2b), (2c) and (2d) where appropriate functional groups exist in these compounds.

Thus intermediates of formula (3) may be obtained by hydrolysis of intermediates of formula (4): 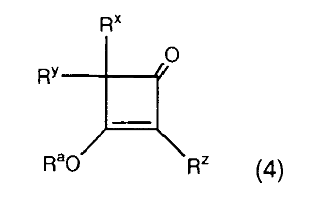 where R^{a} represents a C₁₋₆alkyl group or a silyl group such as a ^{t}butyldimethylsilyl group.

The hydrolysis may be performed using an acid, for example an inorganic acid such as hydrochloric acid in an organic solvent such as an ether e.g. diethylether, or an alcohol e.g. ethanol optionally in the presence of added water at a temperature from about ambient to 80°C.

Intermediates of formula (4) may be obtained by the cycloaddition of an intermediate of formula (5): 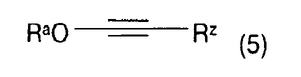 with a ketene of formula (6): 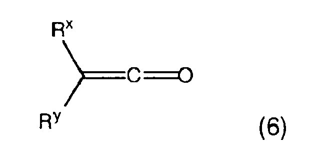 preformed or generated *in situ* during the cycloaddition reaction from an acid chloride of formula (7): 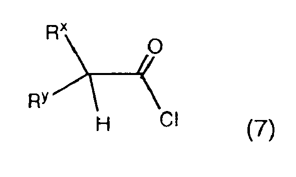 The reaction may be performed in the presence of an organic base such as an amine e.g. triethylamine or N,N-diisopropylethylamine or a cyclic amine such as pyridine or N-methylmorpholine optionally in an organic solvent such as an ether e.g. diethylether or diisopopylether.

Acid chlorides of formula (7) may be obtained from the corresponding acids by a convenient method of generating acid halides, for example by reaction with thionyl chloride or oxalyl chloride under such standard conditions as are well known in the art.

Compounds of formula (1 a) in which R^{z} is for example a halogen atom may be obtained from compounds of formula (1a) in which R^{z} is a hydrogen atom by reaction with a halogen source such as bromine or a halosuccinamide e.g. chloro or bromosuccinamide. The reaction may be performed in a solvent such as an ether e.g. a cyclic ether such as tetrahydrofuran at a temperature from about 0° to 30°. When bromine is used as halogen source the reaction may optionally be performed in the presence of added base such as an amine e.g. triethylamine.

Further compounds of formula (1a) in which R^{z} is a group -L¹(Alk¹)ₙ(R³)ᵥ in which L¹ is for example a Se, S, O or N(R⁸) may be prepared by reaction of an intermediate of formula HL¹(Alk¹)ₙ(R³)ᵥ with a compound of formula (1a) in which R^{z} is a hydrogen atom. The reaction may be performed in an organic solvent such as an ether e.g. a cyclic ether such as tetrahydrofuran at around room temperature optionally in the presence of a base such as an amine e.g. triethylamine.

Intermediate compounds of formula (4) may also be obtained from squaric acid derivations by such well known methods in the art as those of MacDougall, J. M. et al, J. Org. Chem, 64 5979-83 (1999); Hergueta, R. A., J. Org. Chem., 64, 5979-83, (1999); Heileman, M. J. et al, J. Am. Chem. Soc. 120, 3801-2, (1998); Yamamoto, Y. et al, J. Org. Chem, 62, 1292-8 (1997); Zhag, D. et al, J. Org. Chem. 61, 2594-5 (1996); Petasis, N. A. et al, Synlett, 155-6 (1996); Petasis, N. A. et al, Tetrahedron Lett., 36, 6001-4, (1995); Turnbull, P. et al, J. Org. Chem 60, 644-9 (1995); Yerxa, B. R. et al, Tetrahedron, 50, 6173-80 (1994); Ezcurra, J. E. et al, Tetrahedron Lett, 34, 6177-80, (1993); Ohno, M. et al, Tetrahedron Lett., 34, 4807-10, (1993); Yerxa, B. R. et al, Tetrahedron Lett. 33, 7811-14 (1992); Xu, S. L. et al, J. Org. Chem, 57, 326-8 (1992) and Kravs, J .L. et al, Tetrahedron Lett. 28, 1765-8 (1987).

Further compounds of the invention and intermediates thereto may be prepared by alkylation, arylation or heteroarylation. For example, compounds containing a -L¹H or -L²H group (where L¹ and L² is each a linker atom or group) may be treated with a coupling agent R³(Alk¹)ₙX¹ or Ar¹X¹ respectively in which X¹ is a leaving atom or group such as a halogen atom, e.g. a fluorine, bromine, iodine or chlorine atom or a sulphonyloxy group such as an alkylsulphonyloxy, e.g. trifluoromethylsulphonyloxy or arylsulphonyloxy, e.g. p-toluenesulphonyloxy group.

The reaction may be carried out in the presence of a base such as a carbonate, e.g. caesium or potassium carbonate, an alkoxide, e.g. potassium t-butoxide, or a hydride, e.g. sodium hydride, or an organic amine e.g. triethylamine or N,N-diisopropylethylamine or a cyclic amine, such as N-methylmorpholine or pyridine, in a dipolar aprotic solvent such as an amide, e.g. a substituted amide such as dimethylformamide or an ether, e.g. a cyclic ether such as tetrahydrofuran.

Compounds of formula Ar¹X¹ may be prepared from alcohols of formula Ar¹OH by reaction with a halogenating agent, for example a phosphorous oxyhalide such as phosphorous oxychloride at an elevated temperature e.g. 110°C.

Intermediate alcohols of formula Ar¹OH in which, for example, Ar¹ represents a 2,6-naphthyridine may be prepared by methods well known to a person skilled in the art, e.g. by the method of Sakamoto,T. et al [Chem. Pharm. Bull. 33, 626-633, (1985)].

Alternatively alkylating agents of formula Ar¹X¹ in which, for example, Ar¹ represents a 2,6-naphthyridine may be prepared by reaction of a 2,6-naphthyridine N-oxide or N, N'-dioxide with a halogenating agent, e.g. a phosphorous oxyhalide such as phosphorous oxychloride to give a 1-halo or 1,5-dihalo-2,6-napthyridine respectively. In the case of 1,5-dihalo-2,6-napthyridines each halogen atom may be substituted separately by a reagent such as HL²Ar²AlkN(R²)H or HL³(Alk²)ₜL⁴(R⁴)ᵤ by the particular methods just described above.

2,6-Napthyridine N-oxides and N,N'-dioxides may be generated from the corresponding 2,6-napthyridines by the general methods of synthesis of N-oxides described below or they may be synthesised by the methods of Numata, A. et al (Synthesis, 1999, 306-311).

Further alkylating agents of formula Ar¹X¹ in which, for example, Ar¹ represents a 2,6-naphthyridine, may be prepared by the methods of Giacomello G. et al [Tetrahedron Letters, 1117-1121 (1965)], Tan, R. and Taurins, A. [Tetrahedron Lett., 2737-2744, (1965)], Ames, D. E. and Dodds, W. D. [J. Chem. Soc. Perkin 1, 705-710 (1972)] and Alhaique, F. et al [Tetrahedron Lett., 173-174 (1975)].

Intermediate alcohols of formula Ar¹OH in which Ar¹ represents an optionally substituted 2,7-naphthyridin-1-yl group may be prepared by methods well known to a person skilled in the art, e.g. by the method of Sakamoto, T. et al [Chem. Pharm. Bull. 33, 626-633, (1985)] or Baldwin, J, J. et al [J. Org. Chem, 43, 4878-4880, (1978)]. Thus for example the method of Baldwin may be modified to allow the synthesis of intermediate 3-substituted 2,7-naphthyridin-1-yl groups of formula Ar¹OH as depicted in Scheme 1.

Reaction of an optionally substituted 4-methyl-3-cyano pyridine of formula (8) with a N,N-dimethylformamide di-C₁₋₆alkyl acetal, e.g. N,N-dimethylformamide diethyl acetal, in a dipolar solvent such as an amide e.g. a substituted amide such as dimethylformamide at an elevated temperature e.g. 140-150° gives a compound of formula (9) or (10) or a mixture thereof depending on the nature of the group R¹⁶. 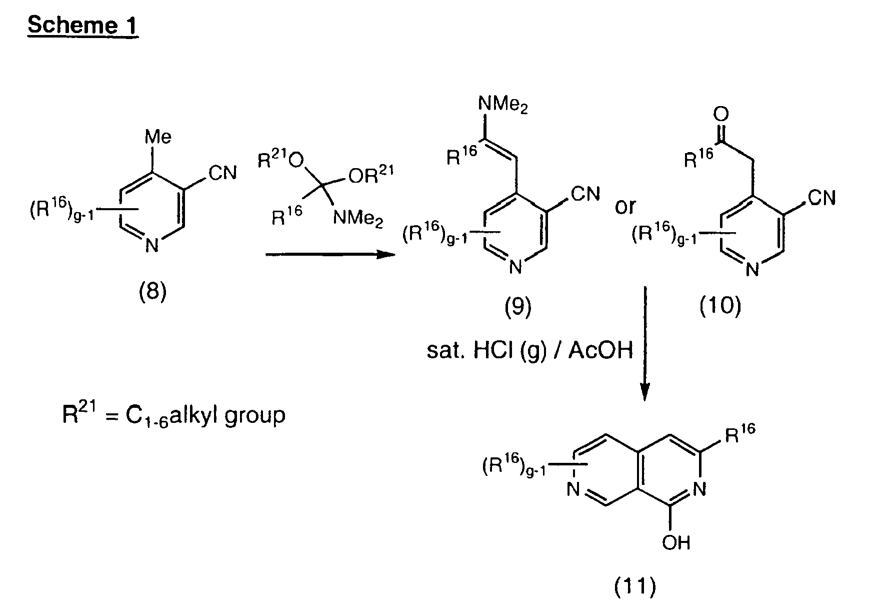 Compounds of formula (9) or (10) may be cyclised to 3-substituted 2,7-naphthyridin-1-yl alcohol of formula (11) by treatment with an acid e.g. an inorganic acid such as hydrochloric acid or hydrobromic acid or an acidic gas such as hydrogen chloride gas in an organic solvent e.g. an organic acid such as acetic acid optionally in the presence of water at a temperature from about ambient to 50°C.

Alternatively alkylating agents of formula Ar¹X¹ in which Ar¹ represents an optionally substituted 2,7-naphthyridin-yl group may be prepared by reaction of a 2,7-naphthyridine N-oxide or N, N'-dioxide with a halogenating agent, e.g. a phosphorous oxyhalide such as phosphorous oxychloride to give a 1-halo or 1,6-dihalo- and/or-1,8-dihalo-2,7-napthyridine respectively. In the case of 1,6-dihalo- and/or 1,8-dialo-2,6-napthyridines each halogen atom may be substituted separately by a reagent such as HL²Ar²AlkN(R²)H or HL³(Alk²)ₜL⁴(R⁴)ᵤ by the particular methods just described above.

2,7-Napthyridine N-oxides and N,N'-dioxides may be generated from the corresponding 2,7-napthyridines by the general methods of synthesis of N-oxides described below or they may be synthesised by the methods of Numata, A. et al (Synthesis, 1999, 306-311).

Further alkylating agents of formula Ar¹X¹ in which, for example, Ar¹ represents a 2,7-naphthyridin-1-yl, may be prepared by the methods of Wenkert E. et al J. Am. Chem. Soc. 89, 6741-5 (1967), and Aust. J. Chem. 433 (1972), and Sheffield D.J. J. Chem. Soc. Perkin. Trans I, 2506 (1972).

Intermediate alcohols of formula Ar¹OH in which Ar¹ represents a 3-substituted isoquinolin-1-yl group may be prepared by methods well known to a person skilled in the art, e.g. by the methods of Wu M.-J. et al Tetrahedron, 55, 13193-200 (1999), Hiebl J. et al Tetrahedron Lett. 40, 7935-8 (1999), Nagarajan A. et al Indian J. Chem., Sect. B, 28B, 67-78 (1989), Brun E. M. et al Synlett, 7, 1088-90 (1999) and Brun, E. M. et al Synthesis, 273-280 (2000).

Further alkylating agents of formula Ar¹X¹ in which, for example, Ar¹ represents a isoquinolin-1-yl group, may be prepared by the methods of Falk H. et al Monatsch. Chem. 25, 325-33 (1994), and Deady, L. W. et al Aust. J. Chem 42, 1029-34 (1989).

In a further example intermediates of formula R¹R²NH may be obtained by reaction of a compound of formula Ar¹L²H with a compound of formula X¹Ar²AlkN(R²)H under the reaction conditions just described

Compounds of formula Ar¹L²H in which, for example Ar¹ represents a 2,6-naphthyridine and L² is a -N(R⁸)- group, may be prepared from substituted 4-cyano-3-cyanomethylpyridines by the methods of Alhaique, F. *et al* (*ibid* and Gazz. Chim. Ital. 1975, 105, 1001-1009) or from 3-fomylpyridines by the methods of Molina, P. at al (Tetrahedron 1992, 48, 4601-4616).

Compounds of formula Ar¹L²H in which, for example Ar¹ represents a 2,7-naphthyridin-1-yl group and L² is a -N(R⁸)- group, may be prepared from substituted 4-formylpyridines by the methods of Molina, P. et al Tetrahedron, 48, 4601-4616, (1992), or by the methods described in US 3,938,367.

Compounds of formula Ar¹ L²H in which, for example Ar¹ represents a 3-substituted isoquinolin-1-yl group and L² is a -N(R⁸)- group, may be prepared by the methods of Bordner, J. et al J. Med. Chem. 31, 1036-9 (1988), Tovar J. D. et al J. Org. Chem., 64, 6499-6504 (1999), Karser E. M. et al Synthesis, 11, 805-6 (1974), and Molino, P et al J. Chem. Soc. Perkin Trans. 1, 1727-31 (1990).

In another example, compounds containing a -L¹H or -L²H or group as defined above may be functionalised by acylation or thioacylation, for example by reaction with one of the alkylating agents just described but in which X¹ is replaced by a -C(O)X², -C(S)X², -N(R⁸)COX² or -N(R⁸)C(S)X² group in which X² is a leaving atom or group as described for X¹. The reaction may be performed in the presence of a base, such as a hydride, e.g. sodium hydride or an amine, e.g. triethylamine or N-methylmorpholine, in a solvent such as a halogenated hydrocarbon, e.g. dichloromethane or carbon tetrachloride or an amide, e.g. dimethylformamide, at for example ambient temperature. Alternatively, the acylation may be carried out under the same conditions with an acid (for example one of the alkylating agents described above in which X¹ is replaced by a -CO₂H group) in the presence of a condensing agent, for example a diimide such as 1-(3-dimethylaminopropyl)-3-ethylcarbodiimide or N,N'-dicyclohexylcarbodiimide, advantageously in the presence of a catalyst such as a N-hydroxy compound e.g. a N-hydroxytriazole such as 1-hydroxybenzotriazole. Alternatively the acid may be reacted with a chloroformate, for example ethylchloroformate, prior to the desired acylation reaction.

In a further example compounds may be obtained by sulphonylation of a compound containing an -OH group by reaction with one of the above alkylating agents but in which X¹ is replaced by a -S(O)Hal or -SO₂Hal group [in which Hal is a halogen atom such as chlorine atom] in the presence of a base, for example an inorganic base such as sodium hydride in a solvent such as an amide, e.g. a substituted amide such as dimethylformamide at for example ambient temperature.

In another example, compounds containing a -L¹H or -L²H group as defined above may be coupled with one of the alkylation agents just described but in which X¹ is replaced by an -OH group in a solvent such as tetrahydrofuran in the presence of a phosphine, e.g. triphenylphosphine and an activator such as diethyl, diisopropyl- or dimethylazodicarboxylate.

In a further example, ester groups -CO₂R⁵, -CO₂R¹¹ or -CO₂Alk⁷ in the compounds may be converted to the corresponding acid [-CO₂H] by acid-or base-catalysed hydrolysis depending on the nature of the groups R⁵, R¹¹ or Alk⁷. Acid- or base-catalysed hydrolysis may be achieved for example by treatment with an organic or inorganic acid, e.g. trifluoroacetic acid in an aqueous solvent or a mineral acid such as hydrochloric acid in a solvent such as dioxan or an alkali metal hydroxide, e.g. lithium hydroxide in an aqueous alcohol, e.g. aqueous methanol.

In a further example, -OR⁵ or -OR¹⁴ groups [where R⁵ or R¹⁴ each represents an alkyl group such as methyl group] in compounds of formula (1) may be cleaved to the corresponding alcohol -OH by reaction with boron tribromide in a solvent such as a halogenated hydrocarbon, e.g. dichloromethane at a low temperature, e.g. around -78°C.

Alcohol [-OH] groups may also be obtained by hydrogenation of a corresponding -OCH₂R¹⁴ group (where R¹⁴ is an aryl group) using a metal catalyst, for example palladium on a support such as carbon in a solvent such as ethanol in the presence of ammonium formate, cyclohexadiene or hydrogen, from around ambient to the reflux temperature. In another example, -OH groups may be generated from the corresponding ester [CO₂Alk⁷ or CO₂R⁵] or aldehyde [-CHO] by reduction, using for example a complex metal hydride such as lithium aluminium hydride or sodium borohydride in a solvent such as methanol.

In another example, alcohol -OH groups in the compounds may be converted to a corresponding -OR⁵ or -OR¹⁴ group by coupling with a reagent R⁵OH or R¹⁴OH in a solvent such as tetrahydrofuran in the presence of a phosphine, e.g. triphenylphosphine and an activator such as diethyl-, diisopropyl-, or dimethylazodicarboxylate.

Aminosulphonylamino [-NHSO₂NHR³ or -NHSO₂NHAr¹] groups in the compounds may be obtained, in another example, by reaction of a corresponding amine [-NH₂] with a sulphamide R³NHSO₂NH₂ or Ar¹NHSO₂NH₂ in the presence of an organic base such as pyridine at an elevated temperature, e.g. the reflux temperature.

In another example compounds containing a -NHCSAr¹, -CSNHAr¹,-NHCSR³ or -CSNHR³ may be prepared by treating a corresponding compound containing a -NHCOAr¹, -CONHAr¹, -NHCOR³ or -CONHR³ group with a thiation reagent, such as Lawesson's Reagent, in an anhydrous solvent, for example a cyclic ether such as tetrahydrofuran, at an elevated temperature such as the reflux temperature.

In a further example amine (-NH₂) groups may be alkylated using a reductive alkylation process employing an aldehyde and a borohydride, for example sodium triacetoxyborohyride or sodium cyanoborohydride, in a solvent such as a halogenated hydrocarbon, e.g. dichloromethane, a ketone such as acetone, or an alcohol, e.g. ethanol, where necessary in the presence of an acid such as acetic acid at around ambient temperature.

In a further example, amine [-NH₂] groups in compounds of formula (1) may be obtained by hydrolysis from a corresponding imide by reaction with hydrazine in a solvent such as an alcohol, e.g. ethanol at ambient temperature.

In another example, a nitro [-NO₂] group may be reduced to an amine [-NH₂], for example by catalytic hydrogenation using for example hydrogen in the presence of a metal catalyst, for example palladium on a support such as carbon in a solvent such as an ether, e.g. tetrahydrofuran or an alcohol e.g. methanol, or by chemical reduction using for example a metal, e.g. tin or iron, in the presence of an acid such as hydrochloric acid.

Aromatic halogen substituents in the compounds may be subjected to halogen-metal exchange with a base, for example a lithium base such as n-butyl or t-butyl lithium, optionally at a low temperature, e.g. around - 78°C, in a solvent such as tetrahydrofuran and then quenched with an electrophile to introduce a desired substituent, for example, a formyl group may be introduced by using dimethylformamide as the electrophile; a thiomethyl group may be introduced by using dimethyldisulphide as the electrophile.

In another example, sulphur atoms in the compounds, for example when present in a linker group L¹ or L² may be oxidised to the corresponding sulphoxide or sulphone using an oxidising agent such as a peroxy acid, e.g. 3-chloroperoxybenzoic acid, in an inert solvent such as a halogenated hydrocarbon, e.g. dichloromethane, at around ambient temperature.

In another example compounds of formula Ar¹ X¹ (where X¹ is a halogen atom such as a chlorine, bromine or iodine atom) may be converted to such compounds as Ar¹CO₂R²⁰ (in which R²⁰ is an optionally substituted alkyl, aryl or heteroaryl group), Ar¹CHO, Ar¹CHCHR²⁰, Ar¹CCR²⁰, Ar¹N(R²⁰)H, Ar¹N(R²⁰)₂, for use in the synthesis of for example compounds of formula Ar¹L²Ar²AlkN(R²)H, using such well know and commonly used palladium mediated reaction conditions as are to be found in the general reference texts Rodd's Chemistry of Carbon Compounds, Volumes 1-15 and Supplementals (Elsevier Science Publishers, 1989), Fieser and Fieser's Reagents for Organic Synthesis, Volumes 1-19 (John Wiley and Sons, 1999), Comprehensive Heterocyclic Chemistry, Ed. Katritzky et al, Volumes 1-8, 1984 and Volumes 1-11, 1994 (Pergamon), Comprehensive Organic Functional Group Transformations, Ed. Katritzky et al, Volumes 1-7, 1995 (Pergamon), Comprehensive Organic Synthesis, Ed. Trost and Flemming, Volumes 1-9, (Pergamon, 1991), Encyclopedia of Reagents for Organic Synthesis, Ed. Paquette, Volumes 1-8 (John Wiley and Sons, 1995), Larock's Comprehensive Organic Transformations (VCH Publishers Inc., 1989) and March's Advanced Organic Chemistry (John Wiley and Sons, 1992).

N-oxides of compounds of formula (1) may be prepared for example by oxidation of the corresponding nitrogen base using an oxidising agent such as hydrogen peroxide in the presence of an acid such as acetic acid, at an elevated temperature, for example around 70°C to 80°C, or alternatively by reaction with a peracid such as peracetic acid in a solvent, e.g. dichloromethane, at ambient temperature.

Salts of compounds of formula (1) may be prepared by reaction of a compound of formula (1) with an appropriate base in a suitable solvent or mixture of solvents e.g. an organic solvent such as an ether e.g. diethylether, or an alcohol, e.g. ethanol using conventional procedures.

Where it is desired to obtain a particular enantiomer of a compound of formula (1) this may be produced from a corresponding mixture of enantiomers using any suitable conventional procedure for resolving enantiomers.

Thus for example diastereomeric derivatives, e.g. salts, may be produced by reaction of a mixture of enantiomers of formula (1) e.g. a racemate, and an appropriate chiral compound, e.g. a chiral base. The diastereomers may then be separated by any convenient means, for example by crystallisation and the desired enantiomer recovered, e.g. by treatment with an acid in the instance where the diastereomer is a salt.

In another resolution process a racemate of formula (1) may be separated using chiral High Performance Liquid Chromatography. Alternatively, if desired a particular enantiomer may be obtained by using an appropriate chiral intermediate in one of the processes described above. Alternatively, a particular enantiomer may be obtained by performing an enantiomer specific enzymatic biotransformation e.g. an ester hydrolysis using an esterase and then purifying only the enantiomerically pure hydrolysed acid from the unreacted ester antipode.

Chromatography, recrystallisation and other conventional separation procedures may also be used with intermediates or final products where it is desired to obtain a particular geometric isomer of the invention.

The following Examples illustrate the invention. All temperatures are in °C. The following abbreviations are used:

| | |
|---|---|
| NMM - N-methylmorpholine; | EtOAc - ethyl acetate; |
| MeOH - methanol; | BOC - butoxycarbonyl; |
| DCM - dichloromethane; | AcOH - acetic acid; |
| DIPEA - diisopropylethylamine; | EtOH - ethanol; |
| Pyr - pyridine; | Ar - aryl; |
| dimethylsulphoxide; | iPr - isopropyl; |
| Et₂O - diethylether; | Me - methyl; |
| THF - tetrahydrofuran, | DMF - N,N-dimethylformamide; |
| FMOC - 9-fluorenylmethoxycarbonyl; | |
| DBU - 1,8-Diazabicyclo[5,4-0]undec-7-ene; | |
| DMAP - 4-(dimethylamino)pyridine. | |
| HOBT - 1-hydroxybenzotriazole | |

All NMR's were obtained either at 300MHz or 400MHz.

All Intermediates and Examples were named with the aid of Beilstein Autonom (available from MDL Information Systems GmbH, Therdor-Heuss-Allee 108D 60486, Frankfurt, Germany) or were given names that seemed consistent, with the exception that propanoates were named by the IUPAC name rather than the trivial name (propionate) and isonicotinoyl (trivial name) is used in place of pyridine-4-carbonyl.

### INTERMEDIATE 1

### (+/-) 3-Ethoxy-4-methyl-4-propyl-2-cyclobuten-1-one

The title compound was prepared using a modification of the method of Wasserman, H.H. et al [J. Org. Chem, 38, 1451-1455, (1973)]; to a solution of 2-methyl pentanoyl chloride (3.91ml) and ethyl ethynylether (5g, 40% solution in hexanes, 28.6mmol) in Et₂O (35ml) at room temperature was added triethylamine (9.9ml), with stirring. The reaction was warmed to 50º and stirred for 72h prior to cooling and filtration. The filtrate was concentrated *in vacuo* and the residual oil chromatographed (SiO₂; hexanes 80: EtOAc 20) to give the title compound as a colourless oil (3.71g, 17.9mmol, 77%). δH (CDCl₃, 300K), 4.84 (1H, s), 4.24-3.98 (2H, m), 2.04 (3H, s), 1.56-1.43 (4H, m), 1.30-1.26 (3H, m), 0.91 (3H, t, J 7.3Hz). m/z (ES⁺, 70V) 178.1 (MH⁺).

### INTERMEDIATE 2

### (+/-) 3-Hydroxy-4-methyl-4-propyl-2-cyclobuten-1-one

Intermediate 1 (1g, 59.5mmol) and conc. hydrochloric acid (2ml) were stirred vigorously at room temperature for 48h. The resulting slurry was filtered and the residue washed with water (3 x10ml) and dried under vacuum to give the title compound as an off-white powder (620mg, 44.2mmol, 74%). δH (CDCl₃, 300K) 3.79 (2H, s), 1.59-1.53 (2H, m), 1.41-1.27 (2H, m), 1.18 (3H, s), 0.85 (3H, t, J 7.3Hz). m/z (ES⁺, 70V) 140.9 (MH⁺).

### INTERMEDIATE 3

### 3-Ethoxy-4,4-dipropyl-2-cyclobuten-1-one

The title compound was prepared using a modification of the method of Wasserman, H.H. et al, [J. Org. Chem, 38, 1451-1455, (1973)]; triethylamine (29ml) was added dropwise at room temperature to a well-stirred solution of di-n-propylacetyl chloride (13.9g, 85.8mmol) and ethyl ethynylether (15g, 40% solution in hexanes, 85.7mmol) in toluene (120ml. The reaction was warmed to 60º and stirred for 48h prior to cooling and filtration. The filtrate was concentrated *in vacuo* and the residual oil chromatographed (SiO₂; hexanes 80: EtOAc 20) to give the title compound as a brown oil (11.2g, 57.1mmol, 67%). δH (CDCl₃, 300K) 5.02 (1H, s), 4.32 (2H, q, J 7.1Hz), 1.69-1.61 (4H, m), 1.45-1.40 (4H, m), 1.02 (6H, t, J 7.3Hz). m/z (ES⁺, 70V) 197.1 (MH⁺).

### INTERMEDIATE 4

### 3-Hydroxy-4,4-dipropyl-2-cyclobuten-1-one

Intermediate 3 (10.2mmol) and 6M hydrochloric acid (10ml) were stirred vigorously at 65º for 72h. The resulting slurry was diluted with DCM (30ml) and distilled water (30ml) and extracted with DCM (3x10ml). The combined extracts were dried (MgSO₄), filtered and concentrated *in vacuo* to give the title compound as a pale yellow oil, which crystallised on standing (1.49g, 8.87mmol, 87%).

### INTERMEDIATE 5

### 3-Ethoxy-2-hexyl-4,4-dimethyl-2-cyclobuten-1-one

The title compound was prepared using a modification of the method of Wasserman, H.H. et al, [J. Org. Chem, 38, 1451-1455, (1973)]; triethylamine (22ml) was added dropwise at room temperature to a well-stirred solution of isobutyryl chloride (7.3ml, 69mmol) and 1-ethoxy-1-octyne [prepared according to the method of Kocienski, P. et al. Tetrahedron Lett. 1833, 30, (1989)] (6.5g, 63mmol) in diethylether (100ml). The reaction was warmed to 35° and stirred for 96h prior to cooling and filtration. The filtrate was concentrated *in vacuo* and the residual oil chromatographed (SiO₂; hexanes 80: EtOAc 20) to give the title compound as a brown oil (8.6g, 38mmol, 61%). δH (CDCl₃, 300K) 4.34 (2H, d, J 7.1Hz), 2.05 (2H, dd, J 5.6Hz, 7.3Hz), 1.44 (3H, t, J 7.1Hz), 1.27-1.12 (8H, m), 1.23 (6H, s), 0.89 (3H, t, J 2.7Hz). m/z (ES⁺, 70V) 225.0 (MH⁺).

### INTERMEDIATE 6

### 2-Hexyl-3-hydroxy-4,4-dimethyl-2-cyclobuten-1-one

Intermediate 5 (7.6g, 34.0mmol) and 6M hydrochloric acid (10ml) were stirred vigorously at 100° for 18h. The resulting slurry was diluted with DCM (30ml) and distilled water (30ml) and extracted with DCM (3 x10ml).

The combined extracts were dried (MgSO₄), filtered and concentrated *in vacuo.* The residue was triturated with hexanes and filtered to give the title compound as an off-white powder (6.5g, 33.0mmol, 98%). δH (CDCl₃, 300K) 2.01 (2H, t, J 7.0Hz), 1.49-1.44 (2H, m), 1.34-1.19 (14H, m), 0.89-0.84 (3H, m). m/z (ES⁺, 70V) 197.0 (MH⁺).

### INTERMEDIATE 7

### (+/-) 4-Benzyl-3-ethoxy-4-methyl-2-cyclobuten-1-one

The title compound was prepared using a modification of the procedure of Wasserman et al [J. Org. Chem, 38, 1451-1455, (1973)]; triethylamine (20ml) was added to a stirred solution containing α-methyl tetrahydrocinnamoyl chloride (5g, 27.5mmol) and ethyl ethynylether (6g, 40% soln. in hexanes, 85.7mmol) and the resulting slurry heated to 35° for 3d. The crude reaction mixture was then filtered and the residue concentrated *in vacuo.* The residual oil was chromatographed (SiO₂, EtOAc 20: hexanes 80) to give the title compound as a pale brown oil (4.91g, 86%). δ□H (CDCl₃, 300K) 7.19-7.05 (5H, m), 4.56 (1H, s), 4.09-4.00 (1H, m), 3.97-3.89 (1H, m), 2.86 (1H, d, J 14.0Hz), 2.86 (1H, d, J 14.0Hz), 1.30 (3H, t, J 7.1Hz), 1.24 (3H, s). m/z (ES⁺, 70V) 216.9 (MH⁺).

### INTERMEDIATE 8

### (+/-) 4-Benzyl-3-hydroxy-4-methyl-2-cyclobuten-1-one

Intermediate 7 (4.5g, 20.9mmol) and hydrochloric acid (6M, 10ml) were stirred at room temperature for 48h. Filtration of the resulting slurry and washing of the residue with water (3x15ml) gave the title compound as a pale brown powder (3.92g, 20.8mmol, 99%). δH (CDCl₃, 300K) 7.03-6.83 (5H, m), 4.24 (1H, s), 2.52 (2H, s), 0.94 (3H, s). m/z (ES⁺, 70V) 189.1 (MH⁺).

### INTERMEDIATE 9

### 3-Cyano-4-(2-(N-N-dimethylamino)ethylen-1-yl)pyridine

A solution of 4-methyl-3-cyanopyridine [prepared acccording to Ref: J. Prakt. Chem. 338, 663 (1996)], (8.0g, 67.8mmol) and *N,N-*dimethylformamide diethyl acetal (11.0g, 74.8mmol) in dry DMF (50ml) was stirred at 140º under N₂ for 2 days. An additional portion of N,N,-dimethylformamide diethyl acetal (5g) was added and stirred at 140º for 4h. The volatiles were removed *in vacuo* and the obtained dark oil partitioned between EtOAc (300ml) and water (50ml). The phases were separated and the aqueous layer re-extracted with EtOAc (3x100m)). The combined organic extracts were washed with brine (30ml), dried (Na₂SO₄), treated with activated charcoal, filtered and evaporated *in vacuo* to afford essentially pure title compound as a dull orange solid (10.1g, 85%). δH (CDCl₃) 8.49 (1H, s), 8.25 (1h, d, J 5.9Hz), 7.29 (1H, d, J 13.2Hz), 7.09 (1H, d, J 5.9Hz), 5.25 (1H, d, J 13.2Hz) and 2.99 (6H, s); m/z (ES⁺, 70V) 174 (MH⁺).

### INTERMEDIATE 10

### 1-Hydroxy-2,7-naphthyridine hydrochloride salt

HCl gas was bubbled through a stirred solution of Intermediate 9 (6.2g, 3.58mmol) in glacial acetic acid (50ml) and water (0.64ml, 3.55mmol) for 1-2min. The reaction mixture was stirred in a stoppered flask at 40º for 18h. The volatiles were removed *in vacuo* affording a dark residue, which was treated with water (3x20ml) and re-evaporated *in vacuo.* The obtained dark semi-solid was treated with 40ml warm ethanol, ice-cooled, and the undissolved solid collected by filtration affording the title compound as a green coloured solid (5.2g, 80%) δH (DMSO-d⁶) 12.5 (1H, br s), 9.38 (1H, s), 8.84 (1H, d, J 7.0Hz), 8.15 (1H, d, J 7.0Hz), 7.89 (1H, br dd, J 7.0, 5.0Hz) and 6.85 (1H, d, J 7.0Hz); m/z (ES⁺, 70V), 147 (MH⁺).

### INTERMEDIATE 11

### 1-Chloro-2,7-naphthyridine

Intermediate 10 (5.2g, 28.5mmol) was stirred with phosphorous oxychloride (75ml) at 110° for 24h. The volatiles were removed *in vacuo* affording a dark oil which was poured into an ice-bath cooled mixture of saturated aqueous NaHCO₃ (100ml containing 20g solid NaHCO₃) and EtOAc (100ml). After thorough mixing the phases were separated and the aqueous layer re-extracted with EtOAc (2x75ml). The combined organic extracts were washed with brine (15ml), dried (Na₂SO₄) and evaporated *in vacuo* to afford the title compound as a yellow solid (4.0g, 85%) δH (CDCl₃) 9.45 (1H, s), 8.81 (1H, d, J 5.7Hz), 8.47 (1H, d, J 5.7Hz), 7.66 (1H, d, J 5.7Hz) and 7.60 (1H, d,J 5.7Hz); m/z (ES⁺, 70V) 165 and 167 (MH⁺).

### INTERMEDIATE 12

### Ethyl (2S)-2-amino-3-[4-(2,7-naphthyridin-1-ylamino)phenyl] propanoate

A solution of ethyl-(*S*)-3-[4-aminophenyl]-2-[*t*-butoxycarbonylamino] propanoate (638mg, 2.07mmol) and Intermediate 11 (310mg, 1.88mmol) in ethoxyethanol (2ml) was stirred at 120º for 15 min and at 100º for 1h under nitrogen. The volatiles were removed *in vacuo* and the dark residue partitioned between EtOAc (70ml) and saturated aqueous NaHCO₃ (10ml). The phases were separated and the aqueous layer re-extracted with EtOAc (2x3Oml). The combined organic extracts were washed with brine (10ml), dried (Na₂SO₄) and evaporated *in vacuo* to afford a dark foam. Chromatography (SiO₂; 5 to 10% MeOH/DCM) afforded a mixture of ethyl-(*S*)-3-[4-(2,7-naphthyridin-1-ylamino)phenyl]-2-[(*t*-butoxycarbonyl) amino]propanoate and some of the title compound (730mg). This mixture was treated with a solution of trifluoroacetic acid (5ml) and DCM (5ml) at room temperature for 1h. The volatiles were removed *in vacuo* and the residue partitioned between EtOAc (75ml) and saturated aqueous NaHCO₃ (20ml). The phases were separated and the aqueous layer re-extracted with EtOAc (3x30ml). The combined organic extracts were washed with brine (10ml), dried (Na₂SO₄) and evaporated *in vacuo* to afford an orange solid. Chromatography (SiO₂; 10% MeOH/DCM) afforded the title compound as a straw-coloured solid (420mg, 60% over two steps). δH (CDCl₃) 10.70 (1H, s), 10.31 (1H, s), 9.44 (1H, d, J 5.6Hz), 8.94 (1H, d, J 5.6Hz), 8.55 (1H, d, J 7.3Hz), 8.54 (2H, d, J 8.5Hz), 8.46 (1H, d, J 5.6Hz), 7.94 (2H, d, J 8.5Hz), 4.84 (2H, q, J 7.1Hz), 4.35 (1H, t, J 6.6Hz), 4.10 (2H, br s), 3.64 (1H, dd, J 13.5, 6.4Hz), 3.56 (1H, dd, J 13.5, 7.0Hz) and 1.95 (3H, t, J 7.1Hz); m/z (ES⁺, 70V) 337 (MH⁺).

### INTERMEDIATE 13

### Methyl (2S)-2-amino3-[4-(2,7-naphthyridin-1-yloxy)phenyl]propanoate

A mixture of *N*-(BOC)-(*S*)-tyrosine methyl ester (1.71g, 5.80 mmol) potassium carbonate (0.80g, 5.80mmol) and Intermediate 11 (1.0g, 6.08mmol) in dry DMF (10ml) was stirred at room temperature for 18h, and at 40º for 18h. The DMF was removed *in vacuo* and the residue partitioned between EtOAc (80ml) and 10% aqueous Na₂CO₃ (20ml). The phases were separated and the aqueous layer re-extracted with EtOAc (2x20ml). The combined organic extracts were washed with brine (10ml), dried (Na₂SO₄) and evaporated *in vacuo* to afford a new colourless oil. Chromatography (silica; 2.5% MeOH/DCM) afforded reasonably pure N-t-butoxycarbonyl protected title compound (1.75g, 71%). This material was dissolved in EtOAc (40ml) and HCl gas was bubbled through the stirred solution for 1min. then the mixture was stirred for an additional 0.5h. The volatiles were removed *in vacuo* affording a yellow solid which was partitioned between EtOAc (80ml) and saturated aqueous NaHCO₃ (20ml). The phases were separated and the aqueous layer re-extracted with EtOAc (2x20ml). The combined organic extracts were washed with brine (10ml), dried (Na₂SO₄) and evaporated *in vacuo.* The obtained oil was chromatographed (SiO₂; 5% MeOH/DCM) to afford the title compound as a near colourless oil (0.83g, 62%) δH (CDCl₃) 9.77 (1H, s), 8.75 (1H, d, J 5.8Hz), 8.10 (1H, d, J 5.8Hz), 7.58 (1H, d, J 5.8Hz), 7.29 (2H, d, J 8.4Hz), 7.25 (1H, d, J 5.9Hz), 7.21 (2H, d, J 8.4Hz), 3.80-3.70 (1H, obscured m), 3.72 (3H, s), 3.15 (1H, dd, J 13.6, 5.1Hz), 2.88 (1H, dd, J 13.6, 8.0Hz) and 0.78 (2H, br s); m/z (ES⁺, 70V) 324 (MH⁺).

### INTERMEDIATE 14

### 4-Acetonyl-3-cyanopyridine

A solution of 4-methyl-3-cyanopyridine (4g, 33.9mmol) and *N,N* dimethylacetamide dimethylacetal (5.4g, 40.6mmol) in dry DMF (20ml) was stirred at 130° for 7h. The volatiles were removed *in vacuo* to afford a dark oil which solidified on standing. This material was chromatographed (SiO₂; 50% EtOAc/Hexane - 100% EtOAc) affording the title compound as an off-yellow solid (3.73g, 69%). δH (CDCl₃) 8.87 (1H, s), 8.74 (1H, d, J 5.2Hz), 7.28 (1H, d, J 5,2Hz), 4.00 (2H, s) and 2.36 (3H, s); m/z (ES⁺, 70V) 161 (MH⁺).

### INTERMEDIATE 15

### 1-Hydroxy-3-methyl-2,7-naphthyridine hydrochloride

HCl gas was bubbled through a stirred solution of Intermediate 14 (3.73g, 23.3mmol) in glacial acetic acid (40ml) for several minutes. The flask was stoppered and reaction stirred for 18h at ambient temperature. The volatiles were removed *in vacuo* affording a straw-coloured solid. This was twice treated with water (30ml portions) and re-evaporated *in vacuo* to dryness, affording the title compound (contaminated with ~25% unidentified by-product) as a dark straw coloured solid (4.1g). δH (DMSO-d⁶) 12.46 (1H, br s), 9.32 (1H, s), 8.71 (1H, d, J 6.5Hz), 7.98 (1H, d, J 6.5Hz), 6.67 (1H,s) and 2.38 (3H, s); m/z (ES⁺, 70V) 161 (MH⁺). Used without further purification.

### INTERMEDIATE 16

### 1-Chloro-3-methyl-2,7-naphthyridine

Intermediate 15 (4.1g) was treated with phosphorus oxychloride (50ml) at 130º for 3h, affording a dark solution. The volatiles were removed *in vacuo* and the obtained dark oil extracted with Et₂O (100ml). Saturated aqueous NaHCO₃ (ice cold; containing 10g additional solid NaHCO₃) was poured (with CARE!) onto the crude product with swirling and ice-bath cooling. After thorough shaking, addition Et₂O (80ml) was added, the mixture reshaken, and the phases separated. The aqueous layer was re-extracted with Et₂O (2x80ml) and the combined ethereal extracts washed with brine (20ml), dried (Na₂SO₄) and evaporated *in vacuo* to afford an orange solid (3.6g). Chromatography (silica; 70% EtOAc/Hexane-100% EtOAc) afforded a more-polar by-product (3-methyl-1H-pyrano[3,4-c]pyridin-1-one, (0.7g) and the title compound as a white solid (2.82g, 79% from intermediate 14) δH (CDCI₃) 9.66 (1H, s), 8.73 (1H, d, J 5.8Hz), 7.56 (1H, d, J 5.8Hz), 7.40 (1H, s) and 2,69 (3H, s); m/z (ES⁺, 70V) 179 and 181 (MH⁺).

### INTERMEDIATE 17

### Ethyl (2S)-2-[(tert-butoxycarbonyl)amino]-3-{4-[(3-methyl[2,7-naphthyridin-1-ylamino]phenyl}propanoate hydrochloride

Acetylchloride (55mg, 50ml, 0.70mmol) was added to absolute ethanol (25ml) and stirred for one minute. Intermediate 16 (2.50g, 14.0mmol) and ethyl-(*S*)-3-[4-aminophenyl]-2-{*tert*-butyloxycarbonyl]propanoate (4.31g, 14.0mmol) were added and the reaction mixture stirred at 60° for 2.75h. The volatiles were removed *in vacuo* to afford a yellow-orange solid. This was treated with EtOAc (~25ml), warmed, re-cooled and the precipitate collected by filtration, with Et₂O washing, affording the title compound as a yellow solid (4.96g, 73%). δH (CDCl₃) 10.44 (1H, br s), 10.33 (1H, br s), 8.60 (1H, d, J 6.5Hz), 8.00 (1H, d, J 6.5Hz), 7.85 (2H, d, J 8.5Hz), 7.28 (1H, d, J 8.0Hz), 7.23 (2H, d, J 8.5Hz), 7.16 (1H, s), 4.19-4.01 (1H, m), 4.08 (2H, q, J 7.0Hz), 2.97 (1H, dd, J 13.8, 5.4 Hz), 2.86 (1H, dd, J 13.8, 10.0Hz), 2.50 (3H, s), 1.34 (9H, s) and 1.15 (3H, t, J 7.0Hz); m/z (ES⁺, 70V) 451 (MH⁺).

### INTERMEDIATE 18

### Ethyl-(2S)-2-amino-3-{4-[(3-methyl[2,7]naphthyridin-1-yl)amino] phenyl}propanoate

HCI gas was bubbled through a stirred solution of Intermediate 17 (4.95g, 10.2mmol) for 1-2min. After 30min stirring at ambient temperature the volatiles were removed *in vacuo* affording a yellow powder. This was treated with saturated aqueous NaHCO₃ (30ml) then extracted with EtOAc (100ml, and 3x50ml). The combined organic extracts were washed with brine (10ml), dried (Na₂SO₄) and evaporated *in vacuo* affording the title compound as a yellow solid (3.56, 100%). δH (CDCl₃) 9.25 (1H, s), 8.50 (1H, d, J 5.6Hz), 7.66 (2H, d, J 8.4Hz), 7.35 (1H, d, J 5.6Hz), 7.34 (1H, masked s), 7.14 (2H, d, J 8.4Hz), 6.81 (1H, s), 4.12 (2H, q, J 7.2Hz), 3.65 (1H, dd, J 7.8, 5.2Hz), 3.02 (1H, dd, J 13.7, 5.2Hz), 2.80 (1H, dd, J 13.7, 7.8Hz), 2.48 (3H, s), 1.56 (2H, br s) and 1.21 (3H, t, J 7.2Hz); m/z (ES⁺, 70V) 351 (MH⁺).

### INTERMEDIATE 19

### Ethyl (2S)-2-[(tert-butoxycarbonyl)amino]-3-{4-[(3-methyl[2,7]naphthyridin-1-yl)oxy]phenyl}propanoate

A mixture of *N*-*t*-butyloxycarbonyl-(*S*)-tyrosine ethyl ester (14.5g, 46.9mmol), caesium carbonate (14.05g, 43.1 mmol) and Intermediate 9 (7.0g, 39.2mmol) in dry DMF (60ml) was stirred at room temperature for 48h. The reaction was diluted with Et₂O (150ml) and filtered off. The filtrate was evaporated under high vacuum and the residue was chromatographed (SiO₂; 40%-60% EtOAc/Hexane) which afforded the title compound as a viscous, straw-coloured oil (16.2g, 77%) δH (CDCl₃) 9.56 (1H, s), 8.58 (1H, d, J 5.8Hz), 7.39 (1H, d, J 5.8Hz), 7.15-7.10 (4H, m), 7.00 (1H, s), 4.99-4.91 (1H,m), 4.54-4.46 (1H, m), 4.09 (2H, q, J 7.1Hz), 3.10-2.99 (2H,m), 2.36 (3H, s), 1.34 (9H, s) and 1.15 (3H, t, J 7.1Hz); m/z (ES⁺, 70V) 452 (MH⁺).

### INTERMEDIATE 20

### Ethyl (2S)-2-amino-3-{4-[(3-methyl[2,7]naphthyridin-1-yl)oxy]phenyl} propanoate

HCl gas was bubbled through a stirred solution of Intermediate 19 (16g) in EtOAc (300ml) until a persistent fine white precipitate formed (~2minutes). After stirring for 0.5h the volatiles were removed *in vacuo.* The obtained solid was partitioned between EtOAc (250ml) and saturated aqueous NaHCO₃ (80ml plus 5g solid NaHCO₃). The phases were separated and the aqueous layer re-extracted with EtOAc (5x50ml). The combined organic extracts were washed with brine (10ml), dried (Na₂SO₄) and evaporated *in vacuo* to afford an oil. Chromatography (SiO₂; 100% EtOAC-10% EtOH/EtOAc) afforded the title compound as a viscous oil (11.1g, 89%). δH (CDCl₃) 9.71 (1H, s), 8.70 (1H, d, J 5.Hz), 7.50 (1H, d, J 5.8Hz), 7.31-7.28 (4H,m), 7.11 (1H, s), 4.23 (2H, q, J 7.1Hz), 3.79-3.72 (1H, m), 3.14 (1H, dd, J 14.1, 5.4Hz), 2.94 (1H, dd, J 14.1, 7.8Hz), 2.47 (3H, s), 1.75-1.50 (2H, br s) and 1.30 (3H, t, J 7.1Hz); m/z (ES⁺, 70V) 352 (MH⁺).

### INTERMEDIATE 21

### 1-Chloro-2,6-naphthyridine

1-Hydroxy-2,6-naphthyridine (550mg) [prepared according to the method of Sakamoto, T. et al Chem. Pharm. Bull. 33, 626, (1985)] was stirred with phosphorous oxychloride (10ml) at 110º for 5h. The volatiles were removed *in vacuo* and the residue treated carefully with ice. After diluting with water (to ~25ml), solid NaHCO₃ was added to effect neutralisation and the product extracted into EtOAc (2x80ml). The combined organic extracts were dried (MgSO₄), evaporated *in vacuo,* and the crude product chromatographed (SiO₂; EtOAc) affording the title compound as a slightly yellow solid (420mg, 68%). δH (CDCl₃) 9.35 (1H, s), 8.82 (1H, d, J 5.9Hz), 8.48 (1H, d, J 5.6Hz), 8.00 (1H, d, J 5.9Hz), 7.74 (1H, d, J 5.6Hz); m/z (ES⁺, 70V) 165 and 167 (MH⁺).

### INTERMEDIATE 22

### Ethyl (2S)-2-[(tert-butoxycarbonyl)amino]3-[4-([2,6]naphthyridin-1-ylaminol)phenyl]propanoate

Ethyl (*S*)-3-(4-aminophenyl)-2-[N-(t-butyloxycarbonyl)aminolpropanoate (600mg, 1.95mmol), Intermediate 21 (350mg, 2.13mmol) and DIPEA (276mg, 372µl, 2.13mmol) in 2-ethoxyethanol (0.5ml) were stirred at 130° under N₂ for several hours. The reaction was partitioned between EtOAc (70ml) and saturated aqueous NaHCO₃ (30ml). The phases were separated and the aqueous layer re-extracted with EtOAc (3x30ml). The combined organic extracts were washed with brine (10ml), dried (MgSO₄) and evaporated *in vacuo* to afford a dark oil. Chromatography (SiO₂; 3% MeOH/DCM) gave the title compound as a dull orange foam (360mg, 42%). δH (CDCl₃) 9.19 (1H, s), 8.67 (1H, d, J 5.9Hz), 8.24 (1H, d, J 5.8Hz), 7.66 (1H, d, J 5.9Hz), 7.65 (2H, d, J 8.5Hz), 7.21 (1H, d, J 5.8Hz), 7.16 (2H, d, J 8.5Hz), 7.15 (1H, obscured s), 5.05-4.97 (1H, m), 4.60-4.51 (1H, m), 4.19 (2H, q, J 7.1Hz), 3.17-3.04 (2H, m), 1.44 (9H, s), 1.27 (3H, t, J 7.1Hz); m/z (ES⁺, 70V) 459 (MNa⁺), 437 (MH⁺).

### INTERMEDIATE 23

### Ethyl (2S)-2-amino-3-[4-([2,6]naphthyridin-1-ylamino)phenyl] propanoate

Intermediate 22 (360mg) was treated with a solution of trifluoroacetic acid (10ml) and DCM (10ml) and stirred at RT for 2h. The volatiles were removed *in vacuo* and the residue was partitioned between EtOAc (80ml) and saturated aqueous NaHCO₃ (30ml). The phases were separated and the aqueous layer re-extracted with EtOAc (3x30ml). The combined organic extracts were dried (MgSO₄) and evaporated *in vacuo* to afford the title compound as a dark orange viscous oil (280mg, 100%). δH (CDCl₃) 9.18 (1H, s), 8.66 (1H, d, J 5.9Hz), 8.22 (1H, d, J 5.8Hz), 7.67 (1H, d, J 5.9Hz), 7.64 (2H, d, J 8.5Hz), 7.22 (2H, d, J 8.5Hz), 7.19 (1H, d, J 5.8Hz), 4.20 (2H, q, J 7.1Hz), 3.73 (1H, dd, J 7.9, 5.1 Hz), 3.10 (1H, dd, J 13.6, 5.2Hz), 2.87 (1H, dd, J 13.6, 7.9Hz), 1.70 (3H, br s), 1.28 (3H, t, 7.1Hz); m/z (ES⁺, 70V) 337 (MH⁺).

### INTERMEDIATE 24

### Methyl (2S)-2-(t-butoxvcarbonyl)amino]-3-[4-([2,6]naphthyridin-1-yloxy)phenyl]propanoate

To *N*-(*t*-butyloxycarbonyl)-(*S*)-tyrosine methyl ester (1.42g, 4.82mmol) in dry DMF (10ml) was added Intermediate 21 (0.79g, 4.82mmol) and cesium carbonate (1.65g, 5.06 mmol) and the reaction stirred at 45º under N₂ for 2 days. The DMF was evaporated, EtOAc added and washed (3x) with water, dried (MgSO₄), and evaporated *in vacuo.* The residue was chromatographed (SiO₂; 40 to 100% EtOAc/isohexane) to afford the title compound as white foam (1.61g, 82%). δH (CDCl₃) 9.29 (1H, s), 8.76 (1H, d, J 5.74Hz), 8.17 (1H, d, J 5.74Hz), 8.11 (1H, d, J 5.8Hz), 7.43 (1H, d, J 5.8Hz), 7.22-7.18 (3H, m), 5.03 (1H, br s), 4.61 (1H, br s), 3.75 (3H, s), 3.15-3.05 (2H, m), 1.44 (9H, s); m/z (ES⁺, 70V) MH⁺ 424.

### INTERMEDIATE 25

### 3,5-Dichloropyridine-4-carboxylic acid

A solution of 3,5-dichloropyridine (5.00g, 33.8mmol) in THF (25ml) was added to a solution of LDA [generated from nBuLi (2.5M solution in hexanes, 14.9ml, 37.2mmol) and diisopropylamine (4.10g, 5.7ml, 40.6mmol)] in THF (25ml) at -78º under nitrogen, to give a yellow/brown slurry. The reaction was stirred for 30min at -78º then CO₂ gas was bubbled through to give a clear brown solution that slowly gave a precipitate, warmed to RT over 2h, then quenched with water (20ml) and partitioned between Et₂O (100ml) and 1M NaOH (100ml). The aqueous layer was separated and acidified to pH 1 with concentrated hydrochloric acid and then extracted with 10% MeOH in DCM (100ml×3). The combined organic layers were dried (MgSO₄) and the solvent removed under vacuum to give a brown solid that was recrystallised from ethanol and dried under vacuum to give the title compound as pinkish crystals (2.63g, 41%). δH (DMSO-d⁶) 8.74 (2H, s). δC (DMSO-d⁶) 163.5, 147.7, 141.0, 126.7.

### INTERMEDIATE 26

### Ethyl (2S)-2-[(tert-butoxycarbonyl)amino]-3-{4-[(3,5-dichloroisonicotinoyl)amino]phenyl}propanoate

A slurry of the compound of Intermediate 25 (51.2g, 0.267mol) in DCM (195ml) and thionyl chloride (195ml, 2.67mol) was treated with DMF (5 drops) and heated to reflux for 4h. The reaction was concentrated *in vacuo* and azeotroped with toluene (2x50ml) to give a yellow solid which was used without further purification. A solution of ethyl-(*S*)-3-(4-aminophenyl)-2-(t-butoxycarbonyl amino)propionate (130.8g, 0.425mol) in DCM (800ml) was cooled to 0º and treated with NMM (56.0ml, 0.51mol), stirred for 5 minutes and then a solution of the acid chloride (98.3g, 0.468mol) in DCM (200ml) was added dropwise keeping the reaction temperature below 5º. The reaction was stirred for 1h, quenched with NaHCO₃ solution (500ml), the organic layer separated, washed with NaHCO₃ solution (500ml), 10% citric acid solution (500ml) and NaHCO₃ solution (500ml), dried (MgSO₄) and concentrated *in vacuo* to give a yellow solid which was recrystallised (EtOAc/hexane) to give the title compound, (140g, 69%). δH (DMSO d⁶), 8.8 (2H, s), 7.55 (2H, d, J 8.5Hz), 7.23 (2H, d, J 8.5Hz), 4.0 (3H, m), 3.4 (2H, b s), 2.9 (1H, m), 2.8 (1H, m), 1.3 (9H, s), 1.25 (3H, t); m/z (ES⁺, 70V) 504 (MNa⁺).

### INTERMEDIATE 27

### Ethyl (2S)-2-amino-3-{4-[(3,5-dichloroisonicotinoyl)amino] phenyl}propanoate hydrochloride

A solution of the compound of Intermediate 26 (70g, 0.146mol) in EtOAc (500ml) and 1,4-dioxan (50ml) was treated with a solution of HCl in EtOAc (500ml, 3M), and stirred at room temperature for 4h. The reaction was concentrated *in vacuo* to give a yellow solid which was triturated with Et₂O then recrystallised (EtOAc/hexane) to give the title compound (59.3g, 92%). δH (DMSO d⁶), 11.10 (1H, s), 8.70 (2H, s), 7.55 (2H, d, J 8.4Hz), 7.25 (2H, d, J 8.4Hz), 4.10 (3H, m), 3.10 (2H, m), 1.10 (3H, m); m/z (ES⁺, 70V) 382 (MH⁺).

### INTERMEDIATE 28

### 3-Ethoxy-7-oxaspiro[3.5]non-2-en-1-one

Tetrahydropyranyl-4-carboxylic acid (14.7g, 0.11 mol) and DMF (0.5ml) in DCM (150ml) was treated dropwise with oxalyl chloride (1.1eq, 10.9ml, 0.12mol). After 1h the reaction mixture was concentrated *in vacuo* and the residual slurry was diluted with Et₂O (200ml) and the resulting precipitate removed by filtration. The filtrate was treated with ethoxyacetylene (40%w/w solution in hexanes, 1.3eq, 18ml) followed dropwise with triethylamine (25ml, 0.19mol) and the reaction stirred for 11d. Filtration and concentration of the filtrate *in vacuo* followed by chromatography (SiO₂, 5:1 EtOAc:hexanes) gave the title compound as a pale yellow oil (12.1g, 59%). δH (CDCl₃, 300K) 4.85 (1H, s), 4.23 (2H, q, J 7.1Hz), 3.89-3.75 (4H, m), 1.88-1.79 (4H, m), 1.47 (3H, t, J 7.1Hz); m/z (ES⁺, 70V) 182.9 (MH⁺).

### INTERMEDIATE 29

### 7-Oxaspiro[3,5]nonane-1,3-dione

Intermediate 28 (12.1g, 0.67mol) and 2M hydrochloric acid (26ml) were stirred vigorously for 24h at room temperature. The resulting solution was concentrated to dryness and the residual slurry was washed with Et₂O (25ml) to give the title compound as an off-white powder (8.93g, 0.062mol). δH (DMSO d⁶, 300K) 4.80 (2H, s), 3.78 (4H, t, J 5.5Hz), 2.62 (4H t J 5.5Hz); m/z (ES⁺, 70V) 154.9 (MH⁺).

### INTERMEDIATE 30

### 3-Ethoxyspiro[3,6]decan-1-one.

A solution of cycloheptyl carbonyl chloride (10.0g, 0.062mol) and ethoxyacetylene (40%w/w solution in hexanes, 6.0g, 0.083mol, 12ml) in diethylether (50ml) was treated dropwise with triethylamine (20ml, 0.14mol) and the reaction stirred for 5d at room temperature. Filtration and concentration of the filtrate *in vacuo* followed by chromatography (SiO₂, 5:1 EtOAc:hexanes) gave the title compound as a pale yellow oil (10.5g, 0.054mol, 87%). δH (CDCl₃, 300K) 4.78 (1H, s), 4.20 (2H, q J 7.1Hz), 1.94-1.87 (2H, m), 1.83-1.77 (2H, m), 1.71-1.66 (2H, m), 1.63-1.52 (6H, m), 1.45 (3H, t J 7.1Hz); m/z (ES⁺, 70V) 194.9 (MH⁺).

### INTERMEDIATE 31

### Spiro[3.6]decane-1,3-dione

Intermediate 30 (8.5g, 0.044mol) and 2M hydrochloric acid (30ml) was stirred vigorously for 24h at room temperature. The resulting slurry was extracted with EtOAc (3x100ml), the extracts combined and concentrated *in vacuo,* and the resulting solid was recrystallised from diethyl ether to give the title compound as an off-white powder (7.1g, 0.043mol, 95%). δH (DMSO d⁶, 300K) 4.58 (2H, s), 1.75-1.29 (12H, m); m/z (ES⁺, 70V) 166.9 (MH⁺).

### INTERMEDIATE 32

### 7-Acetyl-3-ethoxy-7-azaspiro[3.5]non-2-en-1-one.

A solution of 1-acetyl piperidine-4-carbonyl chloride (5.0g, 26.4mmol) and ethoxyacetylene (4.0g, 55.5mmol) in THF (60ml) was treated dropwise with triethylamine (7.6ml, 55.0mmol). The resulting slurry was stirred at room temperature for 5d prior to filtration and concentration of the filtrate *in vacuo.* Chromatography (SiO₂, 100% EtOAc to 95:5 EtOAc:MeOH) gave the title compound as a white powder (3.97g, 17.8mmol, 67%). δH (CDCl₃, 300K) 4.79 (1H, s), 4.17 (2H, q, J 7.1Hz), 3.87-3.81 (1H, m), 3.56-3.42 (3H, m), 2.02 (3H, s), 1.85-1.67 (4H, m), 1.39 (3H, t, J 7.1Hz); m/z (ES⁺, 70V) 223.9 (MH⁺).

### INTERMEDIATE 33

### 7-Acetyl-7-azaspiro[3.5]nonane-1,3-dione

Intermediate 32 (700mg, 0.31 mmol) and hydrochloric acid (2M, 5ml) were stirred at room temperature for 4h. Concentration of the resulting straw-coloured solution in vacuo gave the title compound as a pale brown watersoluble powder (535mg, 0.027mmol, 87%). m/z (ES⁺, 70V) 195.9 (MH⁺).

### INTERMEDIATE 34

### 3-Ethoxy-7-methoxyspiro[3,5]non-2-en-1-one

Was prepared from 4-methoxy cyclohexanecarbonyl chloride (10g, 52.1mmol) and ethoxyacetylene (7.5g, 0.10mol) according to the method of Intermediate 1 to give the title compound as an approx. 1:1 mixture of isomers, as a pale yellow oil (7.2g, 34.4mmol, 65%). δH (CDCl₃, 300K) 4.81-4.79 (1H, s), 4.22-4.20 (2H q, J 7.1Hz), 3.34-3.32 (3H, s), 3.31-3.22 (1H, m), 2.04-1.56 (8H, m), 1.44-1.43 (3H t, J 7.1Hz); m/z (ES⁺, 70V) 211.0 (MH⁺).

### INTERMEDIATE 35

### 7-Methoxyspiro[3.5]nonane-1,3-dione

Intermediate 34 (5.0g, 23.9mmol) and hydrochloric acid (2M, 20ml) were stirred at room temperature for 18h. The resulting slurry was then diluted with water (50ml) and extracted with EtOAc (3x25ml), the extracts were dried (MgSO₄), filtered, and concentrated *in vacuo.* Recrystallisation from diethylether gave the title compound as an off-white powder (4.06g, 22.4mmol, 94%). δH (CDCl₃, 300K) 3.81 (2H, s), 3.25 (4H, m) 1.96-1.90 (2H, m), 1.86-1.79 (2H, m), 1.73-1.66 (2H, m), 1.64-1.56 (2H, m); m/z (ES⁺, 70V) 182.9 (MH⁺).

### INTERMEDIATE 36

### Ethyl (2S)-2-amino-3-hydroxypropanoate hydrochloride

A mixture of (2*S*)-2-amino-3-hydroxypropanoate (25g, 238mmol) and acetyl chloride (34ml, 476mmol) in absolute ethanol (250ml) was stirred at 50° for 18hr. The volatiles were removed *in vacuo* until the volume was reduced to ~100ml. Upon cooling the resultant precipitate was collected, washed with ether and hexane to give the title compound as a white powder (26.3g, 65 %). δH NMR (DMSO d⁶) 8.47 (3H, br s), 5.58 (1H, dd), 4.20 (2H, q), 4.08 (1H, t), 3.81 (2H, dd), 1.23 (3H, t).

### INTERMEDIATE 37

### Ethyl (2S)-2-[(tert-butoxycarbonyl)amino]-3-hydroxypropanoate

Di-*tert*-butyl dicarbonate (10.26g, 47mmol) was added to a stirred mixture of Intermediate 36 (7.98g, 47mmol) and NaHCO₃ (8.70g, 2.2equiv.) in dioxan/water (1:1) (80ml) and stirred for 4.5hr. The bulk of the solvent was removed *in vacuo* and the resultant slurry was treated with EtOAc (150ml). The inorganics were removed by filtration with EtOAc. The filtrate was washed with 10% aq citric acid (30ml), water (30ml), saturated aq. NaHCO₃ (20ml) and brine (20ml) and dried (Na₂SO₄) and evaporared *in vacuo* to afford the title compound as a colourless oil (10.3g, 94%). δH (CDCl₃) 5.45 (1H, br), 4.36 (1H, br), 4.26 (2H, q), 3.94 (2H, br m), 1.47 (9H, s), 1.28 (3H, t); m/z (ES⁺, 70V) 233 (MH⁺), 256 (MNa⁺).

### INTERMEDIATE 38

### Ethyl (2S)-2-[(tert-butoxycarbonyl)amino]-3-[(methylsulfonyl)oxy]propanoate

Methanesulphonyl chloride (730µL, 9.43mmol) was added to a stirred, ice-bath cooled solution of Intermediate 37 (2.0g, 8.5mmol) and 4-methylmorpholine (1.13ml, 10.29mmol) in dry DCM (30ml) and stirred for 6hr. The solvent was removed *in vacuo* and the residue treated with EtOAc (150ml). The organics were washed with water (40ml), 10% aq citric acid (20ml), water (20ml), sat aq NaHCO₃ (20ml), water (20ml), brine (10ml), dried (Na₂SO₄) and evaporated *in vacuo* to afford a colourless glass which solidified on standing. This was treated with hexane and the solid was filtered, washed with hexane and dried under N₂ atmosphere to give the title compound (2.45g, 92 %). δH (CDCl₃), 5.38 (1H, br), 4.63 (3H, br m), 4.27 (2H, q), 3.03 (3H, s), 1.48 (9H, s), 1.33 (3H, t); m/z (ES⁺, 70V) 333 (MNa⁺).

### INTERMEDIATE 39

### Ethyl (2R)-2-[(tert-butoxycarbonyl)amino]-3-iodopropanoate

Intermediate 38 (1.0g, 3.21 mmol) was stirred in acetone (10ml) in a foil covered flask with sodium iodide (723mg, 4.82mmol) at RT for 18 hr. The acetone was removed *in vacuo* and the residue partitioned between EtOAc (100ml) and water (30ml). The organics washed with brine (10ml), dried (Na₂SO₄) and evaporated *in vacuo* to afford a yellow oil. This was purified by chromatography (SiO₂; 30% Et₂O/hexane) to afford the title compound as a colourless oil which solidified to a white solid (597mg, 54%). δH (CDCI₃) 5.36 (1H, br), 4.50 (1H, br m), 4.27 (3H, m), 3.59 (2H, m), 1.48 (9H, s), 1.33 (3H, t); m/z (ES⁺, 70V) 365 (MNa⁺).

### INTERMEDIATE 40

### Ethyl (2S)-2-[(tert-butoxycarbonyl)amino]-3-(5-nitropyridin-2-yl) propanoate

Zinc dust (100mesh) (581 mg, 8.88mmol) was heated under vacuum and then cooled under N₂. 1,2-dibromoethane (32µL, 0.37mmol) and dry THF (1ml) were added with heating to boiling. Heating was stopped and the mixture stirred for 1 min. This heating and stirring was repeated twice more. TMSCl (66µL, 0.52mmol) was added and stirred at 50° for ~10 mins. Intermediate 39. (2.54g, 7.40mmol) in dry THF (4ml) was added and stirred at ~35-40° for 40 minutes. 2-bromo-5-nitropyridine (1.50g, 7.30mmol) and PdCl₂(PPh₃)₂ (260mg, 0.37mmol) and dry THF (2ml) were added and the reaction mixture stirred at 35° for 2hr. The reaction mixture was partitioned between EtOAc (150ml) and sat. aq. NH₄Cl (40ml). The phases were separated and the aqueous phase re-extracted with EtOAc (50ml). The combined organic extracts were washed with brine (10ml), dried (Na₂SO₄) and evaporated in vacuo to afford a dark straw coloured oil. Purification by chromatography (SiO₂; 30-70% Et₂O/hexane) afforded the title compound as a yellow oil (1.52g, 61%). δH (CDCl₃), 9.34 (1H, s), 8.39 (1H. d), 7.38 (1H, d), 5.58 (1H, br), 4.75 (1H, br m), 4.20 (2H, m), 3.47 (2H, m), 1.42 (9H, s), 1.23 (3H, t); m/z (ES⁺, 70V) 339 (MH⁺).

### INTERMEDIATE 41

### Ethyl (2S)-3-(5-aminopyridin-2-yl)-2-[(tert-butoxycarbonyl)amino]propanoate

A stirred solution of Intermediate 40 (1.16g, 3.42mmol) in absolute EtOH (20ml) was hydrogenated at atmospheric pressure with 10% Pd on charcoal (100mg) for 3.5hrs. The catalyst was removed by filtration through a celite pad with DCM. The filtrate was evaporated *in vacuo.* The crude title compound was obtained as a straw-coloured oil (1.03g, 98%) and used without further purification. δH (CDCl₃), 8.01 (1H, s), 6.92 (2H, s), 5.83 (1H, br), 4.59 (1H, br m), 4.13 (2H, m), 3.63 (2H, br), 3.15 (2H, br), 1.43 (9H, s), 1.21 (3H, t); m/z (ES⁺, 70V) 309 (MH⁺).

### INTERMEDIATE 42

### Ethyl(2S)-2-[(tert-butoxycarbonyl)amino]-3-{5-[(3,5-dichloroisonicotinoyl)amino]pyridin-2-yl}propanoate.

3,5-Dichloroisonicotinoyl chloride (0.51 ml, 3.61 mmol) was added to a stirred, ice-bath cooled solution of Intermediate 41 (1.06g, 3.43mmol) and dry pyridine (0.55ml) in dry DCM (20ml) and stirred at RT for 1 hr. After evaporation of the solvent the residue was dissolved in EtOAc (80ml) and washed with satuarted sodium bicarbonate (20ml), water (10ml), brine (10ml), then dried (Na₂SO₄) filtered and concentrated *in vacuo* to a red-brown glass. Chromatography (silica, 75% Et₂O/DCM) afforded a the title compound as tan-coloured solid (1.25g, 75%). δH NMR (DMSO d⁶) 8.69 (2H, s), 8.58 (1H, s), 7.92 (1H, d), 7.20 (1H, d), 4.26 (1H, m), 3.97 (2H, m), 2.93 (2H, m), 1.21 (9H, s), 1.01 (3H, t); m/z (ES⁺, 70V) 483 (MH⁺).

### INTERMEDIATE 43

### Ethyl(2S)-2-amino-3-{5-[(3,5-dichloroisonicotinoyl)amino]pyridin-2-yl}propanoate

Acetyl chloride (6ml) was added to absolute EtOH (20ml) and stirred for 15 min., cooled to RT, then intermediate 42 (2.74g, 5.67mmol) added with and stirring for 3.5hrs. The solvent removed *in vacuo.* The resultant yellow residue was treated with sat. sodium bicarbonate (10ml) and solid sodium bicarbonate till neutralised. Extraction with EtOAc (4 x 50 ml), drying (Na₂SO₄) and concentrated affordet the title compound as a straw-coloured foam (2.1g, 97%). δH NMR (d⁶ DMSO) 8.67 (2H, s), 8.56 (1H, s), 7.85 (1H, d), 7.16 (1H, d), 3.89 (2H, q), 3.57 (1H, dd), 2.86 (1H, dd), 2.82 (1H, dd), 1.73 (2H, br), 1.00 (3H, t). m/z (ES⁺, 70V) 383 (MH⁺).

### INTERMEDIATE 44

### 3-Ethoxy-7,7-dioxo-7λ⁶-thia-spiro[3.5]non-2-en-1-one

A solution of 1,1-dioxo-hexahydro-1λ⁶-thiopyran-4-carboxylic acid (10.2g, 57.3mmol) [Prepared according to the procedure of [Org. Prep. Proc. Int. 1977, 94] and DMF (0.3ml) in DCM (120ml) at rt, was treated dropwise with oxalyl chloride and the resulting slurry stirred for 3d. The crude reaction was then concentrated *in vacuo* to give an oil which was redissolved in THF (100ml), treated with ethoxyacetylene (10ml, 50%w/w) and triethylamine (10ml) and the resulting slurry stirred for 10d at rt. Filtration and concentration of the filtrate *in vacuo* gave a crude oil which was purified by chromatography (SiO2, 30% EtOAc:hexanes) to give the title compound as a yellow oil (8.9g, 38.6mmol, 67%). δH (CDCl₃, 300K) 4.88 (1H, s), 4.27 (2H, q, J 7.1Hz), 3.44-3.37 (2H, m), 3.13-3.05 (2H, m), 2.47-2.40 (2H, m), 2.35-2.29 (2H, m), 1.48 (3H, t, J 7.1Hz), m/z (ES⁺, 70V) 230.9 (MH⁺).

### INTERMEDIATE 45

### 3-Hydroxy-7,7-dioxo-7λ⁶-thia-spiro[3,5]non-2-en-1-one

Intermediate 44 (8.6g, 37.4mmol) was stirred with 1M HCl (100ml) for 3d and the resulting solution concentrated *in vacuo.* The residual solid was triturated with EtOAc to give the title compound as an off-white solid (5.1g, 25.2mmol, 68%). m/z (ES⁺, 70V) 202.9 (MH⁺).

### INTERMEDIATE 46

### 3-Ethoxy-spiro[3,4]octa-2,6-dien-1-one

A solution of cyclopent-3-ene carboxylic acid (4.0g, 36.0mmol) and DMF (0.25ml) in DCM (30ml) at 0º was treated dropwise with oxalyl chloride (3.5ml, 39.0mmol). After 2h the reaction mixture was concentrated *in vacuo,* the residual slurry diluted with Et₂O (100ml) and the resulting precipitate removed by filtration and the filtrate concentrated *in vacuo.* The resulting oil was diluted with Et₂O (50ml), treated with ethoxyacetylene (40%w/w solution in hexanes, 10ml) followed dropwise with triethylamine (6ml, 44.0mmol) and the reaction stirred for 7d. Filtration and concentration of the filtrate *in vacuo* followed by chromatography (SiO₂, 5:1 EtOAc:hexanes) gave the title compound as a pale yellow oil (4.3g, 73%). m/z (ES⁺, 70V) 164.9 (MH⁺).

### INTERMEDIATE 47

### 3-Hydroxy-spiro[3.4]octa-2,6-dien-1-one

Intermediate 46 (2.0g, 12.0mmol) and 2M hydrochloric acid (5ml) were stirred vigorously for 24h at room temperature. The resulting solution was extracted with EtOAc (25ml), the extracts dried (MgSO₄), filtered and concentrated *in vacuo* to give the title compound as an pale brown powder (1.07g, 7.9mmol, 65%). δH (DMSO d⁶, 300K) 5.54 (4H, s), 4.57 (2H, s), 2.52 (2H, s). m/z (ES⁺, 70V) 136.9 (MH⁺).

### INTERMEDIATE 48

### (+,-)-3-Ethoxy-4-methyl-4-phenyl-cyclobut-2-enone

A solution of (+,-) 2-phenylpropionic acid (10.0g, 0.66mmol) and DMF (0.3ml) in DCM (150ml) was treated dropwise with oxalyl chloride (6.4ml, 0.72mmol). After 1h the reaction mixture was concentrated *in vacuo,* the residual slurry diluted with Et₂O (200ml) and the resulting precipitate removed by filtration. The filtrate was treated with ethoxyacetylene (40%w/w solution in hexanes, 18ml) followed dropwise with triethylamine (25ml, 0.19mol) and the reaction stirred for 7d at rt. Filtration and concentration of the filtrate *in vacuo* followed by chromatography (SiO₂, 5:1 EtOAc:hexanes) gave the title compound as a pale yellow oil (6.1g, 45%). δH (CDCl₃, 300K) 7.45-7.24 (5H, m), 5.01 (1H, s), 4.31 (2H, J 7.1Hz), 1.67 (3H, s), 1.51 (3H, t, J 7.1Hz). m/z (ES⁺, 70V) 202.9 (MH⁺).

### INTERMEDIATE 49

### (+,-)-3-Hydroxy-4-methyl-4-phenyl-cyclobut-2-enone

Intermediate 48 (4.5g, 22.2mmol) was hydrolysed according to the method of Intermediate 29 to give the title compound as an off-white powder (3.29g, 18.9mmol, 85%); δH (CDCl₃, 300K) 7.53-7.21 (5H, m), 4.04 (1H, d, J 21.7Hz), 3.93 (1H, d, J 21.7Hz), 1.62 (3H, s). m/z (ES⁺, 70V) 174.9 (MH⁺).

### INTERMEDIATE 50

### Cyclohexylethynyloxy-triisopropyl-silane

Prepared according to the method of Kowalski, Sankar Lal and Haque, JACS, 1986, 108, 7127-7128.

### INTERMEDIATE 51

### 2-Cyclohexyl-3-triisopropylsilanyloxy-spiro[3.5]non-2-en-1-one;

To a stirred solution of the compound of example 89 (5.6g, 20 mmol) in t-butylmethyl ether (50ml) was added cyclohexylcarbonyl chloride (5.3ml, 40 mmol) and triethylamine (13 ml, 100mmol). The mixture was stirred under reflux for 24 hours, allowed to cool and filtered to remove triethylammonium chloride. The filtrate was concentrated under reduced pressure and chromatographed on silica gel, mobile phase 3% EtOAc in hexane to afford the title compound as a brown oil (5.8g, 74%). m/z (ES⁺, 70V) 235.2 (MH⁺ of desilylated compound).

### INTERMEDIATE 52

### 2-Cyclohexyl-spiro[3.5]nonane-1,3-dione

Intermediate 51 was stirred with 5 volumes of 2M hydrochloric acid for 14 days and worked up in a similar manner to Intermediate 4 to afford the title compound as a white crystalline solid in 40% yield. m/z (ES⁺, 70V) 235.0 (MH⁺).

### INTERMEDIATE 53

### 1-Butoxyprop-1-yne

Prepared according to the method of Nooi and Arens; Recl. Trav. Chim. Pays-Bas; 78; 1959; 284 - 287.

### INTERMEDIATE 54

### 1-Butoxybut-1-yne

Prepared in a similar manner to Intermediate 53 from the appropriate starting materials.

### INTERMEDIATE 55

### 1-Butoxypent-1-yne

Prepared in a similar manner to Intermediate 53 from the appropriate starting materials.

### INTERMEDIATE 56

### 3-Butoxy-2,4,4-trimethyl-cyclobut-2-enone

Prepared in a similar manner to Intermediate 1 from Intermediate 53 in 45% yield. δH NMR (d CHCl₃) 4.35 (2H, t, J 6.5Hz), 1.79 (2H, m), 1.66 (3H, s), 1.50 (2H, m), 1.22 (6H,s), 0.99 (3H, t, J 7.4Hz). m/z (ES⁺, 70V) 183.0 (MH⁺)

### INTERMEDIATE 57

### 3-Butoxy-2-ethyl-4,4-dimethyl-cyclobut-2-enone

Prepared in a similar manner to Intermediate 1 from Intermediate 54 in 56% yield. δH NMR (d CHCl₃) 4.31 (2H, t, J 6.5Hz), 2.07 (2H, q, J 7.6Hz), 1.80 (2H, m), 1.52 (2H, m), 1.23 (6H, s), 1.10 (3H, t, J 7.6Hz), 1.00 (3H, t, J 7.3Hz). m/z (ES⁺, 70V) 197.0 (MH⁺).

### INTERMEDIATE 58

### 3-Butoxy-4,4-dimethyl-2-propyl-cyclobut-2-enone

Prepared in a similar manner to Intermediate 1 from Intermediate 55 in 51% yield. δH NMR (d CHCl₃) 4.30 (2H, t, J 6.5Hz), 2.04 (2H, q, J 7.4Hz), 1.75 (2H, m), 1.50 (4H, m), 1.23 (6H, s), 1.00 (3H, t, J 7.4Hz), 0.92 (3H, t, J 7.4Hz). m/z (ES⁺, 70V) 211.0 (MH⁺).

### INTERMEDIATE 59

### 2,2,4-Trimethyl-cyclobutane-1,3-dione

Prepared in a similar manner to Intermediate 2 from Intermediate 56 in 85% yield. δH (d⁶ DMSO) 1.36 (3H, s), 1.07 (6H, s). m/z (ES⁺, 70V) 126.9 (MH⁺).

### INTERMEDIATE 60

### 4-Ethyl-2,2-dimethyl-cyclobutane-1,3-dione

Prepared in a similar manner to Intermediate 2 from Intermediate 57 in 70% yield. δH (d⁶ DMSO) 1.85 (2H, q, J 7.6Hz), 1.07 (6H, s), 0.95 (3H, t, J 7.6Hz). m/z (ES⁺, 70V) 140.9 (MH⁺).

### INTERMEDIATE 61

### 2,2-Dimethyl-4-propyl-cyclobutane-1,3-dione<p>

Prepared in a similar manner to Intermediate 2 from Intermediate 58 in 64% yield. δH (CDCl₃) 1.96 (2H, t, J 7.3 Hz), 1.50 (2H, m), 1.28 (6H, s), 0.90 (3H, t, J 7.3Hz). m/z (ES⁺, 70V) 154.9 (MH⁺).

### INTERMEDIATE 62

### 3-Butoxy-2-methyl-spiro[3.5]non-2-en-1-one

Prepared in a similar manner to Intermediate 1 from Intermediate 53 in 23% yield. δH (CDCl₃) 4.34 (2H, t, J 6.5Hz), 1.77-1.25 (17H, m), 1.00 (3H, t, J 7.4Hz). m/z (ES⁺, 70V) 223.0 (MH⁺)

### INTERMEDIATE 63

### 3-Butoxy-2-propyl-spiro[3.5]non-2-en-1-one

Prepared in a similar manner to Intermediate 1 from Intermediate 55 in 67% yield. δH (CDCl₃) 4.31 (2H, t, J 6.4Hz), 2.07 (2H, t, J 7.2Hz), 1.80-1.40 (13H, m), 1.00 (3H, t, J 7.1Hz), 0.93 (3H, t, J 7.3Hz). m/z (ES⁺, 70V) 251.1 (MH⁺)

### INTERMEDIATE 64

### 2-Methyl-spiro[3.5]nonane-1,3-dione

Prepared in a similar manner to Intermediate 2 from intermediate 62 in 90% yield. δH (d⁶ DMSO) 1.56 (10H, m), 1.37 (3H, s). m/z (ES⁺, 70V) 166.9 (MH⁺).

### INTERMEDIATE 65

### 2-Propyl-spiro[3.5]nonane-1,3-dione

Prepared in a similar manner to Intermediate 2 from Intermediate 63 in 64% yield. δH (d⁶ DMSO) 1.82 (2H, t, J 7.2Hz), 1.58 (8H, m), 1.41 (2H, m), 1.39 (2H, q, J 7.4Hz), 0.85 (3H, t, J 7.3Hz). m/z (ES⁺, 70V) 195.1 (MH⁺).

### INTERMEDIATE 66

### 3-Butoxy-2-methyl-7-oxa-spiro[3.5]non-2-en-1-one

Prepared in a similar manner to Intermediate 1 from Intermediate 53 in 48% yield. δH (CDCl₃) 4.30 (2H, t, J 6.5Hz), 3.76 (4H, m), 1.70 (6H, m), 1.63 (3H, s), 1.36 (2H, m), 0.92 (3H, t, J 7.4Hz). m/z (ES⁺, 70V) 225.0 (MH⁺).

### INTERMEDIATE 67

### 3-Butoxy-2-propyl-7-oxa-spiro[3.5]non-2-en-1-one

Prepared in a similar manner to Intermediate 1 from Intermediate 55 in 79% yield. δH (CDCl₃) 4.33 (2H, t, J 6.4Hz), 3.81 (4H, m), 2.09 (2H, t, J 7.7Hz), 1.81 (6H, m), 1.50 (4H, m), 1.00 (3H, t, J 7.4Hz), 0.94 (3H, t, J 7.3Hz). m/z (ES⁺, 70V) 253.0 (MH⁺).

### INTERMEDIATE 68

### 2-Methyl-7-oxa-spiro[3.5]nonane-1,3-dione

Prepared in a similar manner to Intermediate 2 from Intermediate 66 in 51% yield. δH NMR (d⁶ DMSO) 3.67 (4H, m), 1.68 (4H, m), 1.40 (3H, s). m/z (ES⁺, 70V) 168.9 (MH⁺).

### INTERMEDIATE 69

### 2-Propyl-7-oxa-spirof3.51nonane-1,3-dione

Prepared in a similar manner to Intermediate 2 from Intermediate 67 in 79% yield. m/z (ES⁺, 70V) 196.9 (MH⁺).

### INTERMEDIATE 70

### (3-Ethoxy-prop-2-ynyl)-benzene

To a solution of ethoxy acetylene (9.95g of 50% w/w. solution in hexanes, 70mmol) in THF (100ml) at -78° was added n-butyl lithium (31 ml of 2.5M solution in hexanes, 78mmol). The mixture was stirred at this temperature for 2h. prior to the addition of HMPA (20ml), stirring was continued for a further 15min. before the addition of benzyl bromide (9.2ml). The reaction mixture was allowed to warm to room temperature overnight before partitioning between EtOAc (300ml) and water (200ml). The organics were separated, washed with water (5 x 200ml), brine (200ml), dried (Na₂SO₄), filtered and concentrated *in vacuo* to give the title compound as a mobile brown oil (11.1g, 99%). δH (300MHz, CDCl₃) 7.15-7.57 (5H, m), 4.12 (2H, q, J=7.1Hz), 3.60 (2H, s), 1.41 (3H, t, J=7.1Hz). m/z (ES⁺, 70V) MH⁺ 161.

### INTERMEDIATE 71

### 2-Benzyl-3-ethoxy-4,4-dimethyl-cyclobut-2-enone

To a solution of Intermediate 70 (11g, 68mmol) in THF (200ml) at room temperature was added isobutyryl chloride (11ml) and triethylamine (19ml). The mixture was stirred at this temperature for 65h. filtered, partitioned between EtOAc (400ml) and water (200ml), the organics were separated, washed with brine (200ml), dried (Na₂SO₄), filtered and concentrated *in vacuo.* The crude product was purified by column chromatography (SiO₂: eluant 3:2, hexane:diethyl ether) to give the title compound as a viscous clear oil (11.8g, 75%). δH (300MHz, CDCl₃) 7.18-7.32 (5H, m), 4.27 (2H, q, J=7.1Hz), 3.43(2H, s), 1.36(3H, t, J=7.1Hz), 1.28(6H, s). m/z (ESI, 70V) MH⁺ 231.

### INTERMEDIATE 72

### 4-Benzyl-2,2-dimethyl-cyclobutane-1,3-dione

Intermediate 71 (11.8g, 51.3mmol) was stirred in HCl (200ml, 6M aq.) at room temperature overnight. The solid precipitate was filtered and washed on the sinter with hexane and diethyl ether to give the title compound as a white powder (9.8g, 95%). δH (300MHz, d6 DMSO) □7.13-7.29 (5H, m), 3.20 (2H, s), 1.11 (6H, s). m/z (ESI, 70V). MH+ 213.

### INTERMEDIATE 73

### 4-Bromomethyl-5-methyl-2-oxo-1,3-dioxolene

Prepared according to the method of Sakamoto F., Ikeda S. and Tsukamoto G., Chem. Pharm. Bull., 1984, 32, 2241-2248.

### INTERMEDIATE 74

### Ethyl (2S)-2-tert-Butoxycarbonylamino-3-{4-[(3,5-dichloro-1-oxypyridine-4-carbonyl)aminolphenyl} propanoate

Intermediate 26 (500mg, 1.04mmol) and mCPBA (493mg, 2.0mmol) in DCM (10ml) were stirred together at room temperature for 48hrs. After this time sodium sulfite (10% solution in water, 20ml) was added with stirring for 5 mins, prior to separating between DCM (50ml) and sodium bicarbonate solution (50 ml). The organics were washed with sodium bicarbonate solution (2x50ml) and water (1x50ml), dried (MgSO₄) and reduced in vacuo. The resulting orange solid was recrystalised from EtOAc/hexane to give title compound as a of pale yellow powder (350mg). δH (DMSO d6) 7.78 (2H, s), 6.78 (2H, d, J 8.3Hz), 6.46 (2H, d, J 8.4Hz), 3.55 (1H, m), 3.36 (2H, q, J 7.1Hz), 2.31 (1H, dd J 5.8Hz, 13.8Hz), 2.31 (1H, dd, J 13.6, 8.9Hz), 0.60 (9H, s), 0.43 (3H, t, 3H).

### INTERMEDIATE 75

### (S)-2-Amino-3-{4-[(3,5-dichloro-1-oxy-pyridine-4-carbonyl)-amino]-phenyl}-propionic acid ethyl ester

Intermediate 74 (330mg, 0.55mmol) and HCl in EtOAc (2.6M) were stirred together at room temperature overnight. After this time the formed precipitate was filtered off, washed with Et₂O, (3x50ml) and then made basic by separating between EtOAc (50ml) and sodium bicarbonate solution (50ml). The organics were dried (MgSO₄) and reduced *in vacuo* to give title compound as white solid (185mg). δH (CD₃OD) 8.40 (2H, s), 7.43 (2H, d, J 8.6Hz), 7.05 (2H, d, J 8.6Hz), 3.98 (2H, q, J 7.1Hz), 2.85 (2H, m), 1.04 (3H, t, J 7.1Hz).

### EXAMPLE 1

### Ethyl (2S)-2-[(4,4-dimethyl-3-oxo-1-cyclobutenyl)amino]-3-[4-([2,7]naphthyridin-1-yloxy)phenyl]propanoate

A solution of 3-hydroxy-4,4-dimethyl-2-cyclobutenone (57mg, 0.51mmol) [prepared according to the method of Wasserman, H.H. et al J. Org. Chem, 38, 1451-1455, (1973)] and the ethyl ester prepared according to the method used to prepare Intermediate 13 (164mg, 0.51mmol), in 1,2-dichloroethylene (5ml), was stirred at room temperature for 72h. The volatiles were removed *in vacuo* and the residue chromatographed (SiO₂; EtOAc) affording the title compound as a white solid (188mg, 0.45mmol, 89%). δH (CDCl₃, 300K) 9.92 (1H, s), 8.75 (1H, d, J 5.7Hz), 8.60 (1H, d, J 8.6Hz), 8.04 (1H, d, J 5.8Hz), 7.82 (1H, d, J 5.6Hz), 7.47 (1H, d, J 5.8Hz), 7.27 (2H, d, J 8.5Hz), 7.16 (2H, d, J 8.5Hz), 4.31 (1H, s), 4.30-4.21 (1H, m), 3.68-3.63 (2H, q, J 7.1Hz), 3.17 (1H, dd, J 13.6, 9.4Hz), 2.95 (1H, dd, J 5.0, 13.6Hz), 1.01 (3H, s), 0.93 (3H, s). m/z (ES⁺, 70V) 418.1(MH⁺).

### EXAMPLE 2

### (2S)-2-[(4,4-Dimethyl-3-oxo-1-cyclobutenyl)aminol-3-[4-([2,7]naphthyridin-1-yloxy)phenyl]propanoic acid

The compound of Example 1 (127mg, 0.31mmol) in THF (5ml) was treated in a single portion with LiOH.H₂O (13mg, 0.32mmol) in H₂O (1ml) and the reaction stirred at room temperature for 2h. The reaction was then quenched by the addition of HOAc (glacial, 1ml) and the volatiles removed *in vacuo.* Water (10ml) was then added to the residual foam and stirred vigorously to effect precipitation. The precipitate was then collected by vacuum filtration and the residue washed with water (2 x 5ml). Drying under vacuum gave the title compound as a fine white solid (108mg, 0.27mmol, 88%). δH (DMSO d⁶, 300K) 9.67 (1H, s), 8.78 (1H, d, J 5.7Hz), 8.51 (1H, d, J 8.6Hz), 8.09 (1H, d, J 5.8Hz), 7.86 (1H, d, J 5.6Hz), 7.50 (1H, d, J 5.7Hz), 7.21 (2H, d, J 8.4Hz), 4.17 (2H, d, J 8.4Hz), 4.34 (1H, s), 4.18-4.14 (1H, m), 3.21 (1H, dd, J 4.9, 13.9Hz), 2.98 (1H, dd, J 13.9, 9.3Hz), 1.06 (3H, s), 0.99 (3H, s). m/z (ES⁺, 70V) 404.1 (MH⁺).

### EXAMPLE 3

### Ethyl(2S)-2-[(4,4-dimethyl-3-oxo-1-cyclobutenyl)amino]-3-[4-([2,6]naphthyridin-1-ylamino)phenyl]propanoate

A solution of 3-hydroxy-4,4-dimethyl-2-cyclobutenone (58mg, 5.1 mmol) and Intermediate 23 (1.01g, 2.7mmol) in DCM (15ml), was stirred at room temperature for 48h. The volatiles were removed *in vacuo* and the residue chromatographed (SiO₂; EtOAc) affording the title compound as a white powder (990mg, 2.3mmol, 88%). δH (CDCl₃, 300K) 9.33 (1H, s), 9.24 (1H, s), 8.69 (1H, d, J 5.9Hz), 8.63 (1H, d, J 8.5Hz), 8.42 (1H, dd, J 5.9, 0.8Hz), 8.15 (1H, dd, J 5.7, 1.3Hz), 7.85-7.80 (3H, m), 7.31-7.22 (4H, m), 4.39 (1H, s), 4.24-4.21 (1H, m), 4.17 (2H, q, J 7.1Hz), 3.15 (1H, dd, J 13.8, 5.6Hz), 3.00 (1H, dd, J 13.8, 9.0Hz), 1.19 (3H, t, J 7.1Hz), 1.11 (3H, s), 1.05 (3H, s). m/z (ES⁺, 70V) 431.1 (MH⁺).

### EXAMPLE 4

### (2S)-2-[(4,4-dimethyl-3-oxo-1-cyclobutenyl)amino]-3-[4-([2,6]naphthyridin-1-ylamino)phenyl]propanoic acid

The compound of Example 3 (500mg, 1.16mmol) was hydrolysed in a similar manner to the method of Example 2 to give the title compound as a fine white solid (421 mg, 1.04mmol, 90%). δH (DMSO d⁶, 300K) 9.21 (1H, s), 9.12 (1H, s br), 8.66 (1H, d, J 5.8Hz), 8.38 (1H, d, J 5.8Hz), 8.18 (2H, m), 7.81 (2H, d, J 7.9Hz), 7.27 (2H, d, J 7.9Hz), 7.26 (1H, obscured s), 4.36 (1H, s), 4.13-4.07 (1H, m), 3.20 (1H, dd, J 14.0, 5.1Hz) 3.02 (1H, dd, J 41.0, 8.7Hz), 1.13 (3H, s), 1.09 (3H, s). m/z (ES⁺, 70V) 403.0 (MH⁺).

### EXAMPLE 5

### Ethyl (2S)-2-[(4,4-dimethyl-3-oxo-1-cyclobutenyl)amino]-3-{4-[(3,5-dichloroisonicotinoyl)amino]phenyl}propanoate

A solution of 3-hydroxy-4,4-dimethyl-2-cyclobutenone (58mg, 0.52mmol) [prepared according to the method of Wasserman, H.H. et al J. Org. Chem, 38, 1451-1455, (1973)] and the free base of Intermediate 27 (200mg, 5.2mmol), in DCM (5ml), was stirred at room temperature for 48h. The volatiles were removed *in vacuo* and the residue chromatographed (SiO₂; EtOAc) to give the title compound as a white solid (230mg, 0.48mmol, 93%). δH (CDCl₃, 300K) 8.48 (2H, s), 8.10 (1H, s), 7.51 (2H, d, J 8.2Hz), 7.04 (2H, d, 8.2Hz), 5.91 (1H, s), 4.43 (1H, s), 4.22 (2H, q, J 7.1Hz), 3.17 (1H, dd, J 14.0, 5.1Hz), 3.05 (1H, dd, J 14.0, 5.8Hz), 1.28 (3H, t, J 7.1Hz), 1.15 (3H, s), 1.14 (3H, s). m/z (ES⁺, 70V) 476.0 and 478.0 (MH⁺).

### EXAMPLE 6

### (2S)-2-[(4,4-dimethyl-3-oxo-1-cyclobutenyl)amino]-3-{4-[(3,5-dichloroisonicotinoyl)amino]phenyl}propanoic acid

The compound of Example 5 (100mg, 0,21mmol) was hydrolysed in a similar manner to the method of Example 2 to give the title compound as a fine white solid (76mg, 0.17mmol, 81%). δH (DMSO d⁶, 350K) 10.5 (1H, s), 8.74 (2H, s), 7.80 (1H, broad s), 7.53 (2H, d, J 8.1Hz), 7.25 (2H, d, J 8.1Hz), 7.26 (1H, obscured s), 4.30 (1H, s), 3.88 (1H, m), 3.16 (1H, dd, J 13.5, 4.9Hz), 3.01 (1H, dd, J 13.5, 3.8Hz), 1.11 (3H, s), 1.07 (3H, s). m/z (ES⁺, 70V) 448.0 and 449.9 (MH⁺).

### EXAMPLE 7

### Methyl (2S)-2-[(4R,S)-4-methyl-3-oxo-4-propyl-1-cyclobutenyl]amino-3-{4-[(3-methyl[2,7]naphthyridin-1-yl)oxylphenyl}propanoate

A solution of Intermediate 2 (187mg, 1.33mmol) and Intermediate 20 (450mg, 1.2mmol), in chloroform (10ml), was stirred at 55° for 48h. The volatiles were removed *in vacuo* and the residue chromatographed (SiO₂; EtOAc) to give the title compound as a white solid (539mg, 1.17mmol, 91%) as an approx. 1:1 mixture of diastereomers. δH (CDCl₃, 300K) 9.69 (1H, s), 8.69 (1H, d, J 5.7Hz), 7.51 (1H, dd, J 9.3, 0.5Hz), 7.19-7.11 (4H, m), 5.79 (1H, d, J 7.3Hz), 4.64 (1H, s), 4.36-4.30 (1H, m), 3.84 and 3.82 (3H, s, diastereomeric CH₃), 3.31-3.15 (2H, m), 2.45 (3H, s), 1.59-1.54 (1H, m), 1.50-14 (1H, m), 1.34-1.23 (2H, m), 1.28 and 1.27 (3H, s, diastereomeric CH₃), 0.91-0.86 (3H, m). m/z (ES⁺, 70V) 460.1 (MH⁺).

### EXAMPLE 8

### (2S)-2-[(4R,S)-4-Methyl-3-oxo-4-propyl-1-cyclobutenyl]amino-3-{4-[(3-methyl[2,7]naphthyridin-1-yl)oxy]phenyl}propanoic acid

The compound of Example 7 (230mg, 0.5mmol) was hydrolysed in a similar manner to the method of Example 2 to give the title compound as a fine white solid (198mg, 0.44mmol, 79%) as an approx. 1:1 mixture of diastereomers. δH (DMSO d⁶, 300K) 13.0 (1H, s), 9.60 (1H, d, J 9.7Hz), 8.72 (1H, d, J 5.6Hz), 8.49-8.43 (1H, m NH), 7.76 (1H, d, J 4.7Hz), 7.41-7.34 (2H, m), 7.27-7.21 (2H, m), 4.47 and 4.43 (1H, s), 4.19-4.13 (1H, m), 3.29-3.23 (3H, s, and 1H as obscured m), 3.02-2.97 (1H, m), 2.36 and 2.35 (3H, s), 1.50-1.10 (4H, m), 1.08 and 0.98 (3H, s), 0.84-0.63 (3H, m), m/z (ES⁺, 70V) 446.1 and 447.1 (MH⁺).

### EXAMPLE 9

### Ethyl (2S)-2-[(4,4-dipropyl-3-oxo-1-cyclobutenyl)amino]-3-[4-([2,7]naphthyridin-1-yloxy)phenyl]propanoate

A solution of Intermediate 4 (180mg, 1.07mmol) and the ethyl ester of Intermediate 13 (362mg, 1.07mmol), in chloroform (7ml), was stirred at room temperature for 96h. The volatiles were removed *in vacuo* and the residue chromatographed (SiO₂; EtOAc) to give the title compound as a white solid (406mg, 0.83mmol, 78%). δH (CDCl₃, 300K) 9.72 (1H, s), 8.71 (1H, d J 5.7Hz), 8.04 (1H, d, J 5.8Hz), 7.55 (1H, d, J 5.7Hz), 7.22-7.16 (4H, m), 5.67 (1H, d, J 7.9Hz), 4.64 (1H, s), 4.26-4.16 (3H, m), 3.20 (1H, dd, J 14.1, 5.7Hz), 3.11 (1H, dd, J 14.1, 6.6Hz), 1.58-1.01 (8H, m), 0.81 (6H, t, J 7.0Hz). m/z (ES⁺, 70V) 488.1 and 489.1 (MH⁺).

### EXAMPLE 10

### (2S)-2-[(3-Oxo-4,4-dipropyl-1-cyclobutenyl)aminol]3-[4-([2,7]naphthyridin-1-yloxy)phenyl]propanoic acid

The compound of Example 9 was hydrolysed in a similar manner to the method of Example 2 to give the title compound as a fine off-white powder (35mg, 0.07mmol, 19%), δ□H (DMSO d⁶, 350K) 9.68 (1H, s), 8.83 (1H, d, J 5.7Hz), 8.37 (1,d, J 8.5Hz), 8.14 (1H, d, J 5.8Hz), 7.91 (1H, d, J 5.7Hz), 7.55 (1H, d, J 5.8Hz), 7.39 (2H, d, J 8.4Hz), 7.28 (2H, d, J 8.4Hz), 4.53 (1H, s), 4.14 (1H, dd, J 9.8, 4.3Hz), 3.25 (1H, dd, J 14.0, 4.6Hz), 3.0 (1H, dd, J 10.3, 14.0Hz), 1.50-0.64 (14H, m). m/z (ES⁺, 70V) 460.1 and 461.1 (MH⁺).

### EXAMPLE 11

### Ethyl (2S)-2-[(4R,S)-4-methyl-3-oxo-4-propyl-1-cyclobutenyl]amino-3-[4-([2,7]naphthyridin-1-yloxy)phenyl]propanoate

A solution of Intermediate 2 (300mg, 2.1mmol) and the ethyl ester of Intermediate 13 (724mg, 2.14mmol), in DCM (15ml), was stirred at room temperature for 24h. The reaction was then diluted with DCM (30ml) and distilled water (20ml) and washed successively with 1M aqueous hydrochloric acid (30ml) water (30ml) and saturated, aqueous sodium hydrogen carbonate (30ml). The organic layer was then dried (MgSO₄), filtered and concentrated *in vacuo.* The residual foam was chromatographed (SiO₂; EtOAc) to give the title compound as a white powder (827mg, 1.8mmol, 84%) as an approx. 1:1 mixture of diastereomers. δ□H (CDCl₃, 300K) 9.72 (1H, s), 8.71 (1H, d, J 5.7Hz), 8.04 (1H, d, J 5.8Hz), 7.55 (1H, d, J 5.7Hz), 7.22-7.12 (5H, m), 5.80 (1H, d, J 7.6Hz), 4.57 (1H, s), 4.28-4.20 (3H, m), 3.25-3.07 (2H, m), 1.57-1.21 (7H, m), 1.18 and 1.17 (3H, s) 0.84-0.78 (3H, m). m/z (ES⁺, 70V) 460.1 (MH⁺) and 482.0 (MNa⁺).

### EXAMPLE 12

### (2S)-2-[(4R,S)-4-Methyl-3-oxo-4-propyl-1-cyclobutenyl]amino-3-[4-([2,7]naphthyridin-1-yloxy)phenyl]propanoic acid

The compound of Example 11 (600mg, 1.31 mmol) was hydrolysed in a similar manner to the method of Example 2 to give the title compound as a fine white solid (520mg, 1.21 mmol, 92%) as an approx. 1:1 mixture of diastereomers. δH (DMSO d⁶, 300K) 9.61 and 9.58 (1H, s), 8.72 (1H, d, J 5.7Hz), 8.39-8.33 (1H, m NH), 8.04-8.00 (1H, m), 7.80-7.79 (1H, m), 7.45-7.33 (1H, m), 7.32-7.25 (2H, m), 7.18-7.12 (2H, m), 4.37 and 4.32 (1H, s), 4.10-4.04 (1H, m), 3.17-3.12 (1H, m), 2.94-2.82 (1H, m), 1.41-0.86 (4H, m), 0.99 and 0.91 (3H, s) 0.73 and 0.63 (3H, t, J 7.2Hz). m/z (ES⁺, 70V) 432.0 (MH⁺).

### EXAMPLE 13

### Ethyl (2S)-2-[(4R.S)-4-methyl-3-oxo-4-propyl-1-cyclobutenyl]amino-3-[4-([2,6]naphthyridin-1-ylamino)phenyl]propanoate

Prepared from Intermediate 2 (200mg, 1.43mmol) and Intermediate 23 (400mg, 1.19mmol), in a similar manner to the compound of Example 11 to give the title compound as an approx. 1:1 mixture of diastereomers as a white powder (482mg, 1.05mmol, 89%). δH (CDCl₃, 300K) 9.13 (1H, s), 8.61 (1H, d, J 5.9Hz), 8.17 (1H, d, J 5.8Hz), 7.66-7.60 (3H, m), 7.19-7.04 (5H, m), 5.62 (1H, t, J 4.6Hz), 4.51 and 4.49 (1H, s), 4.25-4.19 (3H, m), 3.16-3.05 (2H, m), 1.51-1.16 (7H, m), 0.85-0.77 (3H, m). m/z (ES⁺, 70V) 459.1 (MH⁺).

### EXAMPLE 14

### (2S)-2-[(4R,S)-4-Methyl-3-oxo-4-propyl-1-cyclobutenyl]amino-3-[4-([2.6]naphthyridin-1-ylamino)phenyl]propanoic acid

The compound of Example 13 (600mg, 1.31 mmol) was hydrolysed in a similar manner to the method of Example 2 to give the title compound as a pale yellow powder (521 mg, 1.21 mmol, 95%) (approx. 1:1 mixture of diastereomers). δH (DMSO d⁶, 300K) 9.10 (1H, s), 8.55-8.53 (1H, m), 8.37 and 8.31 (1H, m NH), 8.27 (1H, d, J 5.9Hz), 7.72-7.65 (2H, m), 7.15-7.08 (3H, m), 4.30 and 4.25 (1H, s), 3.99-3.94 (1H, m), 3.06-2.99 (1H, m), 2.83-2.76 (1H, m), 1.34-0.96 (4H, m), 0.94 and 0.86 (3H, s), 0.68 and 0.55 (3H, t, J 7.0Hz). m/z (ES⁺, 70V) 431.0 (MH⁺).

### EXAMPLE 15

### Ethyl (2S)-2-[(4R,S)-4-methyl-3-oxo-4-propyl-1-cyclobutenyl]amino-3-{4-[(3,5-dichloroisonicotinoyl)amino]phenyl}propanoate

Prepared from Intermediate 2 (120mg, 0.86mmol) and the free base of Intermediate 27 (300mg, 0.79mmol), in a similar manner to the compound of Example 11 to give title compound as an approx. 1:1 mixture of diastereomers as a white powder (318mg, 0.63mmol, 80%). δH (CDCl₃, 300K) 8.56 (2H, s), 8.29 and 8.24 (1H, s NH), 7.61-7.59 (2H, m), 7.16-7.10 (2H, m), 5.82-5.78 (1H, m), 4.56 (1H, s), 4.32-4.26 (3H, m), 3.29-3.23 (1H, m), 3.16-3.09 (1H, m), 1.59-1.13 (7H, m), 0.89-0.84 (3H, m). m/z (ES⁺, 70V) 504.0 and 506.0 (MH⁺).

### EXAMPLE 16

### (2S)-2-[(4R,S)-4-Methyl-3-oxo-4-propyl-1-cyclobutenyl]amino-3-{4-[(3,5-Dichloroisonicotinoyl)amino]phenyl}propanoic acid

The compound of Example 15 (300mg, 0.59mmol) was hydrolysed in a similar manner to the method of Example 2 to give the title compound as a fine white solid (261 mg, 0.55mmol, 92%) (approx. 1:1 mixture of diastereomers). δH (DMSO d⁶, 300K) 10.90 (1H, s), 8.81 (2H, s), 7.60-7.56 (2H, m), 7.31-7.26 (2H, m), 4.45 and 4.42 (1H, s), 4.15-4.41 (1H, m), 3.23-3.14 (1H, m), 2.99-2.89 (1H, m), 1.49-1.12 (3H, m), 1.07 and 0.99 (3H, s), 0.84-0.54 (4H, m). m/z (ES⁺, 70V) 476.0 and 478.0 (MH⁺).

### EXAMPLE 17

### Ethyl (2S)-2-[(4,4-dimethyl-3-oxo-2-hexyl-1-cyclobutenyl)amino]-3-{4-[(3,5-dichloroisonicotinoyl)amino]phenyl}propanoate

Prepared from Intermediate 6 (200mg, 1.0mmol) and the free base of Intermediate 27 (200mg, 0.52mmol), in a similar manner to the compound of Example 11 to give the title compound as a white powder (201 mg, 0.42mmol, 72%). δH (CDCI₃, 300K) 8.99 (1H, s), 8.42 (2H, s), 7.52 (2H, d, J 8.4Hz), 7.02 (2H, d, J 7.6Hz), 5.54 (1H, s), 4.34 (1H, s), 4.19 (2H, q, J 7.1Hz), 3.07 (2H, br s), 1.95-1.81 (2H, br s), 1.27-0.77 (17H, m). m/z (ES⁺, 70V) 560.0 and 562.0 (MH⁺).

### EXAMPLE 18

### (2S)-2-[(4,4-Dimethyl-3-oxo-2-hexyl-1-cyclobutenyl)amino]-3-{4-[(3,5-dichloroisonicotinoyl)amino]phenyl}propanoic acid

The compound of Example 17 (80mg, 0.14mmol) was hydrolysed in a similar manner to the method of Example 2 to give the title compound as an off-white powder (62mg, 0.12mmol, 82%). δH (DMSO d⁶, 300K) 10.53 (1H, s), 8.73 (2H, s), 7.60-7.56 (2H, m), 7.57 (2H, d, J 8.4Hz), 7.30 (2H, d, J 8.4Hz), 4.14-4.12 (1H, m), 3.17 (1H, dd, J 13.9, 4.8Hz), 3.03 (1H, dd, J 13.0, 9.1Hz), 1.87 (2H, t, J 7.3Hz), 1.41-1.25 (9H, m), 1.15-0.86 (8H, m). m/z (ES⁺, 70V) 532.0 and 534.0 (MH⁺).

### EXAMPLE 19

### Ethyl (2S)-2-[(4,4-dimethyl-3-oxo-2-hexyl-1-cyclobutenyl)amino]-3-[4-([2,7]naphthyridin-1-yloxy)phenyl] propanoate

Prepared from Intermediate 6 (200mg, 1.0mmol) and the ethyl ester of Intermediate 13 (200mg, 0.59mmol), in a similar manner to the compound of Example 11 to give the title compound as a white powder (201mg, 0.42mmol, 72%). δH (CDCl₃, 300K) 9.72 (1H, s), 8.71 (1H, d, J 5.7Hz), 8.03 (1H, d, J 5.8Hz), 7.56-7.51 (1H, m), 7.27-7.17 (4H, m), 5.41 (1H, br m), 4.39 (1H, br m), 4.19 (2H, q, J 7.1Hz), 3.15-3.12 (2H, m), 1.91-1.75 (2H, m), 1.39-1.09 (18H, m), 0.81-0.74 (2H, m). m/z (ES ⁺, 70V) 516.1 (MH⁺).

### EXAMPLE 20

### (2S)-2-[(4,4-Dimethyl-3-oxo-2-hexyl-1-cyclobutenyl)amino]-3-[4-([2,7]naphthyridin-1-yloxy)phenyl]propanoic acid

The compound of Example 19 (200mg, 0.39mmol) was hydrolysed in a similar manner to the method of Example 2 to give the title compound as a fine white solid (161mg, 0.33mmol, 85%). δ□H (DMSO d⁶, 360K) 9.62 (1H, s), 8.74 (1H, d, J 5.6Hz), 8.04 (1H, d, J 5.6Hz), 7.82 (1H, d, J 5.6Hz), 7.47 (1H, d, J 5.5Hz), 7.30 (2H, d, J 8.3Hz), 7.17 (2H, d, J 8.3Hz), 4.02 (1H, br s), 3.21-3.18 (1H, m), 2.97-2.91 (1H, m), 1.74 (2H, m), 1.12-0.62 (17H, m). m/z (ES⁺, 70V) 488.1 (MH⁺).

### EXAMPLE 21

### Ethyl (2S)-2-[(4,4-dimethyl-3-oxo-2-hexyl-1-cyclobutenyl)amino]-3-{4-[(3-methyl[2,7]naphthyridin-1-yl)oxy]phenyl}propanoate

Prepared from Intermediate 6 (200mg, 1.0mmol) and Intermediate 18 (300mg, 0.85mmol), in a similar manner to the compound of Example 11 to give the title compound as a white powder (331 mg, 0.63mmol, 73%). δH (CDCl₃, 300K) 9.70 (1H, s), 8.70 (1H, d, J 5.8Hz), 7.51 (1H, d, J 5.8Hz), 7.26-7.19 (4H, m), 5.34 (1H, br s), 4.45 (1H, br s), 4.26 (2H, q, J 7.2Hz), 3.21 (2H, br s), 2.44 (3H, s), 2.10-1.90 (2H, m), 1.47-1.43 (2H, m), 1.33-1.12 (12H, m), 0.87-0.84 (3H, m). m/z (ES⁺, 70V) 530.1 (MH⁺).

### EXAMPLE 22

### (2S)-2-[(4,4-Dimethyl-3-oxo-2-hexyl-1-cyclobutenyl)amino]-3-{4-[(3-methyl[2,7]naphthyridin-1-yl)oxy]phenyl}propanoic acid

The compound of Example 21 (60mg, 0.11mmol) was hydrolysed in a similar manner to the method of Example 2 to give the title compound as a fine white solid (42mg, 0.08mmol, 74%). δH (DMSO d⁶, 360K) 9.59 (1H, s), 8.70 (1H, d, J 5.7Hz), 7.70-7.68 (1H, m), 7.66 (1H, d, J 9.7Hz), 7.37 (2H, d, J 8.6Hz), 7.31 (1H, s), 7.23 (2H, d, J 8.6Hz), 4.18-4.16 (1H, m), 3.24 (1H, dd, J 13.9, 4.4Hz), 3.04 (1H, dd, J 13.9, 9.9Hz), 2.38 (3H, s), 1.86 (2H, t, J 7.3Hz), 1.38-1.19 (8H, m), 1.04 (3H, s), 0.99 (3H, s), 0.83-0.79 (3H, m). m/z (ES⁺, 70V) 502.1 (MH⁺).

### EXAMPLE 23

### Ethyl (2S)-2-[4R,S)-4-benzyl-4-methyl-3-oxo-1-cyclobutenyl]amino-3-{4-[(3-methyl[2,7]naphthyridin-1-yl)oxy]phenyl}propanoate

Prepared from Intermediate 8 (200mg, 1.0mmol) and Intermediate 20 (300mg, 0.85mmol), in a similar manner to the compound of Example 11 to give the title compound as a white powder (412mg, 0.79mmol, 92%) as an approx.1:1 mixture of diastereomers. δH (CDCl₃, 300K) 9.70 (1H, d, J 4.9Hz), 8.71 and 8.70 (1H. d, J 5.8Hz), 7.51 (1H, d, J 5.8Hz), 7.31-7.08 (11H, m), 5.88-5.82 (1H, m), 4.60 and 4.50 (1H, s), 4.33-4.28 (1H, m), 4.26-4.16 (2H, m), 3.25-3.07 (2H, m), 2.98-2.83 (2H, m), 2.45 and 2.40 (3H, s), 1.35-1.21 (6H, m). m/z (ES⁺, 70V) 522.1 (MH⁺).

### EXAMPLE 24

### (2S)-2-[(4R,S)-4-Benzyl-4-methyl-3-oxo-1-cyclobutenyl]amino-3-{4-[(3-methyl[2,7]naphthyridin-1-yl)oxy]phenyl}propanoic acid

The compound of Example 23 (250mg, 0.48mmol) was hydrolysed in a similar manner to the method of Example 2 to give the title compound as a fine white solid (221 mg, 0.45mmol, 94%) as an approx. 1:1 mixture of diastereomers. δH (DMSO d⁶, 360K) 9.72 (1H, m), 8.81 (1H, m), 8.03 (1H, m), 7.82-7.77 (1H, br m), 7.46-7.20 (9H, m), 4.49 and 4.41 (1H, s), 4.21 (1H, m), 3.39-3.30 (1H, m), 3.21-3.14 (1H, m), 3.01-2.87 (2H, m), 2.51 (3H, s), 1.29 and 1.24 (3H, s). m/z (ES⁺, 70V) 494.0 (MH⁺).

### EXAMPLE 25

### Ethyl (2S)-2-[(4R,S)-4-benzyl-4-methyl-3-oxo-1-cyclobutenyl]amino-3-4-[(3,5-dichloroisonicotinoyl)amino]phenylpropanoate

Prepared from Intermediate 8 (185mg, 0.98mmol) and the free base of Intermediate 27 (300mg, 0.79mmol), in a similar manner to the compound of Example 11 to give the title compound as a white powder (387mg, 0.70mmol, 89%) as an approx. 1:1 mixture of diastereomers. δH (CDCl₃, 300K) 9.36 and 9.31 (1H, s), 8.36 and 8.35 (2H, s), 7.54 and 7.45 (1H, d, J 8.4Hz), 7.19-7.02 (8H, m), 6.09-6.03 (1H, m), 4.31 and 4.20 (1H, s), 4.22-4.01 (3H, m), 3.07-2.92 (2H, m), 2.76-2.63 (2H, m), 1.35-1.15 (2H, m), 1.09 and 1.08 (3H, s), m/z (ES⁺, 70V) 551.9 and 553.9 (MH⁺).

### EXAMPLE 26

### (2S)-2-[(4R,S)-4-Benzyl-4-methyl-3-oxo-1-cyclobutenyl]amino-3-{4-[(3,5-dichloroisonicotinoyl)amino]phenyl}propanoic acid

The compound of Example 25 (320mg, 0.58mmol) was hydrolysed in a similar manner to the method of Example 12 to give the title compound as a fine white solid (277mg, 0.53mmol, 91%) as an approx. 1:1 mixture of diastereomers. δH(DMSO d⁶ , 360K) 13.05 (1H, br s), 8.83 and 8.82 (2H, s), 8.67 and 8.62 (1H, d, J 8.9Hz), 7.71 and 7.61 (2H, d, J 8.7Hz), 7.37-6.89 (9H, m), 4.32 and 4.23 (1H, s), 4.09-4.00 (1H, m), 3.20-2.64 (4H, m), 1.24-1.07 (3H, m). m/z (ES⁺, 70V) 523.9 and 525.9 (MH⁺).

### EXAMPLE 27

### Ethyl (2S)-2-[(3-oxospiro[3.5]non-1-en-1-yl)amino]-3-4-[(3,5-dichloroisonicotinoyl)amino]phenyl}propanoate

Prepared from 1-keto-3-hydroxyspiro[3,5]-non-2-ene (400mg, 2.6mmol) [prepared according to the method of Wasserman, H.H. et al, J. Org. Chem., 38, 1451-1455 (1973)] and the free amine of Intermediate 27 (400mg, 1.04mmol), in a similar manner to the compound of Example 11 to give the title compound as a white powder (512mg, 0.99mmol, 95%). δH (CDCl₃, 300K) 10.86 (1H, s), 8.78 (2H, s), 8.34 (1H, d, J 8.5Hz), 7.56 (2H, d, J 8.5Hz), 7.25 (2H, d, J 8.5Hz), 4.36 (1H, s), 4.20-4.11 (3H, m), 3.13 (1H, dd, J 13.8, 5.3Hz), 3.00 (1H, dd, J 9.2, 13.8Hz), 1.67-1.19 (10H, m), 1.17 (3H, t, J 4.1Hz). m/z (ES⁺, 70V) 516.0 and 518.0 (MH⁺).

### EXAMPLE 28

### (2S)-2-[(3-Oxospiro[3.5]non-1-en-1-yl)amino]-3-{4-[(3,5-dichloroisonicotinoyl)amino]phenyl}propanoic acid

The compound of Example 27 (700mg, 1.36mmol) was hydrolysed in a similar manner to the method of Example 2 to give the title compound as a fine white solid (627mg, 1.28mmol, 95%). δH (DMSO d⁶, 360K) 10.54 (1H, s), 8.73 (2H, s), 7.81 (1H, d, J 8.4Hz), 7.56 (2H, d, J 8.5Hz), 7.27 (2H, d, J 8.5Hz), 4.39 (1H, s), 4.12-4.05 (1H, m), 3.19 (1H, dd, J 13.9, 5.1Hz), 3.00 (1H, dd, J 13.9, 8.8Hz), 1.94-1.24 (10H, m). m/z (ES⁺, 70V) 488.0 and 490.0 (MH⁺).

### Example 29

### Ethyl (2S)-2-[(3-oxospiro[3.5]non-1-en-1-yl)amino]-3-{4-[(3-methyl[2,7]naphthyridin-1-yl)oxy]phenyl}propanoate

Prepared from 1-keto-3-hydroxyspiro[3,5]-non-2-ene (400mg, 2.6mmol) and Intermediate 20 (400mg, 1.14mmol), in a similar manner to the compound of Example 11 to give the title compound as a white powder (497mg, 1.02mmol, 89%). δH (CDCl₃, 300K) 9.62 (1H, s), 8.72 (1H, d, J 5.7Hz), 7.99 (1H, d, J 8.6Hz), 7.73 (1H, dd, J 5.7, 0.9Hz), 7.37-7.34 (3H, m), 7.28-7.24 (2H, m), 4.42 (1H, s), 4.26-4.18 (3H, m), 3.25 (1H, dd, J 14.0, 5.6Hz), 3.12 (1H, dd, J 14.0, 9.1Hz), 2.42 (3H, s), 1.72-1.55 (10H, m), 1.25 (3H, t, J 7.1Hz). m/z (ES⁺, 70V) 486.1 (MH⁺).

### EXAMPLE 30

### (2S)-2-[(3-Oxospiro[3.5]non-1-en-1-yl)amino]-3-{4-[(3-methyl[2,7]naphthyridin-1-yl)oxy]phenyl)propanoic acid

The compound of Example 29 (300mg, 0.62mmol) was hydrolysed in a similar manner to the method of Example 2 to give the title compound as a fine white solid (237mg, 0.52mmol, 84%). δH (DMSO d⁶, 360K) 9.62 (1H, s), 8.72 (1H, d, J 5.7Hz), 7.82 (1H, d, J 6.3Hz), 7.73 (1H, d, J 5.5Hz), 7.35 (2H, d, J 8.7Hz), 7.25 (2H, d, J 8.7Hz), 4.39 (1H, s), 4.12 (1H, dd, J 8.7, 13.2Hz), 3.34-3.12 (2H, m), 2.42 (3H, s), 1.72-1.53 (10H, m). m/z (ES⁺, 70V) 458.0 (MH⁺).

### EXAMPLE 31

### Ethyl (2S)-2-[(2-bromo-3-oxospiro[3.5]non-1-en-1-yl)amino]-3-{[4(3,5-dichloroisonicotinoyl)amino]phenyl}propanoate

A solution containing the compound of Example 27 (500mg, 0.97mmol) and triethylamine (2eq, 270µl) in THF (10ml) at 0º was treated dropwise with a solution of bromine (1.1eq, 170mg) in THF (5ml). After 20mins the reaction was allowed to warm to room temperature prior to dilution with EtOAc (100ml). The crude reaction mixture was washed with saturated aqueous NaHCO₃ (20ml) and brine (20ml), dried (MgSO₄) filtered and concentrated *in vacuo.* The residual foam was chromatographed (SiO₂; EtOAc) to give the title compound as a white powder (511mg, 0.86mmol, 95%). δH (CDCI₃, 300K) 8.48 (2H, s), 8.05 (1H, s br), 7.52 (2H, d J 8.4Hz), 7.04 (2H, d J 8.5Hz), 5.81 (1H, d br, J 8.3Hz), 4.98-4.91 (1H, m), 4.21 (2H, q, J 7.1Hz), 3.21 (2H, d J 5.3Hz), 1.70-1.66 (4H, m), 1.53-1.44 (4H, m), 1.28 (3H, t J 7.1Hz), 1.20-1.16 (2H, m). m/z (ES⁺, 70V) 597.9 and 595.0 (MH⁺).

### EXAMPLE 32

### (2S)-2-[(2-Bromo-3-oxospiro[3,5]non-1-en-1-yl)amino]-3-{4-[(3,5-dichloroisonicotinoyl)amino]phenyl}pronanoic acid

The compound of Example 31 (511 mg, 0.86mmol) was hydrolysed in a similar manner to the method of Example 2 (1.3eq, 50mg), to give the title compound as a white powder (421 mg, 0.74mmol, 87%). δH (DMSO d⁶, 390K) 10.34 (1H, s), 8.67 (2H, s), 7.53 (2H, s br), 7.26 (2H, d J 8.26Hz), 4.67 (1H, m), 3.26-3.22 (1H, m), 3.13-3.08 (1H, m), 1.67-1.21 (10H, m). δC (DMSO-d⁶, 300K) 23.86, 25.30, 30.75, 37.79, 57.98, 61.94, 67.02, 119.73, 128.47, 130.38, 133.46, 136.86, 142.85, 148.10, 160.11, 171.80, 173.96, 186.93. m/z (ES⁺, 70V) 569.9 and 567.9 (MH⁺).

### EXAMPLE 33

### Ethyl (2S)-2-[(2-bromo-4,4-dimethyl-3-oxo-1-cyclobutenyl)amino]-3-{4-[(3,5-dichloroisonicotinoyl)amino]phenyl}propanoate

Bromine (1.1eq, 0.32ml) was added dropwise to a stirred solution of the compound of Example 5 (2.7g, 5.67mmol) in THF (25ml) at room temperature. After 25min the reaction was diluted with EtOAc (100ml) and the crude reaction mixture washed with saturated aqueous NaHCO₃ (20ml) and brine (20ml), dried (MgSO₄) filtered and concentrated in *vacuo.* The residual foam was chromatographed (SiO₂; EtOAc) affording the title compound as a pale yellow powder (2.51g, 4.53mmol, 76%). δH (CDCl₃, 300K) 8.46 (2H, s), 8.17 (1H, s br), 7.51 (2H, d J 8.4Hz), 7.04 (2H, d J 8.4Hz), 6.05 (1H, d br, J 8.4Hz), 4.98-4.92 (1H, m), 4.22 (2H, q, J 7.1Hz), 3.21 (2H, d J 5.4Hz), 1.28 (3H, t J 7.1Hz), 1.14 (3H, s), 1.13 (3H, s). m/z (ES⁺, 70V) 555.8 and 557.9 (MH⁺).

### EXAMPLE 34

### (2S)-2-[(2-Bromo-4,4-dimethyl-3-oxo-1-cyclobutenyl)amino]-3-{4-[(3,5-dichloroisonicotinoyl)amino]phenyl}propanoic acid

The compound of Example 33 (198mg, 0.36mmol) was hydrolysed in a similar manner to the method of Example 2 to give the title compound as a white powder (142mg, 0.27mmol, 75%). δH (DMSO-d⁶, 390K) 10.46 (1H, s), 8.74 (2H, s), 7.63 (2H, d J 5.74Hz), 7.35 (2H, d J 8.26Hz), 4.80 (1H, s br), 3.32 (1H, dd J 5.14, 14.2Hz), 3.14 (1H, dd J 8.9Hz 14.2Hz), 1.18 (3H, s), 1.15 (3H, s). m/z (ES⁺, 70V) 527.9 and 529.8 (MH⁺).

### EXAMPLE 35

### Ethyl (2S)-2-[(3-oxospiro[3,5]non-1-en-1-yl)amino]-3-{4-[(2,7)naphthyridin-1-yloxy]phenyl}propanoate

A solution of the ethyl ester of Intermediate 13 (565mg, 1.68mmol) and 1-keto-3-hydroxyspiro[3,5]-non-2-ene (280mg, 1.84mmol) in DCM (20ml) was stirred at roon temperature for 24h. Concentration *in vacuo* and chromatography (SiO₂; EtOAc) to give the title compound as a pale yellow powder (730mg, 1.55mmol, 92%). δH (CDCl₃, 300K) 9.82 (1H, s), 8.82 (1H, d J 5.7Hz), 8.14 (1H, d J 5.9Hz), 7.64 (1H, d J 5.8Hz), 7.25-7.17 (6H, m), 5.77 (1H, d J 7.6Hz), 4.60 (1H, s), 4.25 (2H, q J 7.1Hz), 3.30 (1H, dd J 5.5Hz 13.9Hz), 3.18 (1H, dd J 5.5Hz 13.9Hz), 1.84-1.53 (10H, m), 1.35 (3H, t J 7.1Hz). m/z (ES⁺, 70V) 472.1 (MH⁺).

### EXAMPLE 36

### Ethyl (2S)-2-[(2-bromo-3-oxospiro[3.5]non-1-en-1-yl)amino]-3-{4-(2,7)naphthyridin-1-yloxy]phenyl}propanoate

A stirred solution of the compound of Example 35 (300mg, 0.637mmol) and triethylamine (1.2eq, 100µl) at 0º was treated dropwise with a solution of bromine in DCM (2%wv/v, 2.1ml, 1.2eq). After 12h the reaction was diluted with DCM (50ml) and washed successively with saturated aqueous NaHCO₃, dried (MgSO₄) filtered and concentrated *in vacuo.* The residual foam was triturated with diisopropylether and the resulting solid collected and dried *in vacuo* to give the title compound as a pale yellow powder (325mg, 0.59mmol, 95%). δH (CDCl₃, 300K) 9.83 (1H, s), 8.78 (1H, d J 5.8Hz), 8.16 (1H, d J 5.8Hz), 7.69 (1H, d, J 5.7Hz), 7.32 (1H, d, J 5.8Hz), 7.27 (4H, s), 5.87 (1H, d, J 8.4Hz), 5.10-5.03 (1H, m), 4.30 (2H, q, J 7.1Hz), 3.38-3.32 (2H, m), 1.85-1.69 (4H, m), 1.67-1.50 (6H, m), 1.36 (3H, t, J 7.1Hz). m/z (ES⁺, 70V) 552.0 (MH⁺).

### EXAMPLE 37

### (2S)-2-[(2-Bromo-3-oxospiro[3.5]non-1-en-1-yl)amino]-3-{4-[(2,7)naphthyridin-1-yloxy]phenyl}propanoic acid

The compound of Example 36 (220mg, 0.40mmol) was hydrolysed in a similar manner to the method of Example 2, to give the title compound as a white powder (125mg, 0.24mmol, 60%). δH (DMSO-d⁶, 300K) 9.27 (1H, s), 8.88 (1H, d J 9.4Hz), 8.83 (1H, d J 5.4Hz), 8.12 (1H, d J 5.8Hz), 7.90 (1H, d J 5.7Hz), 7.55 (1H, d J 5.8Hz), 7.38 (2H, d J 8.4Hz), 7.27 (2H, d J 8.4Hz), 4.83-4.79 (1H, m), 3.08-3.03 (2H, m), 1.80-1.37 (8H, m), 1.19-1.12 (2H, m). m/z (ES⁺, 70V) 523.9 (MH⁺).

### EXAMPLE 38

### Ethyl (2S)-2-[(3-oxo-7-oxaspiro[3.5]non-1-en-1-yl)amino]-3-[4-[(3,5-dichloroisonicotinoyl)amino]phenyl}propanoate

Prepared from 7-oxaspiro[3.5]nonane-1,3-dione (1.2g, 7.8mmol) and the free amine of Intermediate 27 (2.67g, 7.0mmol) in a similar manner to the method of Example 11, to give the title compound (3.31g, 6.38mmol, 91%). δH (CDCl₃, 300K) 8.61 (1H, s), 8.33 (2H, s), 7.41 (2H, d J 5Hz), 6.94 (2H, d J 8.5Hz), 6.30 (1H, s br), 4.35 (1H, s), 4.11 (2H, q J 7.1Hz) and (1H, m obscured), 5.72 (4H, m), 3.07 (1H, dd J 14.0, 5.0Hz), 2.94 (1H, dd J 14.0, 6.6Hz), 1.75-1.66 (2H, m), 155-1.48 (2H, m), 1.17 (3H, t J 7.1Hz). m/z (ES⁺, 70V) 517.9 (MH⁺).

### EXAMPLE 39

### Ethyl (2S)-2-[(2-bromo-3-oxo-7-oxaspiro[3,5]non-1-en-1-yl)amino]-3-{4-[(3,5-dichloroisonicotinoyl)amino]phenyl}propanoate

A solution of the compound of Example 38 (1.64g, 3.17mmol) and triethylamine (0.69g, 970µl, 6.8mmol) in THF (15ml) at 0º was treated dropwise with a solution of bromine (560mg, 3.1mmol) in THF (2ml). After 1h the resulting precipitate was removed by filtration, washed several times with cold EtOAc and dried to give the title compound as a white powder (1.53g, 2.56mmol, 81%). δH (DMSO d⁶, 300K) 10.90 (1H, s), 9.07 (1H, d J 9.0Hz), 8.81 (2H, s), 7.60 (2H, d J 8.4Hz), 7.28 (2H, d J 8.4Hz), 4.85-4.80 (1H, m), 4.21 (2H, q J 7.1Hz). 3.81-3.76 (2H, m), 3.63-3.58 (2H, m), 3.23 (1H, dd J 13.8, 4.8Hz), 3.05 (1H, dd J 13.8, 9.4Hz), 2.07-1.94 (2H, m), 1.52-1.49 (1H, m), 1.34-1.31 (1H, m), 1.24 (3H, t J 7.1Hz). m/z (ES⁺, 70V) 597.9 and 599.9 (MH⁺).

### EXAMPLE 40

### (2S)-2-[(2-Bromo-3-oxo-7-oxaspiro[3.5]non-1-en-1-yl)amino]-3-{4-[(3,5-dichloroisonicotinoyl)amino]phenyl}propanoic acid

The compound of Example 39 (575mg, 0.96mmol) was hydrolysed in a similar manner to the method of Example 2, to give the title compound as a white powder (283mg, 0.50mmol, 52%). δH (DMSO d⁶,390K) 10.88 (1H, s), 8.98 (1H, d J 9.2Hz), 8.81 (2H, s), 7.59 (2H, d J 8.5Hz), 7.27 (2H, d J 8.5Hz), 4.78-4.72 (1H, m), 3.82-3.75 (2H, m), 3.64-3.54 (2H, m), 3.24 (1H, dd J 13.9, 4.5Hz), 3.01 (1H, dd J 13.8, 9.5Hz), 2.08-1.93 (2H, m), 1.52-1.48 (1H, m), 1.30-1.26 (1H, m). m/z (ES⁺, 70V) 569.9 and 571.9 (MH⁺).

### EXAMPLE 41

### Methyl (2S)-2-{(3-oxospiro[3.5]non-1-en-1-yl)amino}-3-(2,6-dimethoxy[1,1'-biphenyl]-4-yl)propanoate

To a solution of methyl (2S)-2-amino-3-(2,6-dimethoxy[1,1'-biphenyl]-4-yl)propanoate (0.80g, 2.5mmol) in DCM (10ml) at room temperature was added 1-keto-3-hydroxyspiro[3,5]-non-2-ene (0.38g, 2.5mmol) and the mixture stirred for 48h. Volatiles were removed *in vacuo* and the residue purified by column chromatography (SiO₂; EtOAc) to give the title compound as a white solid (1.05g, 92%). δH (CDCI₃): 7.32-7.26 (3H, m), 7.12 (2H, d, J 8.2Hz), 6.92 (2H, d, J 8.3Hz), 5.90 (1H, br d, J 8.2Hz), 4.60 (1H, s), 4.33 (1H, br), 3.86 (3H,s), 3.73 (6H, s), 3.30 (1H, dd, J 13.9, 5.3Hz), 3.13 (1H, dd, J 13.9, 6.3Hz), 1.82-1.33 (10H, m). m/z (ES⁺, 70V) 450.1 (MH⁺).

### EXAMPLE 42

### (2S)-2-{(3-Oxospiro[3.5]non-1-en-1-yl)amino}-3-(2,6-dimethoxy[1,1'-biphenyl]-4-yl)propanoic acid

The compound of Example 41 (0.40g, 0.9mmol) was hydrolysed in a similar manner to the method of Example 2, to give the title compound as a white solid (0.19g, 45%). δH (DMSO d⁶) 8.25 (1H, d, J 8.6Hz), 7.29-7.19 (3H, m), 7.07 (2H, d, J 7.9Hz), 6.70 (2H, d, J 8.4Hz), 4.32 (1H, s), 4.11 (1H, br), 3.61 (6H, s), 3.18 (1H, dd, J 13.7, 4.7Hz), 2.93 (1H, dd, J 13.7 9.9Hz), 1.67-1.16 (10H, m). m/z (ES⁺, 70V) 436.1 (MH⁺).

### EXAMPLE 43

### Methyl (2S)-2-{(2-bromo-3-oxospiro[3.5]non-1-en-1-yl)amino}-3-(2,6-dimethoxy[1,1'-biphenyl]-4-yl)propanoate

To a cooled solution (0-5⁰) of the compound of Example 41 (0.42g, 0.93mmol) and triethylamine (0.14ml, 1.03mmol) in THF (10ml) was added a solution of bromine (0.16g, 1.0mmol) in DCM (1ml). The mixture was stirred at this temperature for 1h prior to partitioning between EtOAc (100ml) and sodium hydrosulfite (100ml, 5% aq.). The organics were separated, washed with water (50ml), brine (50ml), dried (Na₂SO₄), filtered and concentrated *in vacuo* to give the crude product as pale yellow foam. Column chromatography (SiO₂, 1:1 EtOAc: hexanes) gave the title compound as a white foam (0.45g, 92%). δH (CDCl₃) 7.32-7.26 (3H, m), 7.13 (2H, d, J 8.1Hz), 6.66 (2H, d, J 8.4Hz), 5.80 (1H, br d, J 8.6Hz), 5.15-5.08 (1H, m), 3.87 (3H, s), 3.73 (6H, s), 3.35 (1H, d, J 10.0Hz), 3.31 (1H, d, J 4.9Hz), 1.80-1.33 (10H, m). m/z (ES⁺, 70V) 529.0 and 530.0 (MH⁺).

### EXAMPLE 44

### (2S)-2-{(2-Bromo-3-oxospiro[3.5]non-1-en-1-yl)amino}-3-(2,6-dimethoxy[1,1'-biphenyl]-4-yl)propanoic acid

The compound of Example 43 (0.36g, 0.7mmol) was hydrolysed in a similar manner to the method of Example 2 to give the title compound as a white solid (0.23g, 58%). δH (DMSO d⁶) 8.83 (1H, d, J 9.4Hz), 7.28 (1H, d, J 8.4Hz), 7.24-7.20 (2H, m), 7.10 (2H, d, J 8.1Hz), 6.70 (2H, d, J 8.4Hz), 4.83-4.77 (1H, br), 3.61 (6H, s), 3.25 (1H, dd, J 13.8, 9.8Hz), 2.95 (1H, dd, J 13.8, 10.3Hz), 1.78-1.35 (10H, m). m/z (ES⁺, 70V) 516.0 and 517.0 (MH⁺).

### EXAMPLE 45

### Ethyl (2S)-2-[(3-oxospiro[3.6]dec-1-en-1-yl)amino]-3-{4-[(3,5-dichloro-isonicotinoyl)amino]phenyl}propanoate

Prepared from Intermediate 31 (400mg, 2.4mmol) and the free amine of Intermediate 27 (920mg, 2.4mmol) in a similar manner to the method of Example 11, to give the title compound (1.1g, 20.7mmol, 86%). δH (CDCl₃, 300K) 8.57 (2H, s), 8.28 (1H, s), 7.61 (2H, d J 8.5Hz), 7.14 (2H, d J 8.5Hz), 5.76 (1H, d J 7.5Hz), 4.33-4.23 (3H, m), 3.25 (1H, dd J 5.3, 14.0Hz), 3.12 (1H, dd J 5.7, 13.9Hz), 1.95-1.89 (2H, m), 1.79-1.70 (4H, m), 1.71-1.50 (6H, m), 1.36 (3H, t J 7.1Hz). m/z (ES⁺, 70V) 530.0 (MH⁺).

### EXAMPLE 46

### (2S)-2-[(3-Oxospiro[3.6]dec-1-en-1-yl)amino]3-{4-[(3,5-dichloroisonicotinoyl)amino]phenyl}propanoic acid

The compound of Example 45 (257mg, 0.57mmol) was hydrolysed in a similar manner to the method of Example 2, to give the title compound as a white powder (257mg, 0.51 mmol, 89%). δH ( DMSO d⁶, 390K) 10.83 (1H, s), 8.84 (2H, s), 7.39 (2H, d J 8.5Hz), 7.29 (2H, d J 8.5Hz), 4.30 (1H, s), 4.12-3.98 (1H, m), 3.15 (1H, dd J 13.9, 5.2Hz), 2.97 (1H, dd J 13.8, 9.5Hz), 1.85-1.78 (1H, m), 1.77-1.38 (11H, m). m/z (ES⁺, 70V) 502.0 (MH⁺).

### EXAMPLE 47

### Ethyl (2S)-2-[(2-bromo-3-oxospiro[3.6]dec-1-en-1-yl)amino]-3-{4-[(3,5-dichloroisonicotinoyl)amino]phenyl}propanoate.

A solution of the compound of Example 45 (988mg, 1.87mmol) and triethylamine (520µl, 3.7mmol) in THF (20ml) at 0º was treated dropwise with a solution of bromine (330mg, 2.1mmol) in THF (2ml). After 1h the crude reaction mixture was diluted with EtOAc (50ml), saturated aqueous NaHCO₃ (15ml) and saturated aqueous sodium chloride (15ml) and the crude product extracted with EtOAc (3 x 20ml). The combined extracts were dried (MgSO₄), concentrated *in vacuo* and the crude residue chromatographed (SiO₂, 1:1 EtOAc:hexanes) to give the title compound as a white powder (965mg, 1.58mmol, 85%). δH (CDCl₃, 300K) 8.61 (2H, s), 8.45 (1H, d, J 3.1Hz), 7.63 (2H, d, J 8.2Hz), 7.15 (2H, d, J 8.2Hz), 5.91 (1H, d, J 8.1Hz), 5.05-5.00 (1H, m), 4.30 (2H, q, J 7.1Hz), 3.30 (2H, d, J 5.4Hz), 1.98-1.90 (2H, m), 1.89-1.60 (10H, m), 1.22 (3H, t, J 7.1Hz). m/z (ES⁺, 70V) 609.9 and 611.9 (MH⁺).

### EXAMPLE 48

### (2S)-2-[(2-Bromo-3-oxospiro[3.6]dec-1-en-1-yl)amino]-3-{4-[(3,5-dichloroisonicotinoyl)amino]phenyl}propanoic acid

The compound of Example 47 (560mg, 0.92mmol) was hydrolysed in a similar manner to the method of Example 2, to give the title compound as a white powder (412mg, 0.71mmol, 77%). δH (DMSO d⁶, 380K) 10.40 (1H, s), 8.67 (2H, s), 7.55 (2H, d, J 8.5Hz), 7.26 (2H, d, J 8.5Hz), 4.52 (1H, brs), 3.22 (1H, dd, J14.1, 5.3Hz), 3.11 (1H, dd, J 13.9, 8.0Hz), 1.82-1.29 (12H, m). m/z (ES⁺, 70V) 589.1 and 583.9 (MH⁺).

### EXAMPLE 49

### Ethyl (2S) 2-{[4,4-dimethyl-2-(phenylselenenyl)-3-oxo-1-cyclobutenyl] amino}3-{4-[(3,5-dichloroisonicotinoyl)amino]phenyl}propanoate

A stirred solution of the compound of Example 5 (630mg, 1.41 mmol) in THF (15ml) at room temperature was treated dropwise with a solution of phenylselenenyl chloride (283mg, 1.48mmol). After 10min the crude reaction mixture was diluted with EtOAc (30ml) saturated aqueous NaHCO₃ solution (50ml) and brine (50ml). The mixture was extracted with EtOAc (3 x 50ml), the combined extracts dried (MgSO₄) and concentrated *in vacuo.* The residual slurry was chromatographed (SiO₂, EtOAc) to give the title compound as a white powder (812mg, 1.29mmol, 91%). δH (CDCl₃, 300K) 8.58 (2H, s), 7.75 (1H, s), 7.53 (2H, d, J 8.3Hz), 7.35-7.11 (5H, m), 7.04 (2H, d, J 8.3Hz), 6.11 (1H, d, J 8.5Hz), 5.28-5.25 (1H, m), 4.20 (2H, q, J 7.1Hz), 3.17 (2H, m), 1.31 (6H, s), 1.28 (3H, t, J 7.1Hz). m/z (ES⁺, 70V) 631.9 (MH⁺).

### EXAMPLE 50

### (2S)-2-{[4,4-dimethyl-2-(phenylselenenyl)-3-oxo-1-cyclobutenyl] amino}-3-{4-[(3,5-dichloroisonicotinoyl)amino]phenyl}propanoic acid

The compound of Example 49 (600mg, 0.95mmol) was hydrolysed in a similar manner to the method of Example 2, to give the title compound as a white powder (503mg, 0.83mmol, 87%). δH (DMSO d⁶, 300K) 10.86 (1H, s), 9.11 (1H, d, J 8.9Hz), 8.81 (2H, s), 7.50 (2H, d, J 8.2Hz), 7.21 (2H, d, J 8.2Hz), 4.96-4.92 (1H, br s), 3.13 (1H. dd, J 13.8, 4.5Hz), 2.94 (1H, dd, J 13.6, 8.7Hz), 1.22 (3H, s), 1.14 (3H, s). m/z (ES⁺, 70V) 603.9 (MH⁺).

### EXAMPLE 51

### Ethyl (2S)-2-[(3-oxo-7-acetyl-7-azaspiro[3.5]non-1-en-1-yl)amino]-3-{4-[(3,5-dichloroisonicotinoyl)amino]phenyl}propanoate

Prepared from Intermediate 33 (150mg, 0.77mmol), and the free amine of Intermediate 27 (150mg, 0.39mmol) in a similar manner to the method of Example 11, to give the title compound (143mg, 0.26mmol, 67%). δH (DMSO d⁶, 300K) 10.89 (1H, s), 8.89 (2H, s), 8.55-8.48 (1H, m), 7.58 (2H, d, J 7.9Hz), 7.25 (2H, d, J 7.9Hz), 4.47 (1H, s), 4.29-4.23 (1H, m), 4.16 (2H, q, J 7.1Hz), 3.76-3.72 (1H, m), 3.15 (1H, dd, J 13.8, 5.2Hz), 3.01-2.89 (2H, m), 2.00 (3H, s), 1.90-1.37 (6H, m), 1.21 (3H q J 7.1Hz). m/z (ES⁺, 70V) 559.0 (MH⁺).

### EXAMPLE 52

### (2S)-2-[(3-Oxo-7-acetyl-7-azaspiro[3.5]non-1-en-1-yl)amino]-3-{4-[(3,5-dichloroisonicotinoyl)amino]phenyl}propanoic acid

The compound of Example 51 (200mg, 0.35mmol) was hydrolysed in a similar manner to the method of Example 2, to give the title compound as a white powder (91 mg, 0.16mmol, 46%). δH (CD₃OD, 300K) 8.90 (2H, s), 7.60 (2H, d, J 8.2Hz), 7.30 (2H, J 8.2Hz), 4.49 (1H, s), 4.33-4.27 (2H, m), 3.85-3.77 (1H, m), 3.57-3.45 (1H, m), 3.37-3.31 (1H, m), 3.20-3.11 (1H, m), 3.05-2.99 (1H, m), 2.11 (3H, s), 1.97-1.52 (4H, m). m/z (ES⁺, 70V) 531.0 (MH⁺).

### EXAMPLE 53

### Ethyl (2S)-2-[(7-methoxy-3-oxospiro[3.5]non-1-en-1-yl)amino]-3-{4-[(3,5-dichloroisonicotinoyl)amino]phenyl}propanoate

Prepared from Intermediate 35 (500mg, 2.77mmol) and the free amine of Intermediate 27 (980mg, 2.6mmol) in a similar manner to the method of Example 11, to give the title compound as an inseparable 1:1 mixture of isomers (1.23g, 2.25mmol, 87%). δH (CDCl₃, 300K, 2 isomers) 9.12/8.99 (1H, s), 8.51/8.50 (2H, s), 7.59/7.56 (2H, d, J 8.5Hz), 7.08 (2H, d, J 8.5Hz), 6.21/5.98 (1H, d, J 7.9Hz/7.6Hz), 4.46/4.43 (1H, s), 4.29/4.10 (3H, m), 3.13-3.08 (1H, m), 3.39 (1H, m), 3.30/3.29 (3H, s), 3.23-3.18 (1H, m), 3.13-3.08 (1H, m), 1.97-1.58 (8H, m), 1.35-1.34 (3H, t, J 7.1Hz). m/z (ES⁺, 70V) 546.0 (MH⁺).

### EXAMPLE 54

### (2S)-2-[(7-Methoxy-3-oxospiro[3.5]non-1-en-1-yl)amino]-3-{4-[(3,5-dichloroisonicotinoyl)amino]phenyl}propanoic acid

The compound of Example 53 (950mg, 1.7mmol) was hydrolysed in a similar manner to the method of Example 2, to give the title compound as a white powder, as an approx. 1:1 mixture of isomers (812mg, 1.57mmol, 92%). δH (DMSO d⁶,300K) 10.57 (1H, s), 8.73 (2H, s), 7.93 (1H, br s), 7.56 (2H, d, J 8.2Hz), 7.29-7.21 (2H, m), 4.37 (1H, s), 4.08-4.04 (1H, m), 3.34 (1H, m), 3.25 (3H, s), 3.21-3.02 (2H, m), 1.92-1.34 (8H, m). m/z (ES⁺, 70V) 518.0 (MH⁺).

### EXAMPLE 55

### Ethyl (2S)-2-[(2-bromo-7-methoxy-3-oxospiro[3.5]non-1-en-1-yl)amino]-3-{4-[(3,5-dichloroisonicotinoyl)amino]phenyl}propanoate

Was prepared according to the method of Example 47 from the compound of Example 53 (1.0g, 1.83mmol) and bromine (322mg, 2.0mmol) to give the title compound as a powder (778mg, 1.24mmol, 70%). [Separation of isomers at this stage was achieved chromatographically (SiO₂; 1:1 EtOAc:hexanes to 100% EtOAc)]. δH (CDCl₃, 300K, fast eluting isomer) 10.65 (1H, s), 10.74 (1H, d, J 9.2Hz), 8.58 (2H, s), 7.36 (2H, d, J 8.6Hz), 7.06 (2H, d, J 8.6Hz) 4.54-4.48 (1H, m), 3.18 (1H, m), 3.03-2.98 (1H, m), 3.00 (3H, s), 2.78 (1H, dd, J 13.9, 10.0Hz), 1.18-1.65 (2H, m), 1.61-1.44 (4H, m), 1.18-1.15 (1H, m), 0.92 (1H, m). m/z (ES⁺, 70V) 625.9 (MH⁺).

### EXAMPLE 56

### (2S)-2-[(2-Bromo-7-methoxy-3-oxospiro[3,5]non-1-en-1-yl)amino]-3-{4-[(3,5-dichloroisonicotinoyl)amino]phenyl}propanoic acid

The compound of Example 55 (650mg, 1.04mmol) was hydrolysed in a similar manner to the method of Example 2, to give the title compound as a white powder (512mg, 0.86mmol, 83%). δH (DMSO d⁶, 300K) 10.86 (1H, s), 9.11 (1H, d, J 8.9Hz), 8.81 (2H, s), 7.50 (2H, d, J 8.2Hz), 7.21 (2H, d, J 8.2Hz), 4.96-4.92 (1H, br s), 3.13 (1H, dd, J, 13.8, 4.5Hz), 2.94 (1H, dd, J 13.6, 8.7Hz), 1.22 (3H, s), 1.14 (3H, s). m/z (ES⁺, 70V) 597.9 (MH⁺).

### EXAMPLE 57

### Ethyl (2S)-2-[(2-bromo-3-oxospiro[3.5]non-1-en-1-yl)amino]-3-{4-[(3-methyl[2.7]naphthyridin-1-yl)oxy]phenyl}propanoate

To the compound of Example 29 (0.54g, 1.1mmol) in THF (10ml) at room temperature was added triethylamine (0.2ml, 1.4mmol) and a solution of bromine (224mg, 1.4mmol) in DCM (1ml). The mixture was stirred overnight and then partitioned between EtOAc (50ml) and water (50ml). The organics were separated, washed with sodium hydrosulfite (2 x 50ml, 5% aq.), water (50ml), brine (50ml), dried (Na₂SO₄), filtered and concentrated *in vacuo.* The crude product was subjected to column chromatography (SiO₂; EtOAc) to give the title compound as a white solid (0.46g, 73%). δH (CDCl₃) 9.75 (1H, s), 8.69 (2H, d, J 5.9Hz), 7.64 (2H, d, J 6.0Hz), 7.25 (2H, d, J 8.2Hz), 7.20 (2H, d, J 8.2Hz), 5.89 (1H, d, J 8.3Hz), 5.06 (1H, dt, J 5.4, 8.2Hz), 4.30 (2H, q, J 7.1Hz), 3.35 (2H, m), 2.50 (3H,s), 1.84-1.33 (10H, m). m/z (ES⁺, 70V) 566.1 and 567.1 (MH⁺).

### EXAMPLE 58

### (2S)-2-{(2-Bromo-3-oxospiro[3.5]non-1-en-1-yl)amino}-3-{4-[(3-methyl[2.7]naphthyridin-1-yl)oxy]phenyl}propanoic acid

The compound of Example 57 (0.32g, 0.6mmol) was hydrolysed in a similar manner to the method of Example 2, to give the title compound as a white solid (0.20g, 66%). δH (DMSO d⁶) 9.61 (1H, s), 8.88 (1H, d, J 9.5Hz), 8.72 (1H, d, J 5.7Hz), 7.74 (1H, d, J 5.8Hz), 7.35 (3H, c), 7.24 (2H, d, J 8.6Hz), 4.77 (1H, m), 3.18 (1H, dd, J 13.7, 4.70Hz), 3.01 (1H, dd, J 13.7, 10.4Hz), 2.49 (3H, s), 1.78-1.12 (10H, m). m/z (ES⁺, 70V) 537.1 and 538.1 (MH⁺).

### EXAMPLE 59

### Ethyl (2S)-2-{[2-(phenylsulfanyl)-4,4-dimethyl-3-oxo-1-cyclobutenyl]amino}-3-{4-[(3,5-dichloroisonicotinoyl)amino]phenyl} propanoate

A solution of the compound of Example 5 (340mg, 0.76mmol) in THF (25ml), at room temperature, was treated dropwise with a solution containing phenyl sulphenyl chloride (122mg, 0.84mmol) in THF (2ml). After 10min the reaction mixture was poured into a mixture of EtOAc (150ml) and saturated aqueous NaHCO₃ solution (50ml). The organic layer was extracted and washed with brine (25ml), dried (MgSO₄), filtered and concentrated *in vacuo.* Chromatography (SiO₂; 100% EtOAc) gave the title compound as a white powder (346mg, 0.59mmol, 78%). δH (CDCI₃) 8.45 (2H, s), 8.05 (1H,, s), 7.43 (1H, d, J 8.4Hz), 7.15 (2H, d, J 8.4Hz), 7.11-7.04 (5H, m), 6.25 (1H, d, J 8.5Hz), 5.10-5.05 (1H, m), 4.09 (2H, q, J 7.1Hz), 3.11-3.06 (2H, m), 1.18 (3H, s), 1.15 (3H, s), 1.13 (3H, t, 7.1Hz). m/z (ES⁺, 70V) 584.0 (MH⁺).

### EXAMPLE 60

### (2S)-2-{[(2-(Phenylsulfanyl)-4,4-dimethyl-3-oxo-1-cyclobutenyl]-amino}-3-{4-[(3,5-dichloroisonicotinoyl)amino]phenyl}propanoic acid

Hydrolysis of the ethyl ester (340mg, 0.58mmol) with lithium hydroxide (60mg, 1.4mmol), according to the method of Example 2, gave the title compound (296mg, 0.53mmol, 90%) as a white powder. δH (DMSO d⁶, 390K) 10.30 (1H, br s), 8.68 (2H, s), 7.45 (2H, br s), 7.26-7.22 (2H, m), 7.15-7.08 (7H, m), 4.75-4.66 (1H, m), 3.17 (1H, dd, J 14.0, 5.3Hz), 3.04 (1H, dd J 14.0, 7.7Hz), 1.19 (3H, s), 1.16 (3H, s). m/z (ES⁺, 70V) 556.0, 557.9 (MH⁺).

### EXAMPLE 61

### Ethyl (2S)-2-[(2-chloro-3-oxospiro[3.5]non-1-en-1-yl)amino]-3-{4-[(3,5-dichloroisonicotinoyl)amino]phenyl}propanoate

A solution of the compound of Example 27 (366mg, 0.71mmol) in THF (25ml), at room temperature, was treated portionwise with N-chloro succinimide (100mg, 0.75mmol). After 30min the reaction mixture was poured into a mixture of EtOAc (150ml) and saturated aqueous NaHCO₃ solution (50ml). The organic layer was extracted and washed with brine (25ml), dried (MgSO₄), filtered and concentrated *in vacuo.* Chromatography (SiO₂; 70% EtOAc:hexanes) gave the title compound as a white powder (312mg, 0.56mmol, 80%). δH (CDCI₃) 8.50 (2H, s), 7.73 (1H, s), 7.53 (1H, d, J 8.4Hz), 7.04 (2H, d, J 8.4Hz), 5.73 (1H, d, J 8.0Hz), 4.88-4.81 (1H, m), 4.21 (2H, q, J 7.1Hz), 3.21-3.16 (2H, m), 1.79-1.65 (4H, m), 1.51-1.36 (6H, m), 1.28 (3H, t, J 7.1Hz). m/z (ES⁺,70V) 550.0 (MH⁺).

### EXAMPLE 62

### (2S)-2-[(2-Chloro-3-oxospiro[3.5]non-1-en-1-yl)amino]-3-{4-[(3,5-dichloroisonicotinoyl)amino]phenyl}propanoic acid

Hydrolysis of the compound of Example 61 (300mg, 0.54mmol) with lithium hydroxide (60mg, 1.4mmol), according to the method of Example 2, gave the title compound. δH (DMSO d⁶,390K) 10.44 (1H, br s), 8.69 (2H, s), 8.05-7.85 (1H, s br), 7.54 (2H, d, J 7.8Hz), 7.25 (2H, d, J 7.8Hz), 4.60-4.49 (1H, m), 3.21 (1H, dd, J 14.0, 5.3Hz), 3.04 (1H, dd, J 14.0, 5.1Hz), 1.80-1.21 (10H, m). m/z (ES⁺, 70V) 521.9, 525.9 (MH⁺).

### EXAMPLE 63

### Ethyl (2S)-2-[(2-iodo-3-oxospiro[3.5]non-1-en-1-yl)amino]-3-{4-[(3,5-dichloroisonicotinoyl)amino]phenyl}propanoate

To a stirred solution of the compound of Example 27 (1.0g, 1.9mmol) in THF (10ml) at room temperature was added N-iodosuccinamide (460mg, 2.0mmol) in one portion. After 5 minutes the mixture was concentrated in *vacuo* and the residue triturated with a mixture of ether (10ml) and water (10ml), filtered and washed with ether and water. Oven drying gave the title compound (802mg, 66%) as a yellow solid. δH (DMSO d⁶) 8.90 (1H, d, J 9.1Hz), 8.78 (2H, s), 7.58 (2H, d, J 8.5Hz), 7.25 (2H, d, J 8.5Hz), 4.91 (1H, m), 4.20 (2H, q, J 7.1Hz), 3.30-3.00 (2H, m), 1.80-1.24 (10H, m), 1.21 (3H, t, J 7.1Hz). m/z (ES⁺, 70V) 642.0 (MH⁺).

### EXAMPLE 64

### Ethyl (2S)-3-{4-[(3,5-dichloro-pyridine-4-carbonyl)-amino]-phenyl}-2-(3-oxo-2-pyridin-3-yl-spiro[3.5]non-1-en-1-ylamino)-propanoate

A mixture of the compound of Example 63 (1.0g, 1.6mmol), 10% palladium on charcoal (15mg), triphenylphosphine (100mg, 0.32mmol), copper (1) iodide (30mg, 0.16mmol), 3-pyridyl tributylstannane (560µl, 1.7mmol) in DMF (10ml) was heated to 100º under a nitrogen atmosphere fro 2 hours. The slvent was removed by evaporation in vacuo and the residue purified by column chromatography (SiO₂; 666:333:1 EtOAc:hexane;triethylamine) to give the title compound as a yellow oil (378mg, 41%). δH (DMSO d⁶) 8.76 (2H, s), 8.60 (1H, m), 8.30 (2H, br. s), 7.94 (1H, d, J 8.0Hz), 7.54 (2H, m), 7.34 (2H, m), 7.10 (1H, d, J 8.4Hz), 4.34 (1H, m), 4.24 (2H, q, J 5.3Hz), 3.25-2.95 (2H, m), 1.86-1.40 (10H, m), 1.26 (3H, t, J 5.3Hz). m/z (ES⁺, 70V) 593.0 (MH⁺).

### EXAMPLE 65

### (2S)-3-{4-[(3,5-Dichloro-pyridine-4-carbonyl)-amino]-phenyl}-2-(3-oxo-2-pyridin-3-yl-spiro[3.5]non-1-en-1-ylamino)-propanoic acid

The compound of Example 64 was hydrolysed in a similar manner to the method of Example 2 to give the title compound as a white solid (76%). δH (DMSO d⁶, 400K) 10.28 (1H, s), 8.66 (2H, s), 8.59 (1H, s), 8.34 (1H, m), 7.80 (1H, m), 7.69 (1H, m), 7.51 (2H, m), 7.24 (4H, m), 4.28 (1H, m), 3.25-3.07 (2H, m), 1.90-1.50 (10H, m). m/z (ES⁺, 70V) 565.0 (MH⁺).

### EXAMPLE 66

### Ethyl (2S)-3-{4-[(3,5-Dichloro-pyridine-4-carbonyl)-amino]-phenyl}-2-(2-iodo-4,4-dimethyl-3-oxo-cyclobut-1-enylamino)propanoate

Prepared in a similar manner to the compound of Example 63 from the compound of Example 5 to give the title compound as a white solid (72%). δH (DMSO d⁶) 9.17 (1H, d, J 9.1Hz), 8.79 (2H, s), 7.58 (2H, d, J 8.5Hz), 7.29 (2H, d, J 8.5Hz), 4.94 (1H, m), 4.20 (2H, q, J 7.1Hz), 3.25-3.00 (2H, m), 1.23 (3H, t, J 7.1Hz), 1.12 (3H, s), 1.03 (3H, s). m/z (ES⁺, 70V) 601.8 (MH⁺).

### EXAMPLE 67

### Ethyl (2S)-3-{4-[(3,5-Dichloro-pyridine-4-carbonyl)-amino]-phenyl}-2-(4,4-dimethyl-3-oxo-2-pyridin-3-yl-cyclobut-1-enylamino)propanoate

Prepared in a similar manner to the compound of Example 64 from the compound of Example 66 to give the title compound as a white solid (41%). δH (CDCl₃) 8.85 (1H, m), 8.57 (1H, m), 8.34 (3H, m), 7.92 (2H, m), 7.73 (2H, m), 7.28 (1H, m), 4.33 (1H, m), 4.15 (2H, q, J 7.1Hz), 3.32-3.09 (2H, m), 1.71 (3H, s), 1.33 (3H, s), 1.27 (3H, t, J 7.1Hz). m/z (ES⁺, 70V) 553.0 (MH⁺).

### EXAMPLE 68

### (2S)-3-{4-[(3,5-Dichloro-pyridine-4-carbonyl)-amino]-phenyl}-2-(4,4-dimethyl-3-oxo-2-pyridin-3-yl-cyclobut-1-enylamino)propanoic acid

The compound of Example 67 was hydrolysed in a similar manner to the method of Example 2 to give the title compound as a white solid (43%). δH (DMSO d⁶, 400K) 10.28 (1H, br. s), 8.66 (3H, m), 8.33 (1H, m), 8.09 (1H, m), 7.75 (1H, m), 7.52 (2H, m), 7.27 (3H, m), 4.25 (1H, m), 3.26 (1H, m), 3.14 (1H, m), 1.22 (3H, s), 1.06 (3H, s). m/z (ES⁺, 70V) 524.9 (MH⁺).

### EXAMPLE 69

### (2S)-2-[(2-iodo-3-oxospiro[3.5]non-1-en-1-yl)amino]-3-{4-[(3,5-dichloroisonicotinoyl)amino]phenyl}propanoic acid

The compound of Example 63 was hydrolysed in a similar manner to the method of Example 2 to give the title compound as a white solid (98%). δH (DMSO d⁶) 10.87 (1H, s), 8.84 (1H, d, J 9.3Hz), 8.79 (2H, s), 7.58 (2H, d, J 8.5Hz), 7.27 (2H, d, J 8.5Hz), 4.87 (1H, m), 3.25 (1H, m), 3.02 (1H, m), 1.70-1.25 (10H, m). m/z (ES⁺, 70V) 613.8 (MH⁺).

### EXAMPLE 70

### (2S)-3-{4-[(3,5-Dichloro-pyridine-4-carbonyl)-amino]-phenyl}-2-(2-iodo-4,4-dimethyl-3-oxo-cyclobut-1-enylamino)propanoic acid

The compound of Example 66 was hydrolysed in a similar manner to the method of Example 2 to give the title compound as a white solid (95%). δH (DMSO d⁶) 10.87 (1H, s), 9.08 (1H, d, J 9.3Hz), 8.78 (2H, s), 7.58 (2H, d, J 8.5Hz), 7.26 (2H, d, J 8.5Hz), 4.88 (1H, m), 3.25 (1H, m), 3.04 (1H, m), 1.12 (3H, s), 1.01 (3H, s). m/z (ES⁺, 70V) 573.8 (MH⁺).

### EXAMPLE 71

### Ethyl (2S)-3-{5-[(3,5-dichloroisonicotinoyl)amino]pyridin-2-yl}-2-[(3-oxaspiro[3.5]non-1-en-1-yl)amino]propanoate

To a solution of Intermediate 43 (470mg, 1.22mmol) in DCM (10ml) was added spiro[3.5]nonane-1, 3-dione (187mg, 1.23mmol) with stirred for 18hr. After evaporation of the solvent the crude product was pyrified by chromatography (silica, 3-4 % MeOH/DCM) to afford the title compound as a white foam (610mg, 96%). δH NMR (d⁶ DMSO) 8.81 (2H, s), 8.70 (1H, s), 8.33 (1H, d), 8.02 (1H, d), 7.32 (1H, d), 4.35 (1H, m), 4.13 (2H, m), 3.23 (2H, m), 1.53 (8H, br), 1.37 (2H, br), 1.17 (3H, t). m/z (ES⁺, 70V) 517 (MH⁺).

### EXAMPLE 72

### Ethyl (2S)-2-[(2-bromo-3-oxaspiro[3,5]non-1-en-1-yl)amino]-3-{5-[(3,5-dichloroisonicotinoyl)amino]pyridin-2-yl}propanoate

A solution of NBS (169mg, 0.94mmol) in dry DCM (5ml) was added to a stirred solution of the compound of Example 71 (490 mg, 0.94 mmol) in DCM (10ml) at 0°C (ice-water bath). After 30 min the solvent was evaporated *in vacuo* and the residue partitioned between Et₂O (80ml) and saturated sodium bicarbonate (20ml). The phases were separated and the aqueous layer re-extracted with Et₂O (40ml). The combined organics were washed with water (2 x 10ml), brine (10ml), dried (Na₂SO₄) and evaporated *in vacuo* and the residue purified by chromatography (silica, 50-80 % Et₂O/hexane) to give the title compound as a colourless glass foam (501mg, 88%). δH NMR (d⁶ DMSO) 11.17 (1H, s), 8.83 (2H, s), 8.73 (1H, s), 8.01 (1H, d), 7.34 (1H, d), 5.06 (1H, dd), 4.20 (2H, q), 3.39-3.20 (2H, brm), 1.73 (1H, m), 1.57 (8H, br), 1.34 (1H, br), 1.22 (3H, t). m/z (ES⁺, 70V) 596 (MH⁺).

### EXAMPLE 73

### Ethyl (2S)-2-[(2-bromo-3-oxaspiro[3.5]non-1-en-1-yl)amino]-3-{5-[(3,5-dichloroisonicotinoyl)amino]pyridin-2-yl}propanoate hydrochloride

The compound of Example 72 (300mg, 0.50mmol) was dissolved in EtOAc (20ml) and HCl gas bubbled through for a short time. The resulting white precipitate was collected by filtration, washed with Et₂O and dried to give the title compound as a white powder (155 mg, 48 %). δH NMR (d⁶ DMSO) 11.32 (1H, s), 8.84 (2H, s), 8.81 (1H, s), 8.13 (1H, d), 7.43 (1H, d), 5.06 (1H, dd), 4.19 (2H, q), 3.39 (1H, m), 3.28 (1H, m), 1.74 (1H, m), 1.57 (8H, br), 1.35 (1H, br), 1.22 (3H, t). m/z (ES⁺, 70V) 631 (MH⁺).

### EXAMPLE 74

### (2S)-2-[(2-bromo-3-oxaspiro[3.5]non-1-en-1-yl)amino]-3-{5-[(3,5-dichloroisonicotinoyl)amino]pyridin-2-yl}propanoic acid

The compound of Example 72 (370mg, 0.62mmol) was hydrolysed in a similar manner to the method of Example 2 to give the title compound as a white solid (200 mg, 56 %) as light yellow solid. δH NMR (d⁶ DMSO) 11.16 (1H, s), 8.83 (2H, s), 8.73 (1H, s), 8.05 (1H, d), 7.35 (1H, d), 5.00 (1H, dd), 2.76 (2H, brm), 1.55 (8H, m), 1.27 (1H, br), 1.12 (1H, br). m/z (ES⁺, 70V) 568 (MH⁺).

### EXAMPLE 75

### Ethyl (2S)-2-[(2-chloro-3-oxaspiro[3.5]non-1-en-1-yl)amino]-3-{5-[(3,5-dichloroisonicotinoyl)amino]pyridin-2-yl}propanoate

A solution of NCS (247mg, 1.85mmol) in dry THF (10ml) was added to a stirred (ice-water bath cooled) solution of the compound of Example 71 (800mg, 1.54mmol) in THF (10ml) and DCM (10ml). After 2hr the solvent was evaporated *in vacuo* and the residue partitioned between Et₂O (250ml) and saturated sodium bicarbonate (30ml). The phases were separated and the organic layer was washed with brine (10ml), dried (Na₂SO₄) and evaporated *in vacuo* and the residue purified by chromatography (silica, 70-100 % Et₂O/hexane) to give the title compound as white foam (620mg, 72%). δH NMR (d⁶ DMSO) 8.96 (2H, s), 8.86 (1H, s), 8.20 (1H, d), 7.50 (1H, d), 5.08 (1H, m), 4.32 (2H, q), 3.53-3.31 (2H, brm), 1.72 (9H, m), 1.50 (1H, br), 1.34 (3H, t). m/z (ES⁺, 70V) 551 (MH⁺).

### EXAMPLE 76

### Ethyl (2S)-2-[(2-chloro-3-oxaspiro[3,5]non-1-en-1-yl)amino]-3-{5-[(3,5-dichloroisonicotinoyl)amino]pyridin-2-yl}propanoate hydrochloride

The compound of Example 75 (269mg, 0.48mmol) was dissolved in EtOAc (20ml) and HCl gas bubbled through for a short time. The resulting precipitate was collected by filtration, washed with Et₂O and dried to give the title compound (230 mg, 80.3 %). δH NMR (d⁶ DMSO) 11.21 (1H, s), 8.83 (2H, s), 8.75 (1H, s), 8.08 (1H, d), 7.39 (1H, d), 4.95 (1H, m), 4.20 (2H, q), 3.36 (1H, m), 3.26 (1H, m), 1.71 (1H, m), 1.57 (8H, br), 1.35 (1H, m), 1.21 (3H, t). m/z (ES⁺, 70V) 587 (MH⁺).

### EXAMPLE 77

### (2S)-2-[(2-chloro-3-oxaspiro[3.5]non-1-en-1-yl)amino]-3-{5-[(3,5-dichloroisonicotinoyl)amino]pyridin-2-yl}propanoic acid

The compound of Example 72 (250mg, 0.45mmol) was hydrolysed in a similar manner to the method of Example 2 to give the title compound as a white powder (142mg, 60 %). δH NMR (d⁶ DMSO) 11.19 (1H, s), 8.83 (2H, s), 8.74 (1H, s), 8.07 (1H, d), 7.35 (1H, d), 4.90 (1H, m), 3.37 (1H, m), 3.19 (1H, m), 1.71-1.28 (10H, brm). m/z (ES⁺, 70V) 523 (MH⁺).

### EXAMPLE 78

### Ethyl (2S)-3-{5-[(3,5-dichloroisonicotinoyl)amino]pyridin-2-yl}-2-{[(2-(methylsulfanyl)-3-oxaspiro[3.5]non-1-en-1-yl)amino}propanoate

Sulphuryl chloride (49µL) was added dropwise to a stirred ice-bath cooled. solution of dimethyl sulfide (74µL) in THF (5ml). The ice bath was removed and the solution stirred for 45 min. This solution was added to a stirred solution of the compound of Example 71 (700mg, 1.35mmol) in THF (10ml) and DCM (10ml) and stirred at RT. The reaction was worked up in a similar manner to that of Example 79 to give the title compound.

### EXAMPLE 79

### Ethyl (2S)-3-{5-[(3,5-dichloroisonicotinoyl)amino]pyridin-2-yl}-2-{[(2-(isopropylsulfanyl)-3-oxaspiro[3.5]non-1-en-1-yl)amino}propanoate.

Sulphuryl chloride (218µL) was added dropwise to a stirred ice-bath cooled solution of isopropyl disulphide (432µL) in THF (10ml). The ice bath removed and the mixture stood for 35 min. 5ml of this solution was added to a stirred solution of the compound of Example 71 (700mg, 1.35mmol) in THF (10ml) and DCM (10ml) and stirred at RT for 15 min. The solvent removed and the residue was partitioned between Et₂O (130ml) and saturated sodium bicarbonate (30ml). The phases were separated and the organic layer was washed with brine (10ml), dried (Na₂SO₄) and evaporated *in vacuo* and the residue purified by chromatography (silica, 2-3% EtOH/DCM) to give the title compound as a white foam. m/z (ES⁺, 70V) 591 (MH⁺).

### EXAMPLE 80

### Ethyl (2S)-3-{5-[(3,5-dichloroisonicotinoyl)amino]pyridin-2-yl}-2-{[(4, 4-dimethyl-3-oxo-1-cyclobutenyl)amino}propanoate

To a solution of Intermediate 43 (792mg, 2.06mmol) in DCM (15ml) and THF (5ml) was added 2,2-dimethyl-1,3-cyclobutanedione (0.27g, 2.41 mmol) with stirring for 24hr. After evaporation of the solvent the crude product was purified by chromatography (silica, 4-8% EtOH/DCM) to give the title compound as a white foam (926 mg, 93 %). δH NMR (d⁶ DMSO), 8.78 (2H, s), 8.68 (1H, s), 8.53 (1H, d), 7.30 (1H, d), 4.38 (1H, s), 4.33 (1H, m), 4.11 (2H, q), 3.38-3.10 (2H, m), 1.14 (3H, t), 1.04 (3H, s), 0.94 (3H, s). m/z (ES⁺, 70V) 477 (MH⁺).

### EXAMPLE 81

### Ethyl (2S)-2-[(2-bromo-4, 4-dimethyl-3-oxo-1-cyclobutenyl)amino]-3-{5-[(3,5-dichloroisonicotinoyl)amino]pyridin-2-yl}propanoate

Prepared from the compound of Example 80 (600mg, 1.25mmol) in a similar manner to the method of Example 72 to give the title compound (530mg, 75%) as a white foam. δH NMR (d⁶ DMSO) 9.20 (1H, s), 8.95 (2H, s), 8.87 (1H, s), 8.20 (1H, d), 7.51 (1H, d), 5.19 (1H, m), 4.32 (2H, q), 3.53-3.30 (2H, m), 1.34 (3H, t), 1.26 (3H, s), 1.13 (3H, s). m/z (ES⁺, 70V) 556 (MH⁺).

### EXAMPLE 82

### Ethyl (2S)-2-[(2-bromo-4, 4-dimethyl-3-oxo-1-cyclobutenyl)amino]-3-[5-[(3,5-dichloroisonicotinoyl)amino]-2-pyridinl}propanoate hydrochloride

The compound of Example 81 was dissolved in EtOAc (10m) and HCl gas bubbled through for a short time. The resulting white precipitate was collected by filtration, washed with EtOAc the Et₂O and dried to give the title compound as a white powder (252mg). δH NMR (d⁶ DMSO) 11.30 (1H, s), 9.12 (1H, d), 8.81 (2H, s), 8.80 (1H, s), 8.10 (1H, d), 7.43 (1H, d), 5.04 (1H, m), 4.18 (2H, q), 3.30 (2H, m), 1.20 (3H, t), 1.12 (3H, s), 1.00 (3H, s). m/z (ES⁺, 70V) 592 (MH⁺).

### EXAMPLE 83

### Ethyl (2S)-2-[(2-bromo-4,4dimethyl-3-oxo-1-cyclobutenyl)amino]-3-{5-[(3,5-dichloroisonicotinoyl)amino]pyridin-2-yl}propanoic acid

The compound of Example 81 (200mg, 3.59mmol) was hydrolysed in a similar manner to the method of Example 2 to give the title compound as a white amorphous solid (110 mg, 58 %). δH NMR (d⁶ DMSO), 8.96 (1H, d), 8.81 (2H, s), 8.72 (1H, s), 8.04 (1H, d), 7.34 (1H, d), 4.96 (1H, m), 3.35-3.15 (2H, m), 1.11 (3H, s), 0.96 (3H, s). m/z (ES⁺, 70V) 528 (MH⁺).

### EXAMPLE 84

### Ethyl (2S)-2-[(2-chloro-4,4-dimethyl-3-oxo-1-cyclobutenyl)amino]-3-{5-[(3,5-dichloroisonicotinoyl)amino]pyridin-2-yl}propanoate

A solutionof NCS (294mg, 2.20mmol) in dry DCM (10ml) was added to a solution of the compound of Example 80 (869mg, 1.82mmol) in THF (10ml) at between -10° to 10°with stirring for 1.5 hr. After 30 min the solvent was evaporated in vacuo and the residue purified by chromatography (silica, 4-8% EtOH/ DCM) to give the title compound as a light yellow foam (786 mg, 84 %). δH NMR (d⁶ DMSO) 11.18 (1H, s), 9.02 (1H, d), 8.83 (2H, s), 8.75 (1H, s), 8.08 (1H, d), 7.37 (1H, d), 4.99 (1H, m), 4.20 (2H, q), 3.40-3.21 (2H, m), 1.22 (3H, t), 1.13 (3H, s), 1.01 (3H, s). m/z (ES⁺, 70V) 511 (MH⁺).

### EXAMPLE 85

### (2S)-2-[(2-chloro-4,4-dimethyl-3-oxo-1-cyclobutenyl)amino]-3-{5-[(3,5-dichloroisonicotinoyl)amino]pyridin-2-yl}propanoic acid

The compound of Example 84 (560mg, 1.09mmol) was hydrolysed in a similar manner to the method of Example 2 to give the title compound as an off-white powder (370 mg, 70 %). δH NMR (d⁶ DMSO) 11.17 (1H, s), 9.94 (1H, d), 8.83 (2H, s), 8.75 (1H, s), 8.06 (1H, d), 7.35 (1H, d), 4.91 (1H, m), 3.37-3.16 (2H, m), 1.12 (3H, s), 0.97 (3H, s). m/z (ES⁺, 70V) 483 (MH⁺).

### EXAMPLE 86

### Ethyl (2S)-3-{4-[(3,5-Dichloroisonicotinoyl)amino]phenyl}-2-[2-(1-methyl-pyridin-3-yl)-3-oxo-spiro[3.5]non-1-en-1-ylamino]-propanoate iodide salt

To a stirred solution of the compound of Example 64 (126mg, 0.21mmol) in DMF (1ml) was added iodomethane (14mL, 0.23mmol). After 18 hrs the solvent was removed *in vacuo* to give the crude title compound which was used without further purification. m/z (ES⁺, 70V) 607.0 (MH⁺).

### EXAMPLE 87

### Ethyl (2S)-3-{4-[(3,5-Dichloroisonicotinoyl)amino]phenyl}-2-[2-(1-methyl-piperidin-3-yl)-3-oxo-spiro[3.5]non-1-en-1-ylamino]propanoate

The compound of Example 86 (127mg, 0.21 mmol) was dissolved in EtOH (10ml) and hydrogenated over platinum dioxide (50mg) at room temperature and 1 atmosphere hydrogen for 5 days. The catalyst was removed by evaporation in vacuo to afford the title compound as a yellow oil (129mg, 100%). δH NMR (d⁶ DMSO) 10.48 (1H, br s), 8.70 (2H, s), 7.59 (2H, d, J 8.1Hz), 7.30 (2H, d, J 8.1Hz), 4.25 (1H, m), 4.22 (2H, q, J 4.0Hz), 3.23 (1H, m), 3.08 (1H, m), 1.70-1.50 (22H, m), 1.26 (3H, m). m/z (ES⁺, 70V) 613.2 (MH⁺).

### EXAMPLE 88

### (2S)-3-{4-[(3,5-Dichloroisonicotinoyl)amino]-phenyl}-2-[2-(1-methyl-piperidin-3-yl)-3-oxo-spiro[3.5]non-1-en-1-ylamino]propanoic acid

The compound of Example 87 was hydrolysed in a similar manner to the method of Example 2. The product was purified by passage through a short column (RP-18-silica; 5% aqueous acetonitrile) to give the title compound as a yellow solid (52%). δH NMR (d⁶ DMSO) 10.47 (1H, br s), 8.70 (2H, s), 7.57 (2H, d, J 7.7Hz), 7.27 (2H, d, J 7.7Hz), 4.13 (1H, m), 3.19 (1H, m), 3.02 (1H, m), 2.27 (3H, s), 1.70-1.30 (10H, m). m/z (ES⁺, 70V) 585.1 (MH⁺).

### EXAMPLE 89

### (2S)-Ethyl-2-[(2-chloro-3-oxo-7-oxa-spiro[3,5]non-1-en-1-yl)amino]-3-{4-[(3,5-dichloroisonicotinoyl)amino]phenyl}propanoate

A stirred solution of the compound of Example 38 (800mg, 1.54mmol) in THF (25ml), at rt was treated in several portions with N-chloro succinimide (226mg, 1.69mmol). After 1h the crude reaction was partitioned between EtOAc (150ml) and brine (100ml). The organic layer was removed and washed with a further 100ml of brine and the organic phase dried (MgSO₄), filtered and concentrated *in vacuo.* Chromatography (SiO_{2;} 50% EtOAc:hexanes) gave the title compound as a white powder (625mg, 1.13mmol, 67%). δH (DMSO d⁶, 390K) 10.39 (1H, br s), 8.69 (2H, s), 8.39 (1H, d, J 8.8Hz), 7.57 (2H, s), 7.29 (2H, d, J 8.4Hz), 4.72 (1H, m), 4.24 (2H, q, J 7.1Hz), 3.83-3.77 (2H, m), 3.73-3.62 (2H, m), 3.28 (1H, dd, J 5.5, 14.2Hz), 2.04-1.93 (2H, m), 1.54-1.51 (1H, m), 1.44-1.42 (1H, m), 1.27 (3H, t, J 7.1Hz). m/z (ES⁺,70V) 554 (MH⁺).

### EXAMPLE 90

### (2S)-2-[(2-Chloro-3-oxo-7-oxa-spiro[3,5]non-1-en-1-yl)amino]-3-{4-[(3,5-dichloroisonicotinoyl]-amino]phenyl}propanoic acid

Hydrolysis of the compound of Example 89 (525mg, 0.95mmol) with lithium hydroxide (80mg, 1.7mmol), according to the method of Example 2, gave the title compound (412mg, 0.79mol, 83%). δH (DMSO d⁶, 390K) 10.40 (1H, s), 8.68 (2H, s), 8.30 (1H, br s), 7.55 (2H, d, J 5.8Hz), 7.27 (2H, d, J 5.8Hz), 4.63 (1H, m), 3.80-3.73 (2H, m), 3.69-3.61 (2H, m), 3.26 (1H, dd, J 4.9, 14.1Hz), 3.07 (1H, dd, J 9.1, 14.1Hz), 1.97-1.90 (2H, m), 1.51-1.48 (1H, m), 1.40-1.37 (1H, m). m/z (ES⁺, 70V) 524.0 (MH⁺).

### EXAMPLE 91

### (2S)-Ethyl-2-[(2-Chloro-3-oxo-spiro[3.6]dec-1-en-1-yl)amino]-3-{4-[(3,5-dichloro-isonicotinoyl)-amino]-phenyl}propanoate

Was prepared according to the method of Example 89 from the compound of Example 45 (800mg, 1.51mmol) and N-chloro succinimide (222mg, 1.66mmol) to give the title compound as a white powder (625mg, 1.11mmol, 74%). δH (DMSO d⁶, 390K) 10.40 (1H, s), 8.70 (2H, s), 8.11 (1H, s br), 7.57 (2H, s br), 7.29 (2H, d, J 8.3Hz), 4.68 (1H, m), 4.24 (2H, q, J 7.1Hz), 3.28 (1H. dd, J 5.5, 14.3Hz), 3.12 (1H, dd, J 9.1, 14.3Hz), 1.82-1.52 (12H, m), 1.27 (3H, t, J 7.1Hz). m/z (ES⁺, 70V) 566.0 (MH⁺).

### EXAMPLE 92

### (2S)-2-[(2-Chloro-3-oxo-spiro[3.6]dec-1-en-1-yl)amino]-3-{4-[(3,5-dichloroisonicotinoyl)amino]phenyl}-propanoic acid

Hydrolysis of the compound of Example 91 (600mg, 1.07mmol) with lithium hydroxide (80mg, 1.7mmol), according to the method of Example 2, gave the title compound (512mg, 0.95mol, 89%). δH (DMSO d⁶, 390K) 10.37 (1H, s), 8.67 (2H, s), 7.52 (2H, m), 7.25 (2H, d, J 8.3Hz), 4.44 (1H, m), 3.22 (1H, dd, J 5.2, 14.0Hz), 3.13 (1H, dd, J 8.0, 13.9Hz), 1.98-1.41 (12H, m). m/z (ES⁺, 70V) 536.0 (MH⁺).

### EXAMPLE 93

### (2S)-Ethyl-2-[4,4-dimethyl-2-(1-methyl-1H-tetrazol-5-ylsulfanyl)-3-oxo-cyclobut-1-enylamino]-3-{4-[(3,5-dichloroisonicotinoyl)amino]-phenyl}propanoate

A stirred solution of the compound of Example 5 (1.03g, 2.16mmol) in THF (50ml) at rt, was treated with a slurry of 1-methyl-1H-tetrazol-5-ylsulfanyl chloride (360mg, 2.4mmol) in DCM (2ml). After 30min the crude reaction was partitioned between EtOAc (150ml) and saturated aqueous sodium hydrogen carbonate solution (100ml). The organic phase was removed, washed with brine (100ml), dried (MgSO₄) and concentrated in *vacuo.* Chromatography (SiO₂; EtOAc) gave the title compound as a white powder (1.12g, 1.89mmol, 88%). δH (DMSO d⁶, 390K) 10.40 (1H, s), 9.08 (1H, d, J 2.6Hz), 8.67 (2H, s), 7.55 (2H, d, J 6.2Hz), 7.24 (2H, d, J 6.2Hz), 5.06 (1H, m), 4.17 (2H, q, J 7.1Hz), 4.05 (3H, s), 3.27 (1H, dd, J 5.5, 14.2Hz), 3.12 (1H, dd, J 8.9, 14.2Hz), 1.23 (3H, s), 1.22 (3H, t, J 7.1Hz), 1.20 (3H, s). m/z (ES⁺,70V) 590.0 (MH⁺).

### EXAMPLE 94

### (2S)-2-[4,4-Dimethyl-2-(1-methyl-1H-tetrazol-5-ylsulfanyl)-3-oxo-cyclobut-1-enylamino]-3-{4-[(3,5-dichloroisonicotinoyl)amino]-phenyl}propanoic acid

Hydrolysis of the compound of Example 93 (640mg, 1.08mmol) with lithium hydroxide (80mg, 1.7mmol), according to the method of Example 2, gave the title compound (517mg, 0.92mol, 85%). δH (DMSO d⁶, 390K) 10.41 (1H, s), 9.35 (1H, d, J 2.6Hz), 8.67 (2H, s), 7.51 (2H, d, J 5.9Hz), 7.22 (2H, d, J 7.5Hz), 4.93 (1H, m), 3.97 (3H, s), 3.26 (1H, dd, J 5.5, 14.2Hz), 3.09 (1H, dd, J 8.9, 14.2Hz), 1.17 (3H, s), 1.10 (3H, s). m/z (ES⁺, 70V) 562.0 (MH⁺).

### EXAMPLE 95

### (2S)-Ethyl-2-[(3,7,7-trioxo-7λ⁶-thia-spiro[3.5]non-1-en-1-yl)amino]-3-{4-[(3,5-dichloroisonicotinoyl)amino]phenyl}-propanoate

Prepared from Intermediate 45 (1.1g, 5.4mmol) and the free base of Intermediate 27 (2.08mg, 5.5mmol), in a similar manner to the compound of Example 11 to give the title compound as a white powder (712mg, 1.25mmol, 23%). δH (CDCl₃, 300K) 8.51 (1H, s), 8.33 (2H, s), 7.37 (2H, d, J 8.2Hz), 6.96 (2H, d, J 8.2Hz), 4.25 (1H, s), 4.10 (2H q, J 7.1Hz), 4.01 (1H, m), 3.40-3.33 (2H, m), 3.06 (1H, dd, J 4.5, 14.2Hz), 2.90 (1H, dd, J 14.1, 8.0Hz), 2.79-2.75 (2H, m), 2.38-2.31 (2H, m), 1.99-1.96 (1H, m), 1.86-1.81 (1H, m), 1.16 (3H, t, J 7.1Hz). m/z (ES⁺, 70V) 565.9 (MH⁺).

### EXAMPLE 96

### (2S)-Ethyl-2-[(2-bromo-3,7,7-trioxo-7λ⁶-thia-spiro[3.5]non-1-en-1-yl)amino]-3-{4-[(3,5-dichloroisonicotinoyl)amino]phenyl}-propanoate

A solution of the compound of Example 95 (435mg, 0.77mmol) in THF (18ml) at 0º was treated portionwise with N-bromo succinimide (151mg, 0.85mmol). After 10min the reaction was partitioned between EtOAc (100ml) and saturated aqueous sodium hydrogencarbonate solution (50ml), the organic phase removed, dried (MgSO₄) and concentrated *in vacuo.* Chromatography (SiO₂, 50% EtOAc:hexanes) gave the title compound as a white powder (411mg, 0.64mmol, 83%). δH (DMSO d⁶, 390K) 10.43 (1H, s), 9.00 (1H, d, J 8.4Hz), 8.69 (2H, s), 7.58 (2H, d, J 6.8Hz), 7.28 (2H, d, J 6.8Hz), 4.85 (1H, m), 4.23 (2H, q, J 7.1Hz), 3.37-3.25 (3H, m, overlapping), 3.13-3.03 (3H, m, overlapping), 2.56-2.45 (2H, m), 2.09-1.89 (2H, m), 1.27 (3H, t, J 7.1Hz). m/z (ES⁺, 70V) 645.9 (MH⁺).

### EXAMPLE 97

### (2S)-2-[(2-Bromo-3,7,7-trioxo-7λ⁶-thia-spiro[3.5]non-1-en-1-yl)amino]-3-{4-[(3,5-dichloroisonicotinoyl)amino]phenyl}-propanoic acid

Hydrolysis of the compound of Example 96 (410mg, 0.63mmol) with lithium hydroxide (31 mg, 0.7mmol), according to the method of Example 2, gave the title compound (371mg, 0.60mol, 95%). δH (DMSO d⁶, 390K) 10.41 (1H, s), 9.35 (1H, d, J 2.6Hz), 8.69 (2H, s), 7.55 (2H, d, J 6.7Hz), 7.27 (2H, d, J 6.7Hz), 4.70 (1H, m), 3.37-3.25 (5H, m), 3.05 (1H, dd, J 5.4, 13.3Hz), 2.3 (2H, m), 2.02 (1H, m), 1.92 (1H, m). m/z (ES⁺, 70V) 617.8 (MH⁺).

### EXAMPLE 98

### (2S)-Ethyl-2-[4,4-dimethyl-2-(1-methyl-1H-imidazol-2-ylsulfanyl)-3-oxo-cyclobut-1-enylamino]-3-{4-[(3,5-dichloroisonicotinoyl)amino]phenyl}-propanoate

Prepared from the compound of Example 5 (1.0g, 2.1mmol) and 1-methyl-1H-imidazol-2-ylsulfenyl chloride (340mg, 2.3mmol), in a similar manner to the compound of Example 93 to give title compound as a white powder (1.03g, 1.75mmol, 83%). δH (DMSO d⁶, 390K) 10.84 (1H, s), 9.19 (1H, d, J 9.0Hz), 8.79 (2H, s), 7.53 (2H, d, J 8.5Hz), 7.23 (2H, d, J 8.5Hz), 7.21 (1H, d, J 1.1Hz), 6.88 (1H, d, J 1.1Hz), 5.46 (1H, m), 4.16 (2H, q, J 7.1Hz), 3.62 (3H, s), 3.26 (1H, dd, J 5.2, 14.2Hz), 3.06 (1H, dd, J 8.9, 14.2Hz), 1.20 (3H, t, J 7.1Hz), 1.09 (3H, s), 0.97 (3H, s). m/z (ES⁺, 70V) 590.0 (MH⁺).

### EXAMPLE 99

### (2S)-2-[4,4-Dimethyl-2-(1-methyl-1H-imidazol-2-yllsulfanyl)-3-oxo-cyclobut-1-enylamino]-3-{4-[(3,5-dichloroisonicotinoyl)-amino]-phenyl}-propanoic acid

Hydrolysis of the compound of Example 98 (760mg, 1.29mmol) with lithium hydroxide (100mg, 2.2mmol), according to the method of Example 2, gave the title compound (412mg, 0.74mol, 57%). δH (DMSO d⁶, 390K) 10.83 (1H, s), 9.11 (1H, d, J 9.0Hz), 8.79 (2H, s), 7.51 (2H, d, J 8.5Hz), 7.23 (2H, d, J 8.5Hz), 7.21 (1H, d, J 1.1Hz), 6.90 (1H, d, J 1.1Hz), 5.38 (1H, m), 3.63 (3H, s), 3.25 (1H, dd, J 5.2, 14.2Hz), 3.05 (1H, dd, J 8.9, 14.2Hz), 1.07 (3H, s), 0.96 (3H, s). m/z (ES⁺, 70V) 562.0 (MH⁺).

### EXAMPLE 100

### (2S)-Ethyl-2-[(3-thioxo-spiro[3.5]non-1-en-1-yl)amino]-3-{4-[(3,5-dichloroisonicotinoyl)amino]-phenyl}propanoate

A solution of the compound of Example 27 (700mg, 1.36mmol) and Lawesson's reagent [2,4-bis(4-methoxyphenyl)-1,2,3,4-dithiadiphosphetane 2,4-disulphide] (561 mg, 1.38mmol) in toluene (25ml) was heated to 80º for 24h. The crude reaction was then partitioned between EtOAc (100ml) and brine (100ml). The organic phase was separated, dried (MgSO₄) and concentrated *in vacuo.* Chromatography (SiO₂, EtOAc) gave the title compound as a yellow powder (621 mg, 1.17mmol, 86%). δH (DMSO d⁶, 390K) 10.84 (1H, s), 8.96 (1H, d, J 8.9Hz), 8.78 (2H, s), 7.56 (2H, d, J 7.9Hz), 7.25 (2H, d, J 7.9Hz), 5.48 (1H, s), 4.37 (1H, m), 4.18 (2H, q, J 7.1Hz), 3.20 (1H, dd, J 4.9, 13.9Hz), 3.04 (1H, dd, J 9.9, 13.9Hz), 1.96-1.87 (2H, m), 1.63-1.42 (8H, m), 1.21 (3H, t, J 7.1Hz). m/z (ES⁺, 70V) 532.0 534.0 (MH⁺).

### EXAMPLE 101

### (2S)-2-[(3-Thioxo-spiro[3.5]non-1-en-1-yl)amino]-3-{4-[(3,5-dicloroisonicotinoyl)amino]phenyl}-propanoic acid

Hydrolysis of the compound of Example 100 (340mg, 0.66mmol) with lithium hydroxide (30mg, 0.67mmol), according to the method of Example 2, gave the title compound (287mg, 0.57mol, 86%). δH (DMSO d⁶, 390K) 10.84 (1H, s), 8.87 (1H, d, J 8.8Hz), 8.77 (2H, s), 7.54 (2H, d, J 8.3Hz), 7.24 (2H, d, J 8.3Hz), 5.45 (1H, s), 4.23 (1H, m), 3.21 (1H, dd, J 4.4, 13.9Hz), 3.00 (1H, dd, J 9.9, 13.9Hz), 1.96-1.87 (2H, m), 1.67-1.41 (8H, m). m/z (ES⁺, 70V) 562.0 (MH⁺).

### EXAMPLE 102

### (2S)-2-[(3-oxo-spiro[3.4]oct-1-en-1-yl)amino]-3-{4-[(3,5-dichloroisonicotinoyl)amino]phenyl}-propanoate

A solution of 3-hydroxy-spiro[3.4]oct-2-en-1-one (330mg, 2.39mmol) [prepared according to the method of Wasserman, H.H. et al J. Org. Chem, 38, 1451-1455, (1973)] and the free base of Intermediate 27 (911 mg, 2.39mmol), in DCM (5ml), was stirred at rt for 48h. The volatiles were removed *in vacuo* and the residue chromatographed (SiO₂; EtOAc) to give the title compound as a white solid (1.03g, 2.05mmol, 86%). δH (CDCl₃, 300K) 8.97 (1H, s), 8.41 (2H, s), 7.51 (2H, d, J 8.5Hz), 7.01 (2H, d, J 8.5Hz), 5.89 (1H, d, J 7.5Hz), 4.39 (1H, s), 4.21 (3H, obscured m), 3.15 (1H, dd, J 5.3, 14.0Hz), 3.03 (1H, dd, J 5.8, 14.0Hz), 1.74-1.49 (10H, m), 1.27 (3H, t, J 7.1Hz). m/z (ES⁺, 70V) 502.0 (MH⁺).

### EXAMPLE 103

### (2S)-2-[(3-Oxo-spiro[3.4]oct-1-en-1-yl)amino]-3-{4-[(3,5-dichloroisonicotinoyl)amino]-phenyl}propanoic acid

Hydrolysis of the compound of Example 102 (150mg, 0.30mmol) with lithium hydroxide (30mg, 0.67mmol), according to the method of Example 2, gave the title compound (112mg, 0.23mol, 79%). δH (DMSO d⁶, 390K) 13.08 (1H, s), 10.87 (1H, s), 8.79 (2H, s), 8.39 (1H, d, J 8.5Hz), 7.57 (2H, d, J 8.2Hz), 7.26 (2H, d, J 8.2Hz), 4.39 (1H, s), 4.14 (1H, m), 3.16 (1H, dd, J 4.7, 13.8Hz), 2.98 (1H, dd, J 9.4, 13.8Hz), 1.73-1.58 (10H, m). m/z (ES⁺, 70V) 473.9 (MH⁺).

### EXAMPLE 104

### (2S)-Ethyl-2-[(2-chloro-3-oxo-spiro[3.4]oct-1-en-1-yl)amino]-3-{4-[(3,5-dichloroisonicotinoyl)amino]phenyl}-propanoate

Prepared from the compound of Example 102 (1.25g, 2.49 mmol) and N-chloro succinimide (333mg, 2.7mmol), according to the method of Example 61, to give the title compound as a white powder (1.13g, 2.1mmol, 84%). δH (DMSO d⁶, 390K) 10.41 (1H, s), 8.68 (2H, s), 8.33 (1H, d, J 5.9Hz), 7.57 (2H, m), 7.27 (2H, m), 4.66 (1H, m), 4.21 (2H, q, J 7.1Hz), 3.26 (1H, dd, J 5.3, 14.1Hz), 3.11 (1H, dd, J 9.0, 14.1Hz), 1.98-1.58 (10H, m), 1.23 (3H, t, J 7.1Hz). m/z (ES⁺, 70V) 535.9 (MH⁺).

### EXAMPLE 105

### (2S)-2-[(2-Chloro-3-oxo-spiro[3.4]oct-1-en-1-yl)amino]-3-{4-[(3,5-dichloro-pyridine-4-carbonyl)amino]phenyl}-propanoic acid

Hydrolysis of the compound of Example 104 (1.10g, 2.05mmol) with lithium hydroxide (100mg, 2.2mmol), according to the method of Example 2, gave the title compound (976mg, 1.92mol, 94%). δH (DMSO d⁶, 390K) 10.41 (1H, s), 8.69 (2H, s), 8.26 (1H, s), 7.57 (2H, d, J 6.2Hz), 7.28 (2H, d, J 6.2Hz), 4.61 (1H, m), 3.26 (1H, dd, J 5.0, 14.1Hz), 3.08 (1H, dd, J 9.1, 14.1Hz), 1.92-1.60 (10H, m). m/z (ES⁺, 70V) 509.9 (MH⁺).

### EXAMPLE 106

### (2S)-Ethyl-2-[(2-bromo-3-oxo-spiro[3,4]oct-1-en-1-yl)amino]-3-{4-[(3,5-dichloroisonicotinoyl)amino]phenyl}-propanoate

A solution of the compound of Example 102 (1.25g, 2.49mmol) in THF (25ml) at rt was treated with N-bromo succinimide (443mg, 2.49mmol).

After 30min the reaction was diluted with EtOAc (100ml), washed with saturated aqueous sodium hydrogencarbonate solution (50ml) and the organic phase separated, dried (MgSO₄) and concentrated *in vacuo.* Chromatography (SiO₂, EtOAc) gave the title compound as a white powder (1.27g, 2.18mmol, 87%). δH (DMSO d⁶, 390K) 10.43 (1H, s), 8.69 (2H, s), 8.42 (1H, d, J 8.9Hz), 7.58 (2H, d, J 6.7Hz), 7.29 (2H, d, J 6.7Hz), 4.77 (1H, s), 4.22 (2H, q, J 7.1Hz), 3.26 (1H, dd, J 5.4, 14.1Hz), 3.12 (1H, dd, J 9.0, 14.1Hz), 1.98-1.62 (8H, m), 1.25 (3H, t, J 7.1Hz). m/z (ES⁺, 70V) 582.0 (MH⁺).

### EXAMPLE 107

### (2S)-2-[(2-Bromo-3-oxo-spiro[3,4]oct-1-en-1-yl)amino]-3-{4-[(3,5-dichloroisonicotinoyl)amino]phenyl)-propanoic acid

Hydrolysis of the compound of Example 106 (900mg, 1.54mmol) with lithium hydroxide (100mg, 2.2mmol), according to the method of Example 2, gave the title compound (721 mg, 1.3mmol, 84%). δH (DMSO d⁶, 390K) 10.39 (1H, s), 8.68 (2H, s), 8.12 (1H, s br), 7.54 (2H, d, J 8.2Hz), 7.27 (2H, d, J 8.2Hz), 4.64 (1H, m), 3.25 (1H, dd, J 5.1, 14.1Hz), 3.11 (1H, dd, J 8.6, 14.1Hz), 1.92-1.62 (8H, m). m/z (ES⁺, 70V) 553.9 (MH⁺).

### EXAMPLE 108

### (2S)-Ethyl-2-[(2-methylsulfanyl-3-oxo-spiro[3.5]non-1-en-1-yl)amino]-3-{4-[(3,5-dichloroisonicotinoyl)amino]phenyl}propanoate

A solution of the compound of Example 27 (1.0g, 1.94mmol) in THF (25ml) at rt was treated dropwise with a solution of methanesulfenyl chloride in DCM (2.13ml, 1.0M) [prepared according to the method of Still, I. W. J., et al. J. Org. Chem., 1982, 47, 560]. After 20min the reaction was diluted with EtOAc (100ml) and washed with saturated aqueous sodium hydrogencarbonate solution (50ml). The organic phase was separated, dried (MgSO₄), filtered and concentrated *in vacuo.* Chromatography (SiO₂, 60% EtOAc:hexanes) gave the title compound as a white powder (1.03g, 1.83mmol, 94%). δH (DMSO d⁶, 390K) 10.86 (1H, s), 8.78 (2H, s), 8.70 (1H, d, J 9.2Hz), 7.57 (2H, d, J 8.4Hz), 7.26 (2H, d, J 8.4Hz), 5.11 (1H, m), 4.18 (2H, q, J 7.1Hz), 3.20 (1H, dd, J 4.6, 13.9Hz), 3.00 (1H, dd, J 9.8, 13.9Hz), 1.93 (3H, s), 1.66-1.33 (10H, m), 1.21 (3H, t, J 7.1Hz) . m/z (ES⁺, 70V) 562.1 (MH⁺).

### EXAMPLE 109

### (2S)-2-[(2-Methylsulfanyl-3-oxo-spiro[3.5]non-1-en-1-yl)amino]-3-{4-[(3,5-dichloroisonicotinoyl)amino]phenyl}-propanoic acid

Hydrolysis of the compound of Example 108 (600mg, 1.07mmol) with lithium hydroxide (100mg, 2.2mmol), according to the method of Example 2, gave the title compound as a white powder (421 mg, 0.79mmol, 73%). δH (DMSO d⁶, 390K) 10.84 (1H, s), 8.77 (2H, s), 8.44 (1H, d, J 8.8Hz), 7.54 (2H, d, J 8.4Hz), 7.23 (2H, d, J 8.4Hz), 4.90 (1H, m), 3.19 (1H, dd, J 4.4, 13.7Hz), 2.98 (1H, dd, J 9.1, 13.7Hz), 1.93 (3H, s), 1.79-1.54 (8H, m), 1.36-1.22 (2H, m). m/z (ES⁺, 70V) 534.0 (MH⁺).

### EXAMPLE 110

### (2S)-Ethyl-2-[(2-fluoro-3-oxo-spiro[3.5]non-1-en-1-yl)amino]-3-{4-[(3,5-dichloroisonicotinoyl)amino]phenyl}-propanoate

A solution of the compound of Example 27 (2.02g, 3.91 mmol) in THF (55ml) was treated with Selectfluor™ reagent (1.38g, 3.89mmol) and heated to 70°. After 48h the reaction was diluted with EtOAc (300ml) and washed with saturated aqueous sodium hydrogencarbonate solution (50ml). The organic phase was dried (MgSO₄), filtered and concentrated *in vacuo.* Chromatography (SiO₂, 60% EtOAc:hexanes) gave the title compound as a white powder (1.87g, 3.50mmol, 89%). δH (DMSO d⁶, 390K) 10.89 (1H, s), 8.81 (2H, s), 8.47 (1H, d, J 8.7Hz), 7.59 (12H, d, J 8.5Hz), 7.27 (2H, d, J 8.5Hz), 4.26 (1H, m), 4.19 (2H, q, J 7.1Hz), 3.21 (1H, dd, J 4.9, 13.8Hz), 2.98 (1H, dd, J 9.8, 13.8Hz), 1.70-1.38 (10H, m), 1.22 (3H, t, J 7.1Hz), m/z (ES⁺, 70V) 534.1 (MH⁺).

### EXAMPLE 111

### (2S)-2-(2-Fluoro-3-oxo-spiro[3,5]non-1-en-1-ylamino)3-{4-[(3,5-dichloroisonicotinoyl)amino]phenyl}-propanoic acid

Hydrolysis of the compound of Example 110 (273mg, 0.51mmol) with lithium hydroxide (60mg, 1.3mmol), according to the method of Example 2, gave the title compound as a white powder (197mg, 0.39mmol, 76%). δH (DMSO d⁶, 390K) 10.87 (1H, s), 8.80 (2H, s), 8.30 (1H, d, J 8.7Hz), 7.58 (2H, d, J 8.2Hz), 7.25 (2H, d, J 8.2Hz), 4.15 (1H, m), 3.19 (1H, dd, J 4.5, 13.8Hz), 2.96 (1H, dd, J 9.5, 13.8Hz), 1.92-1.49 (8H, m), 1.37-1.16 (2H, m). m/z (ES⁺, 70V) 506.0 (MH⁺).

### EXAMPLE 112

### (2S)-2-[(2-Fluoro-3-oxo-7-oxa-spiro[3.5]non-1-en-1-yl)amino]-3-{4-[(3,5-dichloroisonicotinoyl)amino]phenyl}-propanoic acid

A mixture of the compound of Example 38 (1.0g, 1.93mmol) and Selectfluor^{TM} reagent (1.0g, 2.8mmol) in THF (25ml) was heated to reflux for 72h. The crude reaction was then diluted with EtOAc (100ml) and washed with saturated aqueous sodium hydrogencarbonate solution (50ml). The organic phase was dried (MgSO₄), filtered and concentrated *in vacuo* to give a gum. This was then redissolved in THF (20ml) and treated with lithium hydroxide (80mg, 1.78mmol) and water (1ml) and stirred for 24h at rt. Acidification with a few drops of AcOH followed by concentration *in vacuo* and chromatography (SiO₂, 10:90:3:2 MeOH:DCM:AcOH:H2O) gave the title compound as a white powder (561 mg, 1.1mmol, 57%). δH (DMSO d⁶, 390K) 10.89 (1H, s), 8.80 (2H, s), 8.58 (1H, d, J 8.8Hz), 7.59 (2H, d, J 8.4Hz), 7.27 (2H, d, J 8.4Hz), 4.23 (1H, m), 3.76 (2H, m), 3.60 (2H, m), 3.23 (1H, dd, J 4.4, 13.9Hz), 2.96 (1H, dd, J 9.8, 13.9Hz), 1.98-1.93 (2H, m), 1.47 (1H, m), 1.30 (1H, m). m/z (ES⁺, 70V) 508.0 (MH⁺).

### EXAMPLE 113

### (2S)-2-(2-Fluoro-4,4-dimethyl-3-oxo-cyclobut-1-enylamino)-3-{4-[(3,5-dichloroisonicotinoyl)amino]phenyl}-propanoic acid

Prepared from the compound of Example 5 (1.25g, 2.49 mmol), Selectfluor™ reagent (1.0g, 2.8mmol) and lithium hydroxide (80mg, 1.8mmol), according to the methods of Examples 61 and 2, to give the title compound as a white powder (1.13g, 2.1mmol, 84%). δH (DMSO d⁶, 390K) 10.88 (1H, s), 8.80 (2H, s), 8.65 (1H, d, J 8.7Hz), 7.60 (2H, d, J 8.2Hz), 7.27 (2H, d, J 8.2Hz), 4.25 (1H, m), 3.22 (1H, dd, J 4.5, 13.9Hz), 2.98 (1H, dd, J 9.3, 13.9Hz), 1.11 (3H, s), 1.03 (3H, s) . m/z (ES⁺, 70V) 466.0 (MH⁺).

### EXAMPLE 114

### (2S)-2-[(4.4-Dimethyl-2-methylsulfanyl-3-oxo-cyclobut-1-enyl)amino]-3-{4-[(3,5-dichloroisonicotinoyl)amino]phenyl}-propanoic acid

A solution of the compound of Example 5 (700mg, 1.47mmol) in THF (15ml) was treated at rt with a 1.0M solution of methanesulfenyl chloride in DCM (1.5ml, 1.5mmol). After 30min the reaction was partitioned between EtOAc (50ml) and saturated aqueous sodium hydrogencarbonate solution (25ml). Separation of the organic phase, followed by drying (MgSO₄), filtration and concentration *in vacuo* gave a solid which was approx. 90% pure. The crude solid was redissolved in THF (20ml) and treated with lithium hydroxide (60mg, 1.3mmol) and water (1ml) and stirred at rt for 24h. The reaction was acidified with a few drops of AcOH and concentrated *in vacuo.* Chromatography (SiO₂, 10:90:3:2 MeOH:DCM:AcOH:H2O gave the title compound as a white powder (289mg, 0.59mmol, 40%). δH (DMSO d⁶, 390K) 10.85 (1H, s), 8.87 (1H, d, J 9.2Hz), 8.80 (2H, s), 7.59 (2H, d, J 8.0Hz), 7.29 (2H, d, J 8.0Hz), 5.04 (1H, m), 2.25 (1H, dd, J 3.5, 13.5Hz), 3.00 (1H, dd, J 9.8, 13.5Hz), 2.00 (3H, s), 1.11 (3H, s), 1.02 (3H, s). m/z (ES⁺, 70V) 493.9 (MH⁺).

### EXAMPLE 115

### (2S)-2-[(2-Isopropylsulfanyl-4,4-dimethyl-3-oxo-cyclobut-1-enyl)amino]-3-{4-[(3,5-dichloroisonicotinoyl)amino]phenyl}-propanoic acid

Was prepared in two steps from the compound of Example 5 (700mg, 1.47mmol), a 1.0M solution of isopropyl sulfenyl chloride in DCM (1.5ml, 1.5mmol) and lithium hydroxide (80mg, 1.8mmol), according to the method of Example 114, to give the title compound as a pale yellow powder (305mg, 0.58mmol, 39%). δH (DMSO d⁶, 390K) 13.24 (1H, s br), 10.87 (1H, s), 8.92 (1H, d, J 9.5Hz), 8.80 (2H, s), 7.58 (2H, d, J 8.4Hz), 7.28 (2H, d, J 8.4Hz), 5.16 (1H, m), 3.22 (1H, dd, J 3.9, 13.8Hz), 2.97 (1H, dd, J 9.7, 13.8Hz), 2.67 (1H, m), 1.14 (3H, s), 1.06-1.04 (9H, m). m/z (ES⁺, 70V) 522.0 (MH⁺).

### EXAMPLE 116

### (2S)-Ethyl-2-[(2-Isopropylsulfanyl-3-oxo-spiro[3.5]non-1-en-1-yl)amino]-3-{4-[(3,5-dichloroisonicotinoyl)amino]phenyl}-propanoate

A stirred solution of the compound of Example 27 (1.0g, 1.93mmol) in THF (50ml) at rt was treated dropwise with a 1.0M solution of isopropyl sulfenyl chloride in DCM until a yellow colouration of the reaction mixture just persisted. The reaction was then diluted with EtOAc (200ml) and washed with saturated aqueous sodium hydrogencarbonate solution (50ml). The organic phase was separated, dried (MgSO₄), filtered and concentrated *in vacuo.* Chromatography (SiO₂, 100% EtOAc) gave the title compound as a pale yellow powder (987mg, 87%). δH (DMSO d⁶, 390K) 10.85 (1H, s), 8.79 (2H, s), 8.73 (1H, d, J 9.5Hz), 7.56 (2H, d, J 8.5Hz), 7.25 (2H, d, J 8.5Hz), 5.20 (1H, m), 4.17 (2H, q, J 7.1Hz), 3.18 (1H, dd, J 4.3, 13.8Hz), 2.97 (1H, dd, J 10.2, 13.8Hz), 2.65 (1H, m), 1.73-1.57 (8H, m), 1.36-1.33 (1H, m), 1.21 (3H, t, J 7.1Hz), 1.17-1.14 (1H, m), 1.02 (6H, d, J 6.6Hz). m/z (ES⁺, 70V) 590.0 (MH⁺).

### EXAMPLE 117

### (2S)-2-[(2-Isopropylsulfanyl-3-oxo-spiro[3.5]non-1-en-1-yl)amino]-3-{4-[(3,5-dichloroisonicotinoyl)amino]phenyl}-propanoic acid

Hydrolysis of the compound of Example 116 (400mg, 0.68mmol) with lithium hydroxide (100mg, 2.2mmol), according to the method of Example 2, gave the title compound as a white powder (333mg, 0.59mmol, 89%). δH (DMSO d⁶, 390K) 10.84 (1H, s), 8.78 (2H, s), 8.62 (1H, d, J 9.4Hz), 7.55 (2H, d, J 8.1Hz), 7.25 (1H, d, J 8.1Hz), 5.12 (1H, m), 3.20 (1H, dd, J 3.6, 13.7Hz), 2.94 (1H, dd, J 10.2, 13.7Hz), 2.62 (1H, m), 1.91-1.64 (8H, m), 1.59-1.56 (1H, m), 1.36-1.33 (1H, m), 1.02 (6H, d, J 6.6Hz). m/z (ES⁺, 70V) 562.0 (MH⁺).

### EXAMPLE 118

### (2S)-Ethyl-2-[(2-isopropylsulfanyl-4,4-dimethyl-3-oxo-cyclobut-1-enyl)amino]-3-[4-([2,7]naphthyridin-1-ylamino)phenyl] propanoate

A solution of 3-hydroxy-4,4-dimethyl-2-cyclobutenone (333mg, 2.97mmol) and Intermediate 12 (1.0g, 2.98mmol) in THF (25ml), was stirred at rt for 48h. The volatiles were removed *in vacuo* and the residue chromatographed (SiO₂; EtOAc) affording 970mg of the coupled product of approx 90% purity. This intermediate was redissolved in THF (35ml) and treated with a 1.0M solution of isopropyl sulfenyl chloride in DCM (3.0ml, 3.0mmol) at rt. After 60min the reaction was diluted with EtOAc (100ml) and washed with saturated aqueous sodium carbonate solution (50ml), the organic phase was dried (MgS04), filtered and concentrated *in vacuo.* Chromatography (SiO₂, EtOAc) gave the title compound as a white powder (817mg, 1.62mmol, 54%). δH (DMSO d⁶, 390K) 9.83 (1H, s), 9.52 (1H, s), 9.01 (1H, d, J 9.4Hz), 8.65 (1H, d, J 5.7Hz), 8.16 (1H, d, J 5.7Hz), 7.81 (2H, d, J 8.5Hz), 7.68 (1H, d, J 5.7Hz), 7.23 (2H, d, J 8.5Hz), 7.13 (1H, d, J 5.7Hz), 5.24 (1H, m), 4.19 (2H, q, J 7.1Hz), 3.20 (1H, dd, J 4.5, 13.8Hz), 2.97 (1H, dd, J 10.0, 13.8Hz), 2.76 (1H, m), 1.22 (3H, t, J 7.1Hz), 1.13 (3H, s), 1.05 (6H, d, J 6.7Hz), 1.04 (3H, s). m/z (ES⁺, 70V) 505.2 (MH⁺).

### EXAMPLE 119

### (2S)-2-[(2-Isopropylsulfanyl-4,4-dimethyl-3-oxo-cyclobut-1-enyl)amino]-3-[4-([2,7]naphthyridin-1-ylamino)phenyl]-propanoate

Hydrolysis of the compound of Example 118 (400mg, 0.79mmol) with lithium hydroxide (100mg, 2.2mmol), according to the method of Example 2, gave the title compound as a white powder (231 mg, 0.49mmol, 62%). δH (DMSO d⁶, 390K) 9.83 (1H, s), 9.51 (1H, s), 8.89 (1H, d, J 9.5Hz), 8.65 (1H, d, J 5.6Hz), 8.15 (1H, d, J 5.7Hz), 7.78 (2H, d, J 8.5Hz), 7.22 (2H, d, J 8.5Hz), 7.11 (1H, d, J 5.7Hz), 5.16 (1H, m), 3.21 (1H, dd, J 4.1, 13.8Hz), 2.94 (1H, dd, J 6.7, 13.8Hz), 2.75 (1H, m), 1.13 (3H, s), 1.06 (6H, d, J 6.6Hz), 1.03 (3H, s). m/z (ES⁺, 70V) 477.1 (MH⁺).

### EXAMPLE 120

### (2S)-Ethyl-2-[(2-isopropylsulfanyl-3-oxo-spiro[3.5]non-1-en-1-yl)amino]-3-[4-([2,7]naphthyridin-1-ylamino)phenyl]-propanoate

Was prepared in two steps from Intermediate 12 (1.0g, 2.98mmol), 1-keto-3-hydroxyspiro[3,5]-non-2-ene (452mg, 2.97mmol) [prepared according to the method of Wasserman, H.H. et al, J. Org. Chem., 38, 1451-1455 (1973)] and a 1.0M solution of isopropyl sulfenyl chloride in DCM (3.5ml, 3.5mmol) according to the method of Example 118 to give the title compound as a yellow powder (931 mg, 1.71mmol, 58%). (DMSO d⁶, 390K) 9.89 (1H, s), 9.58 (1H, s), 8.82 (1H, d, J 9.4Hz), 8.71 (1H, d, J 5.6Hz), 8.21 (1H, d, J 5.6Hz), 7.85 (2H, d, J 8.3Hz), 7.74 (1H, d, J 5.6Hz), 7.28 (2H, d, J 8.3Hz), 7.18 (1H, d, J 5.6Hz), 5.31 (1H, m), 4.24 (2H, q, J 7.1Hz), 3.24 (1H, dd, J 4.1, 13.7Hz), 3.03 (1H, dd, J 10.3, 13.7Hz), 2.80 (1H, m), 1.80-1.71 (8H, m), 1.46-1.43 (1H, m), 1.28 (3H, t, J 7.1Hz), 1.24-1.21 (1H, m), 1.11 (6H, d, J 6.5Hz). m/z (ES⁺, 70V) 545.2 (MH⁺).

### EXAMPLE 121

### (2S)-2-[(2-Isopropylsulfanyl-3-oxo-spiro[3.5]non-1-en-1-yl)amino]-3-[4-([2,7]naphthyridin-1-ylamino)-phenyl]-propanoic acid

Hydrolysis of the compound of Example 120 (900mg, 1.62mmol) with lithium hydroxide (150mg, 3.3mmol), according to the method of Example 2, gave the title compound as a bright yellow powder (790mg, 1.53mmol, 94%). δH (DMSO d⁶, 390K) 9.83 (1H. s), 9.51 (1H, s), 8.67 (1H, d, J 5.6Hz), 8.65 (1H, d, J 5.6Hz), 8.15 (1H, d, J 5.7Hz), 7.77 (2H, d, J 8.4Hz), 7.68 (1H, d, J 5.7Hz), 7.23 (2H, d, J 8.4Hz), 7.12 (1H, J 5.7Hz), 5.20 (1H, m), 3.22 (1H, dd, J 3.9, 13.8Hz), 2.94 (1H, dd, J 10.4, 13.8Hz), 1.79-1.66 (8H, m), 1.40-1.35 (1H, m), 1.20-1.15 (1H, m), 1.05 (3H, d, J 6.3Hz), 1.03 (3H, d, J 6.3Hz). m/z (ES⁺, 70V) 517.2 (MH⁺).

### EXAMPLE 122

### (2S)-Ethyl-2-[(2-isopropylsulfanyl-3-oxo-7-oxa-spiro[3.5]non-1-en-1-yl)amino]-3-[4-([2,7]naphthyridin-1-ylamino)-phenyl]-propanoate

Was prepared in two steps from Intermediate 12 (1.96g, 5.8mmol), 7-oxaspiro[3.5]nonane-1,3-dione (1.0g, 6.5mmol) [prepared according to the method of Wasserman, H.H. et al, J. Org. Chem., 38, 1451-1455 (1973)] and a 1.0M solution of isopropyl sulfenyl chloride in DCM (5.4ml, 5.4mmol) according to the method of 118 to give the title compound as a yellow powder (1.15g, 2.1 mmol, 36%). δH (DMSO d⁶, 390K) 9.83 (1H, s), 9.52 (1H, s), 8.94 (1H, d, J 9.5Hz), 8.65 (1H, d, J 5.6Hz), 8.15 (1H, d, J 5.7Hz), 7.78 (2H, d, J 8.5Hz), 7.68 (1H, d, J 5.6Hz), 7.23 (2H, d, J 8.5Hz), 7.12 (1H, d, J 5.7Hz), 5.26 (1H, m), 4.19 (2H, q, J 7.1Hz), 3.81-3.76 (2H, m), 3.64-3.55 (2H, m), 3.20 (1H, dd, J 4.3, 13.8Hz), 2.96 (1H, dd, J 10.3, 13.8Hz), 2.81-2.74 (1H, m), 2.06-1.93 (2H, m), 1.50-1.47 (1H, m), 1.32-1.28 (1H, m), 1.23 (3H, t, J 7.1Hz), 1.07 (3H, d, J 6.6Hz), 1.05 (3H, d, J 6.6Hz). m/z (ES⁺, 70V) 547.2 (MH⁺).

### EXAMPLE 123

### (2S)-2-[(2-Isopropylsulfanyl-3-oxo-7-oxa-spiro[3,5]non-1-en-1-yl)amino]-3-[4-([2,7]naphthyridin-1-ylamino)-phenyl]-propanoic acid

Hydrolysis of the compound of Example 122 (906mg, 1.66mmol) with lithium hydroxide (150mg, 3.3mmol), according to the method of Example 2, gave the title compound as a pale yellow powder (801 mg, 1.54mmol, 93%). δH (DMSO d⁶, 390K) 9.82 (1H, s), 9.51 (1H, s), 8.86 (1H, d, J 9.5Hz), 8.65 (1H, d, J 5.5Hz), 8.14 (1H, d, J 5.6Hz), 7.76 (2H, d, J 8.1Hz), 7.68 (1H, d, J 5.5Hz), 7.28 (2H, d, J 8.1Hz), 7.12 (1H, d, J 5.6Hz), 5.24-5.19 (1H, m), 3.78 (2H, m), 3.61 (2H, m), 3.21 (1H, dd, J 3.5, 13.8Hz), 2.91 (1H, dd, J 10.7, 13.8Hz), 2.77-2.71 (1H, m), 2.05-1.91 (2H, m), 1.49-1.46 (1H, m), 1.30-1.26 (1H, m), 1.07 (3H, s), 1.03 (3H, s). m/*z* (ES⁺, 70V) 519.1 (MH⁺).

### EXAMPLE 124

### (2S)-Ethyl-2-[(3-oxo-spiro[3.4]octa-1,6-dien-1-yl)amino]-3-{4-[(3,5-dichloroisonicotinoyl)amino]phenyl}propanoate

A solution of Intermediate 47 (1.0g, 7.3mmol) and the free base of Intermediate 27 (2.48g, 7.3mmol), in DCM (25ml), was stirred at room temperature for 48h. The volatiles were removed *in vacuo* and the residue chromatographed (SiO₂; EtOAc) to give the title compound as a white solid (2.14g, 4.28mmol, 59%). δH (CDCI₃, 300K) 9.02 (1H, s), 8.38 (2H, s), 7.49 (2H, d, 8.5Hz), 7.00 (2H, d, J 8.5Hz), 6.03 (1H, d, J 7.8Hz), 5.54 (2H, s), 4.41 (1H, s), 4.21 (2H, q, J 7.1Hz), 4.20 (1H, m), 3.15 (1H, dd, J 5.2, 14.0Hz), 3.03 (1H, dd, J 6.1, 14.0Hz), 1.56-1.51 (2H, m), 2.38-2.34 (2H, m), 1.18 (3H, t, J 7.1Hz). m/z (ES⁺, 70V) 500.0 (MH⁺).

### EXAMPLE 125

### (2S)-2-[(3-Oxo-spiro[3,4]octa-1,6-dien-1-yl)amino]-3-{4-[(3,5-dichloroisonicotinoyl)amino]phenyl}-propanoic acid

Hydrolysis of the compound of Example 124 (970mg, 1.94mmol) with lithium hydroxide (100mg, 2.2mmol), according to the method of Example 2, gave the title compound (896mg, 1.90mol, 98%). δH (DMSO d⁶, 390K) 10.87 (1H, s), 8.80 (2H, s), 8.45 (1H, d, J 8.4Hz), 7.57 (2H, d, J 8.5Hz), 7.27 (2H, d, J 8.5Hz), 5.63 (2H, s), 4.41 (1H, s), 4.11 (1H, m), 3.17 (1H, dd, J 4.8, 13.9Hz), 2.98 (1H, dd, J 9.3, 13.9Hz), 2.46-2.42 (2H, m), 2.36-2.25 (2H, m). m/z (ES⁺, 70V) 471.9 (MH⁺).

### EXAMPLE 126

### (2S)-Ethyl-2-[(2-bromo-3-oxo-spiro[3.4]octa-1,6-dien-1-yl)amino]-3-{4-[(3,5-dichloroisonicotinoyl)aminol]phenyl}-propanoate

Bromination of the compound of Example 124 (206mg, 0.41mmol) with N-bromo succinimide (75mg, 0.57mmol), according to the method of Example 36 afforded the title compound as a white solid (116mg, 0.20mmol, 50%). δH (DMSO d⁶, 390K) 10.36 (1H, s), 8.67 (2H, s), 8.45 (1H, d, J 9.0Hz), 7.58 (2H, d, J 8.5Hz), 7.28 (2H, d, J 8.5Hz), 5.67-5.63 (2H, m), 4.50 (1H, s br), 4.23 (2H, q, J 7.1Hz), 3.28 (1H, dd, J 6.3, 14.1Hz), 3.15 (1H, dd, J 9.0, 14.1Hz), 2.52-2.42 (4H, m), 1.28 (3H, t, J 7.1Hz). m/z (ES⁺, 70V) 579.0 (MH⁺).

### EXAMPLE 127

### (2S)-2-[(2-Bromo-3-oxo-spiro[3.4]octa-1.6-dien-1-yl)amino]-3-{4-[(3,5-dichloroisonicotinoyl)amino]phenyl}-propanoic acid

Hydrolysis of the compound of Example 126 (190mg, 0.33mmol) with lithium hydroxide (60mg, 1.3mmol), according to the method of Example 2, gave the title compound (162mg, 0.29mol, 88%). δH (DMSO d⁶, 390K) 10.89 (1H, s), 9.09-9.04 (1H, m), 8.81 (2H, s), 7.62 (2H, d, J 8.5Hz), 7.29 (2H, d, J 8.5Hz), 5.69-5.60 (2H, m), 4.72 (1H, m), 3.03 (1H, dd, J 9.0, 14.1Hz), 2.98 (1H, dd, J 6.7, 14.1Hz), 2.62-2.20 (4H, m). m/z (ES⁺, 70V) 551.8 (MH⁺).

### EXAMPLE 128

### (2S)-Ethyl-2-[(4,4-dimethyl-3-oxo-2-pentafluorophenylsulfanyl-cyclobut-1-enyl)amino]-3-{4-[(3,5-dichloroisonicotinoyl)amino]phenyl}-propanoate

Was prepared from the compound of Example 5 (500mg, 1.06mmol) and pentafluorophenyl sulphenyl chloride (273mg, 1.17mmol), according to the method of Example 93 to give the title compound as a white powder (459mg, 0.68mmol, 64%). δH (CDCl₃, 300K); 8.41 (2H, s), 7.41 (2H, d, J 8.4Hz), 6.99 (2H, d, J 8.4Hz), 5.98 (1H, d, J 8,8Hz), 5.18-5.12 (1H, m), 4.12 (2H, q, J 7.1Hz), 3.17 (1H, dd, J 5.6, 14.1Hz), 3.10 (1H, dd, J 5.7, 14.1Hz), 1.18 (3H, t, J 7.1Hz), 1.03 (3H, s), 1.01 (3H, s). m/z (ES⁺, 70V) 673.9 (MH⁺).

### EXAMPLE 129

### (2S)-2-[(4,4-Dimethyl-3-oxo-2-pentafluorophenylsulfanyl-cyclobut-1-enyl)amino]-3-{4-[(3,5-dichloroisonicotinoyl)amino]phenyl}-propanoic acid

Hydrolysis of the compound of Example 128 (440mg, 0.65mmol) with lithium hydroxide (60mg, 1.4mmol), according to the method of Example 2, gave the title compound (372mg, 0.57mmol, 88%) as a white powder. δH (DMSO d⁶, 390K) 13.75 (1H, br s), 10.62 (1H, s), 9.16 (1H, d, J 9.5Hz), 8.54 (2H, s), 7.30 (2H, d, J 8.5Hz), 6.97 (2H, d, J 8.5Hz), 4.83-4.77 (1H, m), 3.0 (1H, dd, J 4.1, 14.0Hz), 2.71 (1H, dd, J 10.0, 14.0Hz), 0.83 (3H, s), 0.75 (3H, s). m/z (ES⁺, 70V) 645.9 (MH⁺).

### EXAMPLE 130

### (2S)-Ethyl-2-(4,4-dimethyl-3-oxo-2-pyrazin-2-yl-cyclobut-1-enylamino)-3-{4[(3,5-dichloroisonicotinoyl)amino]pheny}-propanoate

A solution containing vinyl iodide (500mg, 0.81 mmol), palladium dichloride (14mg), triphenyl arsine (101mg), 2-tributylstannyl 1,4-pyridazine (307mg), and copper iodide (15mg) in DMF was heated to 95º for 3h. The reaction was then diluted with saturated aqueous sodium hydrogencarbonate solution (50ml) and EtOAc (100ml). The organic phase was removed, dried (MgSO4), filtered and concentrated *in vacuo.* Chromatography (SiO₂, 50% EtOAc:hexanes) gave the title compound (127mg, 0.23mmol, 28%). δH (CDCl3, 330K) 9.51 (1H, s), 8.72 (1H, s), 8.26 (1H, m), 8.11 (1H, d, J 2.7Hz), 7.97 (1H, d, J 10.9Hz), 7.54 (2H, d, J 8.5Hz), 4.24-4.15 (3H, m), 3.18 (1H, dd, J 5.2, 13.9Hz), 3.11 (1H, dd, J 4.6, 13.9Hz), 1.23 (3H, t, J 7.1Hz), 1.22 (3H, s), 0.98 (3H, s). m/z (ES⁺, 70V) 554.1 (MH⁺).

### EXAMPLE 131

### (2S)-2-[(4,4-Dimethyl-3-oxo-2-pyrazin-2-yl-cyclobut-1-enyl)amino]-3-{4[(3,5-dichloroisonicotinoyl)amino]phenyl}-propanoate

Hydrolysis of the compound of Example 130 (50mg, 0.09mmol) with lithium hydroxide (10mg, 0.23mmol), according to the method of Example 2, gave the title compound (33mg, 0.064mmol, 71%) as a white powder. LCMS indicates the presence of two separate atropisomers. δH (DMSO d⁶, 390K, 1 atropisomer) 10.49 (1H, s), 8.90 (1H, br m), 8.69 (2H, s), 8.42 (1H, br m), 8.21 (1H, br m), 7.43 (2H, d, J 8.3Hz), 7.21 (2H, d, J 8.3Hz), 5.95 (1H, br s), 4.20 (1H, m), 3.27-3.22 (1H, m), 3.06 (1H, m), 1.28 (3H, s), 1.19 (3H, s). m/z (ES⁺, 70V) 526.0 (MH⁺).

### EXAMPLE 132

### (2S)-Ethyl-2-[(4-methyl-3-oxo-4-phenyl-cyclobut-1-enyl)amino]-3-{4-[(3,5-dichlorosinicotinoyl)amino]phenyl]-propanoate

Prepared from Intermediate 49 (300mg, 1.72mmol) and the free base of Intermediate 27 (400mg, 1.04mmol), in a similar manner to the compound of Example 11, to give the title compound as a white powder (329mg, 0.61 mmol, 59%) as an approx. 1:1 mixture of diastereomers. δH (CDCl₃, 300K) 10.95 (1H, s), 10.88 (1H, s), 8.89 (1H, d, J 8.6Hz), 8.81 (2H, s), 8.80 (2H, s), 8.74 (1H, d, J 8.8Hz), 7.63 (2H, d, J 8.5Hz), 7.59 (2H, d, J 8.5Hz), 7.34-7.10 (5H, m), 6.89 (2H, d, J 8.5Hz), 6.85 (2H, d, J 8.5Hz), 4.71 and 4.66 (1H, s), 4.48-4.42 and 4.38-4.33 (1H, m), 4.21 (2H, t, J 7.1Hz), 3.31-3.01 (2H, m), 1.52 and 1.42 (3H, s), 1.25 (3H, t, J 7.1Hz). m/z (ES⁺, 70V) 537.9 (MH⁺).

### EXAMPLE 133

### (2S)-Ethyl-2-[(4-methyl-3-oxo-4-phenyl-cyclobut-1-enyl)amino]-3-{4-[(3,5-dichloroisonicotinoyl)aminolphenyl]-propanoate

The compound of Example 132 (100mg, 0.18mmol) was hydrolysed in a similar manner to the method of Example 2 to give the title compound as a fine white solid (79mg, 0.15mmol, 86%) as an approx. 1:1 mixture of diastereomers. δH(DMSO d⁶, 360K) 10.95 and 10.88 (1H, s), 8.82 and 8.81 (2H, s), 8.65 (1H, d, J 8.7Hz), 7.63 and 7.59 (2H, d, J 8.5Hz), 7.23-7.10 (5H, m), 6.88 and 6.85 (2H, d, J 8.5Hz), 4.70 and 4.63 (1H, s), 4.36-4.25 and 4.25-4.22 (1H, m), 3.31-3.00 (2H, m), 1.41 and 1.25 (3H, s). m/z (ES⁺, 70V) 509.9 (MH⁺).

### EXAMPLE 134

### (2S)-Ethyl-2-[(7-acetyl-2-bromo-3-oxo-7-aza-spiro[3,5]non-1-en-1-yl)amino]-3-{4-[(3,5-dichloroisonicotinoyl)amino]phenyl}-propanoate

A solution of the compound of Example 51 (100mg, 0.18mmol) in THF (10ml) at rt was treated with N-bromo succinimide (32mg, 0.19mmol). After 30min the reaction was diluted with EtOAc (50ml) and saturated aqueous sodium hydrogencarbonate solution (30ml), the organic phase separated and dried (MgSO₄), filtered and concentrated *in vacuo.* Chromatography (SiO₂, EtOAc) gave the title compound as a white powder (79mg, 0.12mmol, 67%). δH (CDCl₃, 300K) 9.54 (1H, s), 8.65 (1H, d, J 9.4Hz), 8.19 (2H, s), 7.49 (2H, d, J 8.4Hz), 7.07 (2H, d, J 8.4Hz), 4.91-4.81 (1H, m), 4.11 (2H, q, J 7.1Hz), 3.49-3.44 (1H, m), 3.25-2.66 (5H, m), 1.89 (3H, s), 1.86-1.25 (4H, m), 1.18 (3H, t, J 7.1Hz). m/z (ES⁺, 70V) 639.0 (MH⁺).

### EXAMPLE 135

### (2S)-2-[(7-Acetyl-2-bromo-3-oxo-7-aza-spiro[3,5]non-1-en-1-yl)amino]-3-{4-[(3,5-dichloroisonicotinoyl)amino]phenyl}-propanoic acid

The compound of Example 134 (50mg, 0.078mmol) was hydrolysed in a similar manner to the method of Example 2, to give the title compound as a white powder (21 mg, 0.034mmol, %). δH(DMSO d⁶, 390K) 10.38 (1H, s), 8.96 (2H, s), 7.57 (2H, s br), 7.28 (2H, d, J 7.9Hz), 4.78 (1H, m), 3.99-3.96 (2H, m), 3.30-3.06 (4H, m), 2.00 (3H, s), 1.94-1.84 (2H, m), 1.48-1.21 (2H, m). m/z (ES⁺, 70V) 610.9 (MH⁺).

### EXAMPLE 136

### Ethyl (2S) 2-(2-benzyl-4,4-dimethyl-3-oxo-cyclobut-1-enylamino)-3-{4-[(3,5-dichloro-isonicotinoyl)amino]-phenyl} propanoate

A solution of the free acid of Intermediate 27 (0.82g, 2.1mmol) and Intermediate 72 (0.48g, 2.3mmol, 1.1eq.) in nitromethane (8ml) was treated with acetic acid (1 drop). The resulting mixture was heated at 100° for 2h. and then partitioned between EtOAc (50ml) and water (25ml), the organics were separated, washed with water (25ml), Na₂CO₃ (25ml, sat. aq.), brine (25ml), dried (Na₂SO₄), filtered and concentrated *in vacuo* to give a crude foam. This was purified by column chromatography (SiO₂, gradient elution 1:1, hexane:EtOAc to EtOAc) to give the title compound as a white solid (0.71g, 59%). δH NMR (DMSO d⁶, 400MHz) 10.87 (1H, s), 8.81 (2H, s), 8.38 (1H, d, J 9.3Hz), 7.53 (2H, d, J 8.4Hz), 7.33 (2H, m), 7.15 (2H, m), 7.09 (2H, d, J 8.5Hz), 7.03 (2H, d, J 7.2Hz), 4.15 (1H, m), 4.04 (2H, dq, J 1.6, 7.1Hz), 3.19 (2H, m), 3.04 (1H, dd, J 13.8, 5.0Hz), 2.89 (1H, dd, J 9.5, 4.8Hz), 1.02-1.26 (8H, m). m/z (ESI, 70V) MH⁺ 566.

### EXAMPLE 137

### 2-(2-Benzyl-4,4-dimethyl-3-oxo-cyclobut-1-enylamino)-3-{4-[(3,5-dichloroisonicotinoyl)amino]-phenyl}propanoic acid

To a solution of the compound of Example 136 (210mg, 0.4mmol) in THF (2ml) was added a solution of lithium hydroxide hydrate (25mg, 0.6mmol, 1.5eq) in water (1ml). The solution was stirred at room temperature for 3h. The reaction mixture was concentrated *in vacuo* and the residue redissolved in water (15ml) and washed with diethyl ether (10ml). The aqueous was concentrated to approx. 5ml and taken to pH 4 with HCI (2M, aq.). The resulting solid was filtered, washed on the sinter with water, diethyl ether and dried *in vacuo* to give the title compound as a white solid (170mg, 85%). δH (400MHz, DMSO d⁶, 380K) 10.42 (1H, br), 8.70 (2H, s), 7.64 (1H, d, J 9.4Hz), 7.52 (2H, d, J 6.6Hz), 7.25 (2H, m), 7.13-7.18 (5H, m), 4.13 (1H, m), 3.26 (2H, d, J 2.9Hz), 3.13 (1H, dd, J 14.0, 4.9Hz), 2.97 (1H, dd, J 14.0, 9.0Hz), 1.12 (3H, s), 1.09 (3H, d, J 15.7Hz). m/z (ESI, 70V) MH⁺ 538.

### EXAMPLE 138

### Isopropyl (2S) 2-(2-Bromo-3-oxo-spiro[3.5]non-1-en-1-ylamino)-3-{4-[(3,5-dichloroisonicotinoyl)amino]phenyl} propanoate

To a solution of the compound of Example 32 (0.5g, 0.9mmol) in DMF (5ml) was added EDC (185mg, 1.1eq), HOBT (135mg, 1.1eq) and *iso*propanol (0.5ml). The mixture was stirred at room temperature for 48h. then partitioned between EtOAc (100ml) and water (50ml). The aqueous was separated and the organics washed with water (5 x 30ml), brine (30ml), dried (Na₂SO₄), filtered and concentrated *in vacuo* to give a crude solid. The crude was triturated with diethyl ether to give the title compound as a white powder (0.35g, 65%) δH NMR (300MHz, DMSO d⁶) 10.69 (1H, br), 8.68 (1H, d, J 9.1Hz), 8.60 (2H, s), 7.39 (2H, d, J 8.5Hz), 7.08 (2H, d, J 8.5Hz), 4.78 (1H, sep, J 6.3Hz), 4.53 (1H, m), 3.01 (1H, dd, J 4.9, 13.8Hz), 2.83 (1H, dd, J 9.5, 13.9Hz), 1.36-1.60 (9H, m), 1.19 (1H, d, J 12.7Hz), 0.98-1.05 (6H, dd). m/z (ESI, 70V) MH⁺ 608.

### EXAMPLE 139

### (2S) 2-(2-Bromo-3-oxo-spiro[3.5]non-1-en-1-ylamino)-3-{4-[3,5-dichloro-isonicotinoyl)amino]phenyl}propanoic acid 1-methyl-piperidin-4-yl ester

Using a similar procedure to that for the preparation the compound of Example 138 from the compound of Example 32 (0.5g, 0.89mmol), EDC (185mg, 1.1eq), HOBT (135mg, 1.1eq), 4-hydroxy-1-methylpiperidine (0.5ml) and DMF (2ml) was prepared the title compound (0.21g, 36%). δH NMR (300MHz, DMSO d⁶) 11.01 (1H, br), 9.01 (1H, d, J 9.4Hz), 8.92 (2H, s), 7.71 (2H, d, J 8.5Hz), 7.40 (2H, d, J 8.5Hz), 4.90 (1H, br), 3.33 (1H, dd, J 13.8, 4.8Hz), 3.16 (1H, dd, J 13.8, 9.6Hz), 2.55 (2H, br), 2.40 (2H, br), 2.24 (3H, d, J 8.0Hz), 1.64-1.95 (12H, m), 1.50 (2H, d, J 12.1Hz), 1.23 (2H, br). m/z (ESI, 70V) MH+ 664.

### EXAMPLE 140

### (2S) 2-(2-Bromo-3-oxo-spiro[3,5]non-1-en-1-ylamino)-3-{4-[(3,5-dichloroisonicotinoyl)amino]phenyl} propanoic acid cyclopentyl ester

Using a similar procedure to that for the preparation the compound of Example 138 from the compound of Example 32 (0.5g, 0.89mmol, EDC (600mg, 3eq), HOBT (400mg, 3eq), cyclopentanol (0.5ml) and DMF (5ml)] was prepared the title compound. δH NMR (400MHz, DMSO d⁶) 10.88 (1H, s), 8.85 (1H, d, J 9.0Hz), 8.78 (2H, s), 7.58 (2H, d, J 8.5Hz), 7.26 (2H, d, J 8.5Hz), 5.15 (1H, m), 4.71 (1H, m), 3.18 (1H, dd, J 5.2, 13.9Hz), 3.02 (1H, dd, J 9.5, 13.9Hz), 1.17-1.84 (18H, m), 1.14 (1H, m). m/z (ESI, 70V) MH⁺ 634.

### EXAMPLE 141

### (2S) 2-(2-Bromo-3-oxo-spiro[3.5]non-1-en-1-ylamino)-3-{4-[3,5-dichloro-isonicotinoyl)amino]phenyl}propanoic acid tetrahydrofuran-3-yl ester

Using a similar procedure to that for the preparation the compound of Example 138 from the compound of Example 32 acid (0.5g, 0.89mmol), EDC (600mg, 3eq), HOBT (400mg, 3eq), (S)-3-hydroxytetrahydrofuran (0.5ml) and dimethylformamide (5ml)] was prepared the title compound. δH NMR (300MHz, DMSO d⁶) 8.87 (1H, d, J 8.9Hz), 8.79 (2H, s), 7.58 (2H, d, J 8.4Hz), 7.27 (2H, d, J 8.5Hz), 5.30 (1H, m), 4.77 (1H, m), 3.72-3.83 (3H, m), 3.65 (1H, d, J 10.4Hz), 3.20 (1H, dd, J 14.0, 5.1Hz), 3.04 (1H, dd, J 14.0, 9.7Hz), 2.11-2.27 (1H, m), 1.87-1.99 (1H, m), 1.37-1.78 (9H, m), 1.07-1.17 (1H, m). m/z (ESI, 70V) MH⁺ 636.

### EXAMPLE 142

### 2-(2-Bromo-3-oxo-spiror[3.5]non-1-en-1-ylamino)-3-{4-[(3,5-dichloroisonicotinoyl)amino]phenyl}propanoic acid 1-methyl-pyrrolidin-3-yl ester

Using a similar procedure to that for the preparation the compound of Example 138 from the compound of Example 32 (0.5g, 0.89mmol), EDC (600mg, 3eq), HOBT (400mg, 3eq), 1-methyl-3-pyrrolidinol (0.6ml) and DMF (5ml) was prepared the title compound. δH NMR (400MHz, DMSO d⁶) 10.88 (1H, s), 8.87 (1H, d, J 9.0Hz), 8.78 (2H, s), 7.58 (2H, d, J 8.3Hz), 7.26 (2H, d, J 8.5Hz), 5.13 (1H.m), 4.74 (1H, m), 3.16 (1H, m), 3.04 (1H, m), 2.43-2.73 (2H, m), 2.07-2.26 (2H, m), 1.55-1.75 (11H, m), 1.11 (1H, m). m/z (ESI, 70V) MH⁺ 649.

### EXAMPLE 143

### 2-(2-Bromo-3-oxo-spiro[3.5]non-1-en-1-ylamino)-3-{4-[(3,5-dichloropyridine-4-carbonyl)-amino]-phenyl}-propanoic acid phenyl ester

Using a similar procedure to that for the preparation the compound of Example 138 from the compound of Example 32 (0.5g, 0.89mmol, EDC (500mg, 2.6eq), HOBT (350mg, 2.6eq), phenol (0.5g) and DMF (5ml)] was prepared the title compound (0.17g, 29%). δH (300MHz, DMSO d⁶) 11.10 (1H, br) ), 9.24 (1H, d, J 8.8Hz), 8.98 (2H, s), 7.81 (2H, d, J 8.5Hz), 7.47-7.67 (5H, c), 7.31 (2H, dd, J 8.4, 0.9Hz), 6.93 (1H, d, J 8.1Hz), 5.20 (1H, br), 3.59 (1H, dd, J 9.3, 6.0Hz), 3.40 (1H, dd, J 14.0, 9.3Hz), 1.60-1.97 (10H, m). m/z (ESI, 70V) MH⁺ 644.

### EXAMPLE 144

### 2-(2-Bromo-3-oxo-spiro[3.5]non-1-en-1-ylamino)-3-{4-[(3,5-dichloroisonicotinoyl)amino]phenyl}propanoic acid pyridin-4-ylmethyl ester

Using a similar procedure to that for the preparation the compound of Example 138 from the compound of Example 32 (0.60g, 1.1mmol), EDC (222mg, 1.1eq), HOBT (162mg, 1.1eq), 4-pyridinemethanol (0.36g, 3.2mmol) and DMF (5ml)] was prepared the title compound (0.48g, 66%). δH NMR (400MHz, DMSO d⁶, 380K) 10.43 (1H, br), 8.69 (2H, s), 8.57 (2H, d, J 6.0Hz), 8.38 (1H, d, J 8.1Hz), 7.57 (2H, m), 7.30 (4H, m), 5.26 (2H, s), 4.95 (1H, br), 3.32 (1H, dd, J 14.2, 5.4Hz), 3.17 (1H, dd, J 14.1, 9.1Hz), 1.46-1.71 (9H, m), 1.21 (1H, br m). m/z (ESI, 70V) MH⁺ 657.

### EXAMPLE 145

### Methyl (2S) 2-[(3-oxo-7-oxaspiro[3.5]non-1-en-yl)amino]-3-(2,6-dimethoxy[1,1'-biphenyl]-4-yl)propanoate

Using a similar procedure to that for the preparation the compound of Example 38 from methyl (2*S*')-2-amino-3-(2,6-dimethoxy[1,1'-biphenyl]-4-yl)propanoate (2.0g, 6.3mmol) and 7-oxaspiro[3.5]nonane-1,3-dione (1.1g, 1.1eq) in DCM (30ml) was prepared the title compound as a white foam (2.4g, 86%). δH NMR (300MHz, DMSO d⁶) 8.53 (1H, d, J 8.7Hz), 7.20-7.30 (3H, m), 7.08 (2H, d, J 8.1Hz), 6.70 (2H, d, J 8.5Hz), 4.44 (1H, s), 4.30 (1H, m), 3.72 (3H, s), 3.61 (6H, s), 3.22 (1H, dd, J 13.7, 5.0Hz), 2.98 (1H, dd, J 13.8, 10.1Hz), 1.74-1.99 (2H, m), 1.48 (1H, d, J 13.6Hz), 1.31 (1H, d, J 13.0Hz). m/z (ESI, 70V) MH⁺ 439.

### EXAMPLE 146

### Methyl (2S) 2-{[2-(isopropylsulfanyl)-3-oxo-7-oxaspiro[3.5]non-1-en-yl) ]amino}-3-(2,6-dimethoxy[1,1'-biphenyl]-4-yl)propanoate

The title compound was prepared in a similar manner to the compound of Example 70 from the bromo derivative of the compound of Example 145 (0.5g, 1.1mmol) as a white foam (0.42g, 73%). δH NMR (400MHz, DMSO d⁶) 8.91 (1H, d, J 9.4Hz), 7.29-7.21 (3H, m), 7.09 (2H, d, J 8.2Hz), 6.70 (2H,d, J 8.4Hz), 5.32 (1H, br), 3.74-3.80 (5H, m), 3.54-3.64 (8H, m), 3.28 (1H, dd, J 4.2, 16.4Hz), 2.97 (1H, dd, J 10.8, 13.6Hz), 2.76 (1H, sep, J 6.7Hz), 1.99 (1H, dt, J 11.5, 4.8Hz), 1.84 (1H, dt, J 4.8, 13.0Hz), 1.48 (1H, d, J 12.1Hz), 1.25 (1H, d, J 12.2Hz), 1.05 (3H, d, J 6.7Hz), 1.02 (3H, d, J 6.7Hz). m/z (ESI, 70V) MH⁺ 526.

### EXAMPLE 147

### Ethyl (2S) 2-[(4,4,-dimethyl-3-oxo-1-cyclobutenyl)amino]-3-(2,6-dimethoxy[1,1'-biphenyl]-4-yl)propanoate

The title compound was prepared in a similar manner to that of the compound of Example 41 from ethyl (2*S*)-2-amino-3-(2,6-d)methoxy[1,1'-biphenyl]-4-yl)propanoate (1.5g, 4.5mol), and 3-hydroxy-4,4-dimethyl-2-cyclobutenone (0.5g, 4.5mmol) in DCM (15ml) as a white foam (1.8g, 93%). δH NMR (300MHz, DMSO d⁶) 8.68 (1H, d, J 8.6Hz), 7.22-7.37 (3H, m), 7.18 (2H, d, J 8.3Hz), 6.78 (2H, d, J 8.4Hz), 4.46 (1H, s), 4.32 (1H, m), 4.21 (2H, q, J 7.1Hz), 3.69 (6H, s), 3.24 (1H, dd, J 13.8, 5.9Hz), 3.09 (1H, dd, J 13.8, 9.1Hz), 1.24 (3H, t, J 7.1Hz), 1.16 (3H, s), 1.08 (3H, s). m/z (ESI, 70V) MH⁺ 424.

### EXAMPLE 148

### Ethyl (2S) 2-{[2-(isopropylsulfanyl)-4,4-dimethyl-3-oxo-1-cyclobutenyl]amino}-3-(2,6-dimethoxy[1,1'-biphenyl]-4-yl)propanoate

The title compound was prepared in a similar manner to the compound of Example 70 as a white foam (0.52g, 87%). δH NMR (400MHz, DMSO d⁶, 380K) 8.99 (1H, d, J 9.5Hz), 7.21-7.29 (3H, m), 7.10 (2H, d, J 8.1Hz), 6.70 (2H, d, J 8.4Hz), 5.29 (1H, m), 4.17 (2H, q, J 7.1Hz), 3.60 (6H, s), 3.23 (1H, dd, J 13.8, 5.0Hz), 2.99 (1H, dd, J 13.8, 10.4Hz), 2.79 (1H, sep, J 6.6Hz), 0.99-1.25 (15H, m). m/z (ESI, 70V) MH⁺ 498.

### EXAMPLE 149

### (2S) 2-{[2-(isopropylsulfanyl)-3-oxo-7-oxaspiro[3.5]non-1-en-yl) ]amino}-3-(2,6-dimethoxy[1,1'-biphenyl]-4-yl propanoic acid

Prepared from the compound of Example 146 (0.25g, 0.46mmol) in a similar manner to that of Example 2 to give the title compound as a white powder (0.21g, 89%). δH NMR (400MHz, DMSO d⁶) 8.80 (1H, d, J 9.5Hz), 7.21-7.28 (3H, m), 7.08 (2H, d, J 8.1Hz), 6.70 (2H, d, J 8.4Hz), 5.25 (1H, m), 3.76 (2H, m), 3.59 (8H, m), 3.30 (1H), 2.93 (1H, dd, J 10.9, 13.6Hz), 2.76 (1H, sep, J 6.8Hz), 1.99 (1H, m), 1.86 (1H, m), 1.46 (1H, d, J 13.1Hz), 1.23 (1H, d, J 12.7Hz), 1.06 (3H, d, J 7.8Hz), 1.03 (3H, d, J 7.8Hz). m/z (ESI, 70V) MH⁺ 512.

### EXAMPLE 150

### (2S) 2-1{[2-(isopropylsulfanyl)-4,4,-dimethyl-3-oxo-1-cyctobutenyl]amino}-3-(2,6-dimethoxy[1,1'-biphenyl]-4-yl)propanoic acid

Prepared from the compound of Example 148 (0.31g, 0.64mmol) in a similar manner to that of Example 2 to give the title compound as a white powder (0.13g, 44%). δH NMR (400MHz, DMSO d⁶) □8.85 (1H, br d), 7.21-7.29 (3H, m), 7.08 (2H, d, J 8.0Hz), 6.70 (2H, d, J 8.4Hz), 5.15 (1H, br), 3.61 (6H, s), 3.91 (1H, dd, J 13.7, 8.6Hz), 2.77 (1H, sep), 0.98-1.14 (6H, m). m/z (ESI, MH⁺) 470.

### EXAMPLE 151

### Ethyl (2S)-2-[(2-Cyclohexyl-3-oxospiro[3.5]non-1-en-1-yl)amino]-3-{4-[(3,5-dichloroisonicotinoyl)amino]phenyl} propanoate

A mixture of the free amine of Intermediate 27 (1000mg, 2.61 mmol), Intermediate 52 (611 mg, 2.61 mmol) and nitromethane (10ml) were heated at reflux for 5 hours. The solvent was removed by evaporation under reduced pressure and the residue chromatographed (SiO₂, 3:2 hexane:EtOAc), to afford the title compound as a colorless oil (310mg, 20%). δH NMR (CDCl₃) 8.51 (2H, s), 7.80 (1H, br s), 7.52 (2H, d, J 8.5Hz), 7.07 (2H, d, J 8.5Hz), 5.15 (1H, d, J 7.6Hz), 4.32 (1H, m), 4.20 (2H, q, J 7.1Hz), 3.07 (2H, d, J 5.6Hz), 1.75-1.40 (20H, m), 1.19 (3H, t, J 7.1Hz).

### EXAMPLE 152

### (2S) 2-[(2-Cyclohexyl-3-oxo-spiro[3.5]non-1-en-1-yl)amino]-3-{4-[(3,5-dichloroisonicotinoyl)amino]phenyl} propanoic acid

The compound of Example 151 was hydrolysed by the method of Example 2, giving the title compound in 78% yield. δH NMR (d⁶ DMSO) 10.84 (1H, br s), 8.78 (2H, s), 7.60 (1H, s), 7.56 (2H, d, J 8.2Hz), 7.25 (2H, d, J 7.9Hz), 4.06 (1H, m), 3.12 (1H, dd, J 3.9, 13.6Hz), 2.92 (1H, dd, J 9.8, 13.4Hz), 1.80-1.00 (21H, m). m/z (ES⁺, 70V) 570.1 (MH⁺)

### EXAMPLE 153

### Ethyl (2S) 3-{4-[(3,5-dichloroisonicotnoyl)amino]phenyl}-2-(2,4,4-trimethyl-3-oxo-cyclobut-1-enylamino) propanoate

Prepared in a similar manner to the compound of Example 151 from Intermediate 59 to give the title compound in 70% yield. δH NMR (CDCl₃) 8.78 (1H, br s), 8.44 (2H, s), 7.52 (2H, d, J 8.4 Hz), 7.04 ( 2H, d, J 8.3Hz), 5.55 (1H,d, J 9.0Hz), 4.39 (1H, m), 4.20 (2H, q, J 7.1Hz), 3.07 (2H, m), 1.41 (3H, s), 1.26 (3H, t, J 7.1Hz), 1.05 (6H, s). m/z (ES⁺, 70V) 490.0 (MH⁺).

### EXAMPLE 154

### (2S) 3-{4-[(3,5-Dichloroisonicotinoyl)amino]phenyl}-2-(2,4,4-trimethyl-3-oxo-cyclobut-1-enylamino) propanoic acid

The compound of Example 153 was hydrolysed by the method of Example 2 to afford the title compound in 69% yield. δH NMR (d⁶ DMSO) 7.86 (2H, s), 6.82 (2H, d, J 8.5Hz), 6.54 (2H, d, J 8.5Hz), 3.69 (1H, m), 2.57 (1H, m), 2.25 (1H, m), 0.68 (3H, s), 0.38 (3H, s), 0.29 (s, 3H). m/z (ES⁺, 70V) 462.0 (MH⁺).

### EXAMPLE 155

### Ethyl (2S) 3-{4-[(3,5-Dichloroisonicotinoyl)amino]phenyl}-2-(2-ethyl-4,4-dimethyl-3-oxo-cyclobut-1-enylamino) propanoate

Prepared in a similar manner to the compound of Example 151 from Intermediate 60 to give the title compound in 73% yield. δH NMR (d⁶ DMSO) 8.78 (2H, s), 8.13 (1H, d, J 9.0Hz), 7.59 (2H, d, J 8.4Hz), 7.30 (2H, d, J 8.3Hz), 4.25 (1H, m), 4.17 (2H, q, J 7.0Hz), 3.12 (1H, m), 3.00 (1H, m), 1.83 (2H, m), 1.20 (4H, m), 1.06 (3H, m), 0.98 (3H, s), 0.84 (3H, t, J 7.5Hz). m/z (ES⁺, 70V) 504.0 (MH⁺).

### EXAMPLE 156

### (2S)3-{4-[(3,5-Dichloroisonicotinoyl)amino]phenyl}-2-(2-ethyl-4,4-dimethyl-3-oxo-cyclobut-1-enylamino) propanoic acid

The compound of Example 155 was hydrolysed by the method of Example 2 to afford the title compound in 78% yield. δH NMR (d⁶ DMSO) 7.83 (2H, s), 6.81 (2H, d, J 8.5Hz), 6.51 (2H, d, J 8.4Hz), 3.52 (1H, m), 2.51 (1H, m), 2.21 (1H, m), 1.10 (2H, q, J 7.3Hz), 0.36 (3H, s), 0.26 (3H, s), 0.10 (3H, t, J 7.5Hz). m/z (ES⁺, 70V) 476.0 (MH⁺).

### EXAMPLE 157

### Ethyl (2S) 3-{4-[(3,5-Dichloroisonicotinoyl)amino]phenyl}-2-(4,4-dimethyl-3-oxo-2-propyl-cyclobut-1-enylamino) propanoate

Prepared in a similar manner to the compound of Example 151 from Intermediate 61 to give the title compound in 75% yield. δH NMR (d ⁶ DMSO) 8.80 (2H, s), 8.11 (1H. d, J 9.3Hz), 7.59 (2H, d, J 8.3Hz), 7.30 (2H, d, J 8.3Hz), 4.26 (1H, m), 4.15(2H, q, J 7.1Hz), 3.12 (1H, m), 3.00 (1H, m), 1.75 (2H, m), 1.23 (2H,m), 1.07 (3H, s), 0.99 (3H,s), 0.73 (3H, t, J 7.3Hz). m/z (ES⁺, 70V) 518.0 (MH⁺).

### EXAMPLE 158

### (2S) 3-{4-[(3,5-Dichloroisonicotinoyl)amino]phenyl}-2-(4,4-dimethyl-3-oxo-2-propyl-cyclobut-1-enylamino)propanoic acid

The compound of Example 157 was hydrolysed by the method of Example 2 to afford the title compound in 93% yield. δH NMR (d⁶ DMSO) 8.78 (2H, s), 8.05 (1H, d, J 9.2Hz), 7.58 (2H, d, J 8.3Hz), 7.30 (2H, d, J 8.3Hz), 4.15 (1H, m), 3.13 (1H, m), 2.95 (1H, m), 1.75 (2H, m), 1.23 (2H, q, J 7.3Hz), 1.06 (3H, s), 0.98 (3H, s), 0.72 (3H, t, J 7.3Hz). m/z (ES⁺, 70V) 490.0 (MH⁺).

### EXAMPLE 159

### Ethyl (2S) 3-{4-[(3,5-Dichloroisonicotinoyl)amino]phenyl}-2-(2-methyl-3-oxo-spiro[3.5]non-1-en-1-ylamino)propanoate

Prepared in a similar manner to the compound of Example 151 from Intermediate 64 to give the title compound in 68% yield. δH NMR (d⁶ DMSO) 8.79 (2H, s), 7.93 (2H, d, J 8.9Hz), 7.58 (2H, d, J 8.4Hz), 7.29 (2H, d, J 8.4Hz), 4.33 (1H, m), 4.16 (2H, q, J 7.1Hz), 3.10 (1H, m), 2.99 ( 1H, m), 1.60 (10H, m), 1.37 (3H, s), 1.18 (3H, t, J 7.1Hz). m/z (ES⁺, 70V) 530.1 (MH⁺).

### EXAMPLE 160

### (2S) 3-{4-[(3,5-Dichloroisonocotinoyl)amino]phenyl}-2-(2-methyl-3-oxo-spiro[3.5]non-1-en-1-ylamino) propanoic acid

The compound of Example 159 was hydrolysed by the method of Example 2 to afford the title compound in 86% yield. δH NMR (d⁶ DMSO) 8.82 (2H, s), 7.81 (1H, d, J 9.1Hz), 7.57 (2H, d, J 8.4Hz), 7.30 (2H, d, J 8.4Hz), 4.28 (1H, m), 3.13 (1H, m), 2.96 (1H, m), 1.70 - 1.49 (8H, m), 1.38 (3H, s), 1.14 (2H, m). m/z (ES⁺, 70V) 501.9 (MH⁺).

### EXAMPLE 161

### Ethyl (2S) 3-{4-[(3,5-Dichloroisonicotinoyl)amino]phenyl}-2-(3-oxo-2-propyl-spiro[3.5]non-1-en-1-ylamino) propanoate

Prepared in a similar manner to the compound of Example 151 from Intermediate 65 to give the title compound in 76% yield. δH NMR (CDCl₃) 8.58 (2H, s), 8.44 (1H, s), 7.65 (2H, d, J 8.5Hz), 7.16 (2H, d, J 8.4Hz), 5.38 (1H, m), 4.46 (1H, m), 4.30 (2H, q, J 7.1Hz). 3.17 (2H, m), 1.85 - 1.42 (14H, m), 1.38 (3H, t, J 7.1Hz), 0.90 (3H, t, J 7.3Hz). m/z (ES⁺, 70V) 558.1 (MH⁺).

### EXAMPLE 162

### (2S) 3-{4-[(3,5-Dichloraisonicotinoyl)amino]phenyl}-2-(3-oxo-2-propyl-spiro[3.5]non-1-en-1-ylamino) propanoic acid

The compound of Example 161 was hydrolysed by the method of Example 2 to afford the title compound in 84% yield. δH NMR (d⁶ DMSO, 380K) 10.42 (1H, br s), 8.69 (2H, s), 7.57 (2H, d, J 7.3Hz), 7.28 (d, 2H, J 8.3Hz), 7.12 (1H, d, J 9.1Hz), 4.21 (1H, m), 3.20 (1H, dd, J 4.9, 14.0Hz), 3.06 (1H, dd, J 8.8, 14.0Hz), 1.87 (2H, m), 1.72 - 1.45 (10H, m), 1.36 (2H, m), 1.23 (1H, m), 0.82 (3H, t, J 7.4Hz). m/z (ES⁺, 70V) 530.1 (MH⁺).

### EXAMPLE 163

### Ethyl (2S) 3-{4-[(3,5-Dichloroisonicotinoyl)amino]phenyl}-2-(2-methyl)-3-oxo-7-oxa-spiro[3,5]non-1-en-1-ylamino) propanoate

Prepared in a similar manner to the compound of Example 151 from Intermediate 68 to give the title compound in 71% yield. δH NMR (d⁶ DMSO) 8.80 (2H, s), 8.11 (1H, d, J 9.1Hz), 7.59 (2H, d, J 8.4Hz), 7.30 (2H, d, J 8.4Hz), 4.38 (1H, m), 4.17 (2H, q, J 7.1Hz), 3.75 (2H, m), 3.60 (2H, m), 3.15 (1H, dd, J 5.1, 13.7Hz), 2.99 (1H, dd, J 9.2, 13.6Hz), 1.90 (2H, m), 1.39 (3H, s), 1.19 (3H, t, J 7.1Hz). m/z (ES⁺, 70V) 532.0 (MH⁺).

### EXAMPLE 164

### (2S) 3-{4-[(3,5-Dichloroisonicotinoyl)amino]phenyl}-2-(2-methyl-3-oxo-7-oxa-spiro[3.5]non-1-en-1-ylamino)propanoic acid

The compound of Example 163 was hydrolysed by the method of Example 2 to afford the title compound in 89% yield. δH NMR (d⁶ DMSO) 8.81 (2H, s), 8.05 (1H, d, J 9.2Hz), 7.60 (2H, d, J 8.4Hz), 7.32 (2H, d, J 8.4Hz), 4.25 (1H, m), 3.75 (2H, m), 3.58 (2H, m), 3.13 (1H, dd, J 4.6, 13.8Hz), 2.98 (1H, dd, J 9.5, 13.8Hz), 1.94 (2H, m), 1.40 (3H, s), 1.29 (2H, m). m/z (ES⁺, 70V) 504.0 (MH⁺).

### EXAMPLE 165

### Ethyl (2S) 3-{4-[(3,5-Dichloroisonicotinoyl)amino]phenyl}-2-(3-oxo-2-propyl-7-oxa-spiro[3,5]non-1-en-1-ylamino) propanoate

Prepared in a similar manner to the compound of Example 151 from Intermediate 69 to give the title compound in 59% yield. δH NMR (d⁶ DMSO) 8.78 (2H, s), 8.08 (1H, d, J 8.8Hz), 7.55 (2H, d, J 8.4Hz), 7.29 (2H, d, J 8.5Hz), 4.23 (1H, m), 4.16 (2H, q, J 7.1Hz), 3.76 (2H, m), 3.58 (2H, m), 3.15 (1H. dd, J 4.8, 13.6Hz), 2.98 (1H, dd, J 9.7, 13.6Hz), 1.80 (2H, m), 1.18 (6H, m), 0.73 (3H, t, J 7.4Hz). m/z (ES⁺, 70V) 560.0 (MH⁺).

### EXAMPLE 166

### Ethyl (2S) 3-{4-[(3,5-Dichloroisonicotinoyl)amino]phenyl}-2-(3-oxo-2-propyl-7-oxa-spiro[3.5]non-1-en-1-ylamino) propanoate

The compound of Example 165 was hydrolysed by the method of Example 2 to afford the title compound in 82% yield. δH NMR (d⁶ DMSO, 380K) 10.39 (1H, br s), 8.68 (2H, s), 7.57 (2H, m), 7.33 (3H, m), 4.23 (1H, m), 3.70 (4H, m), 3.21 (1H, dd, J 4.8, 14.0Hz), 3.04 (1H, dd, J 9.0, 14.0Hz), 2.10 (1H, s), 1.88 (4H, m), 1.87 (3H, t, J 7.0Hz), 1.40 (4H, m). m/z (ES⁺, 70V) 532.1 (MH⁺).

### EXAMPLE 167

### (2S) 2-(2-Bromo-3-oxo-spiro[3.5]non-1-en-1-ylamino)-3-{4-[(3,5-dichloroisonicotinoyl)amino]phenyl}-propanoic acid 2-imidazol-1-yl-ethyl ester

Prepared in a similar manner to the compound of Example 138 from N-2-hydroxyethylimidazole [prepared according to the method of Yoshino et al, J. C. S. Perkin Trans. 1, 1977, 1266-72) to give the title compound in 48% yield. δH NMR (d⁶ DMSO) 10.88 (1H, s), 8.88 (1H, d, J 9.1Hz), 8.79 (2H, s), 7.66 (1H, s), 7.58 (2H, d, J 8.5Hz), 7.29 (2H, d, J 8.5Hz), 6.89 (1H, s), 4.84 (1H, m), 4.39 (2H, m), 4.29 (2H, m), 3.16 (1H, dd, J 4.6, 13.9Hz), 2.96 (1H, dd, J 9.7, 13.9Hz), 1.75 - 1.45 (8H, m), 1.35 (1H, m), 1.13 (1H, m). m/z (ES⁺, 70V) 662.1 (MH⁺).

### EXAMPLE 168

### 2-(2-Bromo-3-oxo-7-oxa-spiro[3.5]non-1-en-1-ylamino)-3-{4-[(3-methyl-[2,7]naphthyridine-1-yl)amino]phenyl}propanoic acid

Prepared by the methods as described herein in 77% yield. δH NMR (d⁶ DMSO) 8.77 (1H, s), 7.67 (1H, d, J 6.3Hz), 6.96 (2H, d, J 8.5Hz), 6.78 (1H, d, J 5.8Hz), 6.45 (2H, d, J 8.5Hz), 6.14 (1H, s), 4.19 (1H, m), 3.05 - 2.85 (4H, m), 2.59 (1H, dd, J 4.4, 14.0Hz), 2.25 (1H, dd, J 9.7, 13.9Hz), 1.66 (3H, s), 1.28 (1H, m), 1.16 (1H, m), 0.83 (1H, d, J 13.5Hz), 0.66 (1H, d, J 13.5Hz). m/z (ES⁺, 70V) 537.9 (MH⁺).

### EXAMPLE 169

### Ethyl 3-{4-[(3,5-Dichloroisonicotinoyl)amino]phenyl}-2-(2-[1,3]dithian-2-yl-3-oxo-spiro[3.5]non-1-en-1-ylamino) propanoate

To a solution of the compound of Example 27 (1.5g, 2.9mmol) in DCM (100ml) was added 1,3-dithienium tetrafluorborate (3g, 14mmol) [prepared by the method of Paterson I; Price L.G. Tet. Lett.1981, 22 (29), 2829]. The mixture was stirred overnight and then partitioned between EtOAc (200ml) and sodium carbonate (100ml, sat. aq.), the organics were separated, washed with water (3 x 50ml), brine (50ml), dried (Na₂SO₄), filtered and concentrated *in vacuo* to give a crude product which was purified by column chromatography (SiO₂: 4:1, EtOAc: hexane) to give the title compound as a pale yellow solid (0.6g, 86%) δH NMR (400MHz, d6 DMSO, 300K) □8.67 (2H, s), 8.15 (1H, d, J 9.5Hz), 7.67 (2H, d, J 8.5Hz), 7.12 (2H, d, J 8.5Hz), 5.06 (1H, m), 4.65 (1H, s),1.10 (1H, m), 4.08 (2H, t, J 7.1Hz), 3.17-2.72 (3H, m), 2.65 (2H, m), 1.95 (1H, m), 1.87 (1H, m), 1.78-1.46 (11H, m), 1.25 (1H, d, J 12.3Hz). m/z (ESI, 70V) MH⁺ 634.

### EXAMPLE 170

### (2S) 3-{4-[(3,5-Dichloroisonicotinoyl)amino]phenyl}-2-(2-[1,3]dithian-2-yl-3-oxo-spiro[3.5]non-1-en-1-ylamino) propanoic acid

To a solution of the compound of Example 169 (0.25g, 0.4mmol) in THF (2ml) was added a solution of lithium hydroxide (25mg, 0.6mmol) in water (1ml). The mixture was stirred at room temperature overnight, concentrated *in vacuo* and the residue dissolved in the minimum amount of water. HCl (2M, aq.) was added until the pH of the solution was 4, the resulting solid was filtered, washed with ether and ethyl acetate to give the product as a pale yellow solid (0.15g, 63%). δH NMR (400MHz, d⁶ DMSO, 300K) □10.85 (1H, s), 8.78 (2H, s), 8.28 (1H, d, J 9.9Hz), 7.55 (2H, d, J 8.5Hz), 7.31 (2H, d, J 8.5Hz), 5.06 (1H, m), 4.75 (1H, s), 3.21-2.78 (3H, m), 2.67 (2H, m), 1.99 (1H, m), 1.75 (1H, m), 1.57 (8H, m), 1.22 (1H, d, J 11.9Hz), 1.08 (1H, m). m/z (ESI, 70V) MH⁺ 606.

### EXAMPLE 171

### Ethyl (2S)-3-{4-[(3,5-Dichloro-1-oxy-pyridine-4-carbonyl)amino]-phenyl}-2-(2-methanesulfinyl-4,4-dimethyl-3-oxo-cyclobut-1-enylamino) propanoate

A solution of the compound of Example 5 (800mg, 1.68mmol) in THF (20ml) was treated at rt with a solution of methanesulfenyl chloride in DCM (1.9ml, 1.0M). After 30min the reacton was partitioned between EtOAc (50ml) and saturated aqueous sodium hydrogencarbonate (25ml). Separation of the organic phase, drying (MgSO₄), filtration and concentration *in vacuo* gave a solid which was approx. 90% pure. The crude solid was redissolved in DCM (20ml) and treated with m-CPBA (1.5g, 57-75%purity) and sodium hydrogencarbonate (500mg) and stirred at rt for 24h. The reaction was diluted with EtOAc (100ml), washed with H₂O (50ml), separated, dried (MgSO₄), filtered and concentrated *in vacuo.* Chromatography (SiO₂, EtOAc) gave the title compound as a white powder (730mg, 1.31mmol, 78%) as an approx. 1:1 mixture of diastereomers. δH (CDCI₃, 330K) 8.44 and 8.41 (2H, s), 8.35 and 8.17 (1H, s), 7.57 and 7.47 (2H, d, J 8.4Hz), 7.15 and 7.08 (2H, d, J 8.4Hz), 7.04 and 7.01 (1H, d, J 8.9Hz), 5.24-5.22 (1H, m), 4.25-4.11 (3H, m), 3.31-3.05 (2H, m), 2.97 and 2.93 (3H, s), 1.30-1.16 (9H, m). m/z (ES⁺, 70V) 553.9 and 556.0 (MH⁺).

### EXAMPLE 172

### (2S)-3-{4-[(3,5-Dichloro-1-oxy-pyridine-4-carbonyl)amino]phenyl}-2-(2-methanesulfinyl-4,4-dimethyl-3-oxo-cyclobut-1-enylamino) propanoic acid

The compound of Example 171 (300mg, 0.54mmol) was hydrolysed in a similar manner to the method of Example 2, to give the title compound as a white powder (239mg, 0.45mmol, 83%) as an approx. 1:1 diasteromeric mixture. δH (DMSO d⁶, 390K) 9.75 (1H, br s), 8.77 (2H, s), 7.56 (2H, d, J 8.5Hz), 7.25 (2H, d, J 8.5Hz), 4.99 (1H. m), 3.18-2.98 (2H, m), 2.85 (3H, s), 1.18 (3H, s), 1.06 (3H, s). m/z (ES⁺, 70V) 525.9 and 527.9 (MH⁺).

### EXAMPLE 173

### (2S)-2-[2-(Methylsulfanyl)-3-oxospiro[3.5]non-1-en-1-yl]amino-3-[4-([2,7]naphthyridin-1-ylamino)phenyl] propanoic acid

The ethyl ester of the title compound was prepared in two steps from the free amine of Intermediate 12 (1.40g, 0.41mmol), spiro[3.5]nonane-1,3-dione (650mg, 0.42mmol) [prepared according to the method of Wasserman, H.H. et al, J. Org. Chem., 38, 1451-1455 (1973)] and a 1.0M solution of methyl sulfenyl chloride in DCM (0.5ml, 0.5mmol) according to the method of Example 108 (1.53g, 0.30mmol, 71%). This ester was then subjected to hydrolysis according to the method of Example 2 to give the title compound as a yellow powder (1.15g, 0.23mmol, 57%). δH (DMSO d⁶, 390K) 9.80 (1H, s), 8.61 (1H, d, J 5.6Hz), 8.13 (1H, d, J 5.4Hz), 7.67 (2H, d, J 8.3Hz), 7.60 (1H, d, J 5.5Hz), 7.18 (1H, d, J 8.3Hz), 7.05 (1H, d, J 5.5Hz), 4.37 (1H, m), 3.22-3.12 (2H, m), 2.14 (3H, s), 1.85-1.10 (10H, m). m/z (ES⁺, 70V) 489.1 (MH⁺).

### EXAMPLE 174

### Ethyl-(2S)-2-[2-(methylsulfanyl)-3-oxo-7-oxaspiro[3.5]non-1-en-1-yl]amino-3-[4-([2,7]naphthyridin-1-ylamino)phenyl]propanoate

Was prepared in two steps from the free amine of Intermediate 12 (1.40g, 0.41mmol), 7-oxaspiro[3.5]nonane-1,3-dione (650mg, 0.42mmol) [prepared according to the method of Wasserman, H.H. et al, J. Org. Chem., 38, 1451-1455 (1973)] and a 1.0M solution of methyl sulfenyl chloride in DCM (0.5ml, 0.5mmol) according to the method of Example 108 to give the title compound as a yellow powder (1.21g, 0.23mmol, 55%). δH (DMSO d⁶, 390K) 9.82 (1H, s), 9.55 (1H, s), 8.93 (1H, d, J 9.2Hz), 8.65 (1H, d, J 5.6Hz), 8.15 (1H, d, J 5.7Hz), 7.78 (2H, d, J 8.5Hz), 7.68 (1H, d, J 5.6Hz), 7.23 (2H, d, J 8.5Hz), 7.13 (1H, d, J 5.7Hz), 5.16-5.10 (1H, m), 4.20 (2H, q, J 7.1Hz), 3.77 (2H, m), 3.59-3.52 (2H, m), 3.21 (1H, dd, J 4.5Hz 13.8Hz), 2.98 (1H, dd, J 10.2Hz 13.8Hz), 1.96 (3H, s), 1.48-1.32 (4H, m), 1.23 (3H, t, J 7.1Hz). m/z (ES⁺, 70V) 519.1 (MH⁺).

### EXAMPLE 175

### (2S)-2-[2-(Methylsulfanyl)-3-oxo-7-oxaspiro[3,5]non-1-en-1-yl]amino-3-[4-([2,7]naphthyridin-1-ylamino)phenyl]propanoic acid

Hydrolysis of the compound of Example 174 (650mg, 0.12mmol) with lithium hydroxide (30mg, 0.7mmol), according to the method of Example 2, gave the title compound as a pale yellow powder (501 mg, 0.10mmol, 85%). δH (DMSO d⁶, 390K) 9.83 (1H, s), 9.54 (1H, s), 8.70 (1H, d, J 8.9Hz), 8.65 (1H, d, J 5.6Hz), 8.14 (1H, d, J 5.7Hz), 7.75 (2H, d, J 8.5Hz), 7.68 (1H, d, J 5.6Hz), 7.21 (2H, d, J 8.5Hz), 7.11 (1H, d, J 5.7Hz), 4.92 (1H, m), 3.76-3.73 (2H, m), 3.62-3.54 (2H, m), 3.23 (1H, d, J 3.9Hz 13.6Hz), 2.94 (1H, dd, J 9.9Hz, 13.6Hz), 1.91 (3H, s), 1.47-1.28 (4H, m). m/z (ES⁺, 70V) 491.1 (MH⁺).

### EXAMPLE 176

### Methyl-(2S)-2-[(2-bromo-4,4-dimethyl-3-oxo-1-cyclobutenyl)amino]-3-[4-([2,7]naphthyridin-1-yloxy)phenyl]propanoate

A stirred solution containing Intermediate 13 (300mg, 0.72mmol) in THF (10ml) at rt was treated dropwise with a 1.0M solution of bromine in THF (1.0ml, 1mmol). After 2h the reaction was diluted with EtOAc (50ml), washed with saturated aqueous sodium hydrogencarbonate solution (2x25ml), dried (MgSO₄) and concentrated *in vacuo.* Chromatography (SiO₂, EtOAc) gave the title compound as a white powder (217mg, 0.43mmol, 61%). δH (DMSO d⁶, 360K) 9.71 (1H, s), 8.71 (1H, d, J 5.7Hz), 8.04 (1H, d, J 5.8Hz), 7.56 (1H, d, J 5.7Hz), 7.23-7.13 (5H, m), 6.19 (1H, d, J 8.1Hz), 5.06-5.02 (1H, m), 3.79 (3H, s), 3.26 (2H, m), 1.19 (6H, s). m/z (ES⁺, 70V) 497.9 (MH⁺).

### EXAMPLE 177

### (2S)-2-[(2-Bromo-4,4-dimethyl-3-oxo-1-cyclobutenyl)amino]-3-[4-([2,7]naphthyridin-1-yloxy)phenyl]propanoic acid

Hydrolysis of the compound of Example 176 (80mg, 0.016mmol) with LiOH (10mg, 0.023mmol) according to the method of Example 2 gave the title compound as a white powder (51 mg, 0.01 mmol, 63%). δH (DMSO d⁶, 360K) 9.71 (1H, s), 8.81 (1H, d, J 5.5Hz), 8.14 (1H, d, J 5.4Hz), 7.87 (1H, d, J 5.2Hz), 7.52 (1H, d, J 5.4Hz), 7.38 (2H, d, J 8.5Hz), 7.28 (2H, d, J 8.5Hz), 4.87-4.84 (1H, m), 3.31-3.15 (2H, m), 1.18 (3H, s), 1.10 (3H, s). m/z (ES⁺, 70V) 483.9 (MH⁺).

### EXAMPLE 178

### N⁴-(4-[3-(2-Bromo-3-oxospiro[3.5]non-1-en-1-yl)-5-oxo-1,3-oxazolan-4-yl]methylphenyl)-3,5-dichloroisonicotinamide

A solution containing the compound of Example 32 (1.0g, 1.76mmol), finely ground potassium carbonate (500mg) and DMAP (50mg, 0.4mmol), in DMF (14ml) was treated dropwise with chloromethyl pivalate (0.5ml) at room temperature. After 24h the reaction was diluted with EtOAc (150ml), washed with brine (3x50ml), dried (MgSO₄) and concentrated *in vacuo.* Chromatography (SiO₂, 1:1 EtOAc:hexanes) gave the title compound as a white powder (475mg, 0.82mmol, 47%). δH (DMSO d⁶, 390K) 10.97 (1H, s), 8.80 (2H, s), 7.63 (2H, d, J 8.5Hz), 7.17 (2H, d, J 8.5Hz), 5.41 (1H, d, J 3.9Hz), 4.95 (1H. m), 4.69 (1H, d, J 3.9Hz), 3.39-3.29 (2H, m), 1.99-1.06 (10H, m). m/z (ES⁺, 70V) 580.9 (MH⁺).

### EXAMPLE 179

### 3-[2-(Isopropylsulfanyl)-3-oxospiro[3.5]non-1-en-1-yl]-4-[4-([2,7]naphthyridin-1-ylamino)benzyl]-1,3-oxazolan-5-one

Prepared from the compound of Example 121 (450mg, 0.88mmol) in a similar manner to the compound of Example 178 to give the title compound as an off-white powder (390mg, 0.73mmol, 84%). δH (DMSO d⁶, 390K) 9.57 (1H, s), 8.64 (1H, d, J 5.7Hz), 8.24 (1H, d, J 5.8Hz), 8.17 (1H, s), 7.74 (2H, d, J 8.4Hz), 7.53 (1H, d, J 5.6Hz), 7.16 (2H, d, J 8.4Hz), 7.06 (1H, d, J 5.8Hz), 5.20 (1H, br s), 4.93 (1H, br s), 4.26 (1H, br s), 3.58 (1H, br s), 3.33 (1H, br s), 3.27 (1H, m), 1.99-1.06 16H, m). m/z (ES⁺, 70V) 529.2 (MH⁺).

### EXAMPLE 180

### Neopentyl (2S)-2-[(2-bromo-3-oxospiro[3.5]non-1-en-1-yl)amino]-3-{4-[(3,5-dichloroisonicotinoyl)amino]phenyl}propanoate

Using a similar procedure to that for the preparation the compound of Example 138 from the compound of Example 32 (0.5g, 0.89mmol), EDC (191mg, 1.0mmol), HOBT (120mg, 0.89mmol), neopentyl alcohol (0.4g, 4.5mmol) and DMF (15ml) was prepared, after purification by chromatography (SiO₂, EtOAc), the title compound as a white powder (400mg, 0.63mmol, 71%). δH (DMSO d⁶, 390K) 10.87 (1H, s), 8.92 (1H, d, J 9.1Hz), 8.79 (2H, s), 7.58 (2H, d, J 8.5Hz), 7.32 (2H, d, J 8.5Hz), 4.88-4.82 (1H, m), 3.86 (1H, d, J 10.4Hz), 3.80 (1H, d, J 10.4Hz), 3.26 (1H, dd, J 13.9, 4.8Hz), 3.03 (1H, dd, J 13.9, 10.1Hz), 1.99-1.06 (10H, m), 0.91 (9H, br s). m/z (ES⁺, 70V) 638.0 (MH⁺).

### EXAMPLE 181

### Isopropyl (2S) 3-{4-[(3,5-dichloroisonicotinoyl)amino]phenyl}-2-(2-methyl-3-oxo-7-oxa-spiro[3.5]non-1-en-1-ylamino) propanoate

Prepared in a similar manner to the compound of Example 138 from the compound of Example 164 to give the title compound in 79% yield. δH (DMSO d⁶) 10.87 (1H, s), 8.80 (2H, s), 8.10 (1H, d, J 8.9Hz), 7.59 (d, 2H, J 8.2Hz), 7.31 (d, 2H, J 8.2Hz), 4.98 (1H, m), 4.30 (1H, m), 3.76 (2H, m), 3.60 (2H, m), 3.11 (1H, dd, J 13.7, 5.3Hz), 3.00 (1H, dd, J 13.2, 9.1Hz), 2.00-1.80 (2H, m), 1.42 (3H, s), 1.17 (6H, m). m/z (ES⁺, 70V) 546.1 (MH⁺).

### EXAMPLE 182

### 5-Methyl-2-oxo-[1,3]dioxol-4-ylmethyl (2S) 2-(2-bromo-3-oxo-spiro[3.5]non-1-en-1-ylamino)-3-{4-[(3,5-dichloroisonicotinoyl)amino]phenyl} propanoate

To a stirred solution of the compound of Example 32 (1.0g, 1.76 mmol) and potassium carbonate (484mg, 3.52mmol) in DMF (20ml) at 0°C was added Intermediate 73 (408mg, 2.12mmol) in one portion. The ice-bath was removed and the mixture allowed to stir at room temperature for 3 hours. The mixture was poured into ice/water and extracted with EtOAc. The extract was washed three times with brine, dried (MgSO₄) and the solvent removed *in vacuo* to afford a yellow solid. Chromatography (SiO₂, 1:1 hexane:EtOAc) gave the title compound as a white solid (686mg, 57%). δH (DMSO d⁶) 10.90 (1H, s), 8.95 (1H, d, J 8.9Hz), 8.80 (2H, s), 7.60 (2H, d, J 8.3Hz), 7.26 (2H, d, J 8.3Hz), 4.86 (1H, m), 3.22 (1H, m, J 4.0, 13.6Hz), 3.06 (1H, m, J 13.2, 10.7Hz), 2.17 (3H, s), 1.74-1.38 (10H, m). m/z (ES⁺, 70V) 680.0 (MH⁺).

### EXAMPLE 183

### 2,3-Dihydroxy-propyl (2S) 2-(2-bromo-3-oxo-spiro[3.5]non-1-en-1-ylamino)-3-{4-[(3,5-dichloroisonicotinoyl)amino]phenyl} propanoate

Prepared in a similar manner to the compound of Example 138 from the compound of Example 32 and glycerol to give the title compound in 48% yield after chromatography on silica gel. δH (DMSO d⁶) 10.91 (1H, s), 8.91 (1H, d, J 9.2Hz), 8.80 (2H, s), 7.60 (2H, d, J 8.5Hz), 7.28 (2H, d, J 8.3Hz), 5.01 (1H, m), 4.85 (1H, m), 4.71 (1H, m), 4.20 (1H, m), 4.09 (1H, m), 3.71 (1H, m), 3.57 (1H, m), 3.26 (1H, dd, J 13.8, 3.9Hz), 3.04 (1H, dd, J 13.8, 9.3Hz), 1.80-1.45 (10H, m). m/z (ES⁺, 70V) 640.0 (MH⁺).

### EXAMPLE 184

### Tetrahydro-furan-3-ylmethyl (2S)-2-(2-bromo-3-oxo-spiro[3.5]non-1-en-1-ylamino)-3-{4-[(3,5-dichloroisonicotinoyl)amino]phenyl} propanoate

Prepared in a similar manner to the compound of Example 138 from the compound of Example 32 and tetrahydrofurfuryl alcohol to give the title compound in 52% yield after chromatography on silica gel. δH (DMSO d⁶) 10.88 (1H, s), 8.93 (1H, d, J 9.1Hz), 8.80 (2H, s), 7.60 (2H, d, J 8.3Hz), 7.28 (2H, d, J 6.9Hz), 4.84 (1H, m), 4.15 (2H, m), 4.05 (1H, m), 3.23 (1H, dd, J 13.8, 4.4Hz), 3.04 (1H, dd, J 13.6, 9.6,Hz), 2.00-1.50 (14H, m). m/z (ES⁺, 70V) 650.1 (MH⁺).

### EXAMPLE 185

### Tetrahydropyran-4-yl (2S)-2-(2-bromo-3-oxo-spiro[3.5]non-1-en-1-ylamino)-3-{4-[(3,5-dichloroisonicotinoyl)amino]-phenyl} propanoate

Prepared in a similar manner to the compound of Example 138 from the compound of Example 32 (0.5g, 0.89mmol), EDC (300mg), HOBT (200mg) and 4-hydroxytetrahydropyran (0.8ml) in DMF (5ml) to give the title compound (0.42g, 74%). δH (300MHz, DMSO d⁶) 11.02 (1H, br), 9.04 (1H, d, J 9.0Hz), 8.92 (2H, s), 7.73 (2H, d, J 8.4Hz), 7.42 (2H, d, J 8.5Hz), 5.13 (1H, br), 4.93 (1H, br), 3.87 (2H, br), 3.60 (2H, br), 3.36 (1H, dd, J 14.0, 5.1Hz), 3.18 (1H, dd, J 13.9, 9.3Hz), 1.61-2.06 (12H, m), 1.52 (2H, d, J 12.6Hz), 1.26 (2H, br). *m*/*z* (ES⁺, 70V) 652 (MH⁺).

### Example 186

### Isopropyl (2S)-2-(2-bromo-3-oxo-7-oxa-spiro[3.5]non-1-en-1-ylamino)-3-{4-[(3,5-dichloroisonicotinoyl)amino]phenyl} propanoate

To a solution of the isopropyl ester of the compound of Example 40 (0.4g, 8.1mmol) [prepared in a similar manner to the compound of Example 138] in THF (5ml) at room temperature was added NBS (0.3g). The mixture was stirred for 2h and then partitioned between water (100ml) and EtOAc (100ml), the organics were separated and washed with water (3x50ml), brine (50ml), dried (Na₂SO₄), filtered and concentrated *in vacuo* to give a crude oil. Purification by column chromatography (3:2, hexane:EtOAc) gave the title compound as a white solid (0.24g, 52%). δH (400MHz, DMSO d⁶) 9.02 (1H, d, J 9.0Hz), 8.79 (2H, s), 7.59 (2H, d, J 8.5Hz), 7.27 (2H, d, J 8.5Hz), 4.97 (1H, m), 4.75 (1H, m), 3.80 (2H, m), 3.58 (2H, q, J 11.7Hz), 3.20 (1H, dd, J 13.0, 5.0Hz), 3.03 (1H, dd, J 13.0, 9.4,Hz), 1.97 (2H, m), 1.49 (1H, dd, J 13.0, 1.6Hz), 1.33 (1H, dd, J 13.2, 1.6Hz), 1.23 (3H, d, J 11.3Hz), 1.19 (3H, d, J 11.4Hz). *m*/*z* (ES⁺, 70V) MH⁺ 612.

### EXAMPLE 187

### Ethyl (2S)-3-{5-[(3,5-dichloroisonicotinoyl)amino]pyridin-2-yl}-2-(4,4-dimethyl-2-methylsulfanyl-3-oxo-cyclobut-1-enylamino) propanoate

A mixture of Intermediate 43 (0.44g, 1.15mmol) and 3-hydroxy-4,4-dimethyl-2-cyclobutenone (0.14g, 1.1 eq) was stirred at room temperature for a period of 17h. The mixture was concentrated *in vacuo* and triturated with diethyl ether and the resulting solid re-dissolved in THF (10ml). The solution was treated with a solution of methanesulfenyl chloride at 0-5°C in DCM until TLC analysis of the mixture indicated complete consumption of starting material. The mixture was partitioned between EtOAc (50ml) and water (50ml), the organics were separated and and washed with water (2x50ml), brine (50ml), dried MgSO4), filtered and concentrated to give a crude white foam. Purification by column chromatography (EtOAc: hexane, 1:1) gave the title compound as a white solid (0.55g, 91%). δH (400MHz, DMSO d⁶) 11.16 (1H, br), 8.87 (1H, d, J 9.0Hz), 8.82 (s, 2H), 8.72 (1H, d, J 2.5Hz), 8.05 (1H, dd, J 8.4, 2.5Hz), □7.35 (1H, d, J 8.4Hz), 5.33 (1H, m), 4.18 (2H, q, J 7.1Hz), 3.36 (1H, dd, J 14.1, 5.0Hz), 3.32 (3H, s), 3.20 (1H, dd, J 14.1, 9.4Hz), 0.94 (3H, s), 1.09 (3H, s), 1.21 (3H, t, J 7.1Hz),. *m*/*z* (ES⁺, 70V) MH⁺ 523.

### EXAMPLE 188

### Isopropyl (2S)-3-{5-[(3,5-Dichloroisonicotinoyl)amino]pyridin-2-yl}-2-(4,4-dimethyl-2-methylsulfanyl-3-oxo-cyclobut-1-enylamino) propanoate

Hydrolysis of the compound of Example 187 (0.35g, 0.67mmol) according to the method of Example 2 and re-esterified with isopropanol (EDC, HOBT, DMF) gave the title compound as a white solid (65mg, 18%). δH (400MHz, DMSO d⁶) □11.16 (1H, br), 8.85 (1H, d, J 8.9Hz), 8.82 (2H, s), 8.72 (1H, d, J 1.8Hz), 8.04 (1H. m), 7.35 (1H. d, J 8.3Hz), 5.28 (1H, m), 5.00 (1H, m), 3.35 (1H, dd, J 14.1, 5.0Hz), 3.32 (3H, s), 3.18 (1H, dd, J 14.1, 9.3Hz), 1.22 (6H, m), 1.09 (3H, d, J 1.2Hz), 0.95 (3H, d, J 1.2Hz). *m*/*z* (ES⁺, 70V) MH⁺ 537.

### EXAMPLE 190

### Ethyl (2S)-3-{4-[(3,5-dichloro-1-oxy-pyridine-4-carbonyl)amino]phenyl}-2-(3-oxo-spiro[3.5]non-1-en-1-ylamino) propanoate

Intermediate 75 (170mg, 0.42 mmol) and 1-keto-3-hydroxy[3,5]-non-2-ene (200mg, 1.3mmol) were stirred together at room temperature overnight in THF (5ml) The reaction mixture was diluted with DCM (50ml), washed with sodium bicarbonate solution (saturated, 2x50ml), dried (MgSO₄) and reduced *in vacuo* to give a yellow solid. The residue was chromatographed (SiO₂ DCM:methanol, 98:2) to give the title compound as a white powder (120mg). δH (CDCI₃) 9.03 (1H, br s), 8.11 (2H, s), 7.64 (2H, d, J 7.9Hz) 7.18 (2H, d, J 7.5Hz), 5.01 (1H, m), 4.22 (1H, m), 4.21 (2H, q, J 7.1Hz), 3.12 (2H, m), 1.45 (10H, m), 1.30 (3H, t, J 7.1Hz). *m*/*z* (ES⁺, 70V) 532.0 (MH⁺).

### Example 191

### (2S)-3-{4-[(3,5-Dichloro-1-oxy-pyridine-4-carbonyl)amino]phenyl}-2-(3-oxo-spiro[3.5]non-1-en-1-ylamino) propanoic acid

The compound of Example 190 (30mg, 0.056mmol) was hydrolysed by the method of Example 2 to afford the title compound as white powder (20mg). δH (CD₃OD) 8.46 (2H, s), 7.47 (2H, d, J 8.5Hz), 7.18 (2H, d, J 8.5Hz), 4.14 (1H, m), 3.21 (1H, m), (obscured by MeOH/water) 2.83 (1H, dd, J 9.6, 4.2Hz), 1.80-1.10 (9H, m), 1.07 (3H, t, J 7.1Hz). *m*/*z* (ES⁺, 70V) 505.0 (MH⁺).

### EXAMPLE 192

### Ethyl (2S)-2-(2-bromo-3-oxo-spiror[3.5]non-1-en-1-ylamino)-3-{4-[(3,5 dichloro-1-oxy-pyridine-4-carbonyl)aminolphenyl) propanoate

Prepared from the compound of Example 190 (85mg, 0.160 mmol) in a similar manner to the compound of Example 31 to give the title compound (80mg). δH (CDCI3) 8.94 (1H, br s), 8.14 (2H, s), 7.62 (2H, d, J 8.4Hz), 7.13 (2H, d, J 8.3Hz), 5.88 (1H, m), 5.00 (1H, m), 4.26 (2H, q, J 7.1Hz), 3.26 (2H, m), 2.03-1.41 (10H, m), 1.35 (3H, t, J 7.1Hz). *m*/*z* (ES⁺, 70V) 612.0 (MH⁺).

### EXAMPLE 193

### (2S)-2-(2-Bromo-3-oxo-spiro[3.5]non-1-en-1-ylamino)-3-{4-[(3,5-dichloro-1-oxy-pyridine-4-carbonyl)amino]phenyl} propanoic acid

The compound of Example 192 was hydrolysed by the method of Example 2 to afford the title compound. δH (DMSO d⁶) 10.80 (1H, s) 8.73 (2H, s), 7.55 (2H, d, J 8.0Hz), 7.24 (2H, d, J 8.4Hz), 4.65 (1H, m), 3.22 (1H, dd, J 13.8, 4.4Hz,), 3.00 (1H, dd, J 13.7, 4.4Hz), 1.82-1.00 (11H, m).

### EXAMPLE 194

### Ethyl (2S)-2-(2-chloro-3-oxo-spiro[3,5]non-1-en-1-ylamino)-3-{4-[(3,5-dichloro-1-oxy-pyridine-4-carbonyl)amino]phenyl} propanoate

Prepared in a similar manner to the compound of Example 61 from the compound of Example 190 (500mg, 0.94 mmol) and N-chlorosuccinimide (150mg, 1.13mmol) to give the title compound as a white powder (220mg). δH 10.85 (1H,s), 8.84 (1H, d, J 9.0Hz), 8.75 (2H, s), 7.58 (2H, d, J 8.5Hz) 7.27 (2H, d, J 8.5Hz), 4.68 (1H, m) 4.20 (2H, q, J 7.0Hz) 3.22 (1H, dd, J 13.8, 4.7Hz) 3.01 (1H, dd, J 13.7, 9.7Hz) 1.74-1.55 (9H, m) 1.38 (1H, m) 1.23 (3H, m, J 7.1Hz) 1.13 (1H, m). *m*/*z* (ES⁺, 70V) 568.0 (MH⁺).

### EXAMPLE 195

### (2S)-2-(2-Chloro-3-oxo-spiro[3,5]non-1-en-1-ylamino)-3-{4-[(3,5-dichloro-1-oxy-pyridine-4-carbonyl)amino]phenyl}propanoic acid

The compound of Example 194 was hydrolysed by the method of Example 2 to afford the title compound. δH (DMSO d⁶) 10.83 (1H, s), 8.75 (m, 3H), 7.57 (2H, d, J 8.4Hz) 7.26 (2H, d, J 8.5Hz), 4.63 (1H, m) 3.22 (1H, dd, J 13.8, 4.5Hz) 3.00 (1H, m), 1.64-1.55 (9H, m) 1.35 (1H, m), 1.15 (1H, m). *m*/*z* (ES⁺, 70V) 538.0 (MH⁺).

### EXAMPLE 196

### Ethyl (2S)-2-[(2-chloro-4,4-dimethyl-3-oxo-1-cyclobutenyl)amino]-3-{4-[(3,5-dichloroisonicotinoyl)amino]phenyl}propanoate

Prepared in a similar manner to the compound of Example 61 from the compound of Example 5 (3.2mmol) and N-chlorosuccinimide (3.5mmol) to give the title compound as a white powder (0.9mmol, 31%). δH (DMSO d⁶, 300K) 9.05 (1H, d, J 9.0Hz), 8.79 (2H, s) 7.60 (2H, d, J 8.5Hz) 7.25 (2H, d, J 8.5Hz) 4.70 (1H, m) 4.19 (2H, q, J 7.1Hz) 3.22 (1H, dd, J 13.9, 5.0Hz) 3.02 (1H, dd, J 13.9, 9.2Hz) 1.21 (3H, q, J 7.1Hz) 1.23 (3H, s) 1.04 (3H, s). *mlz* (ES⁺, 70V) 512.0 (MH⁺).

### EXAMPLE 197

### 1-Methyl-3-pyrrolidinyl (2S)-2-[(2-bromo-4,4-dimethyl-3-oxo-1-cyclobutenyl)amino]-3-{4-[(3,5-dichloroisonicotinoyl) amino]phenyl} propanoate

Prepared in a similar manner to the compound of Example 138 from the compound of Example 34 (0.65 mmol), EDC (0.72mmol), HOBT (0.72mmol) and 1-methyl-3-pyrrolidinol (1.95mmol) in DMF (5ml) to give the title compound (0.25mmol, 40%) δH (CD₃OD) 8.55 (2H, s), 7.52 (2H, d, J 8.5Hz), 7.20 (2H, d, J 8.5 Hz), 4.92, (1H, m), 3.38 (1H, dd, obscured by MeOH) 2.96 (1H, dd, J 13.9, 9.2Hz) 2.50-2.18 (4H, m) 2.18 (3H, s) 1.89-1.56 (m, 4H), 1.12 (3H, s), 1.00 (3H, s). *m*/*z* (ES⁺, 70V) 625.0 (MH⁺).

### EXAMPLE 198

### Isopropyl (2S)-2-(2-bromo-4,4-dimethyl-3-oxo-cyclobut-1-enylamino)-3-{4-[(3,5-dichloroisonicotinoyl)amino]phenyl} propanoate

Prepared in a similar manner to the compound of Example 138 from the compound of Example 34 (0.65 mmol), EDC (0.84mmol), HOBT (0.84mmol) and isopropanol (2.28mmol) in DMF (5ml) to give the title compound (0.25mmol, 34%). δH (DMSO d⁶) 10.88 (1H, s) 9.69 (1H, d, J 9.0Hz) 8.79 (2H, s) 7.59 (2H, J 8.5 Hz) 7.28 (2H, d, J 8.5Hz), 4.97 (1H, m) 4.75 (1H, m) 3.20 (1H, dd, J 13.9, 5.1Hz) 3.03 (1H, dd, J 14.0, 9.2Hz) 1.24 (m, 6H) 1.14 (3H, s) 1.05 (3H, s). *m*/*z* (ES⁺, 70V) 570.0 (MH⁺).

### EXAMPLE 199

### Ethyl (2S)-3-[4-(2,6-Dichlorobenzoylamino)phenyl]-2-(3-oxo-spiro[3.5]non-1-en-1-ylamino) propanoate

The title compound was prepared in a similar manner to that of the compound of Example 27 (coupling of amino acid ethyl ester (1.68mmol), dione (1.68mmol) in DCM (5ml)) to give the title compound as a yellow powder (1.1 mmol, 66%). δH (CD₃OD) 7.83 (2H,d, J 8.3Hz), 7.55-7.35 (4H, m), 7.47 (2H, d, J 8.4Hz), 4.62 (1H, s), 4.50 (3H, m), 3.50 (1H, m), 3.08 (1H, m) 2.05-1.55 (11H, m) 1.49 (3H, t, J 7.1Hz). *m*/*z* (ES⁺, 70V) 501.0 (MH⁺).

### EXAMPLE 200

### Ethyl (2S)-2-(2-bromo-3-oxo-spiro[3.5]non-1-en-1-ylamino)-3-[4-(2,6-dichlorobenzoylamino)phenyl] propanoate

Prepared in a similar manner to the compound of Example 72 from the compound of Example 199 (1.08mmol) to give the title compound as yellow powder (0.86mmol, 80%). δH (CD₃OD) 7.53 (2H, d, J 8.4Hz), 7.38-7.15 (4H, m), 7.17 (2H, d, J 8.4Hz), 5.32 (1H, m) 4.65 (1H, m), 3.22 (1H, dd, J 13.9, 4.4Hz), 3.18 (q, 2H, J 7.1Hz) 2.95 (1H, dd J 13.9, 9.5Hz,),1.85-1.20 (14H, m). ). *m*/*z* (ES⁺, 70V) 581.0 (MH⁺).

### EXAMPLE 201

### (2S)-2-(2-Bromo-3-oxo-spiro[3.5]non-1-en-1-ylamino)-3-[4-(2,6-dichlorobenzoylamino)-phenyl] propanoic acid

Prepared in a similar manner to the compound of Example 2 from the compound of Example 200 (0.85mmol) to give the title compound as white powder (0.60mmol, 60%). δH (DMSO d⁶) 13.39 (1H, br s), 10.70 (1H, d, J 6.1Hz), 8.81 (1H, d, J 9.2Hz), 7.61 (3H, m), 7.56 (1H, m), 7.23 (2H, d, J 8.2Hz), 3.18 (1H, dd, J 13.9, 4.4Hz), 2.98(1H, dd J 13.8, 9.6Hz), 2.89-1.20 (11H, m). *m*/*z* (ES⁺, 70V) 567.0 (MH⁺).

### EXAMPLE 202

### Isopropyl (2S)-2-(2-bromo-3-oxo-spiror[3.5]non-1-en-1-ylamino)-3-[4-(2,6-dichlorobenzoylamino)phenyl] propanoate

Prepared in a similar manner to the compound of Example 138 from the compound of Example 201 (0.78 mmol) to give the title compound (0.49mmol, 63%). δH (DMSO d⁶) 10.71 (1H, s), 8.89(1H, d, J 9.0Hz), 7.62-7.47 (5H, cm), 4.97,(1H,m), 3.17 (1H, dd, J 4.8Hz, 13.8Hz), 3.00 (1H, dd J 9.7Hz, 13.8Hz), 1.79-1.50 (8H, c m) 1.35 (1H,m) 1.24-1.11 (7H,m). *m*/*z* (ES⁺, 70V) 609.0 (MH⁺).

### EXAMPLE 203

### Ethyl (2S)-3-[4-(3-methyl-[2,7]naphthyridin-1-ylamino)phenyl]-2-(3-oxo-spiro[3.5]non-1-en-1-ylamino) propanoate

Prepared in a similar manner to that of the compound of Example 3 to give the title compound as a yellow powder (1.5mmol, 76%). δH (CDCl3) 9.56 (1H, s), 8.53 (1H, d, J 5.8Hz), 7.78 (2H, d, J 8.4Hz), 7.45 (1H, d, J 5.8Hz), 7.08 (2H, d, J 8.5Hz), 6.89 (1H, s), 5.77 (1H, m), 4.57 (1H, s), 4.27 (2H, q, J 7.1Hz), 3.10 (2H, m), 2.54 (3H, s), 1.84-1.23 (14H, m ). *m*/*z* (ES⁺, 70V) 485.2 (MH⁺).

### EXAMPLE 204

### Ethyl (2S)-2-(2-bromo-3-oxo-spiro[3.5]non-1-en-1-ylamino)-3-[4-(3-methyl-[2,7]naphthyridin-1-ylamino)phenyl] propanoate

Prepared in a similar manner to the compound of Example 31 from the compound of Example 203 (0.62mmol), bromine (0.81 mmol) and triethylamine (0.81mmol) in DCM (5ml)) to give the title compound as yellow powder (0.25mmol, 40%). δH (CD₃OD) 9.44 (1H, s), 8.34 (1H, d, J 5.8Hz), 7.43 (1H, d, J 5.8Hz), 7.09 (2H, d, J 8.5 Hz), 6.81 (1H, s), 4.87 (1H, m), 4.89 (1H, m), 4.13 (2H, q, J 7.1Hz), 3.22 (1H, m), (obscured mostly by MeOH), 2.92 (1H, dd, J 14.0, 9.7Hz), 2.34 (3H, s), 1.58-1.26 (10H, m), 1.18 (3H, t, J 7.1Hz). *m*/*z*(ES⁺, 70V) 564.2 (MH⁺).

### EXAMPLE 205

### Ethyl (2S)-2-(2-bromo-3-oxo-spiro[3.5]non-1-en-1-ylamino)-3-[4-(3-methyl-[2,7]naphthyridin-1-ylamino)phenyl] propanoate

Prepared in a similar manner to to the compound of Example 2 from the compound of Example 204 (0.22mmol) to give the title compound as yellow powder (0.20mmol, 90%). δH (DMSO d⁶) 9.76 (1H, s), 9.70 (1H, s), 8.87 (1H, s, J 9.5Hz), 8.56 (1H, d, J 5.6Hz), 7.87 (2H, d, J 8.4Hz), 7.56 (1H, d, J 5.6Hz), 7.20 (2H, d, J 8.4Hz), 6.96 (1H, s), 4.73 (1H, m), 3.22 (1H, dd, J 13.9, 4.0Hz), 2.93 (1H, dd J 13.5, 10.1Hz,), 2.42 (3H, s), 1.80-1.00 (11H, m). *m*/*z* (ES⁺, 70V) 535.0 (MH⁺).

### EXAMPLE 206

### (2S)-3-[4-(3-Methyl-[2,7]naphthyridin-1-ylamino)phenyl]-2-(3-oxo-spiro[3.5]non-1-en-1-ylamino) propanoic acid

Prepared in a similar manner to the compound of Example 2 from the compound of Example 203 (0.62mmol) to give the title compound as white powder (0.27mmol, 43%). δH (DMSO d⁶) 9.81 (1H,s), 9.52 (1H, s), 8.58 (1H, d, J 5.6Hz), 8.30 (1H, J 8.6Hz), 7.86 (2H, d, J 8.4Hz), 7.57(1H, d, J 5.6Hz), 7.22 (2H, d, J 8.5Hz), 6.97(1H, s), 4.08 (1H, m), 4.32 (1H, s), 3.15 (1H, dd, J 13.7, 4.7Hz), 2.97 (1H, dd, J 13.7, 9.5Hz), 2.44 (3H, s), 1.74-1.45 (9H, m), 1.24-1.15 (2H, m). *m*/*z* (ES⁺, 70V) 457.1 (MH⁺).

### EXAMPLE 207

### Ethyl (S)-3-[4-([2,7]naphthyridin-1-ylamino)phenyl]-2-(3-oxo-spiro[3.5]non-1-en-1-ylamino) propanoate

Prepared in a similar manner to the compound of Example 3 to give the title compound as a yellow powder (1.4 mmol, 73%) δH (CDCI3) 9.61 (1H,s), 8.65 (1H, d, J 5.7Hz), 8.25 (1H, d, J 5.8Hz), 7.71 (2H, d, J 8.4Hz), 7.63 (1H, d, J 8.5Hz), 7.12 (2H, d, J 8.5Hz), 7.05 (1H, d, J 5.8Hz), 5.80 (1H, m), 4.55 (1H, s), 4.29 (2H, q, J 7.2Hz), 3.13 (2H, m), 1.87-1.25 (14H, m). *m*/*z* (ES⁺, 70V) 471.1 (MH⁺).

### EXAMPLE 208

### Ethyl (2S)-2-(2-bromo-3-oxo-spiro[3.5]non-1-en-1-ylamino)-3-[4-([27]naphthyridin-1-ylamino)-phenyl] propanoate

Prepared in a similar manner to that of Example 31 from the compound of Example 207 (0.64mmol) to give the title compound as a yellow powder (0.45mmol, 76%). δH (CDCl3) 9.81 (1H, s), 8.64 (1H, d, J 5.7Hz), 8.29 (1H, d, J 5.8Hz), 7.75 (2H, d, J 8.3Hz), 7.60 (1H, d, J 5.8Hz), 7.12 (2H, d, J 8.4Hz), 7.08 (1H, d, J 5.7Hz), 5.91 (1H, m), 5.03 (1H, m), 4.28 (2H, q, J 7.1Hz), 3.29 (2H, m), 1.81-1.39 (10H, m), 1.35 (3H, t, J 7.1Hz). *m*/*z* (ES⁺, 70V) 550.0 (MH⁺).

### EXAMPLE 209

### (S)-2-(2-Bromo-3-oxo-spiro[3.5]non-1-en-1-ylamino)-3-[4-([2,7]naphthyridin-1-ylamino)-phenyl]-propionic acid

Prepared in a similar manner to the compound of Example 2 from the compound of Example 208 (0.40mmol) to give the title compound as white powder (0.25mmol, 64%) δH (DMSO d⁶, 300 K) 9.90 (1H, s), 9.56 (1H, s), 8.86 (1H,d, J 9.3Hz), 8.66 (1H, d, J 5.6Hz), 8.17 (1H, d, J 5.7Hz), 7.81 (2H, d, J 8.2Hz), 7.70 (1H, d, J 5.6Hz), 7.24 (2H, d, J 8.4Hz), 7.14 (1H, d, J 5.7Hz), 4.78 (1H, m) 3.23 (1H, dd, J 13.9, 4.1Hz), 2.99 (1H, dd, J 13.7, 10.0Hz), 1.81-1.04 (11H, m). *m*/*z* (ES⁺, 70V) 522.0 (MH⁺).

### EXAMPLE 210

### (2S)-3-[4-([2,7]Naphthyridin-1-ylamino)phenyl]-2-(3-oxo-spiro[3.5]non-1-en-1-ylamino)propanoic acid

Prepared in a similar manner to the compound of Example 2 from the compound of Example 207 (0.64mmol) to give the title compound as white powder (0.21 mmol, 33%). δH (DMSO d⁶, 300K) 9.85 (1H, s), 9.54 (1H, s), 8.67 (1H,d, J 5.6Hz), 8.28 (1H, d, J 8.6Hz), 8.18 (1H, d, J 5.6Hz), 7.78 (2H, d, J 8.3Hz), 7.70 (1H, d, J 5.6Hz), 7.23 (2H,d, J 8.4Hz), 7.14 (1H, d, J 5.7Hz), 4.34 (1H, s), 4.08 (1H, m), 3.15 (1H, dd, J 13.8, 4.8Hz), 2.95 (1H, dd, J 13.8, 9.4Hz), 1.74-1.39 (9H, m), 1.20 (2H, m). *m*/*z* (ES⁺, 70V) 443.1 (MH⁺).

### EXAMPLE 211

### (2S)-3-[4-[(2,7]Naphthyridin-1-yloxy)phenyl]-2-(3-oxo-spiro[3.5]non-1-en-1-ylamino) propanoic acid

Prepared in a similar manner to the compound of Example 2 (hydrolysis from the compound of Example 35 (0.70 mmol) to give the title compound as a white powder (0.56 mmol, 80%). δH (DMSO d⁶, 300K) 9.70 (1H, s), 8.81 (1H, d, J 5.7Hz), 8.30 (1H, d, J 8.8Hz), 8.10 (1H, d J 5.8Hz), 7.89 (1H, d, J 5.7Hz), 7.53 (1H, d, J 5.9Hz), 7.34 (1H, d, J 8.5Hz), 7.23 (2H, d, J 8.5Hz), 4.34 (1H, s), 4.15 (1H, m), 3.21 (1H, dd, J 14.0, 4.8Hz), 3.00 (1H, dd, J 13.8, 9.7Hz), 1.71-1.50 (11H, m). *m*/*z* (ES⁺, 70V) 444.6 (MH⁺).

### EXAMPLE 212

### Ethyl (2S)-3-{4-[(3,5-dichloroisonicotinoyl)amino]phenyl}-2-(2-hydroxy-3-oxo-spiro[3.5]non-1-en-1-ylamino)-propionic acid ethyl ester

To a solution of the compound of Example 27 (1.0g, 1.9mmol) in DCM (40ml) at -40°C was added lead tetraacetate (0.94g, 1.1 eq). The mixture was allowed to warm to 0°C and stirred at this temperature for 8h. The reaction mixture was partitioned between EtOAc (200ml) and water (100ml), the organics were separated washed with water (2×100ml), brine (50ml), dried (MgSO₄), filtered and concentrated *in vacuo* to give a crude oil. The crude was dissolved in ethanol (10ml) and treated with NaH (100mg). The mixture was stirred at room temperature until TLC analysis indicated that all starting material had been consumed. The reaction was quenched by the addition of NH₄Cl (5ml, sat. aq.), EtOAc (2x20ml) extraction of the mixture followed by washing with water (10ml), brine (10ml), drying (MgSO₄), filtering and concentration *in vacuo* to give a crude product which was purified by column chromatography (SiO₂, EtOAc:Hexane 1:1) to give the title compound as a white foam (0.89g, 86%). δH (DMSO d⁶, 400MHz) 10.83 (1H, br), 8.78 (2H, s), 7.51 (2H, d, J 8.5Hz), 7.12 (2H, d, J 8.5Hz), 4.94 (1H, dd, J 11.4, 5.0Hz), 4.10 (2H, m), 3.33 (1H, dd, J 14.1, 4.9Hz), 3.14 (1H, dd, J 14.0, 11.4Hz), 1.40-1.63 (4H, m), 1.19-1.33 (6H, m), 1.16 (3H, t, J 7.1Hz). *m*/*z* (ES⁺, 70V) 532 (MH⁺).

### EXAMPLE 213

### Ethyl (2S)-3-{4-[(3,5-Dichloroisonicotinoyl)amino]phenyl}-2-(2-methoxy-3-oxo-spiro[3.5]non-1-en-1-ylamino) propanoate

To a solution of the compound of Example 212 (0.8g, 1.5mmol) in acetone (15ml) was added K₂CO₃ (5g) and methyl iodide (2.5ml). The mixture was stirred at room temperature for 5 days. The mixture was filtered and concentrated *in vacuo* and the residue purified by column chromatography (SiO₂, EtOAc:Hexane 1:1) to give the title compound as a white solid (0.45g, 55%). δH (DMSO d⁶, 400MHz) 8.50 (2H, d, J 4.8Hz), 7.21 (2H, d, J 8.4Hz), 7.04 (2H, d, J 8.4Hz), 4.87 (1H, dd, J 11.8, 4.9Hz), 4.00-4.16 (2H, m), 3.34 (3H, s), 3.26 (1H, dd, J 13.9, 4.9Hz), 3.07 (1H, dd, J 13.9, 11.6Hz), 1.15-1.66 (10H, m), 1.12 (3H, t, J 7.0Hz). *m*/*z* (ES⁺, 70V) 546 (MH⁺).

### EXAMPLE 214

### Ethyl (2S)-2-(2-bromo-3-oxo-spiro[3.5]non-1-en-1-ylamino)-3-(2,4,6-trimethoxy[1,1'-biphenyl]-4-yl) propanoate

The title compound was prepared by the methods as described herein. δH (CDCI₃) 7.19 (2H, d, J 8.1Hz), 7.04 (2H, d, J 8.1Hz), 6.14 (2H, s), 5.84 (1H, d, J 8.6Hz), 4.98 (1H, m), 4.20 (2H, q, J 7.1Hz), 3.78 (3H, s), 3.62 (6H, s), 3.21 (2H, d), 1.97-1.40 (10H, m), 1.24 (3H, t, J 7.1Hz). *m*/*z* (ES⁺, 70V) 572 (MH⁺).

### EXAMPLE 215

### (2S)-2-(2-Bromo-3-oxo-spiro[3.5]non-1-en-1-ylamino)-3-(2,4,6-trimethoxy[1,1'-biphenyl]-4-yl) propanoic acid

Prepared from the compound of Example 214 by the method of Example 2 to give the title compound. δH (DMSO d⁶) 12.30 (1H, br s), 8.79 (2H, d, J 10.0Hz), 7.19 (2H, d, J 8.1Hz), 7.08 (2H, d, J 8.1Hz), 6.29 (2H, s), 4.78 (1H, m), 3.81 (3H, s), 3.61 (6H, s), 3.27 (1H, m), 2.98 (1H, dd, J 13.4, 10.2Hz), 1.95-1.00 (10H, m). *m*/*z* (ES⁺, 70V) 544 (MH⁺).

### EXAMPLE 216

### Tetrahydro-furan-2-ylmethyl (2S)-2-(2-Bromo-3-oxo-spiro[3.5]non-1-en-1-ylamino)-3-{4-[(3,5-dichloro-pyridine-4-carbonyl)amino]phenyl}propanoate

Using a similar procedure to that for the preparation of the compound of Example 138 form the compound of Example 32 (0.25g, 0.44mmol), EDC (150mg), HOBT (100mg) and tetrahydrofurfurylalcohol (0.5ml) in DMF (2ml) was prepared the title compound (0.15g, 52%). δH (400MHz, DMSO d⁶) 10.88 (1H, s), 8.93 (1H, d, J 9.1Hz), 8.80 (2H, s), 7.60 (2H, d, J 8.3Hz), 7.28 (2H, d, J 6.9Hz), 4.84 (1H, m), 4.15 (2H, m), 4.05 (1H, m), 3.23 (1H, dd, J 13.8, 4.4Hz), 3.04 (1H, dd, J 13.6, 9.6Hz), 1.50-2.00 (14H, m). *m*/*z* (ES⁺, 70V) 650 (MH⁺).

The following assays can be used to demonstrate the potency and selectivity of the compounds according to the invention. In each of these assays an IC₅₀ value was determined for each test compound and represents the concentration of compound necessary to achieve 50% inhibition of cell adhesion where 100% = adhesion assessed in the absence of the test compound and 0% = absorbance in wells that did not receive cells.

### α₄β₁ Integrin-dependent Jurkat cell adhesion to VCAM-ig

96 well NUNC plates were coated with F(ab)₂ fragment goat anti-human IgG Fcγ-specific antibody [Jackson Immuno Research 109-006-098: 100 µl at 2 µg/ml in 0.1M NaHCO₃, pH 8.4], overnight at 4°*.* The plates were washed (3x) in phosphate-buffered saline (PBS) and then blocked for 1h in PBS/1% BSA at room temperature on a rocking platform. After washing (3x in PBS) 9 ng/ml of purified 2d VCAM-lg diluted in PBS/1% BSA was added and the plates left for 60 minutes at room temperature on a rocking platform. The plates were washed (3x in PBS) and the assay then performed at 37º for 30 min in a total volume of 200 µl containing 2.5 x 10⁵ Jurkat cells in the presence or absence of titrated test compounds.

Each plate was washed (2x) with medium and the adherent cells were fixed with 100µl methanol for 10 minutes followed by another wash. 100µl 0.25% Rose Bengal (Sigma R4507) in PBS was added for 5 minutes at room temperature and the plates washed (3x) in PBS. 100µl 50% (v/v) ethanol in PBS was added and the plates left for 60min after which the absorbance (570nm) was measured.

### α₄β₇ Integrin-dependent JY cell adhesion to MAdCAM-Ig

This assay was performed in the same manner as the α₄β₁ assay except that MAdCAM-lg (150ng/ml) was used in place of 2d VCAM-Ig and a sub-line of the β-lympho blastoid cell-line JY was used in place of Jurkat cells. The IC₅₀ value for each test compound was determined as described in the α₄β₁ integrin assay.

### α₅β₁ Integrin-dependent K562 cell adhesion to fibronectin

96 well tissue culture plates were coated with human plasma fibronectin (Sigma F0895) at 5µg/ml in phosphate-buffered saline (PBS) for 2 hr at 37°C. The plates were washed (3x in PBS) and then blocked for 1h in 100µl PBS/1% BSA at room temperature on a rocking platform. The blocked plates were washed (3x in PBS) and the assay then performed at 37ºC in a total volume of 200µl containing 2.5x 10⁵ K562 cells, phorbol-12-myristate-13-acetate at 10ng/ml, and in the presence or absence of titrated test compounds. Incubation time was 30 minutes. Each plate was fixed and stained as described in the α₄β₁ assay above.

### αₘβ₂-dependent human polymorphonuclear neutrophils adhesion to plastic

96 well tissue culture plates were coated with RPMI 1640/10% FCS for 2h at 37°C. 2 x 10⁵ freshly isolated human venous polymorphonuclear neutrophils (PMN) were added to the wells in a total volume of 200µl in the presence of 10ng/ml phorbol-12-myristate-13-acetate, and in the presence or absence of test compounds, and incubated for 20min at 37ºC followed by 30min at room temperature. The plates were washed in medium and 100µl 0.1% (w/v) HMB (hexadecyl trimethyl ammonium bromide, Sigma H5882) in 0.05M potassium phosphate buffer, pH 6.0 added to each well. The plates were then left on a rocker at room temperature for 60 min. Endogenous peroxidase activity was then assessed using tetramethyl benzidine (TMB) as follows: PMN lysate samples mixed with 0.22% H₂O₂ (Sigma) and 50µg/ml TMB (Boehringer Mannheim) in 0.1M sodium acetate/citrate buffer, pH 6.0 and absorbance measured at 630nm.

### αllb/β₃ -dependent human platelet aggregation

Human platelet aggregation was assessed using impedance aggregation on the Chronolog Whole Blood Lumiaggregometer. Human platelet-rich plasma (PRP) was obtained by spinning fresh human venous blood anticoagulated with 0.38% (v/v) tri-sodium citrate at 220xg for 10 min and diluted to a cell density of 6 x 10⁸/ml in autologous plasma. Cuvettes contained equal volumes of PRP and filtered Tyrode's buffer (g/liter: NaCl 8.0; MgCl₂.H₂O 0.427; CaCl₂ 0.2; KCI 0.2; D-glucose 1.0; NaHCO₃ 1.0; NaHPO₄.2H₂O 0.065). Aggregation was monitored following addition of 2.5µM ADP (Sigma) in the presence or absence of inhibitors.

In the above assays the preferred compounds of the invention such as the compounds of the Examples generally have IC₅₀ values in the α₄β₁ and assay of 1 µM and below and in the α₄β₇ assay of 5µM and below. In the other assays featuring α integrins of other subgroups the same compounds had IC₅₀ values of 50µM and above thus demonstrating the potency and selectivity of their action against α₄ integrins.

Additionally, compounds of the invention, such as the compounds of the Examples, possess advantageous absorption properties as determined by standard tests, which make the compounds particularly suitable for oral dosing.

## Claims

1. A compound of formula (1): 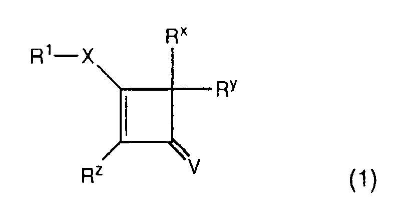 wherein
R¹ is a group Ar¹L²Ar²Alk- in which:
Ar¹ is an optionally substituted aromatic or heteroaromatic group;
L² is a covalent bond or a linker atom or group;
Ar² is an optionally substituted arylene or heteroarylene group; and Alk is a chain
-CH₂-CH(R)-, -CH=C(R)- or 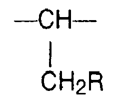
in which R is a carboxylic acid (-CO₂H) or a derivative or biostere thereof;
X is an -O- or -S- atom or -N(R²)- group in which:
R² is a hydrogen atom or a C₁₋₆alkyl group;
V is an oxygen (O) or sulphur (S) atom;
R^{x}, R^{y} and R^{z} which may be the same or different is each an atom or group -L¹(Alk¹)ₙ(R³)ᵥ in which L¹ is a covalent bond or a linker atom or group, Alk¹ is an optionally substituted aliphatic or heteroaliphatic chain, R³ is a hydrogen or halogen atom or group selected from -OR^{3a} [where R^{3a} is a hydrogen atom or an optionally substituted straight or branched C₁₋₆alkyl group or C₃₋₈cycloalkyl group], -SR^{3a}, -CN or an optionally substituted cycloaliphatic, heterocycloaliphatic, polycycloaliphatic, heteropolycycloaliphatic, aromatic or heteroaromatic group, n is zero or the integer 1 and v is the integer 1, 2 or 3 provided that when n is zero and L¹ is a covalent bond v is the integer 1;
or R^{z} is an atom or group as previously defined and R^{x} and R^{y} are joined together to form an optionally substituted spiro linked cycloaliphatic or heterocycloaliphatic group;
and the salts, solvates, hydrates and N-oxides thereof.

2. A compound according to claim 1 in which Alk is a -CH₂CH(R)- or -CH(CH₂R)- chain.

3. A compound according to claim 1 or claim 2 in which R is a carboxylic acid (-CO₂H) group.

4. A compound according to claim 1 or claim 2 in which R is an esterified carboxyl group of formula -CO₂Alk⁷.

5. A compound according to any one of claims 1 to 4 in which X is an -N(R²)- group.

6. A compound according to claim 5 in which R² is a hydrogen atom.

7. A compound according to any one of claims 1 to 6 in which Ar² is an optionally substituted phenylene group or an optionally substituted pyridinediyl group of formula: 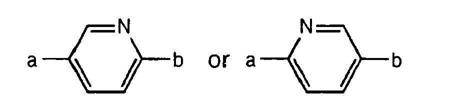 where a and b signify the points of attachment of L² and Alk respectively.

8. A compound according to any one of claims 1 to 7 in which Ar¹ is an optionally substituted phenyl or five-, six- or ten-membered heteroaromatic group.

9. A compound according to claim 8 in which Ar¹ is an optionally substituted pyridyl, pyrimidinyl, naphthyridinyl, quinolinyl or isoquinolinyl group.

10. A compound according to any one of claims 1 to 9 in which R^{z} is a halogen atom.

11. A compound according to any one of claims 1 to 9 in which R^{z} is an optionally substituted C₁₋₈alkyl group.

12. A compound according to any one of claims 1 to 9 in which R^{z} is a group -L¹(Alk¹)ₙR³ in which L¹ is an -0-, -S- or -Se- atom or -S(O)- or -N(R⁸)- group.

13. A compound according to any one of claims 1 to 9 in which R^{z} is a group -L¹(Alk¹)ₙR³ in which L¹ is a covalent bond.

14. A compound according to claim 12 or claim 13 in which n is zero.

15. A compound according to claim 12 or claim 13 in which n is the integer 1 and Alk¹ is an optionally substituted C₁₋₆alkylene chain.

16. A compound according to claim 14 or claim 15 in which R³ is a hydrogen atom or an optionally substituted C₃₋₁₀cycloaliphatic, C₃₋₁₀heterocycloaliphatic, C₆₋₁₂aromatic or C₁₋₉heteroaromatic group.

17. A compound according to any one of claims 1 to 16 in which R^{x} and R^{y} is each an optionally substituted C₁₋₈alkyl group.

18. A compound according to any one of claims 1 to 16 in which R^{x} and R^{y} are joined to form an optionally substituted spiro linked C₃₋₁₀ cycloaliphatic or C₃₋₁₀heterocycloaliphatic group.

19. A compound which is:
(*2S*)-2-[(3-Oxospiro[3.5]non-1-en-1-yl)amino]-3-{4-[(3,5-dichloroisonicotinoyl)amino]phenyl}propanoic acid
(*2S*)-2-[(3-Oxospiro[3.5]non-1-en-1-yl)amino]-3-{4-[(3-methyl[2,7]naphthyridin-1-yl)oxy]phenyl}propanoic acid
(*2S*)-2-[(2-Bromo-3-oxospiro[3.5]non-1-en-1-y)amino]-3-{4-[(3,5-dichloroisonicotinoyl)amino]phenyl}propanoic acid
(2*S*)-2-[(2-Bromo-4,4-dimethyl-3-oxo-1-cyclobutenyl)amino]-3-{4-[(3,5-dichloroisonicotinoyl)amino]phenyl}propanoic acid
(*2S*)-2-[(2-Bromo-3-oxospiro[3.5]non-1-en-1-yl)amino]-3-{4-[(2,7)naphthyridin-1-yloxy]phenyl}propanoic acid
(2*S*)-2-[(2-Bromo-3-oxo-7-oxaspiro[3.5]non-1-en-1-yl)amino]-3-{4-[(3,5-dichloroisonicotinoyl)amino]phenyl}propanoic acid
(2*S*)-2-{(2-Bromo-3-oxospiro[3.5]non-1-en-1-yl)amino}-3-(2,6-dimethoxy[1,1'-biphenyl]-4-yl)propanoic acid
(2*S*)-2-[(3-Oxospiro[3.6]dec-1-en-1-yl)amino]3-{4-[(3,5-dichloroisonicotinoyl)amino]phenyl}propanoic acid
(2*S*)-2-[(2-Bromo-3-oxospiro[3.6]dec-1-en-1-yl)amino]-3-{4-[(3,5-dichloroisonicotinoyl)amino]phenyl}propanoic acid
(2*S*)-2-{[4,4-Dimethy)-2-(phenylselenenyl)-3-oxo-1-cyclobutenyl]amino}-3-{4-[(3,5-dichloroisonicotinoyl)amino]phenyl} propanoic acid
(2*S*)-2-[(2-Bromo-7-methoxy-3-oxospiro[3.5]non-1-en-1-yl)amino]-3-{4-[(3,5-dichloroisonicotinoyl)amino]phenyl}propanoic acid
(2*S*)-2-{(2-Bromo-3-oxospiro[3.5]non-1-en-1-yl)amino}-3-{4-[(3-methyl[2,7]naphthyridin-1-yl)oxy]phenyl}propanoic acid
(2*S*)-2-{[2-(Phenylsulfanyl)-4,4-dimethyl-3-oxo-1-cyclobutenyl] amino}-3-{4-[(3,5-dichloroisonicotinoyl)amino]phenyl}propanoic acid
(*2S*)-2-[(2-Chloro-3-oxospiro[3.5]non-1-en-1-yl)amino]-3-{4-[(3,5-dichloroisonicotinoyl)amino]phenyl}propanoic acid
(2*S*)-3-{4-[(3,5-Dichloroisonicotinoyl)amino]phenyl}-2-(3-oxo-2-pyridin-3-yl-spiro[3.5]non-1-en-1-ylamino)propanoic acid
(2*S*)-2-[(2-Iodo-3-oxospiro[3.5]non-1-en-1-yl)amino]-3-{4-[(3,5-dichloroisonicotinoyl)amino]phenyl}propanoic acid
(2*S*)-2-[(2-Bromo-3-oxospiro[3.5]non-1-en-1-yl)amino]-3-{5-[(3,5-dichloroisonicotinoyl)amino]pyridin-2-yl}propanoic acid
(2*S*)-2-[(2-Chloro-3-oxospiro[3.5]non-1-en-1-yl)amino]-3-{5-[(3,5-dichloroisonicotinoyl)amino]pyridin-2-yl}propanoic acid
(2*S*)-2-[(2-Chloro-3-oxo-7-oxa-spiro[3.5]non-1-en-1-yl)amino]-3-{4-[(3,5-dichloroisonicotinoyl)amino]phenyl}propanoic acid
(2*S*)-2-[(2-Chloro-3-oxo-spiro[3.6]dec-1-en-1-yl)amino]-3-{4-[(3,5-dichloroisonicotinoyl)amino]phenyl}propanoic acid
(2*S*)-2-[4,4-Dimethyl-2-(1-methyl-1H-tetrazol-5-ylsulfanyl)-3-oxo-cyclobut-1-enylamino]-3-{4-[(3,5-dichloroisonicotinoyl)amino]phenyl}propanoic acid
(2*S*)-2-[(2-Bromo-3,7,7-trioxo-7λ⁶-thia-spiro[3.5]non-1-en-1-yl)amino]-3-{4-[(3,5-dichloroisonicotinoyl)amino]phenyl}propanoic acid
(2*S*)-2-[(2-Chloro-3-oxo-spiro[3.4]oct-1-en-1-yl)amino]-3-{4-[(3,5-dichloro-isonicotinoyl)amino]phenyl}propanoic acid
(2*S*)-2-[(2-Bromo-3-oxo-spiro[3.4]oct-1-en-1-yl)amino]-3-{4-[(3,5-dichloroisonicotinoyl)amino]phenyl}propanoic acid
(2*S*)-2-[(2-Methylsulfanyl-3-oxo-spiro[3.5]non-1-en-1-yl)amino]-3-{4-[(3,5-dichloroisonicotinoyl)amino]phenyl}propanoic acid
(*2S*)-2-(2-Fluoro-3-oxo-spiro[3.5]non-1-en-1-ylamino)3-{4-[(3,5-dichloroisonicotinoyl)amino]phenyl}propanoic acid
(2*S*)-2-[(2-Fluoro-3-oxo-7-oxa-spiro[3.5]non-1-en-1-yl)amino]-3-{4-[(3,5-dichloroisonicotinoyl)amino]phenyl}propanoic acid
(2*S*)-2-[(4,4-Dimethyl-2-methylsulfanyl-3-oxo-cyclobut-1-enyl)amino]-3-{4-[(3,5-dichloroisonicotinoyl)amino]phenyl}propanoic acid
(2*S*)-2-[(2-isopropylsulfanyl-4,4-dimethyl-3-oxo-cyclobut-1-enyl)amino]-3-{4-[(3,5-dichloroisonicotinoyl)amino]phenyl}propanoic acid
(2*S*)-2-[(2-Isopropylsulfanyl-3-oxo-spiro[3.5]non-1-en-1-yl)amino]-3-{4-[(3,5-dichloroisonicotinoyl)amino]phenyl}propanoic acid
(2*S*)-2-[(2-Isopropylsufanyl-4,4-dimethyl-3-oxo-cyclobut-1-enyl)amino]-3-[4-([2,7]naphthyridin-1-ylamino)phenyl]propanoic acid
(2*S*)-2-[(2-isopropylsulfanyl-3-oxo-spiro[3.5]non-1-en-1-yl)amino]-3-[4-([2,7]naphthyridin-1-ylamino)phenyl]propanoic acid
(2*S*)-2-[(2-Isopropylsulfanyl-3-oxo-7-oxa-spiro[3.5]non-1-en-1-yl)amino]-3-[4-([2,7]naphthyridin-1-ylamino)phenyl]propanoic acid
(2*S*)-2-[(2-Bromo-3-oxo-spiro[3.4]octa-1 ,6-dien-1-yl)amino]-3-{4-[(3,5-dichloroisonicotinoyl)amino]phenyl}propanoic acid
(2*S*)-2-[(4,4-Dimethyl-3-oxo-2-pentafluorophenylsulfanyl-cydobut-1-enyl)amino]-3-{4-[(3,5-dichloroisonicotinoyl)amino]phenyl}propanoic acid
(*2S*)-2-[(4,4-Dimethyl-3-oxo-2-pyrazin-2-yl-cyclobut-1-enyl)amino]-3-{4[(3,5-dichloroisonicotinoyl)amino]phenyl}propanoic acid
(*2S*)-2-[(7-Acetyl-2-bromo-3-oxo-7-aza-spiro[3.5]non-1-en-1-yl)amino]-3-{4-[(3,5-dichloroisonicotinoyl)amino]phenyl}propanoic acid
(*2S*)-2-{[2-(Isopropylsulfanyl)-3-oxo-7-oxaspiro[3.5]non-1-en-yl)]amino}-3-(2,6-dimethoxy[1,1'-biphenyl]-4-yl)propanoic acid
(*2S*)-2-[(2-Cyclohexyl-3-oxo-spiro[3.5]non-1-en-1-yl)amino]-3-{4-[(3,5-dichloroisonicotinoyl)amino]phenyl}propanoic acid
(*2S*)-3-{4-[(3,5-Dichloroisonicotinoyl)amino]phenyl}-2-(2-methyl-3-oxo-spiro[3.5]non-1-en-1-ylamino)propanoic acid
(*2S*)-3-{4-[(3,5-Dichloroisonicotinoyl)amino]phenyl}-2-(3-oxo-2-propyl-spiro[3.5]non-1-en-1-ylamino)propanoic acid
(*2S*)-3-{4-[(3,5-Dichloroisonicotinoyl)amino]phenyl}-2-(2-methyl-3-oxo-7-oxa-spiro[3.5]non-1-en-1-ylamino)propanoic acid
(*2S*)-3-{4-[(3,5-Dichloroisonicotinoyl)amino]phenyl}-2-(3-oxo-2-propyl-7-oxa-spiro[3.5]non-1-en-1-ylamino)propanoic acid
(*2S*)-3-{4-[(3,5-Dichloroisonicotinoyl)amino]phenyl}-2-(2-[1,3]dithian-2-yl-3-oxo-spiro[3.5]non-1-en-1-ylamino)propanoic acid
(*2S*)-3-{4-[(3,5-Dichloroisonicotinoyl)amino]phenyl}-2-(2-ethyl-3-oxo-spiro[3.5]non-1-en-1-ylamino)propanoic acid
(*2S*)-3-{4-[(3,5-Dichloro-1-oxy-pyridine-4-carbonyl)amino]phenyl}-2-(3-oxo-spiro[3.5]non-1-en-1-ylamino)propanoic acid
(2*S*)-2-(2-Bromo-3-oxo-spiro[3.5]non-1-en-1-ylamino)-3-{4-[(3,5-dichloro-1-oxy-pyridine-4-carbonyl)amino]phenyl}propanoic acid
(2*S*')-2-(2-Chloro-3-oxo-spiro[3.5]non-1-en-1-ylamino)-3-{4-[(3,5-dichloro-1-oxy-pyridine-4-carbonyl)amino]phenyl}propanoic acid
(2*S*)-3-{4-[(3,5-Dichloro-1-oxy-pyridine-4-carbonyl)-amino]-phenyl}-2-(2-methanesulfinyl-4,4-dimethyl-3-oxo-cyclobut-1-enylamino) propanoic acid
(2*S*)-2-(2-Bromo-3-oxo-spiro[3,5]non-1-en-1-ylamino)-3-[4-(3-methyl-[2,7]naphthyridin-1-ylamino)phenyl]propanoic acid
(2*S*)-2-(2-Bromo-3-oxo-spiro[3,5]non-1-en-1-ylamino)-3-[4-([2,7]naphthyridin-1-ylamino)phenyl]propanoic acid
(2*S*)-2-(2-Bromo-4,4-dimethyl-3-oxo-cyclobut-1-enylamino)-3-[4-(3-methyl-[2,7]naphthyridin-1-yloxy)phenyl]propanoic acid
(2*S*)-2-[(2-Bromo-3-oxo-spiro[3,4]oct-1-en-1-yl)amino]-3-{4-[(3,5-dichloroisonicotinoyl)amino]phenyl}propanoic acid
and the salts, solvates, hydrates, N-oxides and carboxylic acid esters thereof.

20. A compound which is:
(2*S*)-2-[(2-Bromo-3-oxospiro[3.5]non-1-en-1-yl)amino]-3-{4-[(3,5-dichloroisonicotinoyl)amino]phenyl}propanoic acid
(2*S*)-2-[(2-Bromo-3-oxo-7-oxaspiro[3.5]non-1-en-1-yl)amino]-3-{4-[(3,5-dichloroisonicotinoyl)amino]phenyl}propanoic acid
(2*S*)-2-[(2-Bromo-3-oxospiro[3.6]dec-1-en-1-yl)amino]-3-{4-[(3,5-dichloroisonicotinoyl)amino]phenyl}propanoic acid
(2*S*)-2-{(2-Bromo-3-oxospiro[3.5]non-1-en-1-yl)amino)-3-{4-[(3-methyt[2,7]naphthyridin-1-yl)oxy]phenyl}propanoic acid
(2*S*)-2-{[2-(Phenylsulfanyl)-4,4-dimethyl-3-oxo-1-cyclobutenyl]-amino}-3-{4-[(3,5-dichloroisonicotinoyl)amino]phenyl}propanoic acid
(2*S*)-2-[(2-Chloro-3-oxospiro[3.5]non-1-en-1-yl)amino]-3-{4-[(3,5-dichloroisonicotinoyl)amino]phenyl}propanoic acid
(2*S*)-3-{4-[(3,5-Dichloroisonicotinoyl)amino]phenyl}-2-(3-oxo-2-pyridin-3-yl-spiro[3.5]non-1-en-1-ylamino)propanoic acid
(2*S*)-2-[(2-lodo-3-oxospiro[3.5]non-1-en-1-yl)amino]-3-{4-[(3,5-dichloroisonicotinoyl)amino]phenyl}propanoic acid
(2*S*)-2-[(2-Bromo-3-oxospiro[3.5]non-1-en-1-yl)amino]-3-{5-[(3,5-dichloroisonicotinoyl)amino]pyridin-2-yl}propanoic acid
(2*S*)-2-[(2-Chloro-3-oxospiro[3.5]non-1-en-1-yl)amino]-3-{5-[(3.5-dichloroisonicotinoyl)amino]pyridin-2-yl}propanoic acid
(2*S*)-2-[(2-Chloro-3-oxo-7-oxa-spiro[3.5]non-1-en-1-yl)amino]-3-{4-[(3,5-dichloroisonicotinoyl)amino]phenyl}propanoic acid
(2S)-2-[(2-Chloro-3-oxo-spiro[3.6]dec-1-en-1-yl)amino]-3-{4-[(3,5-dichloroisonicotinoyl)amino]phenyl}propanoic acid
(2*S*)-2-[(2-Bromo-3,7,7-trioxo-7λ⁶-thia-spiro[3.5]non-1-en-1-yl)amino]-3-{4-[(3,5-dichloroisonicotinoyl)amino]phenyl}propanoic acid
(2*S*)-2-[(2-Chloro-3-oxo-spiro[3.4]oct-1-en-1-yl)amino]-3-{4-[(3,5-dichloroisonicotinoyl)amino]phenyl}propanoic acid
(2*S*)-2-[(2-Bromo-3-oxo-spiro[3.4]oct-1-en-1-yl)amino]-3-{4-[(3,5-dichloroisonicotinoyl)amino]phenyl}propanoic acid
(2*S*)-2-[(2-Methylsulfanyl-3-oxo-spiro[3.5]non-1-en-1-yl)amino]-3-{4-[(3,5-dichloroisonicotinoyl)amino]phenyl}propanoic acid
(2*S*)-2-(2-Fluoro-3-oxo-spiro[3.5]non-1-en-1-ylamino)3-{4-[(3,5-dichloroisonicotinoyl)amino]phenyl}propanoic acid
(2*S*)-2-[(2-Fluoro-3-oxo-7-oxa-spiro[3.5]non-1-en-1-yl)amino]-3-{4-[(3,5-dichloroisonicotinoyl)amino]phenyl}propanoic acid
(2*S*)-2-[(4,4-Dimethyl-2-methylsulfanyl-3-oxo-cyclobut-1-enyl)amino]-3-{4-[(3,5-dichloroisonicotinoyl)amino]phenyl}propanoic acid
(2*S*)-2-[(2-Isopropylsulfanyl-4,4-dimethyl-3-oxo-cyclobut-1-enyl)amino]-3-{4-[(3,5-dichloroisonicotinoyl)amino]phenyl}propanoic acid
(2*S*)-2-[(2-Isopropylsulfanyl-3-oxo-spiro[3.5]non-1-en-1-yl)amino]-3-{4-[(3,5-dichloroisonicotinoyl)amino]phenyl}propanoic acid
(2*S*)-2-[(2-Isopropylsulfanyl-4,4-dimethyl-3-oxo-cyclobut-1-enyl)amino]-3-[4-([2,7]naphthyridin-1-ylamino)phenyl]propanoic acid
(2*S*)-2-[(2-Isopropylsulfanyl-3-oxo-spiro[3.5]non-1-en-1-yl)amino]-3-[4-([2,7]naphthyridin-1-ylamino)phenyl]propanoic acid
(2*S*)*-*2-[(2*-*Isopropylsulfanyl-3-oxo-7-oxa-spiro[3.5]non-1-en-1-yl)amino]-3-[4-([2,7]naphthyridin-1-ylamino)phenyl]propanoic acid
(2*S*)-2-[(2-Bromo-3-oxo-spiro[3.4]octa-1,6-dien-1-yl)amino]-3-{4-[(3,5-dichloroisonicotinoyl)amino]phenyl}propanoic acid
(*2S*)-*2*-[(7-Acetyl-2-bromo-3-oxo-7-aza-spiro[3.5]non-1-en-1-yl)amino]-3-{4-[(3,5-dichloroisonicotinoyl)amino]phenyl}propanoic acid
(*2S*)-2-[(2-Cyclohexyl-3-oxo-spiro[3.5]non-1-en-1-yl)amino]-3-{4-[(3,5-dichloroisonicotinoyl)amino]phenyl}propanoic acid
(*2S*)3-{4-[(3,5-Dichloroisonicotinoyl)amino]phenyl}-2-(2-methyl-3-oxo-spiro[3.5]non-1-en-1-ylamino)propanoic acid
(*2S*)-3-{4-[(3,5-Dichloroisonicotinoyl)amino]phenyl}-2-(3-oxo-2-propyl-spiro[3.5]non-1-en-1-ylamino)propanoic acid
(2S)-3-{4-[(3,5-Dichloroisonicotinoyl)amino]phenyl}-2-(2-[1,3]dithian-2-yl-3-oxo-spiro[3.5]non-1-en-1-ylamino)propanoic acid
(2*S*)-2-(2-Bromo-3-oxo-spiro[3.5]non-1-en-1-ylamino)-3-{4-[(3,5-dichloro-1-oxy-pyridine-4-carbonyl)amino]phenyl}propanoic acid
2-(2-Chloro-3-oxo-spiro[3.5]non-1-en-1-ylamino)-3-{4-[(3,5-dichloro-1-oxy-pyridine-4-carbonyl)amino]phenyl}propanoic acid
and the salts, solvates, hydrates, N-oxides and carboxylic acid esters thereof.

21. A compound which is:
Ethyl (2S)-2-(2-bromo-3-oxo-spiro[3.5]non-1-en-1-ylamino)-3-{4-[(3,5-dichloroisonicotinoyl)amino]phenyl}propanoate
Isopropyl (2S)-2-(2-bromo-3-oxo-spiro[3.5]non-1-en-1-ylamino)-3-{4-[(3,5-dichloroisonicotinoyl)amino]phenyl}propanoate
t-Butyl (2S)-2-(2-bromo-3-oxo-spiro[3.5]non-1-en-1-ylamino)-3-{4-[(3,5-dichloroisonicotinoyl)amino]phenyl}propanoate
1-Methyl-piperidin-4-yl (*2S*)-2-(2-bromo-3-oxo-spiro[3.5]non-1-en-1-ylamino)-3-{4-[(3,5-dichloroisonicotinoyl)amino]phenyl}propanoate
Phenyl (*2S*)-2-(2-bromo-3-oxo-spiro[3.5]non-1-en-1-ylamino)-3-{4-[(3,5-dichloroisonicotinoyl)amino]phenyl}propanoate
Cyclopentyl (2*S*)-2-(2-bromo-3-oxo-spiro[3.5]non-1-en-1-ylamino)-3-{4-[(3,5-dichloroisonicotinoyl)amino]phenyl}propanoate
2-Imidazol-1-yl-ethyl (2*S*)-2-(2-bromo-3-oxo-spiro[3.5]non-1-en-1-ylamino)-3-{4-[(3,5-dichloroisonicotinoyl)amino]phenyl)propanoate
Neopentyl (2*S*)-2-(2-bromo-3-oxo-spiro[3.5]non-1-en-1-ylamino)-3-{4-[(3,5-dichloroisonicotinoyl)amino]phenyl}propanoate
Tetrahydro-furan-3-yl (2*S*)-2-(2-bromo-3-oxo-spiro[3.5]non-1-en-1-ylamino)-3-{4-[(3,5-dichloroisonicotinoyl)amino]phenyl}propanoate
Pyridin-4-ylmethyl (2*S*)-2-(2-bromo-3-oxo-spiro[3.5]non-1-en-1-ylamino)-3-{4-[(3,5-dichloroisonicotinoyl)amino]phenyl}propanoate
Tetrahydropyran-4-yl (2*S*)-2-(2-bromo-3-oxo-spiro[3.5]non-1-en-1-ylamino)-3-{4-[(3,5-dichloroisonicotinoyl)amino]phenyl}propanoate
5-Methyl-2-oxo-[1,3]dioxol-4-ylmethyl (2*S*)-2-(2-bromo-3-oxo-spiro[3.5]non-1-en-1-ylamino)-3-{4-[(3,5-dichloroisonicotinoyl)amino]-phenyl}propanoate
1-Methyl-pyrrolidin-3-yl (2*S*)-2-(2-bromo-3-oxo-spiro[3.5]non-1-en-1-ylamino)-3-{4-[(3,5-dichloroisonicotinoyl)amino]phenyl}propanoate
2,3-Dihydroxyoropyl (2*S*)-2-(2-bromo-3-oxo-spiro[3.5]non-1-en-1-ylamino)-3-{4-[(3,5-dichloroisonicotinoyl)amino]phenyl}propanoate Tetrahydrofuran-2-ylmethyl (2*S*)-2-(2-bromo-3-oxo-spiro[3.5]non-1-en-1-ylamino)-3-{4-[(3,5-dichloroisonicotinoyl)amino]phenyl}propanoate and the salts, solvates, hydrates and N-oxides thereof.

22. A pharmaceutical composition comprising a compound according to claim 1 together with one or more pharmaceutically acceptable carriers, excipients or diluents.
